# EUROPEAN PATENT APPLICATION

(11) **EP 4 455 132 A1**
(43) Date of publication of application: **30.10.2024**
(21) Application number: 22910073.0
(22) Date of filing: 21.12.2022
(51) Int. Cl.: C07D 401/14, C07D 413/14, A61K 31/506, A61P 35/00

(54) **PYRIMIDINE HETEROCYCLIC COMPOUND, PREPARATION METHOD THEREFOR AND USE THEREOF IN MEDICINE**

(30) Priority: 22.12.2021 CN 202111583828; 27.01.2022 CN 202210098381; 23.05.2022 CN 202210562007; 07.12.2022 CN 202211568373
(71) Applicant: Insilico Medicine IP Limited, Central, Hong Kong (HK)
(72) Inventor: LU, Hongfu, Shanghai 201203 (CN); PENG, Jingjing, Shanghai 201203 (CN); DING, Xiao, Shanghai 201203 (CN); LIU, Jinxin, Shanghai 201203 (CN); LIU, Yingtao, Shanghai 201203 (CN); REN, Feng, Shanghai 201203 (CN); ZHOU, Fusheng, Shanghai 201203 (CN); JIANG, Tao, Shaoxing Binhai New City Zhejiang 312000 (CN); LIANG, Tao, Shanghai 201203 (CN); LAN, Jiong, Shanghai 201203 (CN); LU, Qiang, Shanghai 201203 (CN)
(74) Representative: Mewburn Ellis LLP
(86) International application number: PCT/CN2022/140669
(87) International publication number: WO 2023/116761

(57) **Abstract**

The present invention discloses a pyrimidine heterocyclic compound, and a preparation method and medical use thereof. Specifically, the present invention discloses a pyrimidine heterocyclic compound with a structure shown in Formula (A) or a pharmaceutically acceptable salt thereof (groups are each defined in the specification), a pharmaceutical composition comprising the compound, and use of the compound in treatment of cell proliferative diseases (such as cancers).

## Description

The present invention claims the priority of Chinese patent application CN202111583828.0 filed on December 22, 2021, the priority of Chinese patent application CN202210098381.6 filed on January 27, 2022, the priority of Chinese patent application CN202210562007.7 filed on May 23, 2022, and the priority of Chinese patent application CN202211568373.X filed on December 7, 2022. The present application cites part or all of the above Chinese patent applications.

### TECHNICAL FIELD

The present invention belongs to the field of medicine, and in particular relates to a pyrimidine heterocyclic compound, and a preparation method and medical use thereof.

### BACKGROUND OF THE INVENTION

Cyclin dependent kinase (CDK)/cyclin complex is identified as a conservative component of an RNA polymerase II transcription mechanism. At present, there are 20 mammalian CDKs. In mammalian CDKs, the CDK7 has solid kinase activity, and only the CDK7 has dual-functions of regulation of cell cycle progression and transcription. In the cytosol, the CDK7, as a heterotrimer complex, is considered to play a role of the CDK1/2/4/6 activating kinase (CAK), and therefore, the phosphorylation of the CDK7 to the conserved residues in the CDK1/2/4/6 is necessary for fully catalytic CDK activity and cell cycle progression. In the cell nucleus, the CDK7 forms a kinase core of an RNA polymerase II transcription factor complex and is responsible for phosphorylating the C-terminal domain (CTD) of the RNA polymerase II, which is a necessary step for gene transcription initiation. Two functions (namely CAK and CTD phosphorylation) of the CDK7 support the key aspects of cell proliferation, cell cycle and transcription together.

As an general regulatory factor of transcription, the CDK7 can be used as a therapeutic target for treating many diseases and syndromes. The CDK7 can interact with multiple transcription factors, cofactors, chromatin regulatory factors and mutants in non-coding RNA in a transcriptional regulatory region to regulate transcription. These transcription factors, cofactors, chromatin regulatory factors or mutants in non-coding RNA can cause cancers, autoimmune diseases, nervous system disorders, developmental syndromes, diabetes, cardiovascular diseases, obesity, etc. Some of these transcription factors can control RNA polymerase II mediated transcription initiation and elongation, and when their expressions or functions change, produce invasive tumor cells (such as those caused by c-Myc) or some forms of autoimmunity (such as those caused by AIRE) may occur. Therefore, the CDK7 kinase can promote the abnormal expression of some tumor-related transcription factors by regulating the overall transcription process, and promote tumor development by regulating the phosphorylation of key kinases in the cell cycle. More importantly, the CDK7 regulates the expression of oncogenic transcription factors more significantly than other housekeeping genes in cancer cells. The inhibition of the CDK7 can differentially affect the transcription of some oncogenes and housekeeping genes, thus ensuring the therapeutic window. Transcription regulation and cell cycle regulation by regulating CDK7 mediated phosphorylation may treat diseases with abnormal proliferation, including cancers. As an general regulatory factor of transcription, the CDK7 can also be used as a therapeutic target for treating diseases such as inflammations, viral replication such as HIV and EBV, cancers and cardiac hypertrophy.

The high sequence and structure similarity of kinase domains of CDK family member hinder the discovery of CDK7 selective inhibitors. Therefore, the development of selective CDK7 inhibitors is of great value for clinical applications.

### SUMMARY OF THE INVENTION

A first aspect of the present invention provides a compound shown in Formula (A) or a pharmaceutically acceptable salt thereof: 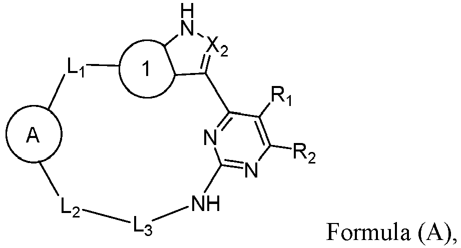 wherein 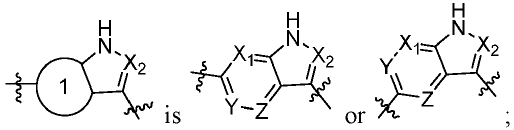
ring A is none; or the ring A is selected from the group consisting of 3-14 membered carbocycle, 3-14 membered heterocycle, 5-12 membered heteroaryl and C₅₋₁₂ aryl; the ring A is unsubstituted or substituted by 1, 2, 3, 4 or 5 groups selected from a group S1; the 3-14 membered heterocycle has 1, 2, 3 or 4 heteroatoms independently selected from nitrogen, oxygen and sulfur; the 5-12 membered heteroaryl has 1, 2, 3 or 4 heteroatoms independently selected from nitrogen, oxygen and sulfur;
L₃ is none, C₁₋₆ alkylene, C₂₋₆ alkenylene or ring B (the structure is 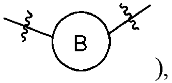 wherein 1, 2, 3, 4 or 5 hydrogen atoms on the C₁₋₆ alkylene may be each independently and optionally substituted by R¹³; 1, 2, 3, 4 or 5 hydrogen atoms on the C₂₋₆ alkenylene may be each independently and optionally substituted by R¹³;
the ring B is selected from the group consisting of 3-14 membered carbocycle, 3-14 membered heterocycle, 5-12 membered heteroaryl and C₅₋₁₂ aryl; the ring B is unsubstituted or substituted by 1, 2, 3, 4 or 5 groups selected from the group S1; the 3-14 membered heterocycle has 1, 2, 3 or 4 heteroatoms independently selected from nitrogen, oxygen and sulfur; the 5-12 membered heteroaryl has 1, 2, 3 or 4 heteroatoms independently selected from nitrogen, oxygen and sulfur;
L₁ is none, C₁₋₆ alkylene, C₂₋₆ alkenylene, -O-, -NR¹⁰-, -C=N-, C₅₋₁₂ aryl, 5-12 membered heteroaryl, -C(O)-, -C(O)-NR¹⁰- or -NR¹⁰-C(O)-, wherein 1, 2, 3, 4 or 5 hydrogen atoms on the C₁₋₆ alkylene may be each independently and optionally substituted by R¹³; 1, 2, 3, 4 or 5 hydrogen atoms on the C₂₋₆ alkenylene may be each independently and optionally substituted by R¹³; the 5-12 membered heteroaryl has 1, 2, 3 or 4 heteroatoms independently selected from nitrogen, oxygen and sulfur; the C₅₋₁₂ aryl and the 5-12 membered heteroaryl are each independently unsubstituted or substituted by 1, 2, 3, 4 or 5 groups selected from the group S 1;
L₂ is selected from the group consisting of:
   -(O)ₘ₂-(CR¹³R¹⁴)ₘ₁-(NR¹²)ₘ₃-,
   -(NR¹¹)ₘ₂-(CR¹³R¹⁴)ₘ₁-(NR¹²)ₘ₃-,
   -(O)ₘ₂-(CR¹³R¹⁴)ₘ₅-(3-7 membered carbocyclyl)ₘ₄-(CR¹³R¹⁴)ₘ₆-(NR¹²)ₘ₃-,
   -(NR¹¹)ₘ₂-(CR¹³R¹⁴)ₘ₅-(3-7 membered carbocyclyl)ₘ₄-(CR¹³R¹⁴)ₘ₆-(NR¹²)ₘ₃-,
   -(O)ₘ₂-(CR¹³R¹⁴)ₘ₅-(3-7 membered heterocyclyl)ₘ₄-(CR¹³R¹⁴)ₘ₆-(NR¹²)ₘ₃-,
   -(NR¹¹)ₘ₂-(CR¹³R¹⁴)ₘ₅-(3-7 membered heterocyclyl)ₘ₄-(CR¹³R¹⁴)ₘ₆-(NR¹²)ₘ₃-,
   -(O)ₘ₂-(CR¹³R¹⁴)ₘ₅-NR¹⁰-(CR¹³R¹⁴)ₘ₆-(NR¹²)ₘ₃-,
   -(NR¹¹₎ₘ₂-(CR¹³R¹⁴)ₘ₅-NR¹⁰-(CR¹³R¹⁴)ₘ₆-(NR¹²)ₘ₃-,
   -(O)ₘ₂-(CR¹³R¹⁴)ₘ₅-NR¹⁰C(O)-(CR¹³R¹⁴)ₘ₆-(NR¹²)ₘ₃-,
   -(NR¹¹)ₘ₂-(CR¹³R¹⁴)ₘ₅-NR¹⁰C(O)-(CR¹³R¹⁴)ₘ₆-(NR¹²)ₘ₃-,
   -(O)ₘ₂-(CR¹³R¹⁴)ₘ₅-C(O)NR¹⁰-(CR¹³R¹⁴)ₘ₆-(NR¹²)ₘ₃-,
   -(NR¹¹)ₘ₂-(CR¹³R¹⁴)ₘ₅-C(O)NR¹⁰-(CR¹³R¹⁴)ₘ₆-(NR¹²)ₘ₃-,
   -(O)ₘ₂-(CR¹³R¹⁴)ₘ₅-O-(CR¹³R¹⁴)ₘ₆-(NR¹²)ₘ₃-,
   -(NR¹¹)ₘ₂-(CR¹³R¹⁴)ₘ₅-O-(CR¹³R¹⁴)ₘ₆-(NR¹²)ₘ₃-,
   -(O)ₘ₂-(CR¹³R¹⁴)ₘ₅-OC(O)-(CR¹³R¹⁴)ₘ₆-(NR¹²)ₘ₃-,
   -(NR¹¹)ₘ₂-(CR¹³R¹⁴)ₘ₅-OC(O)-(CR¹³R¹⁴)ₘ₆-(NR¹²)ₘ₃-,
   -(O)ₘ₂-(CR¹³R¹⁴)ₘ₅-C(O)O-(CR¹³R¹⁴)ₘ₆-(NR¹²)ₘ₃-,
   -(NR¹¹)ₘ₂-(CR¹³R¹⁴)ₘ₅-C(O)O-(CR¹³R¹⁴)ₘ₆-(NR¹²)ₘ₃-,
   -(O)ₘ₂-(CR¹³R¹⁴)ₘ₅-(CH=CH)ₘ₇-(CR¹³R¹⁴)ₘ₆-(NR¹²)ₘ₃-, and
   -(NR¹¹)ₘ₂-(CR¹³R¹⁴)ₘ₅-(CH=CH)ₘ₇-(CR¹³R¹⁴)ₘ₆-(NR¹²)ₘ₃-;
   each R¹⁰ is independently hydrogen, C₁₋₆ alkyl, deuterated C₁₋₆ alkyl, -3-7 membered carbocyclyl, -C(O)-C1-6 alkyl, -C(O)-C₁₋₄ alkyl-C₁₋₆ alkoxy, -C(O)-3-7 membered carbocyclyl, -C₁₋₄ alkyl-hydroxyl, -C₁₋₄ alkyl-cyano, -C₁₋₄ alkyl-C₁₋₆ alkoxy, -C₁₋₄ alkyl-NHC(O)-C₁₋₆ alkyl, -C₁₋₄ alkyl-NHC(O)-C₁₋₄ alkyl-C₁₋₆ alkoxy, -C₁₋₄ alkyl-NHC(O)-3-7 membered carbocyclyl, or -C₁₋₄ alkyl-NRR';
   each 3-7 membered heterocyclyl independently has 1, 2 or 3 heteroatoms independently selected from nitrogen, oxygen and sulfur; the 3-7 membered carbocycle is each independently unsubstituted or substituted by 1, 2, 3, 4 or 5 groups selected from the group S 1;
   each 3-7 membered carbocyclyl is independently unsubstituted or substituted by 1, 2, 3, 4 or 5 groups selected from the group S1;
   each R¹¹ is independently hydrogen, C₁₋₆ alkyl or deuterated C₁₋₆ alkyl;
   each R¹² is independently hydrogen, C₁₋₆ alkyl or deuterated C₁₋₆ alkyl;
   each R¹³ is independently hydrogen, cyano, hydroxyl, halogen, C₁₋₆ alkyl, C₁₋₆ alkoxy, halogenated C₁₋₆ alkyl, halogenated C₁₋₆ alkoxy, -3-7 membered carbocyclyl, -C₀₋₆ alkylene-NRR', -C₁₋₆ alkylene-hydroxyl or -C₀₋₆ alkylene-cyano;
   each R¹⁴ is independently hydrogen, cyano, hydroxyl, halogen, C₁₋₆ alkyl, C₁₋₆ alkoxy, halogenated C₁₋₆ alkyl, halogenated C₁₋₆ alkoxy, -3-7 membered carbocyclyl, -C₀₋₆ alkylene-NRR', -C₁₋₆ alkylene-hydroxyl or -C₀₋₆ alkylene-cyano;
   each m1 is independently 1, 2, 3, 4, 5 or 6;
   each m2 is independently 0 or 1;
   each m3 is independently 0 or 1;
   each m4 is independently 1 or 2;
   each m5 is independently 0, 1, 2, 3, 4, 5 or 6;
   each m6 is independently 0, 1, 2, 3, 4, 5 or 6;
   each m7 is independently 1 or 2;
   R₁ is hydrogen, halogen, cyano, C₁₋₆ alkyl or 3-14 membered carbocyclyl; the C₁₋₆ alkyl and the 3-14 membered carbocyclyl are independently unsubstituted or substituted by 1, 2, 3, 4 or 5 groups selected from the group consisting of oxo, hydroxyl, halogen, C₁₋₆ alkyl and halogenated C₁₋₆ alkyl;
   R₂ is hydrogen, halogen, cyano, C₁₋₆ alkyl or 3-14 membered carbocyclyl; the C₁₋₆ alkyl and the 3-14 membered carbocyclyl are independently unsubstituted or substituted by 1, 2, 3, 4 or 5 groups selected from the group consisting of oxo, hydroxyl, halogen, C₁₋₆ alkyl and halogenated C₁₋₆ alkyl;
   X₂ is N or C(R₃);
   X₁ is N or C(R₄);
   Y is N or C(R₄);
   Z is N or C(R₃);
   each R₃ is independently hydrogen, halogen, cyano, C₁₋₆ alkyl or halogenated C₁₋₆ alkyl;
   each R₄ is independently hydrogen, halogen, hydroxyl, cyano, -C₂₋₄ alkenylene-phenyl, - C₂₋₄ alkynylene-phenyl, -S(O)-OH, -S(O)₂-OH, -S-(C₁₋₆ alkyl), C₁₋₆ alkyl, -O-C₁₋₆ alkyl, -C₁₋₆ alkylene-O-C₁₋₆ alkyl, -O-C₁₋₆ alkylene-O-C₁₋₆ alkyl, -C₀₋₆ alkylene-NRR', -C₀₋₆ alkylene-C(O)OH, -C₀₋₆ alkylene-C(O)-C₁₋₆ alkyl, -C₀₋₆ alkylene-C(O)-NRR', -C₀₋₆ alkylene-NR-C(O)-C₁₋₆ alkyl, -C₀₋₆ alkylene-S(O)₂-C₁₋₆ alkyl, -C₀₋₆ alkylene-S(O)₂-NRR', -C₀₋₆ alkylene-NR-S(O)₂-C₁₋₆ alkyl, -C₀₋₆ alkylene-NR-S(O)₂-NRR', -C₀₋₆ alkylene-P(O)O-(C₁₋₆ alkyl)₂, -C₀₋₆ alkylene-P(O)-(C₁₋₆ alkyl)(O-C₁₋₆ alkyl), -C₀₋₆ alkylene-P(O)-(C₁₋₆ alkyl)₂, -C₀₋₆ alkylene-3-14 membered carbocyclyl, -C₀₋₆ alkylene-3-14 membered heterocyclyl, -C₀₋₆ alkylene-5-12 membered heteroaryl, -C₀₋₆ alkylene-C₅₋₁₂ aryl, -C₀₋₆ alkylene-C(O)-3-14 membered heterocyclyl, -C₀₋₆ alkylene-C(O)-5-12 membered heteroaryl, -O-C₀₋₆ alkylene-O-C₁₋₆ alkyl, - O-C₀₋₆ alkylene-3-14 membered carbocyclyl, -O-C₀₋₆ alkylene-3-14 membered heterocyclyl, - O-C₀₋₆ alkylene-5-12 membered heteroaryl, -O-C₀₋₆ alkylene-C₅₋₁₂ aryl, and -S(O)-C₁₋₆ alkyl, wherein the C₁₋₆ alkyl, the C₀₋₆ alkylene, the C₂₋₄ alkenylene, the 3-14 membered carbocyclyl, the 3-14 membered heterocyclyl, the 5-12 membered heteroaryl and the C₅₋₁₂ aryl are independently unsubstituted or substituted by 1, 2, 3, 4 or 5 groups selected from the group S1; the 3-14 membered heterocycle has 1, 2, 3 or 4 heteroatoms independently selected from nitrogen, oxygen and sulfur; the 5-12 membered heteroaryl has 1, 2, 3 or 4 heteroatoms independently selected from nitrogen, oxygen and sulfur;
   the groups in the group S1 include: oxo, halogen, hydroxyl, cyano, C₁₋₆ alkyl, -O-C₁₋₆ alkyl, -C₁₋₆ alkylene-O-C₁₋₆ alkyl, -O-C₁₋₆ alkylene-O-C₁₋₆ alkyl, -C₀₋₆ alkylene-NRR', -C₀₋₆ alkylene-C(O)OH, -C₀₋₆ alkylene-C(O)-C₁₋₆ alkyl, -C₀₋₆ alkylene-C(O)-NRR', -C₀₋₆ alkylene-NR-C(O)-C₁₋₆ alkyl, -C₀₋₆ alkylene-S(O)₂-C₁₋₆ alkyl, -C₀₋₆ alkylene-S(O)₂-NRR', -C₀₋₆ alkylene-NR-S(O)₂-C₁₋₆ alkyl, -C₀₋₆ alkylene-NR-S(O)₂-NRR', -C₀₋₆ alkylene-P(O)O-(C₁₋₆ alkyl)₂, -C₀₋₆ alkylene-P(O)-(C₁₋₆ alkyl)(O-C₁₋₆ alkyl), -C₀₋₆ alkylene-P(O)-(C₁₋₆ alkyl)₂, -C₀₋₆ alkylene-3-14 membered carbocyclyl, -C₀₋₆ alkylene-3-14 membered heterocyclyl, -C₀₋₆ alkylene-5-12 membered heteroaryl, -C₀₋₆ alkylene-C₅₋₁₂ aryl, -C₀₋₆ alkylene-C(O)-3-14 membered heterocyclyl, -C₀₋₆ alkylene-C(O)-5-12 membered heteroaryl, -O-C₀₋₆ alkylene-O-C₁₋₆ alkyl, -O-C₀₋₆ alkylene-3-14 membered carbocyclyl, -O-C₀₋₆ alkylene-3-14 membered heterocyclyl, -O-C₀₋₆ alkylene-5-12 membered heteroaryl, -O-C₀₋₆ alkylene-C₅₋₁₂ aryl and - S(O)-C₁₋₆ alkyl;
   R and R' are each independently hydrogen, C₁₋₆ alkyl or deuterated C₁₋₆ alkyl, or R and R' optionally form the 3-14 membered heterocyclyl or 5-12 membered heteroaryl together with nitrogen atoms connected to R and R', wherein the heterocyclyl and the heteroaryl each independently contain 0, 1 or 2 heteroatoms selected from N, O and S in addition to the existing nitrogen atoms;
   in the above groups, 2 hydrogen atoms on any one carbon atom on the C₀₋₆ alkylene may further be optionally substituted by 3-7 membered carbocycle or 3-7 membered heterocyclic spirocycle at the same time; and
   in the above groups, 2 hydrogen atoms on any one carbon atom on the C₁₋₆ alkylene may further be optionally substituted by 3-7 membered carbocycle or 3-7 membered heterocyclic spirocycle at the same time.

In some embodiments, R₁ is hydrogen, halogen, cyano, C₁₋₄ alkyl or 3-7 membered carbocyclyl; and the C₁₋₄ alkyl and the 3-7 membered carbocyclyl are independently unsubstituted or substituted by 1, 2, 3, 4 or 5 groups selected from the group consisting of oxo, hydroxyl, halogen, C₁₋₃ alkyl and halogenated C₁₋₃ alkyl.

In some embodiments, R₁ is hydrogen, halogen, cyano, C₁₋₄ alkyl (such as methyl), halogenated C₁₋₄ alkyl (such as trifluoromethyl), 3-7 membered carbocyclyl or halogenated 3-7 membered carbocyclyl.

In some embodiments, R₂ is hydrogen, halogen, cyano, C₁₋₄ alkyl or 3-7 membered carbocyclyl; and the C₁₋₄ alkyl and the 3-7 membered carbocyclyl are independently unsubstituted or substituted by 1, 2, 3, 4 or 5 groups selected from the group consisting of oxo, hydroxyl, halogen, C₁₋₃ alkyl and halogenated C₁₋₃ alkyl.

In some embodiments, R₂ is hydrogen, halogen, cyano, C₁₋₄ alkyl (such as methyl), halogenated C₁₋₄ alkyl (such as trifluoromethyl), 3-7 membered carbocyclyl or halogenated 3-7 membered carbocyclyl.

In some embodiments, X₁ is N.

In some embodiments, X₁ is C(R₄), wherein R₄ is hydrogen, halogen, hydroxyl, cyano, - C₂₋₄ alkenylene-phenyl, -C₂₋₄ alkynylene-phenyl, -S(O)-OH, -S(O)₂-OH, -S-(C₁₋₆ alkyl) or R₈; R₈ is C₁₋₆ alkyl, -O-C₁₋₆ alkyl, -C₁₋₆ alkylene-O-C₁₋₆ alkyl, -O-C₁₋₆ alkylene-O-C₁₋₆ alkyl, -C₀₋₆ alkylene-NRR', -C₀₋₆ alkylene-C(O)OH, -C₀₋₆ alkylene-C(O)-C₁₋₆ alkyl, -C₀₋₆ alkylene-C(O)-NRR', -C₀₋₆ alkylene-NR-C(O)-C₁₋₆ alkyl, -C₀₋₆ alkylene-S(O)₂-C₁₋₆ alkyl, -C₀₋₆ alkyleneS(O)₂-NRR', -C₀₋₆ alkylene-NR-S(O)₂-C₁₋₆ alkyl, -C₀₋₆ alkylene-NR-S(O)₂-NRR', -C₀₋₆ alkylene-P(O)O-(C₁₋₆ alkyl)₂, -C₀₋₆ alkylene-P(O)-(C₁₋₆ alkyl)(O-C₁₋₆ alkyl), -C₀₋₆ alkylene-P(O)-(C₁₋₆ alkyl)₂, -C₀₋₆ alkylene-3-14 membered carbocyclyl, -C₀₋₆ alkylene-3-14 membered heterocyclyl, -C₀₋₆ alkylene-5-12 membered heteroaryl, -C₀₋₆ alkylene-C₅₋₁₂ aryl, -C₀₋₆ alkylene-C(O)-3-14 membered heterocyclyl, -C₀₋₆ alkylene-C(O)-5-12 membered heteroaryl, -O-C₀₋₆ alkylene-O-C₁₋₆ alkyl, -O-C₀₋₆ alkylene-3-14 membered carbocyclyl, -O-C₀₋₆ alkylene-3-14 membered heterocyclyl, -O-C₀₋₆ alkylene-5-12 membered heteroaryl, -O-C₀₋₆ alkylene-C₅₋₁₂ aryl and -S(O)-C₁₋₆ alkyl, wherein the C₁₋₆ alkyl, the C₀₋₆ alkylene, the C₂₋₄ alkenylene, the 3-14 membered carbocyclyl, the 3-14 membered heterocyclyl, the 5-12 membered heteroaryl and the C₅₋₁₂ aryl are independently unsubstituted or substituted by 1, 2, 3, 4 or 5 groups selected from the group S1; the 3-14 membered heterocycle has 1, 2, 3 or 4 heteroatoms independently selected from nitrogen, oxygen and sulfur; the 5-12 membered heteroaryl has 1, 2, 3 or 4 heteroatoms independently selected from nitrogen, oxygen and sulfur; and the R, the R' and the groups in the group S1 are defined as above.

In some embodiments, X₁ is C(R₄), wherein R₄ is -C₀₋₆ alkylene-NRR', -C₀₋₆ alkylene-C(O)OH, -C₀₋₆ alkylene-C(O)-C₁₋₆ alkyl, -C₀₋₆ alkylene-C(O)-NRR', -C₀₋₆ alkylene-NR-C(O)-C₁₋₆ alkyl, -C₀₋₆ alkylene-S(O)₂-C₁₋₆ alkyl, -C₀₋₆ alkylene-S(O)₂-NRR', -C₀₋₆ alkylene-NR-S(O)₂-C₁₋₆ alkyl, -C₀₋₆ alkylene-NR-S(O)₂-NRR', -C₀₋₆ alkylene-P(O)O-(C₁₋₆ alkyl)₂, -C₀₋₆ alkylene-P(O)-(C₁₋₆ alkyl)(O-C₁₋₆ alkyl) or -C₀₋₆ alkylene-P(O)-(C₁₋₆ alkyl)₂, wherein the C₁₋₆ alkyl and the C₀₋₆ alkylene are independently unsubstituted or substituted by 1, 2, 3, 4 or 5 groups selected from the group S1; and the R, the R' and the groups in the group S1 are defined as above.

In some embodiments, X₁ is C(R₄), wherein R₄ is -C₀₋₆ alkylene-P(O)O-(C₁₋₆ alkyl)₂, - C₀₋₆ alkylene-P(O)-(C₁₋₆ alkyl)(O-C₁₋₆ alkyl) or -C₀₋₆ alkylene-P(O)-(C₁₋₆ alkyl)₂, wherein the C₁₋₆ alkyl and the C₀₋₆ alkylene are independently unsubstituted or substituted by 1, 2, 3, 4 or 5 groups selected from the group S1; and the groups in the group S1 are defined as above.

In some embodiments, X₁ is C(R₄), wherein R₄ is -P(O)O-(C₁₋₆ alkyl)₂, -P(O)-(C₁₋₆ alkyl)(O-C₁₋₆ alkyl) or -P(O)-(C₁₋₆ alkyl)₂, wherein the C₁₋₆ alkyl is independently unsubstituted or substituted by 1, 2, 3, 4 or 5 groups selected from the group S1; and the groups in the group S1 are defined as above.

In some embodiments, X₁ is C(R₄), wherein R₄ is hydrogen, halogen or cyano.

In some embodiments, X₁ is C(R₄), wherein R₄ is -P(O)O-(CH₃)₂, -P(O)-(CH₃)(O-CH₃) or -P(O)-(CH₃)₂.

In some embodiments, X₂ is C(R₃), wherein R₃ is hydrogen.

In some embodiments, Y is C(R₄), wherein R₄ is hydrogen.

In some embodiments, Z is C(R₃), wherein R₃ is hydrogen.

As mentioned herein, 2 hydrogen atoms on the same carbon atom on the C₀₋₆ alkylene or C₀₋₆ alkylene are substituted by the 3-7 membered carbocycle or 3-7 membered heterocyclic spirocycle at the same time, which means that after the 2 hydrogen atoms on the carbon atom are substituted by a bivalent substituent, the bivalent substituent and the carbon atom together form a 3-7 membered carbocycle or 3-7 membered heterocycle. For example, 2 hydrogen atoms on the carbon atom on -CH₂- are substituted by cyclopropyl ring spirocycle, which means that after 2 hydrogen atoms on the carbon atom on -CH₂- are substituted by bivalent ethyl (that is, ethylidene), the ethylidene and the carbon atom together form cyclopropyl, and so on.

In some embodiments,
the compound shown in Formula (A) is a compound shown in Formula (I-A): 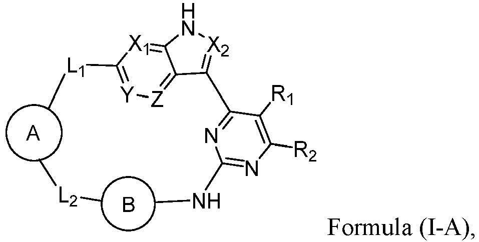
wherein X₁, Y, Z, X₂, R₁, R₂, L₁, L₂, ring A and ring B are respectively defined as above;
   or
the compound shown in Formula (A) is a compound shown in Formula (I-B): 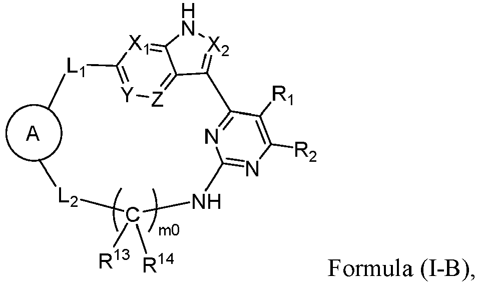
wherein X₁, Y, Z, X₂, R₁, R₂, L₁, L₂, ring A, R¹³ and R¹⁴ are respectively defined as above, and m0 is 1, 2, 3, 4, 5 or 6;
   or
the compound shown in Formula (A) is a compound shown in Formula (A1): 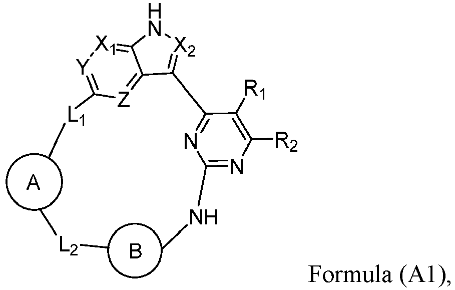
wherein X₁, Y, Z, X₂, R₁, R₂, L₁, L₂, ring A and ring B are respectively defined as above;
   or
the compound shown in Formula (A) is a compound shown in Formula (A2): 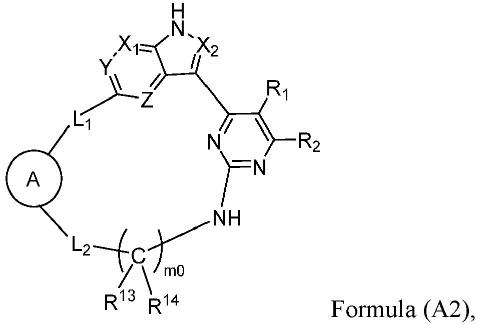
wherein X₁, Y, Z, X₂, R₁, R₂, L₁, L₂, ring A, R¹³ and R¹⁴ are respectively defined as above, and m0 is 1, 2, 3, 4, 5 or 6.

In some embodiments, the compound shown in Formula (A) is a compound shown in Formula (Ia), a compound shown in Formula (Ib), a compound shown in Formula (Ic), a compound shown in Formula (Id), a compound shown in Formula (Ie) or a compound shown in Formula (If): 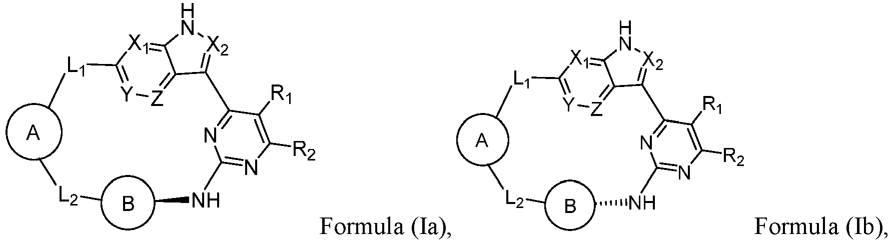 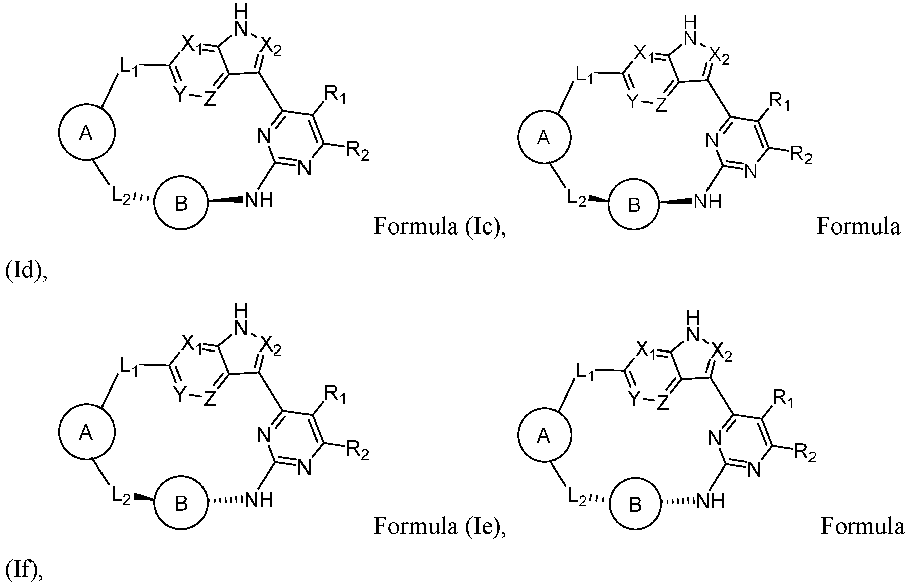 wherein X₁, Y, Z, X₂, R₁, R₂, L₁, L₂, ring A and ring B are respectively defined as above.

In some embodiments,
the compound shown in Formula (A) is a compound shown in Formula (II-A): 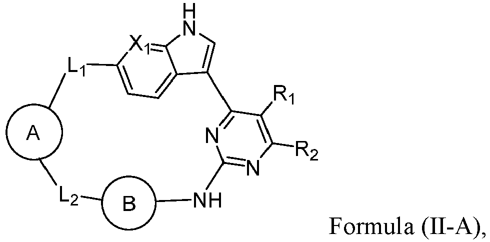
wherein R₁, R₂, L₁, L₂, ring A, ring B and X₁ are respectively defined as above;
   or
the compound shown in Formula (A) is a compound shown in Formula (II-B): 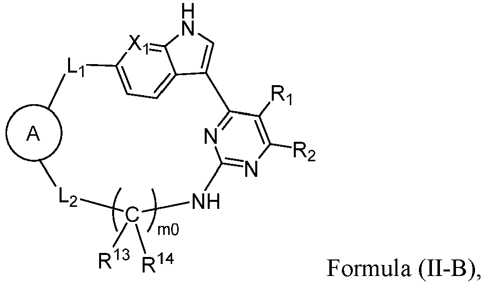
wherein X₁, R₁, R₂, L₁, L₂, ring A, R¹³ and R¹⁴ are respectively defined as above, and m0 is 1, 2, 3, 4, 5 or 6;
   or
the compound shown in Formula (A) is a compound shown in Formula (A3): 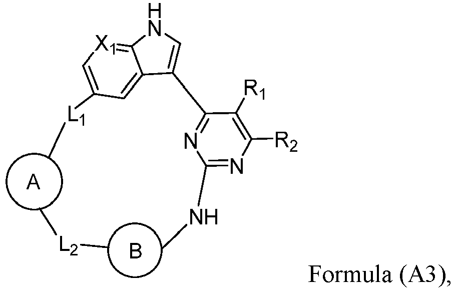
wherein X₁, R₁, R₂, L₁, L₂, ring A and ring B are respectively defined as above;
   or
the compound shown in Formula (A) is a compound shown in Formula (A4): 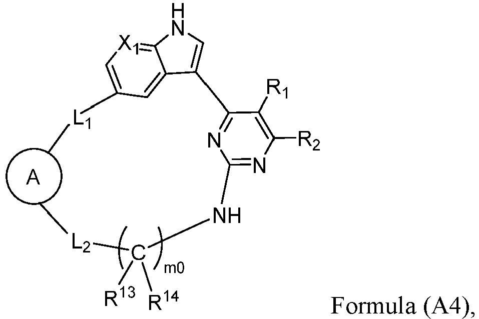
wherein X₁, R₁, R₂, L₁, L₂, ring A, R¹³ and R¹⁴ are respectively defined as above, and m0 is 1, 2, 3, 4, 5 or 6.

In some embodiments, the compound shown in Formula (A) is a compound shown in Formula (IIa), a compound shown in Formula (IIb), a compound shown in Formula (IIc), a compound shown in Formula (IId), a compound shown in Formula (IIe) or a compound shown in Formula (IIf): 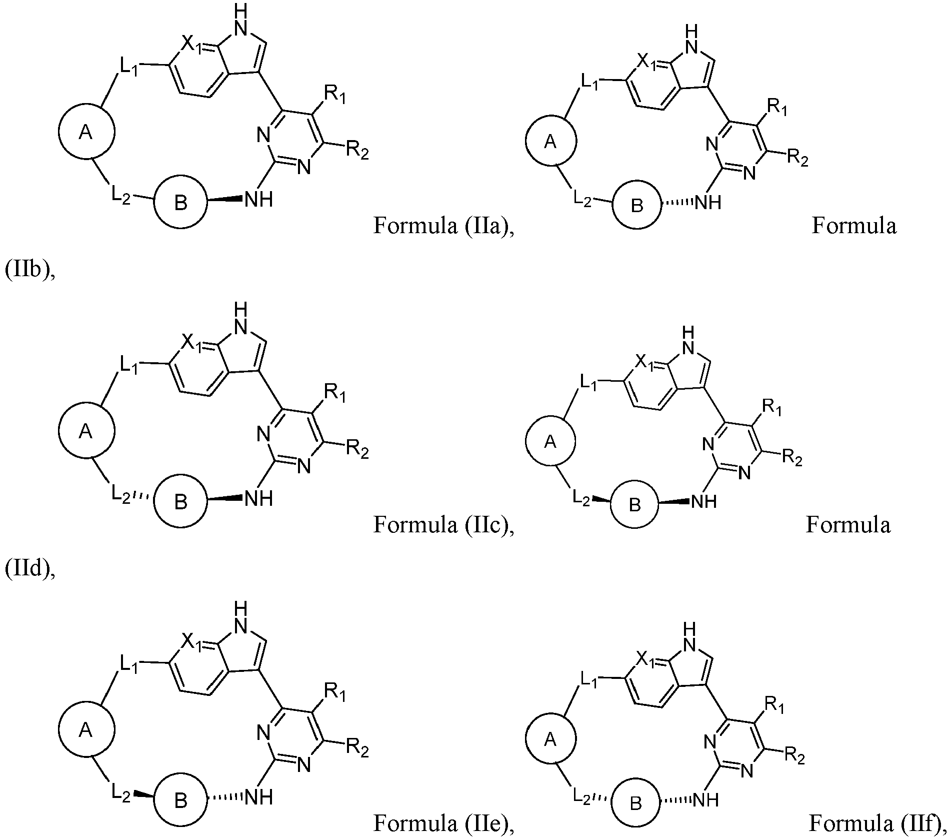 wherein X₁, R₁, R₂, L₁, L₂, ring A and ring B are respectively defined as above.

In some embodiments,
the compound shown in Formula (A) is a compound shown in Formula (III-A): 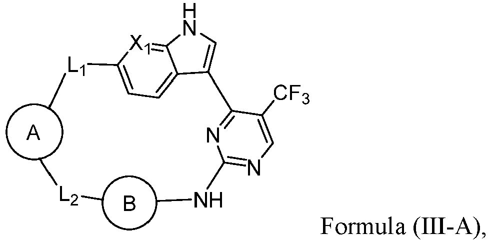
wherein L₁, L₂, ring A, ring B and X₁ are respectively defined as above;
   or
the compound shown in Formula (A) is a compound shown in Formula (III-B): 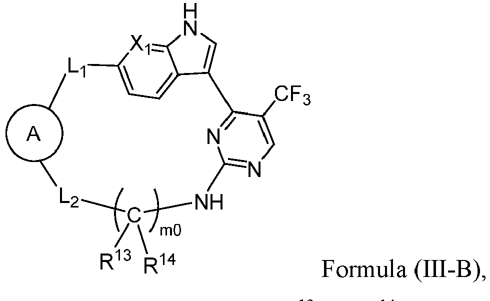
wherein X₁, L₁, L₂, ring A, R¹³ and R¹⁴ are respectively defined as above, and m0 is 1, 2, 3, 4, 5 or 6;
   or
the compound shown in Formula (A) is a compound shown in Formula (A5): 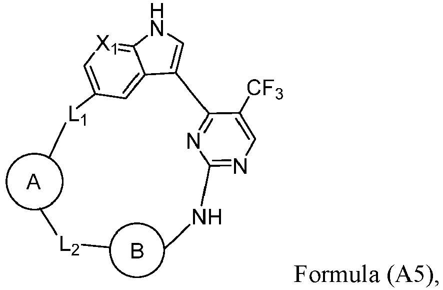
wherein X₁, L₁, L₂, ring A and ring B are respectively defined as above;
   or
the compound shown in Formula (A) is a compound shown in Formula (A6): 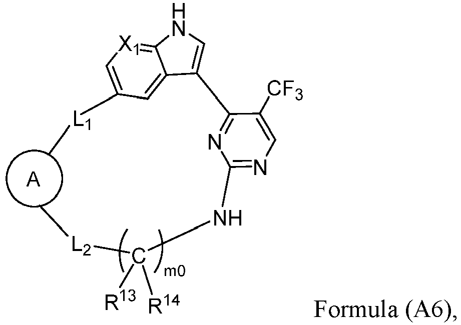
wherein X₁, L₁, L₂, ring A, R¹³ and R¹⁴ are respectively defined as above, and m0 is 1, 2, 3, 4, 5 or 6.

In some embodiments, the compound shown in Formula (A) is a compound shown in Formula (III-1) or a compound shown in Formula (III-2): 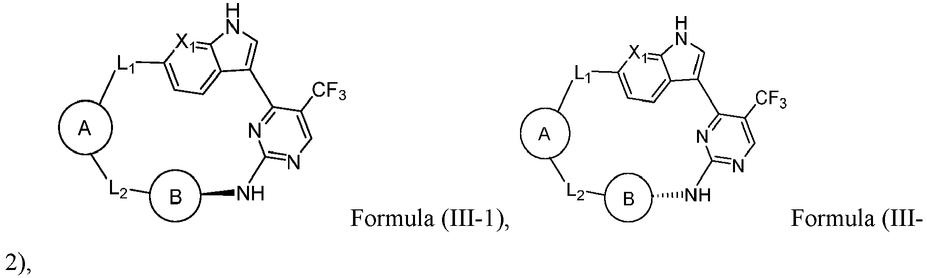 wherein L₁, L₂, ring A, ring B and X₁ are respectively defined as above.

In some embodiments, the compound shown in Formula (A) is a compound shown in Formula (III-a), a compound shown in Formula (III-b), a compound shown in Formula (III-c) or a compound shown in Formula (III-d): 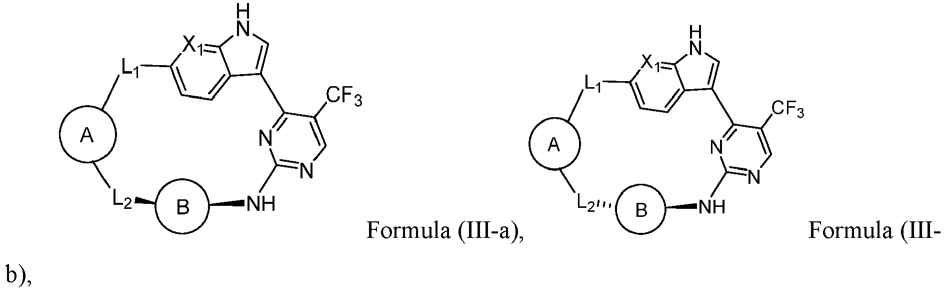 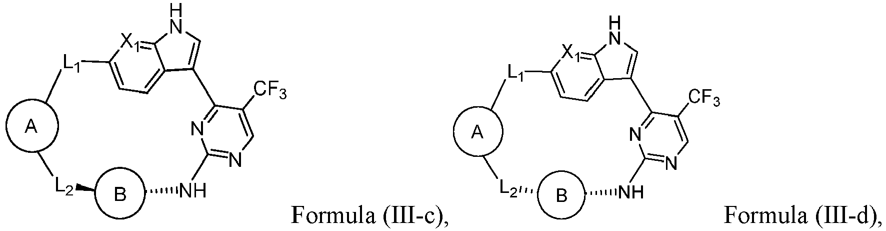 wherein L₁, L₂, ring A, ring B and X₁ are respectively defined as above.

In some embodiments, the compound shown in Formula (A) is a compound shown in Formula (IV-a), a compound shown in Formula (IV-b) or a compound shown in Formula (IV-c): 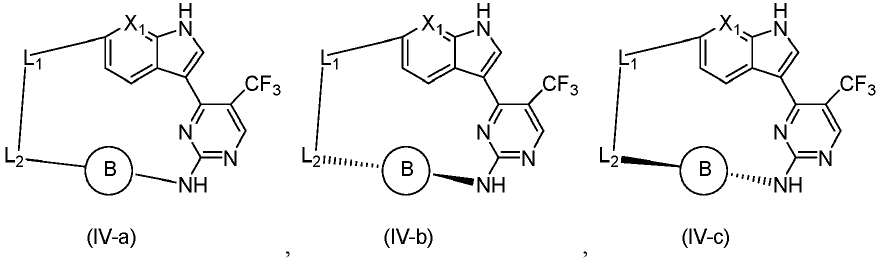 ,wherein L₁, L₂, ring B and X₁ are respectively defined as above.

In some embodiments, the ring A is none; or the ring A is selected from the group consisting of 3-6 membered carbocycle, 3-6 membered heterocycle, 5-6 membered heteroaryl and phenyl; the ring A is unsubstituted or substituted by 1, 2 or 3 groups selected from OH, C₁₋₆ alkyl and halogen; and other variables are defined as in the present invention.

In some embodiments, the ring A is none; or the ring A is selected from the group consisting of cyclobutyl ring, cyclopentyl ring, cyclohexyl ring, piperidinyl ring, piperazinyl ring, tetrahydropyrrolidinyl ring, pyrazolyl ring and imidazolyl ring; the ring A is unsubstituted or substituted by 1, 2, 3, 4 or 5 groups selected from the group S1; and other variables are defined as in the present invention.

In some embodiments, the ring A is none; or the ring A is selected from the group consisting of cyclobutyl ring, cyclopentyl ring, cyclohexyl ring, piperidinyl ring, piperazinyl ring, tetrahydropyrrolidinyl ring, pyrazolyl ring and imidazolyl ring; the ring A is unsubstituted or substituted by 1, 2, 3, 4 or 5 groups selected from the group S1; the groups in the group S include: hydroxyl, methyl and halogen; and other variables are defined as in the present invention.

In some embodiments, the ring A is none; or the ring A is selected from the group consisting of: 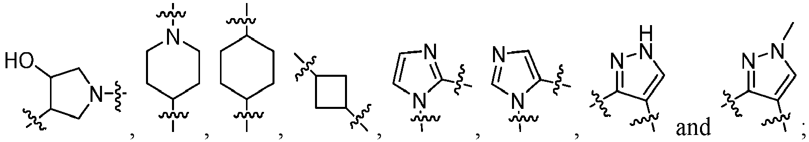 and other variables are defined as in the present invention.

In some embodiments, the ring A is none; or the ring A is selected from the group consisting of: 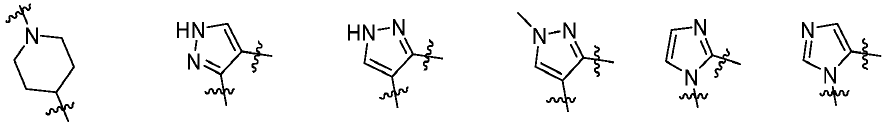 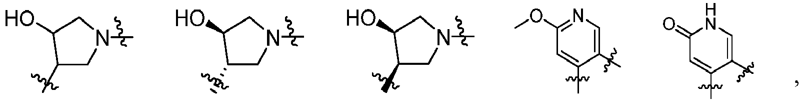 and other variables are defined as in the present invention.

In some embodiments, the ring B is selected from the group consisting of 3-6 membered carbocycle and 3-6 membered heterocycle; the ring B is unsubstituted or substituted by 1, 2 or 3 groups selected from OH, C₁₋₆ alkyl and halogen; and other variables are defined as in the present invention.

In some embodiments, the ring B is selected from the group consisting of cyclobutyl ring, cyclopentyl ring, cyclohexyl ring, piperidinyl ring, piperazinyl ring, tetrahydropyrrolidinyl ring, pyrazolyl ring and imidazolyl ring; the ring B is unsubstituted or substituted by 1, 2, 3, 4 or 5 groups selected from the group S1; and other variables are defined as in the present invention.

In some embodiments, the ring B is selected from the group consisting of 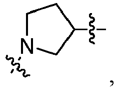 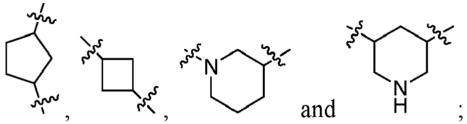 and other variables are defined as in the present invention.

In some embodiments, the ring B is selected from the group consisting of: 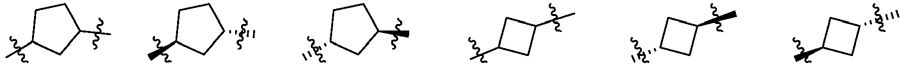 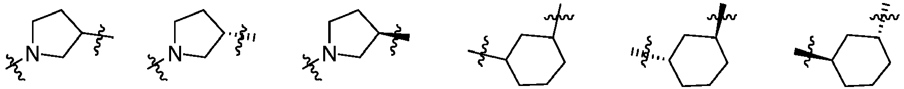 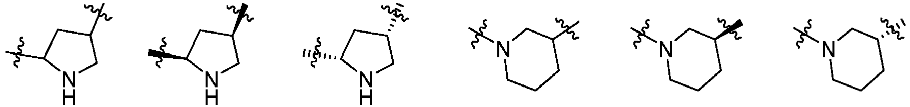 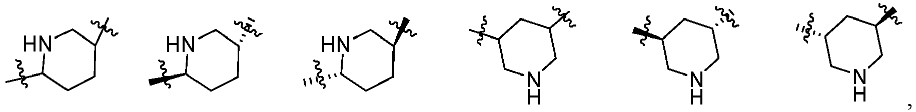 and other variables are defined as in the present invention.

In some embodiments, L₂ is -NR¹¹-(CR¹³R¹⁴)ₘ₁-NR¹²-; R¹¹ and R¹² are independently hydrogen or methyl; each R¹³ and R¹⁴ are independently hydrogen, halogen or methyl; m1 is 1, 2, 3, 4, 5 or 6; and other variables are defined as in the present invention.

In some embodiments, L₂ is -O-(CR¹³R¹⁴)ₘ₁-NR¹²-; R¹² is hydrogen or methyl; each R¹³ and R¹⁴ are independently hydrogen, halogen or methyl; m1 is 1, 2, 3, 4, 5 or 6; and other variables are defined as in the present invention.

In some embodiments, L₂ is -NR¹¹-(CR¹³R¹⁴)ₘ₁-; R¹¹ is hydrogen or methyl; each R¹³ and R¹⁴ are independently hydrogen, halogen or methyl; m1 is 1, 2, 3, 4, 5 or 6; and other variables are defined as in the present invention.

In some embodiments, L₂ is -(CR¹³R¹⁴)ₘ₁-NR¹² -; R¹² is hydrogen or methyl; each R¹³ and R¹⁴ are independently hydrogen, halogen or methyl; m1 is 1, 2, 3, 4, 5 or 6; and other variables are defined as in the present invention.

In some embodiments, L₂ is -(CR¹³R¹⁴)ₘ₁-; each R¹³ and R¹⁴ are independently hydrogen, halogen or methyl; m1 is 1, 2, 3, 4, 5 or 6; and other variables are defined as in the present invention.

In some embodiments, L₂ is -NR¹¹-(CR¹³R¹⁴)ₘ₅-3-7 membered carbocycle-(CR¹³R¹⁴)ₘ₆-NR¹²-; R¹¹ and R¹² are independently hydrogen or methyl; each R¹³ and R¹⁴ are independently hydrogen, halogen or methyl; m5 is 0, 1, 2, 3 or 4; m6 is 0, 1, 2, 3 or 4; the 3-7 membered carbocycle is unsubstituted or substituted by 1, 2, 3, 4 or 5 groups selected from the group S1; and other variables are defined as in the present invention.

In some embodiments, L₂ is -O-(CR¹³R¹⁴)ₘ₅-3-7 membered carbocycle-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; the 3-7 membered carbocycle is unsubstituted or substituted by 1, 2, 3, 4 or 5 groups selected from the group S1; and other variables are defined as in the present invention.

In some embodiments, L₂ is -(CR¹³R¹⁴)ₘ₅-3-7 membered carbocycle-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; and the 3-7 membered carbocycle is unsubstituted or substituted by 1, 2, 3, 4 or 5 groups selected from the group S1. In some embodiments, the 3-7 membered carbocycle is cyclobutyl, cyclopentyl or cyclohexyl; and other variables are defined as in the present invention.

In some embodiments, L₂ is -NR¹¹-(CR¹³R¹⁴)ₘ₅-3-7 membered heterocycle-(CR¹³R¹⁴)ₘ₆-NR¹²; R¹¹ and R¹² are independently hydrogen or methyl; each R¹³ and R¹⁴ are independently hydrogen, halogen or methyl; m5 is 0, 1, 2, 3 or 4; m6 is 0, 1, 2, 3 or 4; the 3-7 membered heterocycle is unsubstituted or substituted by 1, 2, 3, 4 or 5 groups selected from the group S1; and other variables are defined as in the present invention.

In some embodiments, L₂ is -O-(CR¹³R¹⁴)ₘ₅-3-7 membered heterocycle-(CR¹³R¹⁴)ₘ₆-NR¹²; R¹² is hydrogen or methyl; each R¹³ and R¹⁴ are independently hydrogen, halogen or methyl; m5 is 0, 1, 2, 3 or 4; m6 is 0, 1, 2, 3 or 4; the 3-7 membered heterocycle is unsubstituted or substituted by 1, 2, 3, 4 or 5 groups selected from the group S1; and other variables are defined as in the present invention.

In some embodiments, L₂ is -(CR¹³R¹⁴)ₘ₅-3-7 membered heterocycle-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; the 3-7 membered heterocycle is unsubstituted or substituted by 1, 2, 3, 4 or 5 groups selected from the group S1; and other variables are defined as in the present invention.

In some embodiments, L₂ is -NR¹¹-(CR¹³R¹⁴)ₘ₅-NR¹⁰-(CR¹³R¹⁴)ₘ₆-NR¹²-; R¹¹ and R¹² are independently hydrogen or methyl; each R¹³ and R¹⁴ are independently hydrogen, halogen or methyl; R¹⁰ is hydrogen or methyl; m5 is 0, 1, 2, 3 or 4; m6 is 0, 1, 2, 3 or 4; and other variables are defined as in the present invention.

In some embodiments, L₂ is -O-(CR¹³R¹⁴)ₘ₅-NR¹⁰-(CR¹³R¹⁴)ₘ₆-NR¹²-; R¹² is hydrogen or methyl; each R¹³ and R¹⁴ are independently hydrogen, halogen or methyl; R¹⁰ is hydrogen or methyl; m5 is 0, 1, 2, 3 or 4; m6 is 0, 1, 2, 3 or 4; and other variables are defined as in the present invention.

In some embodiments, L₂ is -(CR¹³R¹⁴)ₘ₅-NR¹⁰-(CR¹³R¹⁴)ₘ₆-NR¹²-; R¹² is hydrogen or methyl; each R¹³ and R¹⁴ are independently hydrogen, halogen or methyl; R¹⁰ is hydrogen or methyl; m5 is 0, 1, 2, 3 or 4; m6 is 0, 1, 2, 3 or 4; and other variables are defined as in the present invention.

In some embodiments, L₂ is -NR¹¹-(CR¹³R¹⁴)ₘ₅-NR¹⁰C(O)-(CR¹³R¹⁴)ₘ₆-NR¹²-; R¹¹ and R¹² are independently hydrogen or methyl; each R¹³ and R¹⁴ are independently hydrogen, halogen or methyl; R¹⁰ is hydrogen or methyl; m5 is 0, 1, 2, 3 or 4; m6 is 0, 1, 2, 3 or 4; and other variables are defined as in the present invention.

In some embodiments, L₂ is -O-(CR¹³R¹⁴)ₘ₅-NR¹⁰C(O)-(CR¹³R¹⁴)ₘ₆-NR¹²-; R¹² is hydrogen or methyl; each R¹³ and R¹⁴ are independently hydrogen, halogen or methyl; R¹⁰ is hydrogen or methyl; m5 is 0, 1, 2, 3 or 4; m6 is 0, 1, 2, 3 or 4; and other variables are defined as in the present invention.

In some embodiments, L₂ is -(CR¹³R¹⁴)ₘ₅-NR¹⁰C(O)-(CR¹³R¹⁴)ₘ₆-NR¹²-; R¹² is hydrogen or methyl; each R¹³ and R¹⁴ are independently hydrogen, halogen or methyl; R¹⁰ is hydrogen or methyl; m5 is 0, 1, 2, 3 or 4; m6 is 0, 1, 2, 3 or 4; and other variables are defined as in the present invention.

In some embodiments, L₂ is -NR¹¹-(CR¹³R¹⁴)ₘ₅-C(O)NR¹⁰-(CR¹³R¹⁴)ₘ₆-NR¹²-; R¹¹ and R¹² are independently hydrogen or methyl; each R¹³ and R¹⁴ are independently hydrogen, halogen or methyl; R¹⁰ is hydrogen or methyl; m5 is 0, 1, 2, 3 or 4; m6 is 0, 1, 2, 3 or 4; and other variables are defined as in the present invention.

In some embodiments, L₂ is -O-(CR¹³R¹⁴)ₘ₅-C(O)NR¹⁰-(CR¹³R¹⁴)ₘ₆-NR¹²-; R¹² is hydrogen or methyl; each R¹³ and R¹⁴ are independently hydrogen, halogen or methyl; R¹⁰ is hydrogen or methyl; m5 is 0, 1, 2, 3 or 4; m6 is 0, 1, 2, 3 or 4; and other variables are defined as in the present invention.

In some embodiments, L₂ is -(CR¹³R¹⁴)ₘ₅-C(O)NR¹⁰-(CR¹³R¹⁴)ₘ₆-NR¹²-; R¹² is hydrogen or methyl; each R¹³ and R¹⁴ are independently hydrogen, halogen or methyl; R¹⁰ is hydrogen or methyl; m5 is 0, 1, 2, 3 or 4; m6 is 0, 1, 2, 3 or 4; and other variables are defined as in the present invention.

In some embodiments, L₂ is -NR¹¹-(CR¹³R¹⁴)ₘ₅-O-(CR¹³R¹⁴)ₘ₆-NR¹²-; R¹¹ and R¹² are independently hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -O-(CR¹³R¹⁴)ₘ₅-O-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -(CR¹³R¹⁴)ₘ₅-O-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -NR¹¹-(CR¹³R¹⁴)ₘ₅-OC(O)-(CR¹³R¹⁴)ₘ₆-NR¹²-; R¹¹ and R¹² are independently hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -O-(CR¹³R¹⁴)ₘ₅-OC(O)-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -(CR¹³R¹⁴)ₘ₅-OC(O)-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -NR¹¹-(CR¹³R¹⁴)ₘ₅-C(O)O-(CR¹³R¹⁴)ₘ₆-NR¹²-; R¹¹ and R¹² are independently hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -O-(CR¹³R¹⁴)ₘ₅-C(O)O-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -(CR¹³R¹⁴)ₘ₅-C(O)O-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -NR¹¹-(CR¹³R¹⁴)ₘ₅-CH=CH-(CR¹³R¹⁴)ₘ₆-NR¹²-; R¹¹ and R¹² are independently hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -O-(CR¹³R¹⁴)ₘ₅-CH=CH-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, L₂ is -(CR¹³R¹⁴)ₘ₅-CH=CH-(CR¹³R¹⁴)ₘ₆-NR¹²-; R¹² is hydrogen or methyl; each R¹³ and R¹⁴ are independently hydrogen, halogen or methyl; m5 is 0, 1, 2, 3 or 4; m6 is 0, 1, 2, 3 or 4; and other variables are defined as in the present invention.

In some embodiments, the groups in the group S 1 include: oxo, hydroxyl, cyano, halogen, methyl, ethyl and propyl; and other variables are defined as in the present invention.

In some embodiments, the 3-7 membered carbocycle is cyclopropyl, cyclobutyl, cyclopentyl or cyclohexyl; and other variables are defined as in the present invention.

In some embodiments, the 3-7 membered heterocycle is azacyclobutyl, oxacyclobutyl, tetrahydropyrrolyl, tetrahydrofuranyl, piperidyl or piperazinyl; and other variables are defined as in the present invention.

In some embodiments, L₂ is selected from one of the following groups: 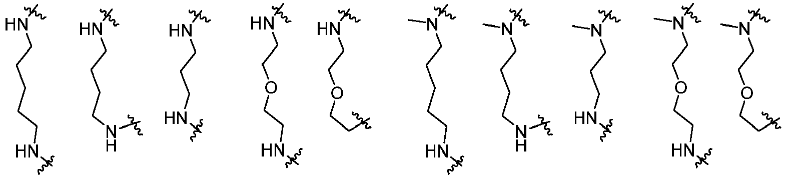 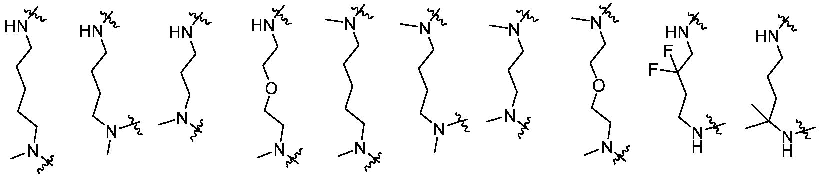 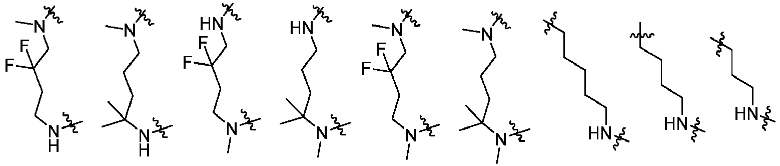 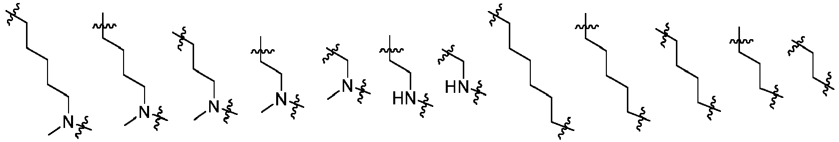 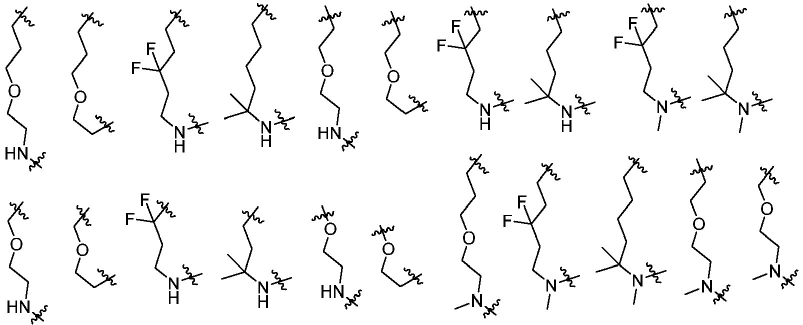 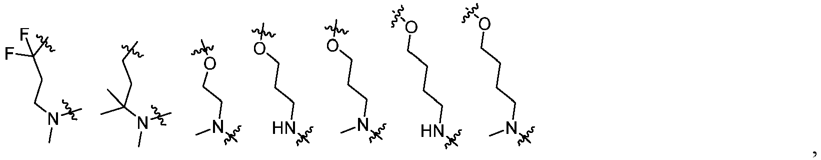 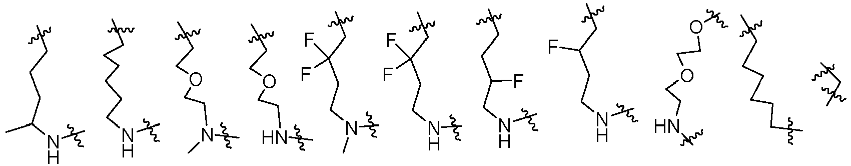 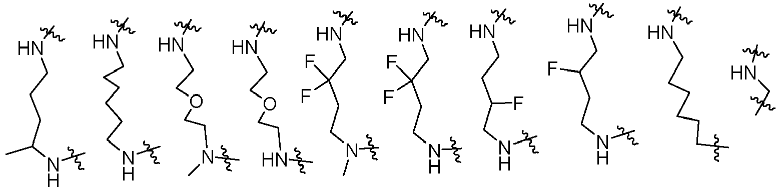 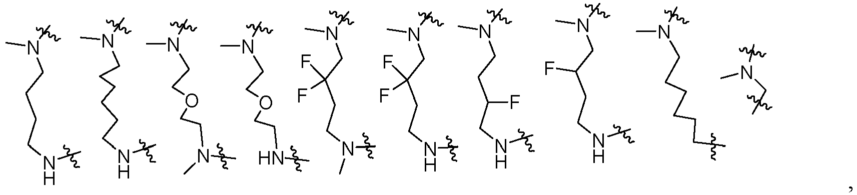 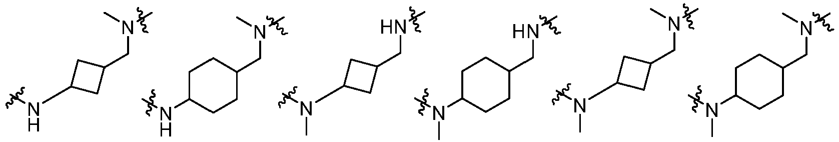 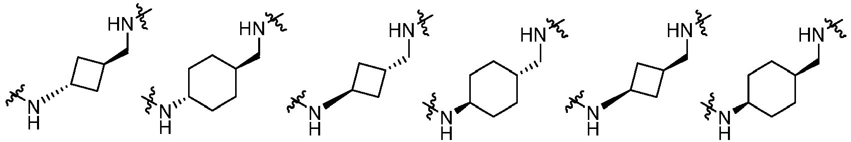 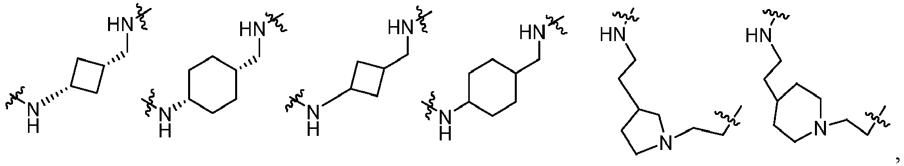 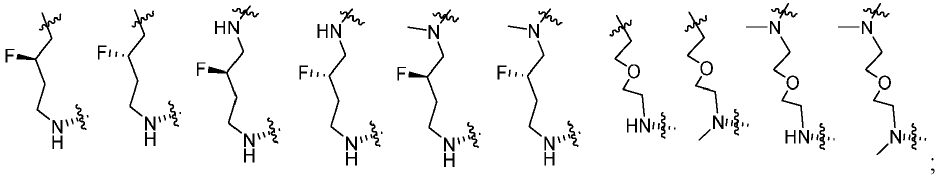 and other variables are defined as in the present invention.

In some embodiments, L₂ is selected from the group consisting of: 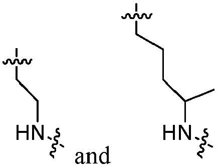 ; and other variables are defined as in the present invention.

In some embodiments, L₁ is none, -O-, -N(CH₃)-, -CH=CH-, -C=N-, -CH₂-, -CH(CH₃), - CH₂CH₂-, -CH(CH₃)CH₂-, -CH₂CH₂CH₂-, -CH₂CH₂CH(CH₃)-, -C(O)-, -NH-C(O)-, - N(CH₃)-C(O)-, -C(O)-N(CH₃)-, -C(O)-NH-CH₂- or -C(O)-NH-; and other variables are defined as in the present invention.

In some embodiments, L₁ is -N(CD₃)-, -CH(CD₃), -N(CD₃)-C(O)- or -C(O)-N(CD₃); and other variables are defined as in the present invention.

In some embodiments, X₁ is N or C(R₄), wherein R₄ is hydrogen, halogen, cyano, - S(O)₂CH₃ or -P(O)(CH₃)₂; and other variables are defined as in the present invention.

In some embodiments, the compound shown in Formula (A) is selected from one of the compounds shown in Table (I) below:

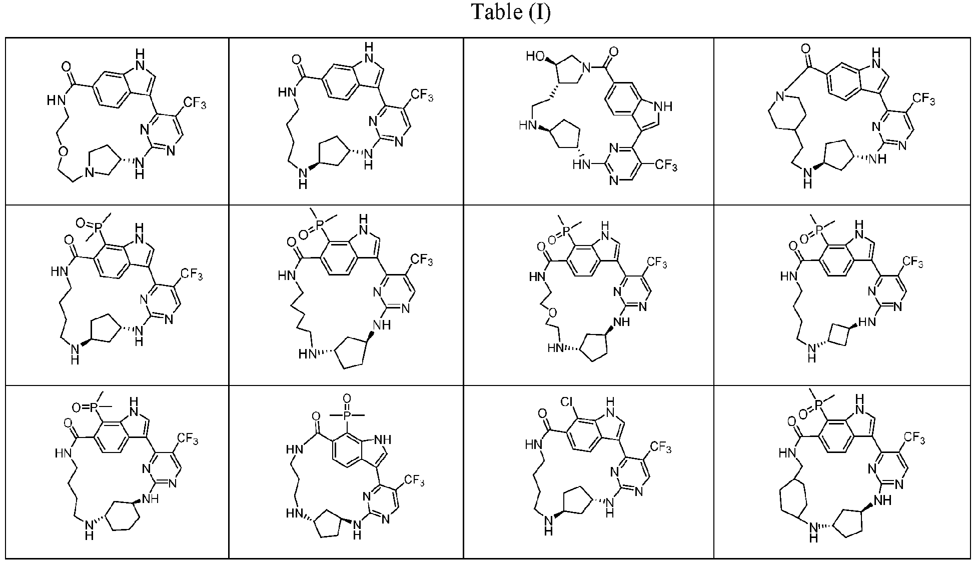

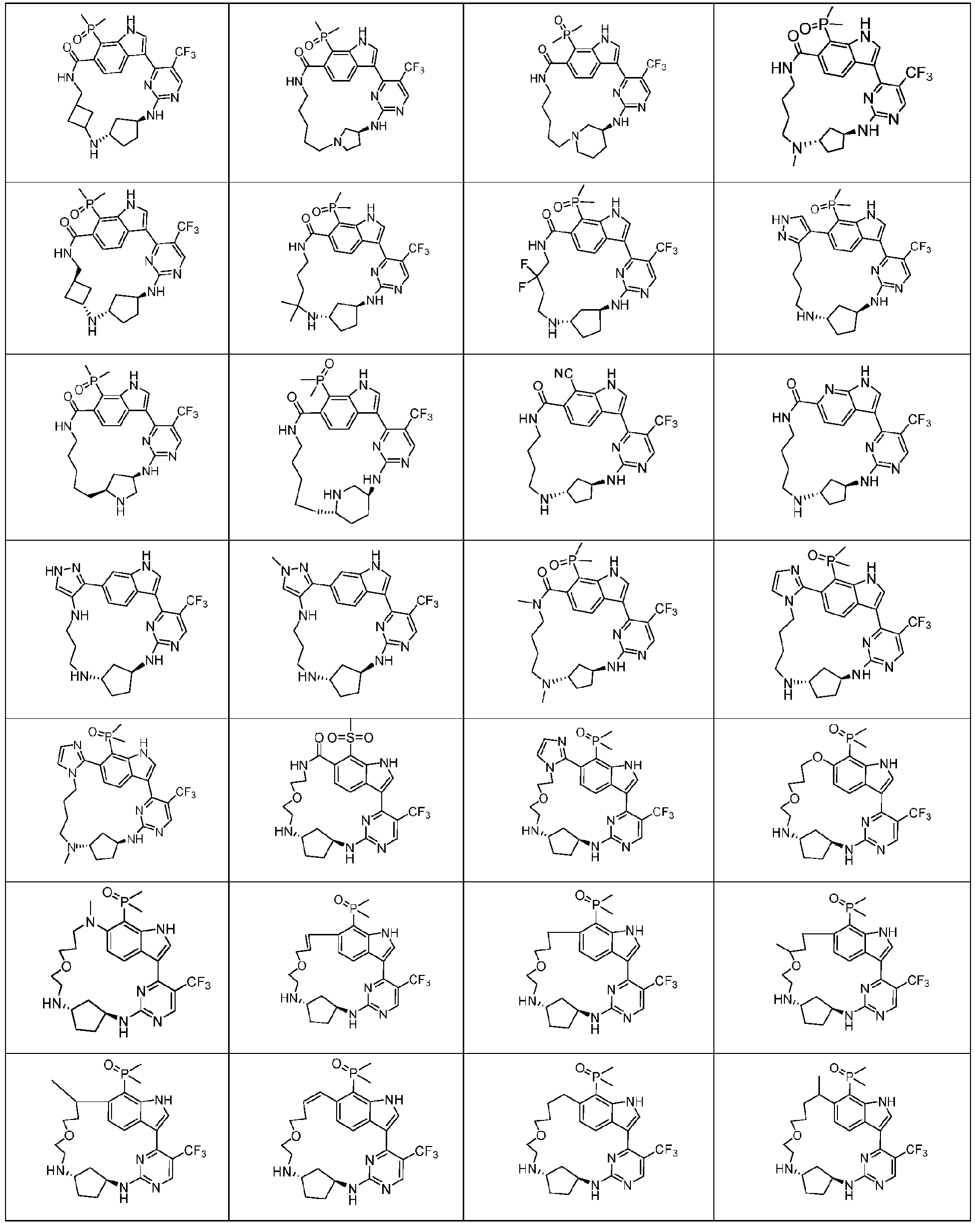

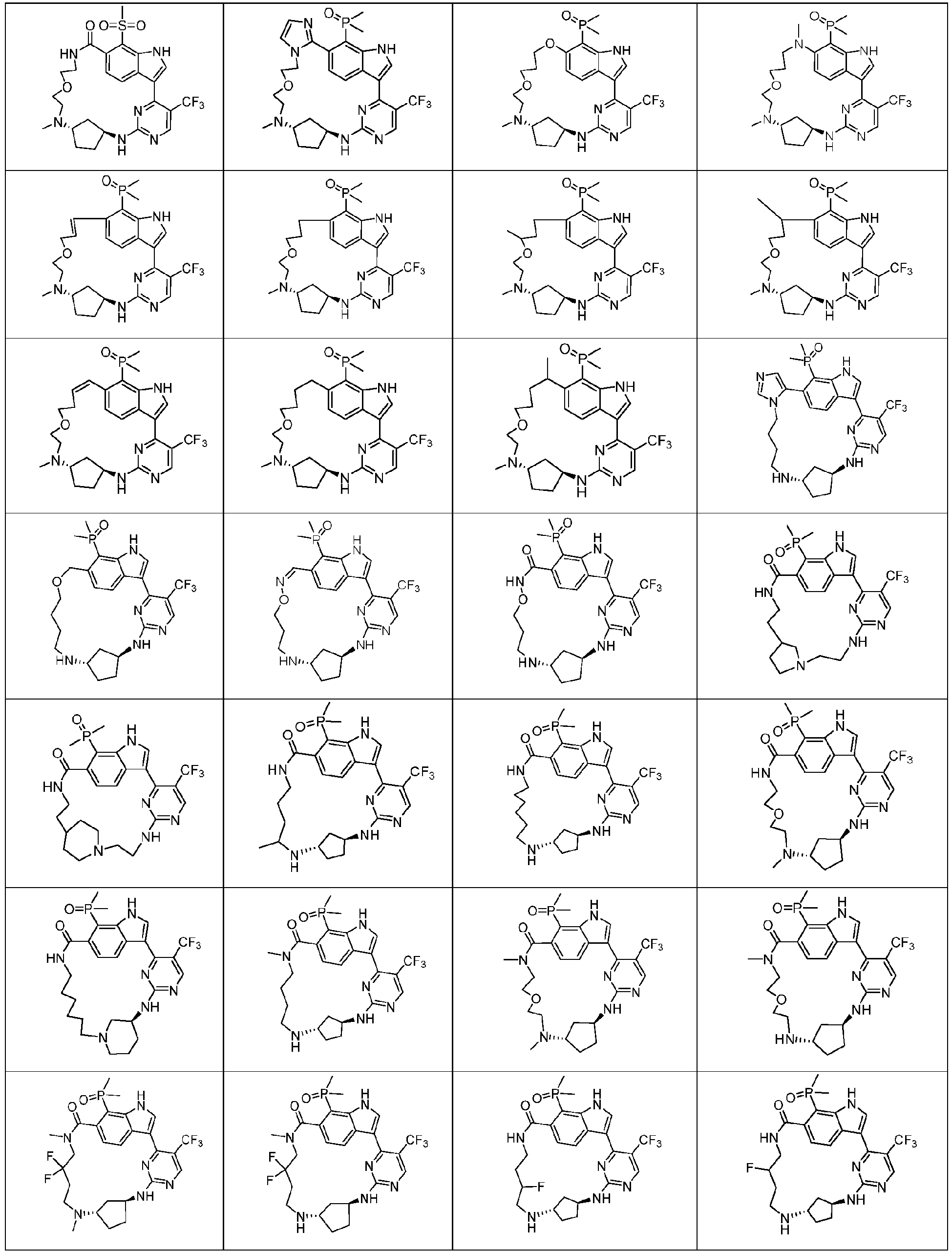

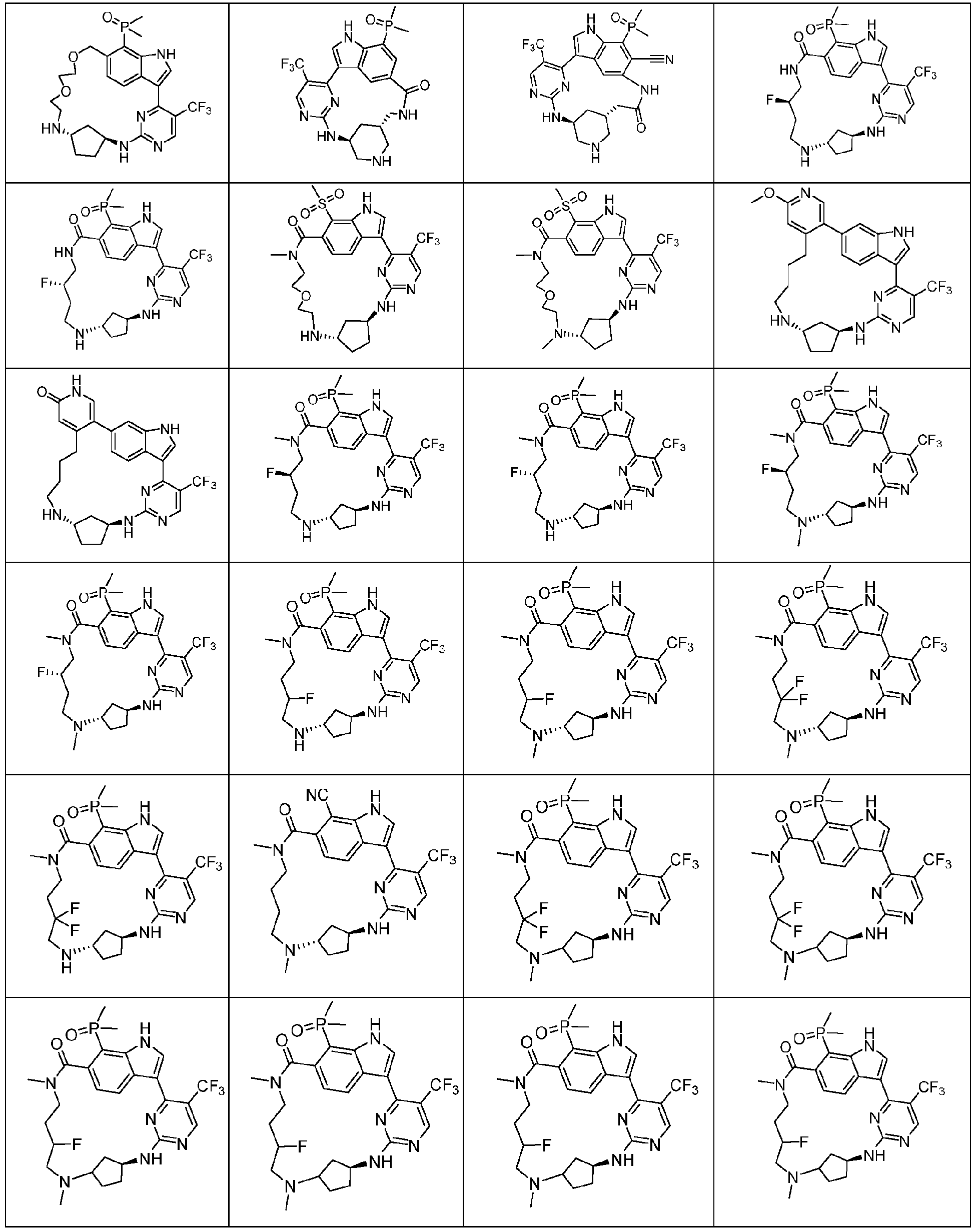

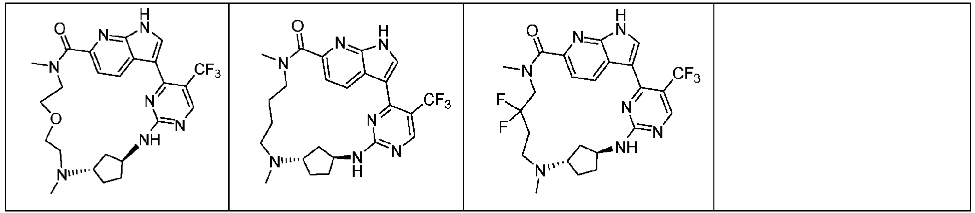

A second aspect of the present invention provides a pharmaceutical composition, comprising the compound described in the first aspect, a stereoisomer, a prodrug or a pharmaceutically acceptable salt thereof, and a pharmaceutically acceptable carrier.

As used herein, the term "active material of the present invention" or "compound of the present invention" refers to the compound described in the first aspect, a stereoisomer, a prodrug or a pharmaceutically acceptable salt thereof.

As used herein, the term "pharmaceutically acceptable carrier" refers to a representative carrier of any preparation or carrier medium that can deliver an effective dose of the active material of the present invention, does not interfere with the biological activity of the active material, and has no toxic and side effects on hosts or subjects, including water, oil, vegetables, minerals, cream bases, lotion bases, ointment bases, etc. These bases include suspending agents, tackifiers, transdermal enhancers, etc. The preparations thereof are well known to technicians in the field of cosmetics or local drugs.

The pharmaceutical composition can be administered in any of the following ways: oral administration, spray inhalation, rectal administration, nasal administration, buccal administration, local administration, and parenteral administration, such as subcutaneous, intravenous, intramuscular, intraperitoneal, intrathecal, intraventricular, intrasternaland intracranial injection or infusion, or administration by virtue of an external implant, wherein oral administration, intraperitoneal administration or intravenous administration is preferred. During oral administration, the compound of the present invention can be prepared into any orally acceptable preparation, including, but not limited to, troches, capsules, aqueous solutions or aqueous suspensions. Carriers used for troches generally include lactose and corn starch, and lubricants such as magnesium stearate can also be added. Diluents used for capsules generally include lactose and dried corn starch. Aqueous suspensions are generally used by mixing active ingredients with appropriate emulsifiers and suspending agents. If necessary, some sweeteners, aromatics or colorants can be added to the above oral preparations. During local administration, especially treatment of the affected surfaces or organs that are easy to reach by local external application, such as eyes, skin or lower intestinal nervous diseases, the compound of the present invention can be prepared into different local pharmaceutical preparations according to different affected surfaces or organs. During local application to eyes, the compound of the present invention can be prepared into a micronized suspension or solution, and the carrier used is isotonic sterile saline with a certain pH, wherein preservatives such as benzyl chloride alkoxide can be optionally added. For eye use, the compound can also be prepared into ointments, such as a vaseline ointment. During local application to skin, the compound of the present invention can be prepared into appropriate ointments, lotions or creams, wherein active ingredients are suspended or dissolved in one or more carriers. Carriers used for ointments include, but are not limited to, mineral oil, liquid vaseline, white vaseline, propylene glycol, polyethylene oxide, polypropylene oxide, emulsified wax and water. Carriers used for lotions or creams include, but are not limited to, mineral oil, dehydrated sorbitol monostearate, Tween 60, hexadecane wax, hexadecane aromatic alcohol, 2-octyldodecanol, benzyl alcohol and water. The compound of the present invention can also be administered in the form of sterile injection preparations, including sterile injection water or oil suspensions or sterile injection solutions. The available carriers and solvents include water, Ringer's solutions and isotonic sodium chloride solutions. In addition, sterilized non-volatile oil can also be used as a solvent or suspending medium, such as glyceride or diglyceride.

In another aspect, the present invention provides use of the compound described in the first aspect, a stereoisomer, a prodrug or a pharmaceutically acceptable salt thereof in preparation of drugs for treating and/or preventing CDK7-related diseases.

In another aspect, the present invention provides use of the compound described in the first aspect, a stereoisomer, a prodrug or a pharmaceutically acceptable salt thereof in preparation of drugs for treating and/or preventing cancers.

In another aspect, the present invention provides a method for treating cancers, including the step of administering an effective therapeutic dose of the compound described in the first aspect, a stereoisomer, a prodrug or a pharmaceutically acceptable salt thereof or any combination of the above, or administering the pharmaceutical composition described in the second aspect to subjects in need.

In another aspect, the present invention provides use of the compound described in the first aspect, a stereoisomer, a prodrug or a pharmaceutically acceptable salt thereof in preparation of CDK7 inhibitors.

In some embodiments, the CDK7-related diseases are proliferative diseases (such as tumors or cancers), infectious diseases, immune diseases, autoimmune diseases and inflammatory diseases.

In some embodiments, the tumors or cancers include solid tumors and hematologic tumors. In some embodiments, the tumors or cancers include melanomas, bone cancers (such as osteosarcomas and Ewing's sarcomas), breast cancers (such as hormone receptor positive (HR+) breast cancers (such as estrogen receptor positive (ER+) or progesterone receptor positive (PR+) breast cancers), hormone receptor negative breast cancers, triple negative breast cancers (TNBC; ER-/PR-/HER2-)), colorectal cancers, brain cancers, lung cancers (such as small cell lung cancers and non-small cell lung cancers), pancreatic cancers, ovarian cancers, endometrial cancers, cervical cancers, esophageal cancers, gastric cancers, bile duct cancers, prostate cancers, liver cancers, renal cell cancers, chronic lymphoblastic leukemia (CLL), acute lymphoblastic leukemia (ALL), T-cell acute lymphoblastic leukemia (T-ALL), chronic myeloid leukemia (CML), acute myeloid leukemia (AML), lymphomas, multiple myelomas, glioblastomas and neuroblastomas.

In some embodiments, the infectious diseases include AIDS, cholera, conjunctivitis, dengue fever, encephalitis, enterovirus infection (such as poliomyelitis and non-poliomyelitis), E. coli infection, foot-and-mouth disease, hepatitis, herpes zoster, influenza, measles, etc.

In some embodiments, the immune diseases and/or autoimmune diseases include asthma, diabetes, rheumatic diseases, AIDS, rejection of transplanted organs and tissues, rhinitis, chronic obstructive pulmonary disease, osteoporosis, ulcerative colitis, lupus erythematosus, allergies, rheumatoid arthritis, myasthenia gravis, Crohn's disease, psoriasis, etc.

In some embodiments, the inflammatory diseases are selected from the inflammatory diseases of the central nervous system (CNS), inflammatory rheumatic diseases, inflammatory diseases of the blood vessels, inflammatory diseases of the middle ear, inflammatory bowel diseases, inflammatory diseases of the skin, uveitis inflammatory diseases and inflammatory diseases of the throat.

As used herein, the term "subject" refers to an animal, especially a mammal, preferably a human.

As used herein, the term "effective dose" or "therapeutic effective dose" refers to the sufficient dose of non-toxic drugs or medicaments that can achieve the desired effect. In the embodiments of the present invention, when patients are treated according to the present invention, the dose of drugs depends on many factors, such as specific administration schemes, the type and severity of diseases or symptoms, and the uniqueness of the subjects or hosts to be treated (such as body weight). However, according to specific surrounding conditions, such as the used specific drugs, the route of administration, the treated symptoms and the treated subjects or hosts, the administration dose can be conventionally determined by the methods known in the art. Generally, the administration dose used for treatment of adults is typically 0.02-5000 mg/day, such as about 1-1500 mg/day. The required dose can be conveniently expressed as one dose, or divided doses of simultaneous administration (or in a short time) or at appropriate intervals, such as two, three, four or more divided doses per day. It can be understood by those skilled in the art that although the above dose range is given, the specific effective dose can be appropriately adjusted according to the conditions of patients and in combination with the diagnosis of doctors.

As used herein, the term "pharmaceutically acceptable salt" refers to a salt of a compound of the present invention that is pharmaceutically acceptable and has the pharmacological activity of a parent compound. Such salts include: acid addition salt formed with inorganic acids or organic acids, wherein the inorganic acids include nitric acid, phosphoric acid, carbonic acid, etc., and the organic acids include propionic acid, caproic acid, cyclopentanoic acid, glycolic acid, pyruvic acid, gluconic acid, stearic acid, muconic acid, etc.; or salts formed when acidic protons existing on the parent compound are substituted by metal ions, such as alkali metal ions or alkaline earth metal ions; or coordination compounds formed with organic alkalis, wherein the organic alkalis include ethanolamine, diethanolamine, triethanolamine, N- methylglucosamine, etc. The pharmaceutically acceptable salts of the present invention can be synthesized by conventional chemical methods from parent compounds containing acid radicals or basic groups. Generally, such salts are prepared by enabling these compounds in the form of free acids or alkalis to react with stoichiometric appropriate alkalis or acids in water or an organic solvent or a mixture of both. Generally, non-aqueous media such as ether, ethyl acetate, ethanol, isopropanol and acetonitrile are preferred. In addition to the form of salts, the compound provided by the present invention also has a prodrug form. The prodrug of the compound described herein can easily undergo chemical changes under physiological conditions so as to be converted into the compound of the present invention. In addition, the prodrug can be converted into the compound of the present invention by chemical or biochemical methods in the in-vivo environment.

As used herein, the term "stereoisomers" include conformational isomers and configurational isomers, wherein the configurational isomers typically include cis-trans isomers and optical isomers. The compound of the present invention can exist in the form of stereoisomers, and therefore covers all possible forms of stereoisomers, including but are not limited to, cis-trans isomers, tautomers, enantiomers, diastereomers, atropisomers, etc. The compound of the present invention can also exist in the form of any combination of the above-mentioned stereoisomers, or any mixture such as an equivalent mixture of mesomers, racemates and atropisomers, such as a single enantiomer, a single diastereomer or a mixture of the above, or a single atropisomer or a mixture thereof. When the compound of the present invention contains olefin double bonds, unless otherwise specified, the compound includes cis isomers and trans isomers, as well as any combination thereof. The atropisomer of the present invention is an axial or planar chiral stereoisomer generated based on the restriction of intramolecular rotation. The compound shown in Formula (A) has optical isomers derived from asymmetric carbon, axial asymmetry, etc. If necessary, the single isomer can be obtained by separation by the methods known in the art, such as crystallization or chiral chromatography.

As used herein, the term "hydrocarbyl" refers to a linear or branched saturated aliphatic hydrocarbyl group. The term "C₁₋₆ hydrocarbyl" refers to linear or branched hydrocarbyl with 1-6 carbon atoms, such as C₁₋₆ alkyl, C₂₋₆ alkenyl and C₂₋₆ alkynyl.

As used herein, the term "C₁₋₆ alkyl" refers to linear or branched alkyl with 1-6 carbon atoms, preferably C₁₋₄ alkyl, and more preferably C₁₋₃ alkyl. Specific examples include, but are not limited to, methyl, ethyl, n-propyl, isopropyl, n-butyl, isobutyl, tertiary butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and various branched isomers thereof.

As used herein, the term "alkenyl" refers to alkyl as defined above having one or more carbon-carbon double bonds at any site of the chain, and the term "C₂₋₆ alkenyl" refers to alkenyl with 2-6 carbon atoms and at least one (such as 1 to 2) carbon-carbon double bond, preferably C₂₋₄ alkenyl (that is, alkenyl with 2-4 carbon atoms and 1-2 carbon-carbon double bonds). Specific examples include, but are not limited to, vinyl, 1-propenyl, 2-propenyl, 1-, 2-or 3-butenyl, pentenyl, hexenyl, butadienyl, etc.

As used herein, the term "alkynyl" refers to alkyl as defined above having one or more carbon-carbon triple bonds at any site of the chain, and the term "C₂₋₆ alkynyl" refers to alkynyl with 2-6 carbon atoms and at least one (such as 1 to 2) carbon-carbon triple bonds, more preferably C₂₋₄ alkynyl (that is, alkynyl with 2-4 carbon atoms and 1-2 carbon-carbon triple bonds). Specific examples include, but are not limited to, ethynyl, 1-propynyl, 2-propynyl, 1-, 2- or 3-butynyl, etc.

As used herein, the term "halogen" refers to fluorine, chlorine, bromine or iodine.

As used herein, the term "halogenated" refers to fluoro, chloro, bromo or iodo.

As used herein, the term "halogenated hydrocarbyl" refers to hydrocarbyl in which one or more (such as 1, 2, 3, 4 or 5) hydrogen atoms are substituted by halogen, wherein the hydrocarbyl is defined as above. The term "halogenated C₁₋₆ alkyl" refers to halogenated alkyl with 1-6 carbon atoms, preferably halogenated C₁₋₄ alkyl, and more preferably halogenated C₁₋₃ alkyl. Specific examples include, but are not limited to, monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, trifluoroethyl, etc.

As used herein, the term "deuterated hydrocarbyl" refers to hydrocarbyl in which one or more (such as 1, 2, 3, 4 or 5) hydrogen atoms are substituted by deuterium atoms, wherein the hydrocarbyl is defined as above. The term "deuterated C₁₋₆ alkyl" refers to deuterated alkyl with 1-6 carbon atoms, preferably deuterated C₁₋₄ alkyl, and more preferably deuterated C₁₋₃ alkyl. Specific examples include, but are not limited to, monodeuteromethyl, dideuteromethyl, trideuteromethyl, etc.

As used herein, the terms "carbocyclyl" and "carbocycle" can be used interchangeably and refer to saturated or partially unsaturated monocyclic or polycyclic hydrocarbyl. The term "3-14 membered carbocyclyl" or "C₃₋₁₄ carbocyclyl" refers to saturated or partially unsaturated monocyclic or polycyclic hydrocarbyl with 3-14 carbon atoms, such as monocyclic carbocyclyl (such as 3-7 membered carbocyclyl or "C₃₋₇ carbocyclyl") or polycyclic carbocyclyl (such as spirocarbocyclyl, fused carbocyclyl and bridged carbocyclyl).

The terms "3-7 membered carbocyclyl" and "C₃₋₇ carbocyclyl" can be used interchangeably and both refer to saturated or partially unsaturated monocyclic hydrocarbyl with 3-7 ring carbon atoms, preferably 3-6 membered monocyclic carbocyclyl, preferably 4-6 membered monocyclic carbocyclyl, and more preferably 3, 4, 5 and 6 membered monocyclic carbocyclyl. Specific examples of the monocyclic carbocyclyl include, but are not limited to, cyclopropyl, cyclobutyl, cyclopentyl, cyclohexyl, etc.

The terms "spirocarbocyclyl" and "spirocarbocyclyl ring" refer to polycyclic hydrocarbyl formed by sharing one carbon atom (called spiro atom) between two or more monocyclic rings. According to the number of spiro atoms shared between rings, the spirocarbocyclyl is divided into mono-spirocarbocyclyl, bi-spirocarbocyclyl and multi-spirocarbocyclyl. The term "6-12 membered spirocarbocyclyl" refers to polycyclic hydrocarbyl with 6-12 ring carbon atoms, wherein the monocyclic ring sharing spiro atoms is 3-7 membered monocyclic carbocycle, preferably 7-12 membered spirocarbocyclyl, more preferably 7-12 membered mono-spirocarbocyclyl, and further preferably 7 membered (4 membered monocyclic carbocyclyl ring/4 membered monocyclic carbocyclyl ring), 8 membered (4 membered monocyclic carbocyclyl ring/5 membered monocyclic carbocyclyl ring), 9 membered (4 membered monocyclic carbocyclyl ring/6 membered monocyclic carbocyclyl ring, 5 membered monocyclic carbocyclyl ring/5 membered monocyclic carbocyclyl ring), 10 membered (5 membered monocyclic carbocyclyl ring/6 membered monocyclic carbocyclyl ring) or 11 (6 membered monocyclic carbocyclyl ring/6 membered monocyclic carbocyclyl ring) mono-spirocarbocyclyl. Specific examples of the spirocarbocyclyl include, but are not limited to, 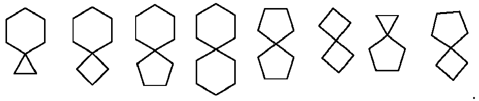 These spirocarbocyclyls can be connected to other parts of molecules by any ring atom.

The terms "fused carbocyclyl" and "fused carbocyclyl ring" refer to polycyclic hydrocarbyl formed by sharing a pair of adjacent carbon atoms between two or more monocyclic rings. According to the number of rings formed, the fused carbocyclyl can be divided into bicyclic, tricyclic, tetracyclic or polycyclic fused carbocyclyl. The term "5-12 membered fused carbocyclyl" refers to polycyclic hydrocarbyl with 5-12 ring carbon atoms, wherein the monocyclic ring sharing a pair of adjacent carbon atoms is 3-7 membered monocyclic carbocycle, preferably 7-12 membered fused carbocyclyl, more preferably 7-10 membered bi-fused carbocyclyl, and the most preferably 8 membered (5 membered monocyclic carbocyclyl ring and 5 membered monocyclic carbocyclyl ring fused), 9 membered (5 membered monocyclic carbocyclyl ring and 6 membered monocyclic carbocyclyl ring fused) or 10 membered (6 membered monocyclic carbocyclyl ring and 6 membered monocyclic carbocyclyl ring fused) bi-fused carbocyclyl. Specific examples of the fused carbocyclyl include, but are not limited to, 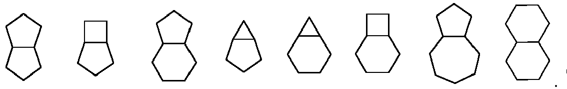 These fused carbocyclyls can be connected to other parts of molecules by any ring atom.

The terms "bridged carbocyclyl" and "bridged carbocyclyl ring" refer to polycyclic hydrocarbyl formed by sharing two carbon atoms that are not directly connected between two or more monocyclic rings. According to the number of rings formed, the bridged carbocyclyl can be divided into bicyclic, tricyclic, tetracyclic or polycyclic bridged carbocyclyl. The term "5-12 membered bridged carbocyclyl" refers to polycyclic hydrocarbyl with 5-12 ring carbon atoms, wherein any two rings share two carbon atoms that are not directly connected, preferably 7-12 membered bridged carbocyclyl. Specific examples of the bridged carbocyclyl include, but are not limited to, 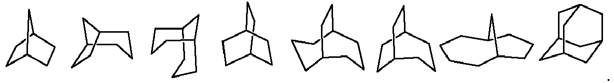 These bridged carbocyclyls can be connected to other parts of molecules by any ring atom.

The carbocyclyl ring can be fused on the aryl, heteroaryl or heterocyclyl ring, wherein the ring connected with a parent structure is the carbocyclyl ring, and non-limiting examples include indanyl, tetralyl, benzocycloheptyl, etc. In the present invention, the above various carbocyclyls can be optionally substituted. When the carbocyclyl is substituted, the substituent is preferably one or more substituent groups recited in the present application.

As used herein, the terms "heterocyclyl" and "heterocycle" can be used interchangeably and refer to saturated or partially unsaturated monocyclic or polycyclic hydrocarbyl, such as monocyclic heterocyclyl, spiroheterocyclyl, fused heterocyclyl and bridged heterocyclyl. The ring carbon atom of the heterocyclyl in the present invention can be optionally substituted by 1, 2 or 3 oxo substituents to form a cyclic ketone, cyclic lactone or cyclic lactam structure. The term "3-14 membered heterocyclyl" refers to saturated or partially unsaturated monocyclic or polycyclic hydrocarbyl with 3-14 ring atoms, wherein one or more (preferably 1, 2, 3 or 4) ring atoms are heteroatoms selected from nitrogen, oxygen or S(=O)_{m'} (wherein m' is an integer from 0 to 2), but do not include the cyclic part of -O-O-, -O-S- or -S-S-, and the remaining ring atoms are carbon. When the ring atom is a nitrogen atom, the nitrogen atom can be substituted or unsubstituted (that is, N or NR, R is hydrogen or other substituents defined herein). The 3-14 membered heterocyclyl described in the present invention includes monocyclic heterocyclyl (such as 3-7 membered heterocyclyl), spiroheterocyclyl, fused heterocyclyl and bridged heterocyclyl.

The terms "3-7 membered heterocyclyl" and "3-7 membered heterocyclyl ring" refer to saturated or partially unsaturated monocyclic hydrocarbyl with 3-7 ring atoms in which 1, 2 or 3 ring atoms are heteroatoms selected from nitrogen, oxygen or S(=O)_{m'} (wherein m' is an integer from 0 to 2), preferably 4-7 membered monocyclic heterocyclyl with 4-7 ring atoms in which 1 or 2 ring atoms are heteroatoms, and more preferably 5 or 6 membered monocyclic heterocyclyl with 5 or 6 ring atoms in which 1 or 2 ring atoms are heteroatoms. When the heteroatom is a nitrogen atom, the nitrogen atom can be substituted or unsubstituted (that is, N or NR, R is hydrogen or other substituents defined herein). When the heteroatom is a sulfur atom, the sulfur atom can be optionally oxidized (that is, S(=O)_{m'}, wherein m' is an integer from 0 to 2). The ring carbon atom of the monocyclic heterocyclyl can be optionally substituted by 1, 2 or 3 oxo substituents to form a cyclic ketone, cyclic lactone or cyclic lactam structure. Specific examples of the monocyclic heterocyclyl include, but are not limited to, ethylene imide, ethylene oxide, azetidine, azetidin-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahydro-2H-pyran-2-one, imidazolidine, tetrahydrofuran, tetrahydrothiophene, pyrrolidine, 1,3-dioxolan-2-one, oxazolidin-2-one, imidazolidin-2-one, piperidine, piperazine, piperazin-2-one, morpholine, morpholin-3-one, morpholin-2-one, thiomorpholin-3-one 1,1-dioxide, thiomorpholine, thiomorpholin-1,1-dioxide, tetrahydropyran, 1,2-dihydroazoheterocyclic butadiene, 1,2-dihydroxyheterocyclic butadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazine, hexahydropyrimidine, 1,4-dioxane, tetrahydropyrimidin-2(1H)-one, 1,4-dioxan-2-one, 5,6-dihydro-2H-pyran-2-one, 5,6-dihydropyrimidin-4(3H)-one, 3,4-dihydropyridin-2(1H)-one, 5,6-dihydropyridin-2(1H)-one, 5,6-dihydropyrimidin-4(1H)-one, pyrimidin-4(3H)-one, pyrimidin-4(1H)-one, 4,5-dihydro-1H-imidazole, 2,3-dihydro-1H-imidazole, 2,3-dihydroxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazol-2(3H)-one, 1,3-dihydro-2H-imidazol-2-one, furan-2,5-dione, 3,6- dihydropyridin-2(1H)-one, pyridin-2,6-(1H, 3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine, 1,2,3,4-tetrahydropyrimidine, etc.

2 connected ring atoms on the above monocyclic heterocyclyl ring, including C-C and N-C, can be optionally fused with the monocyclic carbocyclyl ring, monocyclic heterocyclyl ring, monoaryl ring, 5 or 6 membered monocyclic heteroaryl ring, and other carbocyclyls, heterocyclyls, aryls or heteroaryls defined in the present invention to form a fused polycyclic ring. The 2 connected ring atoms on the monocyclic heterocyclyl forming a fused ring with other rings are preferably C-C.

The terms "spiroheterocyclyl" and "spiroheterocyclyl ring" refer to polycyclic heterocyclyl formed by sharing one carbon atom (called spiro atom) between two or more saturated or partially unsaturated monocyclic rings, wherein one or more (such as 1, 2 or 3) ring atoms are heteroatoms selected from nitrogen, oxygen or S(=O)_{m'} (wherein m' is an integer from 0 to 2), and the remaining ring atoms are carbon. When the heteroatom is a nitrogen atom, the nitrogen atom can be substituted or unsubstituted (that is, N or NR, R is hydrogen or other substituents defined herein). Each monocyclic ring can contain one or more double bonds, but no ring has a completely conjugated pi-electron system. According to the number of spiro atoms shared between rings, the spiroheterocyclyl is divided into mono-spiroheterocyclyl, bi-spiroheterocyclyl and multi-spiroheterocyclyl. The term "7-14 membered spiroheterocyclyl" refers to spiroheterocyclyl with 7-14 ring atoms in which 1 or 2 ring atoms are heteroatoms, preferably 7 membered (4 membered monocyclic heterocyclyl ring/4 membered monocyclic heterocyclyl ring or 4 membered monocyclic heterocyclyl ring/4 membered monocyclic carbocyclyl or 4 membered monocyclic carbocyclyl ring/4 membered monocyclic heterocyclyl ring), 8 membered (4 membered monocyclic heterocyclyl ring/5 membered monocyclic heterocyclyl ring), 9 membered (4 membered monocyclic heterocyclyl ring/6 membered monocyclic heterocyclyl ring, 5 membered monocyclic heterocyclyl ring/5 membered monocyclic heterocyclyl ring), 10 membered (5 membered monocyclic heterocyclyl ring/6 membered monocyclic heterocyclyl ring) or 11 membered (6 membered monocyclic heterocyclyl ring/6 membered monocyclic heterocyclyl ring) mono-spiroheterocyclyl. Specific examples of the spiroheterocyclyl include, but are not limited to,

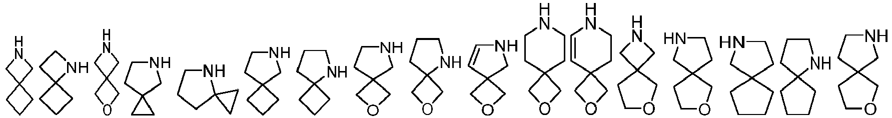

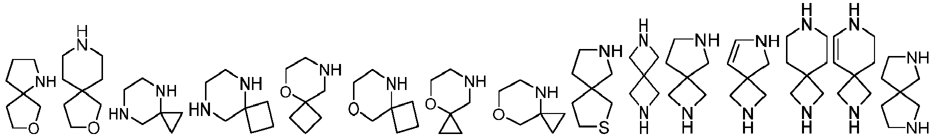

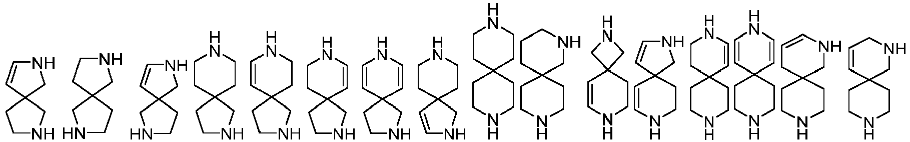

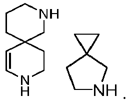

These spiroheterocyclyls can be connected to other parts of molecules by any appropriate ring atom.

The terms "fused heterocyclyl" and "fused heterocyclyl ring" refer to polycyclic heterocyclyl formed by sharing a pair of adjacent ring atoms between two or more saturated or partially unsaturated monocyclic rings, wherein one or more (such as 1, 2 or 3) ring atoms are heteroatoms selected from nitrogen, oxygen or S(=O)_{m'} (wherein m' is an integer from 0 to 2), and the remaining ring atoms are carbon. When the heteroatom is a nitrogen atom, the nitrogen atom can be substituted or unsubstituted (that is, N or NR, R is hydrogen or other substituents defined herein). Each monocyclic ring can contain one or more double bonds, but no ring has a completely conjugated pi-electron system. The shared adjacent ring atom pair can be C-C or N-C. According to the number of rings formed, the fused heterocyclyl can be divided into bicyclic, tricyclic, tetracyclic or polycyclic fused heterocyclyl. The term "5-14 membered fused heterocyclyl" refers to fused heterocyclyl with 5-14 ring atoms, preferably 6-10 membered fused heterocyclyl with 6-10 ring atoms in which 1 or 2 ring atoms are heteroatoms, more preferably 8-10 membered fused heterocyclyl with 8-10 ring atoms in which 1 or 2 ring atoms are heteroatoms, and the most preferably 8 membered (5 membered monocyclic heterocyclyl ring and 5 membered monocyclic heterocyclyl ring fused), 9 membered (5 membered monocyclic heterocyclyl ring and 6 membered monocyclic heterocyclyl ring fused) or 10 membered (6 membered monocyclic heterocyclyl ring and 6 membered monocyclic heterocyclyl ring fused) bicyclic fused heterocyclyl. Specific examples of the fused heterocyclyl include, but are not limited to,

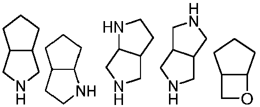

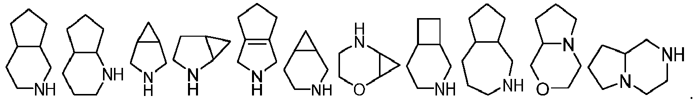

These fused heterocyclyls can be connected to other parts of molecules by any appropriate ring atom.

The terms "bridged heterocyclyl" and "bridged heterocyclyl ring" refer to polycyclic heterocyclyl formed by sharing two ring atoms that are not directly connected between two or more saturated or partially unsaturated monocyclic rings, wherein one or more (such as 1, 2 or 3) ring atoms are heteroatoms selected from nitrogen, oxygen or S(=O)_{m'} (wherein m' is an integer from 0 to 2), and the remaining ring atoms are carbon. According to the number of rings formed, the bridged carbocyclyl can be divided into bicyclic, tricyclic, tetracyclic or polycyclic bridged heterocyclyl. The term "5-14 membered bridged heterocyclyl" refers to saturated or partially unsaturated polycyclic heterocyclyl with 5-14 ring atoms, wherein any two rings share two ring atoms that are not directly connected, each monocyclic ring can contain one or more double bonds, but no ring has a completely conjugated pi-electron system, preferably 5-10 membered bridged heterocyclyl. Specific examples of the bridged heterocyclyl include, but are not limited to,

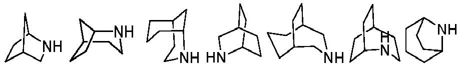

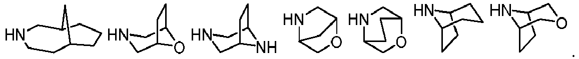

These bridged heterocyclyls can be connected to other parts of molecules by any appropriate ring atom.

In the present invention, the above various heterocyclyls can be optionally substituted. When the heterocyclyl is substituted, the substituent is preferably one or more substituent groups recited in the present application.

As used herein, the terms "aryl", "aryl ring" and "aromatic ring" can be used interchangeably and refer to an all-carbon monocyclic, all-carbon non-fused polycyclic (rings are connected by covalent bonds and are not fused) or all-carbon fused polycyclic (that is, rings sharing adjacent carbon atom pairs) group, and at least one ring in the group is aromatic, that is, a conjugated pi-electron system is provided. The term "C₅₋₁₂ aryl" refers to aryl with 5-12 ring atoms, preferably C₆₋₁₀ aryl. The C₅₋₁₂ aryl in the present invention includes monocyclic aryl (such as phenyl), non-fused polycyclic aryl (such as biphenyl) and aromatic fused polycyclic rings.

In some embodiments, when the C₅₋₁₂ aryl is an aromatic fused polycyclic ring, the aromatic fused polycyclic ring can be a polycyclic group formed by fusing a monoaryl ring with one or more monoaryl rings, and non-limiting examples include naphthyl, anthryl, etc.

In some embodiments, when the C₅₋₁₂ aryl is an aromatic fused polycyclic ring, the aromatic fused polycyclic ring can also be a polycyclic group formed by fusing a monoaryl ring (such as phenyl) with one or more non-aromatic rings, wherein the ring connected to a parent structure is an aromatic ring or a non-aromatic ring. The non-aromatic rings include, but are not limited to, 3-7 membered monocyclic heterocyclyl rings (preferably a 5 or 6 membered monocyclic heterocyclyl ring, the ring carbon atom of the monocyclic heterocyclyl ring can be substituted by 1-2 oxo substituents to form a cyclic lactam or cyclic lactone structure), and 3-7 membered monocyclic carbocyclyl rings (preferably a 5 or 6 membered monocyclic carbocyclyl ring, the ring carbon atom of the monocyclic carbocyclyl ring can be substituted by 1-2 oxo substituents to form a cyclic ketone structure). The above polycyclic group formed by fusing a monoaryl ring with one or more non-aromatic rings can be connected to other groups or parent structures by nitrogen atoms or carbon atoms, and the ring connected to the parent structure is a monoaryl ring or a non-aromatic ring.

As used herein, the terms "heteroaryl", "heteroaryl ring" and "heteroaromatic ring" can be used interchangeably and refer to monocyclic or fused polycyclic (that is, rings sharing adjacent ring atom pairs, and shared adjacent ring atom pairs can be C-C or N-C) groups in which ring atoms are substituted by at least one heteroatom independently selected from nitrogen, oxygen or sulfur, wherein nitrogen and sulfur atoms can be optionally oxidized, and nitrogen atoms can be optionally quaternized. The heteroaryl has shared 6, 10 or 14 pi-electrons, and at least one ring in the group is aromatic. The term "5-12 membered heteroaryl" refers to heteroaryl with 5-12 ring atoms in which 1, 2, 3 or 4 ring atoms are heteroatoms selected from nitrogen, oxygen or S(=O)_{m'} (wherein m' is an integer from 0 to 2), preferably 5-10 membered heteroaryl with 5-10 ring atoms in which 1, 2, 3 or 4 ring atoms are heteroatoms. The heteroaryl herein can be monocyclic heteroaryl (such as 5 or 6 membered monocyclic heteroaryl), fused bicyclic heteroaryl (such as 8-10 membered bicyclic heteroaryl) or fused tricyclic heteroaryl.

As used herein, the term "5 or 6 membered monocyclic heteroaryl" or "5 or 6 membered heteroaryl" refers to monocyclic heteroaryl with 5 or 6 ring atoms in which 1, 2 or 3 ring atoms are heteroatoms selected from nitrogen, oxygen or S(=O)_{m'} (wherein m' is an integer from 0 to 2). Specific examples of the monocyclic heteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine, etc.

As used herein, the term "8-10 membered bicyclic heteroaryl" refers to fused bicyclic heteroaryl with 8-10 ring atoms in which 1, 2, 3, 4 or 5 ring atoms are heteroatoms selected from nitrogen, oxygen or S(=O)_{m'} (wherein m' is an integer from 0 to 2). The fused bicyclic heteroaryl can be a bicyclic group formed by fusing a monoaryl ring (such as phenyl) with a monocyclic heteroaryl ring (preferably a 5 or 6 membered monocyclic heteroaryl ring), or a bicyclic group formed by fusing a monocyclic heteroaryl ring (preferably a 5 or 6 membered monocyclic heteroaryl ring) with a monocyclic heteroaryl ring (preferably a 5 or 6 membered monocyclic heteroaryl ring).

Any 2 connected ring atoms on the above monocyclic heterocyclyl ring, including C-C, N-C and N-N, can be fused with the monocyclic carbocyclyl ring, monocyclic heterocyclyl ring, monoaryl ring, 5 or 6 membered monocyclic heteroaryl ring, and other carbocyclyls, heterocyclyls, aryls or heteroaryls defined in the present invention to form a fused polycyclic ring. Non-limiting examples of the 8-10 membered bicyclic heteroaryl include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyridino[3,2-d]pyrimidine, pyridino[2,3-d]pyrimidine, pyridino[3,4-d]pyrimidine, pyridino[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine, etc.

The above monocyclic heteroaryl, or the bicyclic heteroaryl formed by fusing a benzene ring with a monocyclic heteroaryl ring, or the bicyclic heteroaryl formed by fusing a monocyclic heteroaryl ring with a monocyclic heteroaryl ring can be connected to other groups or parent structures by nitrogen atoms or carbon atoms. Specific examples of the bicyclic heteroaryl include, but are not limited to, 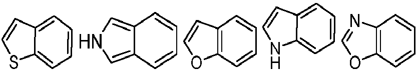 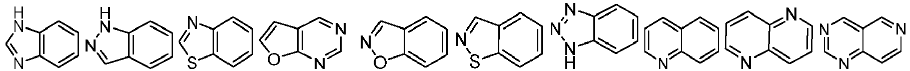 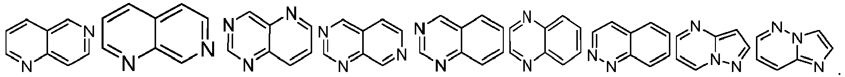 These groups can be connected to other parts of molecules by any appropriate ring atom. The ring connected to the parent structure can be a monocyclic heteroaryl ring or a benzene ring.

In some embodiments, the fused bicyclic heteroaryl or fused tricyclic heteroaryl can be a polycyclic group formed by fusing a monocyclic heteroaryl ring (preferably a 5 or 6 membered monocyclic heteroaryl ring) with one or more non-aromatic rings, wherein the ring connected to the parent structure is a monocyclic heteroaryl ring or a non-aromatic ring. The non-aromatic rings include, but are not limited to, 3-6 membered monocyclic heterocyclyl rings (preferably a 5 or 6 membered monocyclic heterocyclyl ring, the ring carbon atom of the monocyclic heterocyclyl ring can be substituted by 1-2 oxo substituents to form a cyclic lactam or cyclic lactone structure), 3-6 membered monocyclic carbocyclyl rings (preferably a 5 or 6 membered monocyclic carbocyclyl ring, the ring carbon atom of the monocyclic carbocyclyl ring can be substituted by 1-2 oxo substituents to form a cyclic ketone structure), etc. The above polycyclic group formed by fusing a monocyclic heteroaryl ring with one or more non-aromatic rings can be connected to other groups or parent structures by nitrogen atoms or carbon atoms, and the ring connected to the parent structure is a monocyclic heteroaryl ring or a non-aromatic ring. For example, the term "8-10 membered heteroaryl heterocyclyl" or "8-10 membered heteroaryl heterocyclyl ring" refers to 8-10 membered fused bicyclic heteroaryl formed by fusing a 5 or 6 membered monocyclic heteroaryl ring with a 5 or 6 membered monocyclic heterocyclyl ring, that is, two adjacent substituent groups on the 5 or 6 membered monocyclic heteroaryl and the connected ring atoms form a fused 5 or 6 membered monocyclic heterocyclyl ring, wherein the 5 or 6 membered monocyclic heterocyclyl ring is defined as above. For another example, as used herein, the term "8-10 membered heteroaryl carbocyclyl" or "8-10 membered heteroaryl carbocyclyl ring" refers to 8-10 membered fused bicyclic heteroaryl formed by fusing a 5 or 6 membered monocyclic heteroaryl ring with a 5 or 6 membered monocyclic carbocyclyl ring, that is, two adjacent substituent groups on the 5 or 6 membered monocyclic heteroaryl and the connected ring atoms form a fused 5 or 6 membered monocyclic carbocyclyl ring, wherein the 5 or 6 membered monocyclic carbocyclyl ring is defined as above. Non-limiting examples of the "8-10 membered heteroaryl heterocyclyl" or the "8-10 membered heteroaryl carbocyclyl" include: 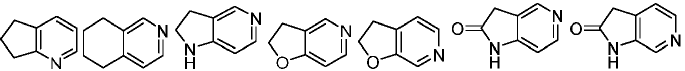 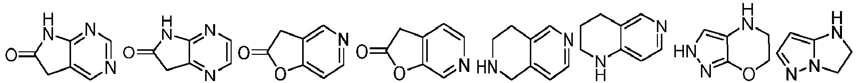 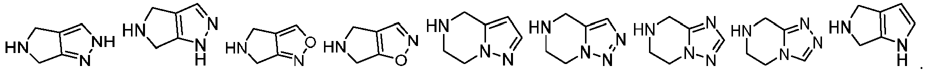 The above groups can be connected to other parts of molecules by any appropriate ring atom.

In the present invention, the above various heteroaryls can be substituted or unsubstituted. When the heteroaryl is substituted, the substituent is preferably one or more substituent groups recited in the present application.

As used herein, the term "hydroxyl" refers to -OH.

As used herein, the term "amino" refers to -NH₂.

As used herein, the term "cyano" refers to -CN.

As used herein, the term "nitro" refers to -NO₂.

As used herein, the term "benzyl" refers to -CH₂-benzene.

As used herein, the term "oxo" refers to =O.

As used herein, the term "alkylene" represents a divalent bound form of alkyl. For example, "C₀₋₆ alkylene" represents alkylene with 0-6 carbon atoms. For example, "C₁ alkylene" represents alkylene with 1 carbon atom, such as -CH₂-. For example, "C₂ alkylene" represents alkylene with 2 carbon atoms, such as -CH₂CH₂-, and so on.

As used herein, the term "alkenylene" represents a divalent bound form of alkenyl. For example, "C₂₋₆ alkenylene" represents alkenylene with 2-6 carbon atoms. For example, "C₂ alkenylene" represents alkenylene with 2 carbon atoms, such as -CH=CH-, and so on.

As used herein, the term "substitution" refers to the substitution of any one or more hydrogen atoms on a specific atom by a substituent, which can include heavy hydrogen and hydrogen variants, as long as the valence state of the specific atom is normal and the substituted compound is stable. When the substituent is an oxo substituent (that is, =O), it means that 2 hydrogen atoms are substituted. The substitution of the oxo substituent cannot occur on aryl. The term "optionally substitute" or "optionally substituted" refers to substituted or unsubstituted. Unless otherwise specified, the type and number of substituents can be arbitrary on the basis of chemical realization.

When any variable (such as R) appears more than once in the composition or structure of a compound, the definition thereof in each case is independent. Therefore, for example, if a group is substituted by 0-2 R, the group can be optionally substituted by at most two R, and R in each case has independent options. In addition, the combination of substituents and/or variants thereof is allowed only if such combination can produce a stable compound.

As used herein, the term "none" means that two connected groups are directly connected, such as 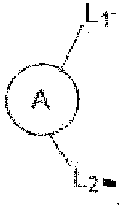 When the ring A is none, L₁ and L₂ are directly connected, that is, "L₁-L₂".

When the atom through which the listed substituent is connected to a substituted group is not specified, the substituent can be bonded by any atom. For example, pyridinyl as a substituent can be connected to a substituted group by any carbon atom on a pyridinyl ring.

When the connecting direction of the listed connecting group is not specified, the connecting direction is arbitrary. For example, in 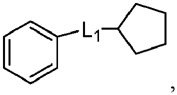 L is selected from -C(O)-NH-, at that time, -C(O)-NH- can connect the benzene ring and cyclopentane in the same direction as the reading order from left to right to form 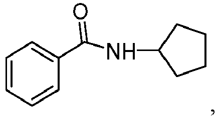 and can also connect the benzene ring and cyclopentane in the direction opposite to the reading order from left to right to form 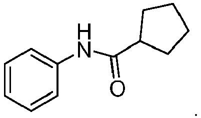 The combination of the connecting groups, substituents and/or variants thereof is allowed only if such combination can produce a stable compound.

The compound shown in Formula (A) in the present invention can be prepared by using the synthesis methods known in the art or by combining the methods known in the art with the methods recited in the present invention. The solvents, temperatures and other reaction conditions given in the present invention are exemplary and can be changed according to the methods well known in the art. The compounds in the examples recited in the present invention can be synthesized according to the specific structures of the compounds with appropriate starting materials by the methods recited in the examples, or synthesized by the methods similar to those recited in the examples. The starting materials for synthesizing the compounds in the examples of the present invention can be prepared by known synthesis methods or similar methods recited in the literature, or obtained from commercial sources. The compounds can be further separated into stereoisomers thereof by the methods well known in the art, such as crystallization and chromatography, according to needs, and separation conditions are easily obtained by those skilled in the art by conventional means or limited tests. As a further explanation, the compound shown in Formula (A) in the present invention can be synthesized by the following methods, wherein the solvents, temperatures and other reaction conditions in each step can be the same with or similar to those recited in the following examples, or the reaction conditions known in the art can be used.

### DETAILED DESCRIPTION OF THE INVENTION

The compounds of the present invention can be prepared by various synthesis methods well known to those skilled in the art, including the specific implementations listed below, the implementations formed by combining them with other chemical synthesis methods, and the equivalent replacement methods well known to those skilled in the art. Preferred implementations include, but are not limited to, examples of the present invention.

The present invention is described in detail below through examples, but it does not mean any adverse limitation to the present invention. The present invention has been described herein in detail, and its specific examples have also been disclosed. For those skilled in the art, it will be obvious to make various changes and improvements to the specific implementations of the present invention without departing from the spirit and scope of the present invention. If the specific conditions are not indicated in the examples, the general conditions or the conditions recommended by the manufacturer shall be followed. Reagents or instruments used without indicating the manufacturer are conventional products that are commercially available.

### Example 1 Synthesis of compound Z1

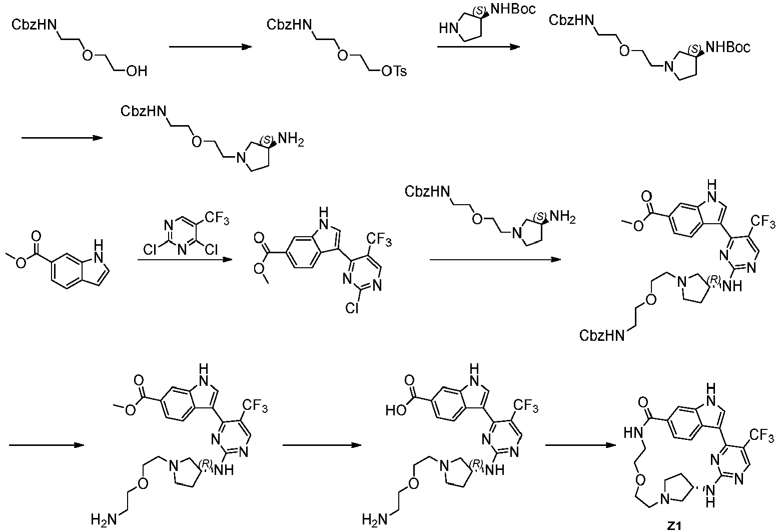

Step 1: [2-(2-hydroxyethoxy)ethyl]benzyl carbamate (5.0 g, 20.92 mmol) and dichloromethane (25 mL) were added into a three-neck flask, stirring was carried out, and triethylamine (6.34 g, 62.76 mmol) and 4-dimethylaminopyridine (510 mg, 4.18 mmol) were added. The mixture was cooled to 0°C, and paratoluensulfonyl chloride (3.97 g, 20.92 mmol) was slowly added. Stirring was carried out at room temperature to react for 2 h, and the complete reaction of the raw materials was monitored by LC-MS. The mixture was poured into saturated sodium bicarbonate (100 mL), and extracting was carried out with dichloromethane (50 mL ×3). Organic phases were combined, dried over anhydrous sodium sulfate, and concentrated. The crude product was purified with a silica gel column (ethyl acetate/petroleum ether: 20-50%) to obtain an oily product 2-(2-(((benzyloxy)carbonyl)amino)ethoxy)ethyl 4-methylbenzenesulfonate (3.84 g, and yield of 46.7%). ¹H NMR (400 MHz, CDCl₃) δ 7.79 (d, *J* = 8.4 Hz, 2H), 7.42 - 7.28 (m, 7H), 5.10 (s, 3H), 4.35 - 4.07 (m, 2H), 3.68 - 3.58 (m, 2H), 3.47 (t, *J* = 5.0 Hz, 2H), 3.32 (dd, *J* = 10.4, 5.2 Hz, 2H), 2.41 (s, 3H).

Step 2: Potassium iodide (0.056 mL, 0.508 mmol) and potassium carbonate (702.54 mg, 5.083 mmol) solids were added into a solution of 2-(2-(((benzyloxy)carbonyl)amino)ethoxy)ethyl 4-methylbenzenesulfonate (1,000 mg, 2.542 mmol) and (S)-tert-butyl pyrrolidin-3-yl carbamate (394 mg, 2.12 mmol) in N,N-dimethylformamide (15.0 mL) at room temperature. The reaction mixture was reacted at 70 °C for 16 h while stirring. Pouring into ice water (100 mL) was carried out after reaction, and extracting was carried out with ethyl acetate for multiple times (50 mL ×3). Organic phases were combined, washed with a saturated brine, dried over anhydrous sodium sulfate, and concentrated. The crude product was purified with silica gel column chromatography (ethyl acetate/petroleum ether: 10-100%) to obtain a yellow oily product (S)-(2-(2-(3-(((tert-butoxycarbonyl)amino)pyrrolidin-1-yl)ethoxy)ethyl)benzyl carbamate (630 mg, 1.314 mmol, and yield of 51.70%). LCMS m/z (ESI): [M+H]⁺=408.2.

Step 3: Trifluoroacetic acid (4.0 mL, 53.85 mmol) was added into a solution of (S)-(2-(2-(3-((tert-butoxycarbonyl)amino)pyrrolidin-1-yl)ethoxy)ethyl)benzyl carbamate (730 mg, 1.791 mmol) in dichloromethane (6.0 mL) at room temperature. The reaction mixture was stirred at room temperature for 3 h until realizing complete reaction. The reaction solution was concentrated, and ethyl acetate (50 mL ×3) and saturated sodium bicarbonate (50 mL ×3) were added for extraction. Organic phases were combined, washed with a saturated brine, dried over anhydrous sodium sulfate, and concentrated. The crude product was purified with silica gel column chromatography (methanol/dichloromethane: 0-10%) to obtain a yellow oily product compound (S)-(2-(2-(3-aminopyrrolidin-1-yl)ethoxy)ethyl)benzyl carbamate (520 mg, 1.66 mmol, and yield of 92.55%). LCMS m/z (ESI): [M+H]⁺=308.2.

Step 4: Aluminum trichloride (12.9 g, 85.62 mmol) was added into a solution of 2,4-dichloro-5-(trifluoromethyl)pyrimidine (7.44 g, 34.29 mmol) in 1,2-dichloroethane (30.0 mL) at room temperature. Heating was carried out to 90°C and reacting was carried out for 30 min. A solution of methyl indole-6-carboxylate (5.0 g, 28.54 mmol) in 1,2-dichloroethane (30.0 mL) was slowly dropwise added into the reaction solution. The reaction mixture continued to react at 90 °C for 18 h. The reaction mixture was cooled and poured into ice water (200 mL), and extracting was carried out with ethyl acetate (100 mL ×3). Organic phases were combined, washed with a saturated brine, dried over anhydrous sodium sulfate, and concentrated. The crude product was purified with silica gel column chromatography (methanol/dichloromethane: 0-10%) to obtain a yellow solid methyl 3-(2-chloro-5-(trifluoromethyl)pyrimidin-4-yl)-1H-indole-6-carboxylate (2.5 g, 6.26 mmol, and yield of 21.92%). ¹H NMR (400 MHz, DMSO-d₆) δ 12.50 (s, 1H), 9.08 (d, *J* = 3.4 Hz, 1H), 8.39 (d, *J* = 8.4 Hz, 1H), 8.28 (d, *J* = 2.6 Hz, 1H), 8.20 (t, *J* = 6.4 Hz, 1H), 7.89 - 7.77 (m, 1H), 3.89 (s, 3H).

Step 5: N,N-diisopropylethylamine (1.56 mL, 9.4 mmol) was added into a solution of the compound methyl 3-(2-chloro-5-(trifluoromethyl)pyrimidin-4-yl)-1H-indole-6-carboxylate (450 mg, 1.27 mmol) and the compound (S)-(2-(2-(3-aminopyrrolidin-1-yl)ethoxy)ethyl)benzyl carbamate (583 mg, 1.9 mmol) in dioxane (6 mL) under nitrogen protection at room temperature. The reaction mixture was heated to 120°C and reacting was carried out for 4 h. The reaction mixture was cooled and poured into ice water (50 mL), and extracting was carried out with ethyl acetate (50 mL ×3). Organic phases were combined, washed with a saturated brine (100 mL), dried over anhydrous sodium sulfate, and concentrated. The crude product was purified with silica gel column (methanol/dichloromethane: 0-10%) to obtain a yellow oily product methyl (R)-3-(2-((1-(2-(2-(((benzyloxy)carbonyl)amino)ethoxy)ethyl)pyrrolidin-3-yl)amino)-5-(trifluoromethyl)pyrimidin-4-yl)-1H-indole-6-carboxylate (286 mg, 0.32 mmol, and yield of 25.25%). LCMS m/z (ESI): [M+H]⁺=627.3.

Step 6: Palladium 10% on carbon (100 mg, 0.945 mmol) was added into a solution of the compound methyl (R)-3-(2-((1-(2-(2-(((benzyloxy)carbonyl)amino)ethoxy)ethyl)pyrrolidin-3-yl) amino)-5-(trifluoromethyl)pyrimidin-4-yl)-1H-indole-6-carboxylate (660 mg, 1.05 mmol) in methanol (50 mL) at room temperature. The reaction mixture was stirred in a hydrogen atmosphere to react for about 4 h. Filtering and concentrating were carried out, and a crude product was purified with a silica gel column (methanol/dichloromethane: 0-20%) to obtain a grey black oily product methyl (R)-3-(2-((1-(2-(2-aminoethoxy)ethyl)pyrrolidin-3-yl)amino)-5-(trifluoromethyl)pyrimidin-4-yl)-1H-indole-6- carboxylate (460 mg, 0.65 mmol, yield of 62.08%). LCMS m/z (ESI): [M+H]⁺=493.0.

Step 7: Lithium hydroxide (197.3 mg, 4.7 mmol) was added into a solution of the compound methyl (R)-3-(2-((1-(2-(2-aminoethoxy)ethyl)pyrrolidin-3-yl)amino)-5-(trifluoromethyl)pyrimidin-4-yl)-1H-indole-6-carboxylate (386 mg, 0.76 mmol) in methanol/water (10/5 mL) under nitrogen protection at room temperature. The reaction mixture was heated to 70 °C and reacting was carried out for 3 h. Cooling and concentrating were carried out. Reversed-phase column purification was carried out on a crude product (acetonitrile/water: 0-70%) to obtain a white solid product (R)-3-(2-((1-(2-(2-aminoethoxy)ethyl)pyrrolidin-3-yl)amino)-5-(trifluoromethyl)pyrimidin-4-yl)-1H-indole-6-carboxylic acid (106 mg, 0.19 mmol, and yield of 24.03%). LCMS m/z (ESI):
[M+H]⁺=479.2.

Step 8: N,N-diisopropylethylamine (0.11 mL, 0.66 mmol) was added into a solution of N,N,N',N'-tetramethyl-O-(7-azabenzotriazol-1-yl)uronium hexafluorophosphate (126 mg, 0.33 mmol) in N,N-dimethylformamide (15 mL) under nitrogen protection at room temperature. A solution of the compound (R)-3-(2-((1-(2-(2-aminoethoxy)ethyl)pyrrolidin-3-yl)amino)-5-(trifluoromethyl)pyrimidin-4-yl)-1H-indole-6-carboxylic acid (106 mg, 0.22 mmol) in N,N-dimethylformamide (1 mL) was added while stirring the mixture. Reactants were reacted at room temperature for 30 min. The reactants were poured into ice water (50 mL), and extracting was carried out with ethyl acetate (50 mL ×3). Organic phases were combined, washed with a saturated brine, dried, and concentrated. A crude product was purified by preparation (chromatographic column: Waters Xbridge C18 10 um OBD 19X250 mm; mobile phase: water/acetonitrile (0.1% ammonium bicarbonate); flow rate: 20 mL/min; detection wavelength: 254 nm/214 nm; and column temperature: room temperature), thereby obtaining a product (4³S)-2⁵-(trifluoromethyl)-1¹H-7-oxa-3,10-diaza-1(3,6)-indola-2(4,2)-pyrimidina-4(3,1)-pyrrolidinacycloundecaphan-11-one (Z1, 2.3 mg, yield of 2.25%). LCMS m/z (ESI): [M+H]⁺=461.2. ¹H NMR (400 MHz, DMSO-d₆) δ 11.91 (s, 1H), 8.58 (s, 1H), 8.22 (d, *J* = 8.6 Hz, 1H), 8.06 (s, 1H), 7.87 (s, 1H), 7.61 (s, 2H), 7.39 (s, 1H), 4.08 (s, 1H), 3.57 (s, 3H), 3.25 (s, 3H), 3.08 (s, 2H), 2.71 (d, *J* = 82.0 Hz, 3H), 2.00 (d, *J* = 90.2 Hz, 3H). ¹⁹F NMR (400 MHz, DMSO) δ -56.63.

### Example 2 Synthesis of compound Z2

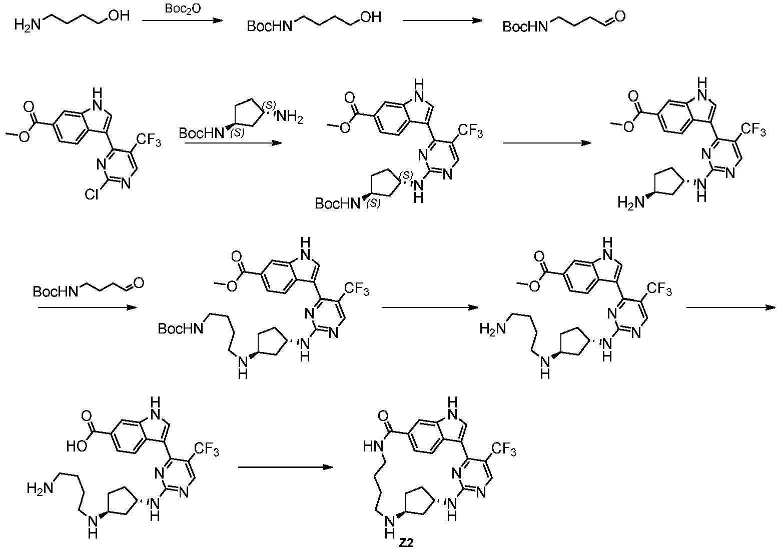

Step 1: Di-tert-butyl dicarbonate (3.67 g, 16.856 mmol) was added into a solution of 4-amino-1-butanol (3.0 g, 33.7 mmol) in ethanol (30 mL) at room temperature. The reaction mixture was stirred at room temperature to react for 4 h. The reaction solution was concentrated. The crude product was extracted with ethyl acetate (100 mL×3) and sodium bicarbonate (150 mL). Organic phases were combined, dried over anhydrous sodium sulfate, and concentrated. The crude product was purified with silica gel column chromatography (ethyl acetate/petroleum ether: 0-100%) to obtain a colorless oily product tert-butyl (4-hydroxybutyl)carbamate (5.8 g, 30.69 mmol, and yield of 91%).

Step 2: Oxalyl chloride (13 mL, 23 mmol, 2 M dichloromethane) was dropwise added into a reaction solution of dimethyl sulfoxide (3.7 mL) in dichloromethane (50 mL) at -70 °C under nitrogen protection, and stirring continued for 5 min after dropwise adding. A solution of tert-butyl (4-hydroxybutyl)carbamate (4.1 g, 21.6 mmol) in dichloromethane (35.0 mL) was dropwise added. The reaction mixture was gradually heated to 0°C and was stirred for 25 min. The reaction mixture was poured into ice water after reaction, and extracting was carried out with ethyl acetate (50 mL ×3). Organic phases were combined, washed with a saturated brine (150 mL), dried over anhydrous sodium sulfate, and concentrated. The crude product was purified with silica gel column (ethyl acetate/petroleum ether: 0-50%) to obtain a yellow oily product tert-butyl (4-oxobutyl)carbamate (4.0 g, 24.59 mmol, the yield of 84.55%).

Step 3: N,N-diisopropylethylamine (2.78 ml, 16.78 mmol) was added into a solution of a compound methyl 3-(2-chloro-5-(trifluoromethyl)pyrimidin-4-yl)-1H-indole-6-carboxylate (2.0 g, 5.62 mmol) and a compound tert-butyl ((1S,3S)-3-aminocyclopentyl) carbamate (1.02 g, 5.06 mmol) in N-methylpyrrolidone (30 mL) under nitrogen protection at room temperature. The reaction mixture was heated to 140°C and reacting was carried out for 0.5 h. The reaction mixture was cooled and poured into ice water, and was extracted with ethyl acetate (50 mL ×3). Organic phases were combined, washed with a saturated brine (150 mL), dried over anhydrous sodium sulfate, and concentrated. The crude product was purified with silica gel column (methanol/dichloromethane: 0-10%) to obtain a gray solid methyl 3-(2-(((1S,3 S)-3-((tert-butoxycarbonyl)amino)cyclopentyl)amino)-5-(trifluoromethyl)pyrimidin-4-yl)-1H-indole-6-carboxylate (2.0 g, 3.84 mmol, and yield of 68.47%). LCMS m/z (ESI): [M+H]⁺=519.2. ¹H NMR (400 MHz, DMSO-d₆) δ 12.11 (s, 1H), 8.64 - 8.51 (m, 1H), 8.48 - 8.31 (m, 1H), 8.15 (d, *J* = 5.8 Hz, 1H), 8.01 (d, *J* = 21.1 Hz, 2H), 7.88 -7.69 (m, 1H), 7.06 - 6.83 (m, 1H), 4.44 (s, 1H), 4.07 - 3.81 (m, 4H), 2.16 - 1.77 (m, 4H), 1.51 (d, *J* = 33.4 Hz, 2H), 1.41 - 1.34 (m, 9H).

Step 4: Trifluoroacetic acid (1.0 mL, 13.56 mmol) was added into a solution of the compound methyl 3-(2-(((1S,3 S)-3-((tert-butoxycarbonyl)amino)cyclopentyl)amino)-5-(trifluoromethyl)pyrimidin-4-yl)-1H-indole-6-carboxylate (520 mg, 1.0 mmol) in dichloromethane (10 mL) at room temperature. The reaction mixture was stirred at room temperature for 1 h until realizing complete reaction. The reaction solution was concentrated, and ethyl acetate (50 mL ×3) and saturated sodium bicarbonate (100 mL) were added for extraction. The organic phases was combined, washed with a saturated brine (100 mL), dried over anhydrous sodium sulfate, and concentrated to obtain a yellow oily crude product methyl 3-(2-(((1S,3S)-3-aminocyclopentyl)amino)-5-(trifluoromethyl)pyrimidin-4-yl)-1H-indole-6-carboxylate (520 mg, 1.66 mmol, yield of 92.55%), and the product was directly used in the next reaction without purification. LCMS m/z (ESI): [M+H]⁺=419.1.

Step 5: The compound tert-butyl (4-oxobutyl)carbamate (192.86 mg, 1.03 mmol) was added into a solution of the compound methyl 3-(2-(((1S,3S)-3-aminocyclopentyl)amino)-5-(trifluoromethyl)pyrimidin-4-yl)-1H-indole-6-carboxylate (480 mg, 1.0 mmol) in tetrahydrofuran (5 mL) at room temperature. The mixture was stirred at room temperature for 30 min, and then sodium cyanoborohydride (143.84 mg, 2.29 mmol) was added. The reaction mixture was stirred at room temperature for 2 h until realizing complete reaction. The reaction solution was concentrated, and ethyl acetate (50 mL ×3) and saturated sodium bicarbonate (100 mL) were added for extraction. Organic phases were combined, washed with a saturated brine (100 mL), dried over anhydrous sodium sulfate, and concentrated. Column chromatography purification was carried out on a crude product (methanol/dichloromethane: 0-10%) to obtain a yellow solid compound methyl 3-(2-(((1S,3S)-3-((4-((t-butyloxycarbonyl)amino)butyl)amino)cyclopentyl)amino)-5-(trifluoromethyl)pyrimidin-4-yl)-1H-indole-6-carboxylate (356 mg, 0.6 mmol, and the yield of 52.66%). LCMS m/z (ESI): [M+H]⁺ =590.2.

Step 6: Trifluoroacetic acid (0.5 mL, 6.75 mmol) was added into a solution of the compound 3-(2-(((1S,3S)-3-((4-((t-butyloxycarbonyl)amino)butyl)amino)cyclopentyl)amino)-5-(trifluoromethyl)pyrimidin-4-yl)-1H-indole-6-carboxylate (356 mg, 0.76 mmol) in dichloromethane (5 mL) at room temperature. The reaction mixture was stirred at room temperature for 1 h until realizing complete reaction. The reaction solution was concentrated, and ethyl acetate (50 mL ×3) and saturated sodium bicarbonate (100 mL) were added for extraction. The organic phases were combined, washed with a saturated brine (100 mL), dried over anhydrous sodium sulfate, and concentrated to obtain a yellow oily crude compound methyl 3-(2-(((1S,3S)-3-((4-aminobutyl)amino)cyclopentyl)amino)-5-(trifluoromethyl)pyrimidin-4-yl)-1H-indole-6-carboxylate (387 mg, 0.47 mmol, yield of 78.54%), and the product was directly used in the next reaction without purification. LCMS m/z (ESI): [M+H]⁺=490.2.

Step 7: Lithium hydroxide (98.54 mg, 2.35 mmol) was added into a solution of the methyl 3-(2-(((1S,3S)-3-((4-aminobutyl)amino)cyclopentyl)amino)-5-(trifluoromethyl)pyrimidin-4-yl)-1H-indole-6-carboxylate (384 mg, 0.78 mmol) in tetrahydrofuran/water (10/5 mL) under nitrogen protection at room temperature. The reaction mixture was stirred to react for 0.5 h. Cooling and concentrating were carried out. Reversed-phase column purification was carried out on a crude product (acetonitrile/water: 0-70%) to obtain a white solid product 3-(2-(((1S,3S)-3-((4-aminobutyl)amino)cyclopentyl)amino)-5-(trifluoromethyl)pyrimidin-4-yl)-1H-indole-6-carboxylic acid (208 mg, 0.44 mmol, yield of 55.76%). LCMS m/z (ESI): [M+H]⁺=476.2.

Step 8: N,N-diisopropylethylamine (0.139 mL, 0.84 mmol) was added into a solution of N,N,N',N'-tetramethyl-O-(7-azabenzotriazol-1-yl)uronium hexafluorophosphate (239 mg, 0.63 mmol) in N,N-dimethylformamide (5 mL) under nitrogen protection at room temperature. A solution of the compound 3-(2-(((1S,3S)-3-((4-aminobutyl)amino)cyclopentyl)amino)-5-(trifluoromethyl)pyrimidin-4-yl)-1H-indole-6-carboxylic acid (106 mg, 0.22 mmol) in N,N-dimethylformamide (5 mL) was added while stirring the mixture. Reactants were reacted at room temperature for 30 min. The reactants were poured into ice water (50 mL), and extracting was carried out with ethyl acetate (50 mL ×3). Organic phases were combined, washed with a saturated brine (100 mL), dried over anhydrous sodium sulfate, and concentrated. A crude product was purified by preparation (chromatographic column: Waters Xbridge C18 10um OBD 19X250 mm; mobile phase: water/acetonitrile (0.1% ammonium bicarbonate); flow rate: 20 mL/min; detection wavelength: 254 nm/214 nm; column temperature: 25°C), thus obtaining a product (4¹S, 4³S)-2⁵-(trifluoromethyl)-1¹H-3,5,10-triaza-1(3,6)-indola-2(4,2)-pyrimidina-4(1,3)-cyclopentanacycloundecaphan-11-one (Z2, 1.02 mg, and yield of 0.35%). LCMS m/z (ESI): [M+H]⁺=458.2. ¹H NMR (400 MHz, CD₃OD) δ 8.39 (s, 1H), 7.79 (s, 1H), 7.47 (s, 1H), 7.34 - 7.24 (m, 1H), 7.23 - 7.07 (m, 1H), 2.95 (s, 2H), 2.27 (d, *J* = 23.4 Hz, 2H), 2.08 (d, *J* = 8.5 Hz, 2H), 1.41 (m, 4H), 1.19 (m, 6H). ¹⁹F NMR (377 MHz, CD₃OD) δ -59.28 (s).

### Example 3 Synthesis of compound Z3

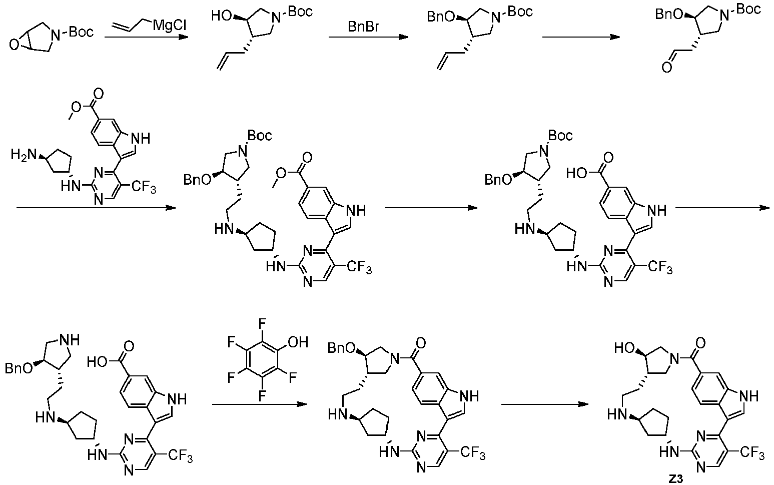

Step 1: Allyl magnesium chloride (60.739 mL, 121.48 mmol, 2 M/L) was slowly dropwise added into a solution of 3-N-tert-butyloxycarbonyl-6-oxa-3-azabicyclo[3.1.0]hexane (5 g, 26.99 mmol) and cuprous (I) bromide dimethyl sulfide complex (60.739 mL, 121.48 mmol) in tetrahydrofuran (150 mL) at -30°C under nitrogen protection, and the reaction solution was stirred to react for 1 h at -10°C under nitrogen protection. The reaction solution was quenched with a 10% ammonium chloride aqueous solution (100 mL) and ethyl acetate (100 mL), and stirring was carried out at room temperature for 1 h. The reaction solution was extracted with ethyl acetate (50 mL×3), the combined organic layer was washed with a saturated brine, dried over anhydrous sodium sulfate, filtered, and concentrated to dryness. The obtained crude product was purified with silica gel column chromatography (petroleum ether/ethyl acetate = 10/1-55/45) to obtain a yellow oily substance tert-butyl (3R,4S)-3-hydroxy-4-allylpyrrolidine-1-carboxylate (1.55 g, and yield of 25.26%). LCMS m/z (ESI): [M+H-56] ⁺=172.

Step 2: Sodium hydride (0.32 g, 7.92 mmol, purity of 60%) was added into a solution of tert-butyl (3R,4S)-3-hydroxy-4-allylpyrrolidine-1-carboxylate (1.5 g, 6.60 mmol) in N,N-dimethylformamide (8 mL) at 0°C under nitrogen protection, and the reaction solution was stirred at room temperature under nitrogen protection to react for 0.5 h. Benzyl bromide (0.947 mL, 7.92 mmol) was added, and the obtained reaction solution was stirred at room temperature overnight. The reaction solution was poured into water (50 mL), extracting was carried out with ethyl acetate (50 mL ×3), the combined organic layer was washed with a saturated brine, dried over anhydrous sodium sulfate, filtered and concentrated to dryness. The obtained crude product was purified with silica gel column chromatography (petroleum ether/ethyl acetate = 10/1-9/1) to obtain a yellow oily substance tert-butyl (3R,4S)-3-benzyloxy-4-allylpyrrolidine-1-carboxylate (0.9 g, yield of 42.96%). LCMS m/z (ESI): [M+H-56] ⁺=262, [M+H-100] ⁺=218. ¹H NMR (400 MHz, CDCl₃) δ 7.38 - 7.25 (m, 5H), 5.82 - 5.66 (m, 1H), 5.11 - 4.97 (m, 2H), 4.60 - 4.44 (m, 2H), 3.83 - 3.72 (m, 1H), 3.64 - 3.49 (m, 2H), 3.42 - 3.32 (m, 1H), 3.20 - 3.03 (m, 1H), 2.37 - 2.25 (m, 1H), 2.24 - 2.14 (m, 1H), 2.09 - 1.93 (m, 1H), 1.45 (s, 9H).

Step 3: Potassium osmate (92.86 mg, 0.25 mmol) was added into a mixed solution of tert-butyl (3R,4S)-3-benzyloxy-4-allylpyrrolidine-1-carboxylate (800 mg, 2.52 mmol) and sodium periodate (1.62 g, 7.56 mmol) in acetonitrile (15 mL) and water (6 mL), and the reaction solution was stirred at room temperature to react for 2 h. Water (10 mL) was added into the reaction solution, extracting was carried out with ethyl acetate (10 mL ×3), the combined organic layer was washed with a saturated brine, dried over anhydrous sodium sulfate, filtered, and concentrated to dryness. The obtained crude product was purified with silica gel column chromatography (petroleum ether/ethyl acetate = 10/1-55/45) to obtain a yellow oily substance tert-butyl (3R,4S)-3-benzyloxy-4-(2-acetaldehyde)pyrrolidine-1-carboxylate (500 mg, yield of 62.11%). LCMS: MS m/z (ESI): [M+H-56] ⁺=264, [M+H-100] ⁺=220.

Step 4: A small amount of acetic acid (0.086 mL, 1.50 mmol) was added into a solution of tert-butyl (3R,4S)-3-benzyloxy-4-(2-acetaldehyde)pyrrolidine-1-carboxylate (480 mg, 1.50 mmol) and methyl 3-(2-(((1S,3S)-3-amino)cyclopentyl)amino)-5-(trifluoromethyl)pyrimidin-4-yl)-1H-indole-6-formate (630 mg, 1.50 mmol) in 1,2-dichloroethane (15 mL), and the reaction solution was stirred at room temperature for reaction for 0.5 h. Sodium borohydride acetate (951 mg, 4.51 mmol) was added, and the obtained reaction solution was stirred at room temperature overnight. Water (20 mL) was added into the reaction solution, extracting was carried out with ethyl acetate (10 mL ×3), the combined organic layers were washed with a saturated brine, dried over anhydrous sodium sulfate, filtered and concentrated to dryness. The obtained crude product was purified with silica gel column chromatography (dichloromethane/methanol = 100/1-95/5) to obtain a yellow oily substance methyl 3-(2-(((1S,3S)-3-((2-((3S,4R)-4-(benzyloxy)-1-(t-butyloxycarbonyl)pyrrol-3-yl)ethyl)amino)cyclopentyl)amino)-5-(trifluoromethyl)pyrimidin-4-yl)-1H-indole-6-formate (460 mg, yield of 42.37%). LCMS: MS m/z (ESI): [M+H] ⁺=723.

Step 5: A solution of lithium hydroxide (139 mg, 3.32 mmol) in water (2 mL) was added into a mixed solution of methyl 3-(2-(((1S,3S)-3-((2-((3S,4R)-4-(benzyloxy)-1-(t-butyloxycarbonyl)pyrrol-3-yl)ethyl)amino)cyclopentyl)amino)-5-(trifluoromethyl)pyrimidin-4-yl)-1H-indole-6-formate (480 mg, 0.66 mmol) in tetrahydrofuran (2 mL) and methanol (2 mL), and the reaction solution was stirred at room temperature to react overnight. The reaction solution was acidified with 0.5 M hydrochloric acid aqueous solution until reaching pH value of 3-4, extracting was carried out with ethyl acetate (5 mL ×3), the combined organic layer was washed with a saturated brine, dried over anhydrous sodium sulfate, filtered and concentrated to dryness. The obtained crude product was purified with silica gel column chromatography (dichloromethane/methanol = 100/1-85/15) to obtain a yellow oily substance 3-(2-(((1S,3S)-3-((2-((3S,4R)-4-(benzyloxy)-1-(tert-butyloxycarbonyl)pyrrol-3-yl)ethyl)amino)cyclopentyl)amino)-5-(trifluoromethyl)pyrimidin-4-yl)-1H-indole-6-formic acid (460 mg, yield of 98.2%). LCMS: MS m/z (ESI): [M+H] ⁺=709.

Step 6: A hydrochloric acid/dioxane (10 mL, 4 N) solution was added into 3-(2-(((1S,3S)-3-((2-((3 S,4R)-4-(benzyloxy)-1-(tert-butyloxycarbonyl)pyrrol-3-yl)ethyl)amino)cyclopentyl)amino)-5-(trifluoromethyl)pyrimidin-4-yl)-1H-indole-6-formic acid (390 mg, 0.55 mmol), and the reaction solution was stirred at room temperature to react for 1 h. The reaction solution was concentrated to dryness, thereby obtaining a yellow solid 3-(2-(((1S,3S)-3-((2-((3S,4R)-4-(benzyloxy)pyrrol-3-yl)ethyl)amino)cyclopentyl)amino)-5-(trifluoromethyl)pyrimidin-4-yl)-1H-indole-6-formic acid (320 mg, yield of 95.55%). LCMS: MS m/z (ESI): [M+H] ⁺=609.

Step 7: Pentafluorophenol (194 mg, 1.05 mmol) and N,N'-dicyclohexylcarbodiimide (217 mg, 1.05 mmol) were sequentially added into a solution of 3-(2-(((1S,3S)-3-((2-((3S,4R)-4-(benzyloxy)pyrrol-3-yl)ethyl)amino)cyclopentyl)amino)-5-(trifluoromethyl)pyrimidin-4-yl)-1H-indole-6-formic acid (320 mg, 0.53 mmol) in N,N-dimethylformamide (180 mL), and the reaction solution was stirred at room temperature to react overnight. The reaction solution was concentrated to dryness, and the obtained crude product was purified with silica gel column chromatography (dichloromethane/methanol = 100/1-90/10) to obtain a brown solid (4¹S,4³S,8³S,8⁴R)-8⁴-(benzyloxy)-2⁵-(trifluoromethyl)-1¹H-3,5-diaza-1(3,6)-indola-2(4,2)-pyrimidina-8(3,1)-pyrrolidina-4(1,3)-cyclopentanacyclononaphan-9-one (100 mg, yield of 32.26%). LCMS: MS m/z (ESI): [M+H] ⁺=591.

Step 8: A boron trichloride (0.25 mL, 1 M) solution was slowly dropwise added into a solution of (4¹S,4³S,8³S,8⁴R)-8⁴-(benzyloxy)-2⁵-(trifluoromethyl)-1¹H-3,5-diaza-1(3,6)-indola-2(4,2)-pyrimidina-8(3,1)-pyrrolidina-4(1,3)-cyclopentanacyclononaphan-9-one (30 mg, 0.05 mmol) in dichloromethane (2 mL) at 0°C, and the reaction solution was stirred and reacted for 1.5 h at 0°C under nitrogen protection. The reaction solution was quenched with methanol (0.1 mL), and was concentrated to dryness, and the obtained crude product was purified through Prep-HPLC (chromatographic column: Xbridge Prep C18 10 um OBO^{™} 19X250 nm; mobile phase: acetonitrile/water (0.05% ammonium hydroxide); flow rate: 20 mL/min) to obtain (4¹S,4³S,8³S,8⁴R)-8⁴-hydroxy-2⁵-(trifluoromethyl)-1¹H-3,5-diaza-1(3,6)-indola-2(4,2)-pyrimidina-8(3,1)-pyrrolidina-4(1,3)-cyclopentanacyclononaphan-9-one (Z3 of 2.6 mg, yield of 8.0%). LCMS: MS m/z (ESI): [M+H] ⁺=501. ¹H NMR (400 MHz, CD₃OD) δ 8.79 - 8.19 (m, 2H), 7.86 (d, J = 76.7 Hz, 1H), 7.51 - 7.45 (m, 1H), 7.25 - 7.15 (m, 1H), 4.64 - 4.48 (m, 1H), 4.29 - 4.18 (m, 1H), 4.15 - 3.87 (m, 2H), 3.58 - 3.37 (m, 1H), 3.26 - 3.18 (m, 1H), 2.86 - 2.67 (m, 1H), 2.59 - 2.47 (m, 1H), 2.29 - 2.19 (m, 2H), 2.13 - 1.96 (m, 2H), 1.88 - 1.74 (m, 1H), 1.70 - 1.58 (m, 2H), 1.56 - 1.44 (m, 1H), 1.36 - 1.09 (m, 2H).

### Example 4 Synthesis of compound Z4

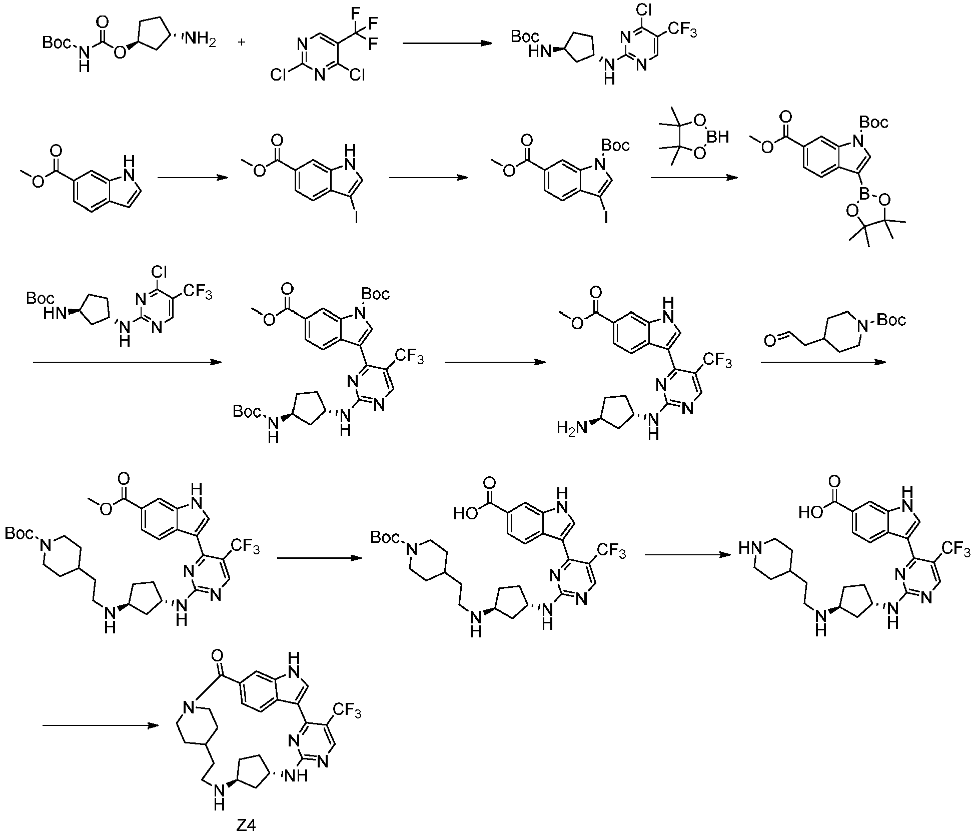

Step 1: N,N-diisopropylethylamine (2.61 g, 20.0 mmol) and 2,4-dichloro-5-(trifluoromethyl)pyrimidine (1.10 g, 5.0 mmol) were added into a solution of tert-butyl ((1S,3S)-3-aminocyclopentyl)carbamate (1 g, 5.0 mmol) in tetrahydrofuran (25 mL), and the reaction solution was stirred at 25 °C to react for 14 h. After monitoring complete reaction by LCMS, the reaction solution was directly concentrated to obtain a crude product. The crude product was purified with silica gel column chromatography (petroleum ether/ethyl acetate = 30/1-15/1) to obtain tert-butyl N-[(1S,3S)-3-{[4-chloro-5-(trifluoromethyl)pyrimidin-2-yl]amino}cyclopentyl]carbamate (0.76 g, yield of 40%). LCMS: MS m/z (ESI): [M+H]⁺ =381.2, t_{R} = 1.56 min.

Step 2: 1-iodo-pyrrolidin-2,5-dione (4.62 g, 20.55 mmol) was added into a solution of methyl 1H-indole-6-carboxylate (3.00 g, 17.12 mmol) in N,N-dimethylformamide (30 mL) at 0°C. The reaction was stirred for 3 h at room temperature. Complete reaction was monitored by TLC (petroleum ether:ethyl acetate =1:1). Extracting was carried out with ethyl acetate (200mL ×3) and water (60 mL). Then a saturated sodium chloride solution was added for washing, drying was carried out with anhydrous sodium sulfate, and filtering and concentrating were carried out. Purified with silica gel column chromatography (petroleum ether:ethyl acetate =1:1) to obtain a compound methyl 3-iodo-1H-indole-6-carboxylate (3.5 g, yield of 67.89%).

Step 3: Triethylamine (3.23 mL, 23.25 mmol) and 4-dimethylaminopyridine (0.36 g, 2.91 mmol) were added into a solution of methyl 3-iodo-1H-indole-6-carboxylate (3.50 g, 11.62 mmol) in dichloromethane (35 mL), and di-tert-butyl dicarbonate (2.99 mL, 13.95 mmol) was added at 0°C. The reaction was stirred for 18 h at room temperature. Complete reaction was monitored by TLC (petroleum ether:ethyl acetate =5:1). The reaction solution was extracted with dichloromethane (200 mL×3) and water (60 mL), a saturated sodium chloride solution was added for washing, anhydrous sodium sulfate was added for drying, and filtering and concentrating were carried out. Purified with silica gel column chromatography (petroleum ether/ethyl acetate =3:1) to obtain a compound 1-(tert-butyl) 6-methyl-3-iodo-1H-indole-1,6-diformate (3.8 g, yield of 81.48%).

Step 4: Potassium acetate (1.76 g, 17.95 mmol), palladium dichloride (0.66 g, 0.91 mmol) and 4,4,5,5-tetramethyl-1,3,2-dioxaborane (1.95 mL, 13.46 mmol) were added into a solution of 1-(tert-butyl) 6-methyl-3-iodo-1H-indole-1,6-diformate (3.60 g, 8.97 mmol) in dioxane (36 mL), and stirring was carried out at 70°C for 3 h. Complete reaction was monitored by TLC (petroleum ether:ethyl acetate =5:1). The mixture was collected, and vacuum concentration was carried out. The crude product was purified with silica gel column chromatography (petroleum ether/ethyl acetate =20: 1) to obtain a compound 1-(tert-butyl) 6-methyl-3-(4,4,5,5-tetramethyl-1,3,2-dioxaboran-2-yl)-1H-indole-1,6-diformate (2.5 g, yield of 69.43%).

Step 5: Potassium carbonate (1.58 g, 11.46 mmol), palladium dichloride (0.28 g, 0.38 mmol) and tert-butyl N-[(1S,3 S)-3-{ [4-chloro-5-(trifluoromethyl)pyrimidin-2-yl]amino}cyclopentyl]carbamate (1.46 g, 3.82 mmol) were added into a solution of 1-(tert-butyl) 6-methyl-3-(4,4,5,5-tetramethyl-1,3,2-dioxaboran-2-yl)-1H-indole-1,6-diformate (2.3 g, 5.73 mmol) in dioxane and water solution (23 mL), reacting was carried out, and stirring was carried out for 3 h at 100 °C. Complete reaction was monitored by TLC (petroleum ether:ethyl acetate =3:1). The mixture was collected, and vacuum concentration was carried out. The crude product was purified with silica gel column chromatography (petroleum ether/ethyl acetate elution =3:1) to obtain the compound 1-(tert-butyl) 6-methyl-3-(2-(((1S,3 S)-3-((t-butyloxycarbonyl)amino)cyclopentyl)amino)-5-(trifluoromethyl)pyrimidin-4-yl)-1H-indole-1,6-diformate (2.2 g, yield of 90%).

Step 6: Dioxane (15 mL, 3.55 mmol) was added into a flask containing 1-(tert-butyl)6-methyl-3-(2-(((1S,3S)-3-((t-butyloxycarbonyl)amino)cyclopentyl)amino)-5-(trifluoromethyl)pyrimidin-4-yl)-1H-indole-1,6-diformate (2.20 g, 3.55 mmol), and then hydrogen chloride (15 mL, 3.55 mmol) was added. The mixture was stirred at 25°C for 2 h. Complete reaction was monitored by TLC (dichloromethane:methanol = 10:1). Concentrating was carried out, the obtained solid was dissolved with methyl tert-butyl ether, and filtering was carried out to obtain a light yellow solid compound methyl 3-(2-(((1S,3S)-3-aminocyclopentyl)amino)-5-(trifluoromethyl)pyrimidin-4-yl)-1H-indole-6-carboxylate (2.6 g, crude product, hydrochloride). ES-API: [M+H]⁺ = 419.2. ¹H NMR (400 MHz, DMSO-d₆) δ 12.17 (s, 1H), 8.60 (d, *J=* 15.4 Hz, 1H), 8.38 (d, *J* = 30.4 Hz, 1H), 8.05 (s, 2H), 7.78 *(d, J=* 24.6 Hz, 1H), 4.53 (d, *J =* 6.1 Hz, 1H), 3.88 (s, 3H), 3.56 (s, 2H), 2.16 (s, 2H), 2.00 (d, *J*= 9.2 Hz, 3H), 1.63 (s, 2H), 1.23 (s, 2H).

Step 7: Sodium borohydride (0.39 mL, 11.92 mmol) and tert-butyl 4-(2-oxyethyl)piperidine-1-carboxylate (0.54 g, 2.38 mmol) were added into a solution of methyl 3-(2-(((1S,3 S)-3-aminocyclopentyl)amino)-5-(trifluoromethyl)pyrimidin-4-yl)-1H-indole-6-carboxylate (1.00 g, 2.38mmol) in 1,2-dichloroethane (10 mL), and the reaction was stirred at room temperature overnight. The reaction solution was extracted with dichloromethane (60 mL ×3) and a saturated sodium bicarbonate solution, washing was carried out with a saturated sodium chloride solution, dried over anhydrous sodium sulfate, filtered and concentrated. Thin-layer chromatography purification was carried out on a crude product (dichloromethane:methanol =30:1) to obtain methyl 3-(2-(((1S,3S)-3-((2-(1-(tert-butyloxycarbonyl)piperidin-4-yl)ethyl)amino)cyclopentyl)amino)-5-(trifluoromethyl)pyrimidin-4-yl)-1H-indole-6-carboxylate (300 mg, yield of 19.46%). ES-API: [M+H]⁺= 630.31.

Step 8: Lithium hydroxide (0.02 mL, 0.71 mmol) was added into a solution of methyl 3-(2-(((1S,3S)-3-((2-(1-(tert-butyloxycarbonyl)piperidin-4-yl)ethyl)amino)cyclopentyl)amino)-5-(trifluoromethyl)pyrimidin-4-yl)-1H-indole-6-carboxylate (150 mg, 0.24 mmol) in tetrahydrofuran/methanol/water (1:1:1), and the reaction was stirred at room temperature overnight. The pH value of the reactant was adjusted to be acidic with dilute hydrochloric acid. The reaction was diluted with ethyl acetate (6 mL ×3) and water (2 mL), and then a saturated sodium chloride solution was added for separation. An organic layer was collected, dried over anhydrous sodium sulfate, and concentrated to obtain a compound 3-(2-(((1S,3S)-3-((2-(1-(tert-butyloxycarbonyl)piperidin-4-yl)ethyl)amino)cyclopentyl)amino)-5-(trifluoromethyl)pyrimidin-4-yl)-1H-indole-6-carboxylic acid (150 mg, yield of 98.79%). ¹H NMR (400 MHz, DMSO-d₆) δ 12.26 (d, *J* = 14.1 Hz, 1H), 9.16 (s, 3H), 8.85 (s, 1H), 8.58 (s, 1H), 8.32 (s, 1H), 8.16 - 7.98 (m, 3H), 7.76 (d, *J=* 17.5 Hz, 1H), 4.52 (s, 1H), 3.21 (d, J= 12.1 Hz, 4H), 2.84 (d, *J* = 29.6 Hz, 5H), 2.19 (d, *J* = 6.3 Hz, 3H), 1.78 (s, 3H), 1.60 (s, 4H), 1.28 (d, *J*= 40.2 Hz, 9H).

Step 9: Dioxane (3 mL, 0.52 mmol) was added into a flask containing 3-(2-(((1S,3S)-3-((2-(1-(tert-butyloxycarbonyl)piperidin-4-yl)ethyl)amino)cyclopentyl)amino)-5-(trifluoromethyl)pyrimidin-4-yl)-1H-indole-6-carboxylic acid (330 mg, 0.52 mmol), and then hydrogen chloride (3 mL) was added. The mixture was stirred at 25 °C for 2 h. Complete reaction was monitored by TLC (dichloromethane:methanol = 7:1). An organic layer was concentrated in vacuum. The obtained solid was dissolved with methyl tert-butyl ether, and filtering was carried out to obtain a light yellow solid compound 3-(2-(((1S,3S)-3-((2-(piperidin-4-yl)ethyl)amino)cyclopentyl)amino)-5-(trifluoromethyl)pyrimidin-4-yl)-1H-indole-6-carboxylic acid (350 mg, yield of 126.00%). ES-API: [M+H]⁺ =517.4. ¹H NMR (400 MHz, DMSO-d₆) δ 12.54 (s, 1H), 9.39 (d, *J=* 41.9 Hz, 3H), 9.13 (s, 1H), 8.59 (d, *J=* 12.7 Hz, 1H), 8.37 (dd, *J=* 40.4, 8.0 Hz, 1H), 8.07 (d, *J=* 60.1 Hz, 3H), 7.76 (dd, *J=* 33.4, 8.0 Hz, 1H), 4.51 (s, 1H), 3.18 (d, *J* = 11.9 Hz, 3H), 2.91 - 2.71 (m, 4H), 2.20 (dd, *J=* 13.6, 6.8 Hz, 3H), 1.79 (d, *J=* 8.9 Hz, 3H), 1.66 (d, *J=* 19.7 Hz, 4H), 1.37 (d, *J=* 9.5 Hz, 2H). Step 10: 2-(7-azabenzotriazole)-N,N,N',N'-tetramethyluronium hexafluorophosphate (72 mg, 0.19 mmol) and N,N-diisopropylethylamine (88 mg, 0.68 mmol) were added into a solution of 3-(2-((1S,3S)-3-((2-(piperidin-4-yl)ethyl)amino)cyclopentyl)amino)-5-(trifluoromethyl)pyrimidin-4-yl)-1H-indole-6-carboxylic acid (90 mg, 0.17 mmol) in N,N-dimethylformamide (5 mL), and the reaction solution was stirred at room temperature for 16 h. After monitoring complete reaction by LCMS, the reaction solution was concentrated to dryness, and reversed-phase preparation was carried out on the obtained crude product (chromatographic column: Ultimate XBC18, 50X250 mm, 10 um (PARP-06); flow rate: 70 mL/min; mobile phase: acetonitrile-water (0.1% trifluoroacetic acid); column temperature: 25 °C) to obtain (4¹S,4³S)-2⁵-(trifluoromethyl)-1¹H-3,5-diaza-1(3,6)-indola-2(4,2)-pyrimidina-8(4,1)-piperidina-4(1,3)-cyclopentanacyclononaphan-9-one (Z4) trifluoroacetate (1.7 mg, yield of 1.3%). LCMS: MS m/z (ESI): [M+H]⁺=499.2, t_{R} = 1.22 min. ¹H NMR (400 MHz, CD₃OD): δ 8.64 (d, *J* = 8.0 Hz, 1H), 8.56 (s, 1H), 8.12 (s, 1H), 7.52 (s, 1H), 7.39 (d, *J* = 8.0 Hz, 1H), 4.53 - 4.50 (m, 1H), 4.48 - 4.37 (m, 1H), 3.72 (s, 1H), 3.26 - 3.19 (m, 1H), 3.09 - 2.97 (m, 1H), 2.80 - 2.55 (m, 3H), 2.42 - 2,40 (m, 2H), 2.11 - 1.97 (m, 2H), 1.92 - 1.60 (m, 8H), 1.36 - 1.29 (m, 1H).

### Example 5 Synthesis of compound Z5

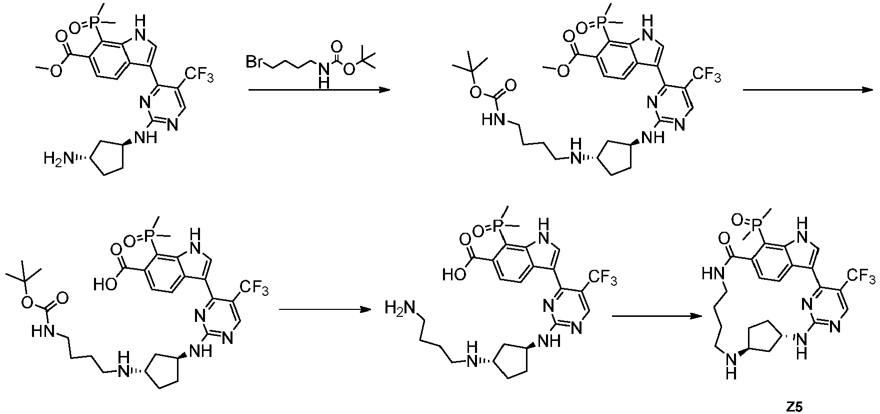

Step 1: Tert-butyl (4-bromobutyl)carbamate (138 mg, 0.55 mmol) and N,N-diisopropylethylamine (284 mg, 2.20 mmol) were sequentially added into a solution (5 mL) of methyl 3-(2-(((1 S,3 S)-3-aminocyclopentyl)amino)-5-(trifluoromethyl)pyrimidin-4-yl)-7-(dimethylphosphoryl)-1H-indole-6-carboxylate (270 mg, 0.55 mmol) in N,N-dimethylformamide, and the reaction solution was stirred at 80 °C under nitrogen protection to react for 16 h. After monitoring complete reaction by LCMS, 10% ammonium chloride aqueous solution (100 mL) was added into the reaction solution, extracting was carried out with ethyl acetate (100 mL ×3), the combined organic layer was washed with a saturated brine, dried over anhydrous sodium sulfate, filtered, and concentrated. The obtained crude product was purified with silica gel column chromatography (petroleum ether/ethyl acetate = 2/1-1/1) to obtain white solid methyl 3-(2-(((1S,3S)-3-((4-((t-butyloxycarbonyl)amino)butyl)amino)cyclopentyl)amino)-5-(trifluoromethyl)pyrimidin-4-yl)-7-(dimethylphosphoryl)-1H-indole-6-carboxylate (170 mg, yield of 46%). LCMS: MS m/z (ESI): [M+H]⁺=667.3, t_{R} = 1.28 min.

Step 2: A solution of lithium hydroxide (66.0 mg, 1.56 mmol) in water (2 mL) was added into a mixed solution of methyl 3-(2-(((1 S,3 S)-3-((4-((t-butyloxycarbonyl)amino)butyl)amino)cyclopentyl)amino)-5-(trifluoromethyl)pyrimidin-4-yl)-7-(dimethylphosphoryl)-1H-indole-6-carboxylate (170 mg, 0.26 mmol) in tetrahydrofuran (2 mL) and methanol (2 mL), and the reaction solution was stirred at 40 °C for reaction overnight. After monitoring complete reaction by LCMS, the reaction solution was acidified with a 1.0 M hydrochloric acid aqueous solution until reaching pH of 3-4, extracting was carried out with dichloromethane (10 mL ×3), the combined organic layer was washed with a saturated brine, dried over anhydrous sodium sulfate, filtered, and concentrated to obtain a white solid 3-(2-(((1S,3S)-3-((4-((t-butyloxycarbonyl)amino)butyl)amino)cyclopentyl)amino)-5-(trifluoromethyl)pyrimidin-4-yl)-7-(dimethylphosphoryl)-1H-indole-6-carboxylic acid (160 mg, yield of 94%), and the product was directly used in the next reaction without purification. LCMS: MS m/z (ESI): [M+H]⁺ =653.2, t_{R} = 1.12 min.

Step 3: A hydrochloric acid/methanol (5 mL, 4N) solution was added into a solution of 3-(2-(((1S,3S)-3-((4-((t-butyloxycarbonyl)amino)butyl)amino)cyclopentyl)amino)-5-(trifluoromethyl)pyrimidin-4-yl)-7-(dimethylphosphoryl)-1H-indole-6-carboxylic acid (160 mg, 0.25 mmol) in methanol (5 mL) at 25°C, and the reaction solution was stirred at 25°C to react for 16 h. After monitoring complete reaction by LCMS, the reaction solution was concentrated to obtain a white solid 3-(2-(((1S,3S)-3-((4-aminobutyl)amino)cyclopentyl)amino)-5-(trifluoromethyl)pyrimidin-4-yl)-7-(dimethylphosphoryl)-1H-indole-6-carboxylic acid (120 mg, yield of 87%). LCMS: MS m/z (ESI): 553.2 [M+H]⁺, t_{R} = 1.00 min.

Step 4: 2-(7-azabenzotriazole)-N,N,N',N'-tetramethyluronium hexafluorophosphate (91.2 mg, 0.24 mmol) and N,N-diisopropylethylamine (114 mg, 0.88 mmol) were sequentially added into a solution of 3-(2-(((1S,3 S)-3-((4-aminobutyl)amino)cyclopentyl)amino)-5-(trifluoromethyl)pyrimidin-4-yl)-7-(dimethylphosphoryl)-1H-indole-6-carboxylic acid (120 mg, 0.22 mmol) in N,N-dimethylformamide (5 mL), and the reaction solution was stirred at 80°C for 16 h. After monitoring complete reaction by LCMS, the reaction solution was concentrated, and reversed-phase preparation was carried out (chromatographic column: Ultimate XBC18, 50X250 mm, 10 um (PARP-06); mobile phase: acetonitrile-water (0.1% trifluoroacetic acid); flow rate: 70 mL/min; column temperature: 25°C) on the obtained crude product to obtain 4¹S,4³S)-1⁷-(dimethylphosphoryl)-2⁵-(trifluoromethyl)-1¹H-3,5,10-triaza-1(3,6)-indola-2(4,2)-pyrimidina-4(1,3)-cyclopentanacycloundecaphan-11-one (Z5) trifluoroacetate (17.2 mg, yield of 17%). LCMS: MS m/z (ESI): [M+H]⁺ =535.2, t_{R} = 1.55 min.¹HNMR (400 MHz, DMSO-*d₆*): δ 12.00 (s, 1H), 8.61 (s, 1H), 8.53 - 8.31 (m, 3H), 8.18 (d, *J=* 4.0 Hz, 1H), 8.13 - 8.05 (m, 1H), 8.00 (s, 1H), 7.29-7.26 (m, 1H), 4.34 - 4.20 (m, 1H), 2.85 - 2.75 (m, 1H), 2.73 - 2.57 (m, 4H), 2.20-2.12 (m, 2H), 1.95 - 1.85 (m, 6H), 1.81 - 1.52 (m, 6H), 1.50 - 1.40 (m, 1H).

### Example 6 Synthesis of compound Z6

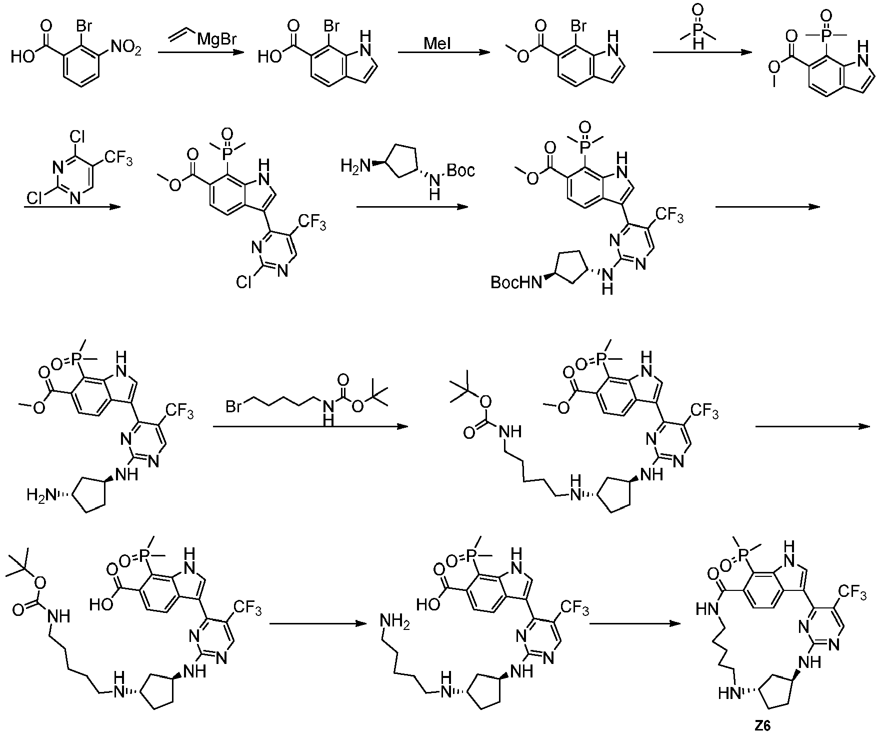

Step 1: A solution (1 M, 163 mL, 163 mmol) of vinyl magnesium bromide in tetrahydrofuran was slowly dropwise added into a solution of 2-bromo-3-nitrobenzoic acid (10.0 g, 40.6 mmol) in tetrahydrofuran (150 mL) at -78°C, continuous stirring and reacting were carried out at -78 °C for 3 h under nitrogen protection after the dropwise adding, natural warming was carried out to reach room temperature, and stirring was carried out for 16 h. After monitoring complete reaction by LCMS, a saturated ammonium chloride aqueous solution (300 mL) was slowly added into the reaction solution for quenching, the pH was adjusted to 4-5 with 1 M hydrochloric acid, extracting was carried out with dichloromethane (300 mL × 3), the combined organic layer was washed with a saturated brine, dried over anhydrous sodium sulfate, filtered and concentrated. The resulting crude product was purified by column chromatography on silica gel (dichloromethane/methanol = 30/1) to obtain7-bromo-1H-indole-6-carboxylic acid as a yellow solid (9.4 g, and yield of 96.5%). LCMS: m/z (ESI): 239.8 [M+H]⁺, t_{R} = 1.21 min.

Step 2: Iodomethane (6.66 g, 46.9 mmol) and potassium carbonate (10.8 g, 78.3 mmol) were added into a solution of 7-bromo-1H-indole-6-carboxylic acid (9.40 g, 39.2 mmol) in N,N-dimethylformamide (80 mL) at 25°C. The reaction solution was stirred at 25°C for 16 h under nitrogen protection. After monitoring complete reaction by LCMS, water (200 mL) was added into the reaction solution, then extracting was carried out with ethyl acetate (200 mL × 3), the combined organic layer was washed with a saturated brine, dried over anhydrous sodium sulfate, filtered and concentrated. The resulting crude product was purified by column chromatography on silica gel (petroleum ether/ethyl acetate = 10/1) to obtain methyl 7-bromo-1H-indole-6-carboxylate as a yellow solid (3.90 g, yield of 37%). LCMS: m/z (ESI): 254.0 [M+H]⁺, t_{R} = 1.46 min. ¹H NMR (400 MHz, DMSO-*d₆*): δ 11.63 (s, 1H), 7.62 (dd, *J=* 13.6, 5.6 Hz, 2H), 7.51 (d, *J=* 8.0 Hz, 1H), 6.66 (d, *J=* 3.2 Hz, 1H), 3.87 (s, 3H).

Step 3: Solutions of tris(dibenzylideneacetone)dipalladium (242 mg, 0.393 mmol), xantphos (455 mg, 0.787 mmol) and 1,4-dioxane (20 mL) were sequentially added into a sealed tank at 25°C, stirring was carried out for 30 min under nitrogen protection, and then methyl 7-bromo-1H-indole-6-carboxylate (2.0 g, 7.87 mmol), potassium phosphate (1.67 g, 7.87 mmol) and dimethyl phosphine oxide (0.61 g, 7.87 mmol) were sequentially added. The reaction solution was stirred for 14 h at 110 °C. After monitoring complete reaction by LCMS, the reaction solution was directly concentrated, and the resulting crude product was purified by column chromatography on silica gel (petroleum ether/ethyl acetate = 1/1-pure ethyl acetate) to obtain methyl 7-(dimethylphosphoryl)-1H-indole-6-carboxylate as a yellow solid (1.00 g, yield of 51%). LCMS: m/z (ESI): [M+H]⁺ =252.0, t_{R} = 1.30 min. ¹H NMR (400 MHz, DMSO-*d₆*): δ 12.12 (s, 1H), 7.86 (d, *J* = 8.4 Hz, 1H), 7.69 (dd, *J* = 8.4, 3.6 Hz, 1H), 7.65 (t, *J=* 2.8 Hz, 1H), 6.59 (s, 1H), 3.88 (s, 3H), 1.95 (s, 3H), 1.92 (s, 3H).

Step 4: Aluminum trichloride (1.06 g, 7.96 mmol) was added into a dried 50 mL three-neck bottle under a nitrogen atmosphere, then a solution of methyl 7-(dimethylphosphoryl)-1H-indole-6-carboxylate (1.00 g, 3.98 mmol) in 1,2-dichloroethane (10 mL) was added by an injector, stirring was carried out at 80 °C for 30 min, then a solution of 2,4-dichloro-5-(trifluoromethyl)pyrimidine (0.95 g, 4.38 mmol) in 1,2-dichloroethane (1 mL) was added, and the reaction solution was reacted at 80°C for 16 h. After monitoring complete reaction by LCMS, water (20 mL) was added into the reaction liquid, then extracting was carried out with ethyl acetate (20 mL × 3), the combined organic layer was washed with a saturated brine, dried over anhydrous sodium sulfate, filtered and concentrated, and the resulting crude product was purified by column chromatography (petroleum ether/ethyl acetate = 3/5) to obtain methyl 3-(2-chloro-5-(trifluoromethyl)pyrimidin-4-yl)-7-(dimethylphosphoryl)-1H-indole-6-carboxylate as a white solid (800 mg, yield of 47%). LCMS: m/z (ESI): [M+H]⁺ =432.0, t_{R} = 1.58 min. ¹H NMR (400 MHz, CDCl₃): δ 13.15 (s, 1H), 8.83 (s, 1H), 8.77 (dd, J = 8.8, 1.6 Hz, 1H), 8.20 (d, *J* = 2.0 Hz, 1H), 8.07 (dd, *J=* 8.8, 4.0 Hz, 1H), 3.96 (s, 3H), 2.10 (d, *J* = 14.4 Hz, 6H).

Step 5: methyl 3-(2-chloro-5-(trifluoromethyl)pyrimidin-4-yl)-7-(dimethylphosphoryl)-1H-indole-6-carboxylate (770 mg, 1.78 mmol), N-methyl pyrrolidone (4 mL), N,N-diisopropylethylamine (0.59 mL, 3.57 mmol) and tert-butyl ((1S,3S)-3-aminocyclopentyl)carbamate (392 mg, 1.96 mmol) were sequentially added into a 10 mL microwave tube, the reaction solution was stirred at 80°C for dissolving, and then microwave reaction was carried out at 130°C for 2 h. After monitoring complete reaction by LCMS, water (20 mL) was added into the reaction solution, then extracting was carried out with ethyl acetate (20 mL × 3), the combined organic layer was washed with a saturated brine, dried over anhydrous sodium sulfate, filtered and concentrated, and the resulting crude product was purified by column chromatography (petroleum ether/ethyl acetate = 1: 1) to obtain methyl 3-(2-(((1S,3S)-3-((t-butyloxycarbonyl)amino)cyclopentyl)amino)-5-(trifluoromethyl)pyrimidin-4-yl)-7-(dimethylphosphoryl)-1H-indole-6-carboxylate as an off-white solid (500 mg, yield of 58%). LCMS: m/z (ESI): [M+H]⁺ =595.8, t_{R} = 1.58 min. ¹H NMR (400 MHz, DMSO-*d₆*): δ 12.67 (s, 1H), 8.59 (s, 2H), 8.16 (s, 1H), 8.01 - 7.68 (m, 2H), 6.78 (s, 1H), 4.46 (dd, *J* = 13.8, 6.8 Hz, 1H), 3.94 (s, 3H), 2.23 - 2.10 (m, 2H), 2.00 (d, *J* = 14.2 Hz, 6H), 1.96 - 1.87 (m, 3H), 1.66 - 1.47 (m, 2H), 1.40 (s, 9H).

Step 6: Methyl 3-(2-(((1S,3S)-3-((t-butyloxycarbonyl)amino)cyclopentyl)amino)-5-(trifluoromethyl)pyrimidin-4-yl)-7-(dimethylphosphoryl)-1H-indole-6-carboxylate (400 mg, 0.67 mmol) was dissolved in a mixed solution of hydrogen chloride/dioxane (5 mL, 4 M) and methanol (2 mL) at room temperature, and reacting was carried out at 20°C for 16 h. After monitoring complete reaction by LCMS, a saturated sodium bicarbonate solution (40 mL) was added into the reaction solution, then extracting was carried out with dichloromethane (20 mL × 3), the combined organic layer was washed with a saturated brine, dried over anhydrous sodium sulfate, filtered and concentrated, and the resulting crude product was purified by column chromatography (dichloromethane/methanol = 30: 1) to obtain methyl 3-(2-(((1S,3 S)-3-aminocyclopentyl)amino)-5-(trifluoromethyl)pyrimidin-4-yl)-7-(dimethylphosphoryl)-1H-indole-6-carboxylate as a white solid (160 mg, yield of 48%). LCMS: m/z (ESI): 496.2 [M+H]⁺, t_{R} = 1.40 min.

Step 7: methyl 3-(2-(((1S,3S)-3-aminocyclopentyl)amino)-5-(trifluoromethyl)pyrimidin-4-yl)-7-(dimethylphosphoryl)-1H-indole-6-carboxylate (500 mg, 1.0 mmol) was dissolved in N,N-dimethylformamide (3 mL), tert-butyl (5-bromopentyl)carbamate (269 mg, 1.0 mmol) and N,N-diisopropylethylamine (0.67 mL, 4.0 mmol) were sequentially added, reacting was carried out, and stirring was carried out at 80°C for 16 h. After monitoring complete reaction by LCMS, spin drying was carried out, and purifying was carried out by column chromatography (dichloromethane/methanol = 100/7) to obtain methyl 3-(2-(((1S,3S)-3-((5-((t-butyloxycarbonyl)amino)pentyl)amino)cyclopentyl)amino)-5-(trifluoromethyl)pyrimidin-4-yl)-7-(dimethylphosphoryl)-1H-indole-6-carboxylate as an off-white solid (420 mg, yield of 61%). LCMS: m/z (ESI): [M+H]⁺ =681.2, t_{R} = 1.54 min.

Step 8: Methyl 3-(2-(((1S,3S)-3-((5-((t-butyloxycarbonyl)amino)pentyl)amino)cyclopentyl)amino)-5-(trifluoromethyl)pyrimidin-4-yl)-7-(dimethylphosphoryl)-1H-indole-6-carboxylate (360 mg, 0.53 mmol) was dissolved in a mixed system of water (1 mL), methanol (1 mL) and tetrahydrofuran (1 mL), then lithium hydroxide monohydrate (111 mg, 2.65 mmol) was added, and stirring was carried out at 40°C for 16 h. After monitoring complete reaction by LCMS, water (20 mL) was added, the pH was adjusted to 4-5 with hydrochloric acid (1 M), extracting was carried out with dichloromethane (20 mL × 9), the combined organic phase was washed with a saturated brine, dried over anhydrous sodium sulfate, filtered and concentrated to obtain 3-(2-(((1S,3S)-3-((5-((t-butyloxycarbonyl)amino)pentyl)amino)cyclopentyl)amino)-5-trifluoromethyl)pyrimidin-4-yl)-7-(dimethylphosphoryl)-1H-indole-6-carboxylic acid as a white solid crude product (320 mg, yield of 91%), and the product was directly used in the next reaction without purification. LCMS: m/z (ESI): [M+H]⁺ =667.2, t_{R} = 1.50 min.

Step 9: 3-(2-(((1S,3 S)-3-((5-((t-butyloxycarbonyl)amino)pentyl)amino)cyclopentyl)amino)-5-trifluoromethyl)pyrimidin-4-yl)-7-(dimethylphosphoryl)-1H-indole-6-carboxylic acid (320 mg, 0.47 mmol) was dissolved in hydrogen chloride/methanol (5 mL, 4 M), and the system was stirred at 25°C for 4 h. After monitoring complete reaction by LCMS, the solvent was spin-dried to obtain 3-(2-(((1S,3 S)-3-((5-aminopentyl)amino)cyclopentyl)amino)-5-(trifluoromethyl)pyrimidin-4-yl)-7-(dimethylphosphoryl)-1H-indole-6-carboxylic acid as a white solid (260 mg, yield 98%), and the product was directly used in the next reaction without purification. LCMS: m/z (ESI): [M+H]⁺ =567.3, t_{R} = 0.92 min.

Step 10: 2-(7-azabenzotriazole)-N,N,N',N'-tetramethyluronium hexafluorophosphate (134 mg, 0.35 mmol) and N,N-diisopropylethylamine (0.23 mL, 1.41 mmol) were sequentially added into a solution of 3-(2-(((1S,3 S)-3-((5-aminopentyl)amino)cyclopentyl)amino)-5-(trifluoromethyl)pyrimidin-4-yl)-7-(dimethylphosphoryl)-1H-indole-6-carboxylic acid (200 mg, 0.35 mmol) in N,N-dimethylformamide (20 mL), and the reaction solution was stirred at 60°C for 16 h. After monitoring complete reaction by LCMS, the reaction solution was concentrated, and reversed-phase preparation was carried out (chromatographic column: Ultimate XB-C18, 50× 250 mm, 10 um (PARP-02); mobile phase: acetonitrile-water (0.1% trifluoroacetic acid); flow rate: 70 mL/min; column temperature: 25°C) on the resulting crude product to obtain (4¹S,4³S)-1⁷-(dimethylphosphoryl)-2⁵-(trifluoromethyl)-1¹H-3,5,11-triaza-1(3,6)-indola-2(4,2)-pyrimidina-4(1,3)-cyclopentanacyclododecaphan-12-one (Z6) trifluoroacetate (34.3 mg, yield of 15%). LCMS: m/z (ESI): [M+H]⁺ =549.2, t_{R} = 0.70 min. ¹H NMR (400 MHz, DMSO-*d₆*): δ 11.84 - 11.94 (m, 1H), 8.70 - 8.30 (m, 3H), 8.30 - 8.07 (m, 3H), 7.87 - 8.10 (m, 1H), 7.62 - 7.22 (m, 1H), 4.39 - 4.18 (m, 1H), 3.50 - 3.05 (m, 1H), 3.01 - 2.80 (m, 3H), 2.70 - 2.52 (m, 1H), 2.45 - 2.33 (s, 1H), 2.22 - 2.01 (m, 2H), 2.00 - 1.37 (m, 15H).

### Example 7 Synthesis of compound Z7

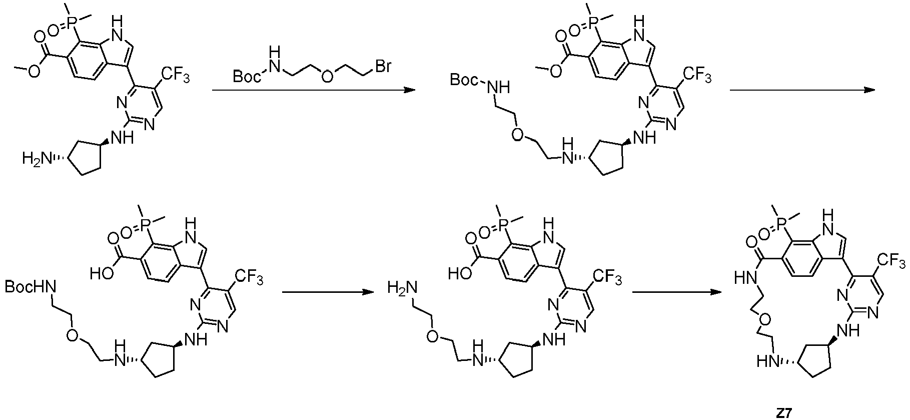

Step 1: Tert-butyl (2-(2-bromoethoxy)ethyl)carbamate (270 mg, 1.00 mmol) and N,N-diisopropylethylamine (0.67 mL, 4.00 mmol) were sequentially added into a solution (5 mL) of methyl 3-(2-(((1S,3 S)-3-aminocyclopentyl)amino)-5-(trifluoromethyl)pyrimidin-4-yl)-7-(dimethylphosphoryl)-1H-indole-6-carboxylate (500 mg, 1.00 mmol) in N,N-dimethylformamide at room temperature, and the reaction solution was reacted under nitrogen protection at 70°C for 16 h while stirring. After monitoring complete reaction by LCMS, a 10% ammonium chloride aqueous solution (100 mL) was added, extracting was carried out with ethyl acetate (100 mL × 3), the combined organic layer was washed with a saturated brine, dried over anhydrous sodium sulfate, filtered and concentrated. The resulting crude product was purified by column chromatography on silica gel (dichloromethane/methanol = 50/1-10/1) to obtain methyl 3-(2-(((1S,3S)-3-((2-(2-((t-butyloxycarbonyl)amino)ethoxy)ethyl)amino)cyclopentyl)amino)-5-(trifluoromethyl)pyrimidin-4-yl)-7-(dimethylphosphoryl)-1H-indole-6-carboxylate as a yellow solid (170 mg, yield of 46%). LCMS: MS m/z (ESI): [M+H]⁺ =683.4, t_{R} = 1.18 min.

Step 2: A solution of lithium hydroxide (86.0 mg, 2.05 mmol) in water (2 mL) was added into a mixed solution of methyl 3-(2-(((1S,3 S)-3-((2-(2-((t-butyloxycarbonyl)amino)ethoxy)ethyl)amino)cyclopentyl)amino)-5-(trifluoromethyl)pyrimidin-4-yl)-7-(dimethylphosphoryl)-1H-indole-6-carboxylate (350 mg, 0.51 mmol) in tetrahydrofuran (2 mL) and methanol (2 mL), and the reaction solution was reacted for 16 h while stirring at 40°C. After monitoring complete reaction by LCMS, the reaction solution was acidified with a 1.0 M hydrochloric acid aqueous solution until reaching pH of 3-4, extracting was carried out with dichloromethane/methanol (10: 1, 60 mL × 6), the combined organic layer was washed with a saturated brine, dried over anhydrous sodium sulfate, filtered and concentrated to obtain 3-(2-(((1S,3 S)-3-((2-(2-((t-butyloxycarbonyl)amino)ethoxy)ethyl)amino)cyclopentyl)amino)-5-(trifluoromethyl)pyrimidin-4-yl)-7-(dimethylphosphoryl)-1H-indole-6-carboxylic acid as a yellow solid (314 mg, yield of 92%). the crude product was directly used in the next reaction without purification. LCMS: MS m/z (ESI): [M+H]⁺=669.2, t_{R} = 1.10 min.

Step 3: A hydrochloric acid/methanol (5 mL, 4M) solution was added into a solution of 3-(2-(((1S,3S)-3-((2-(2-((t-butyloxycarbonyl)amino)ethoxy)ethyl)amino)cyclopentyl)amino)-5-(trifluoromethyl)pyrimidin-4-yl)-7-(dimethylphosphoryl)-1H-indole-6-carboxylic acid (300 mg, 0.45 mmol) in methanol (5 mL) at 25°C, and the reaction solution was stirred at 40°C for 4 h. After monitoring complete reaction by LCMS, the reaction solution was directly concentrated to obtain 3-(2-(((1S,3 S)-3-((2-(2-aminoethoxy)ethyl)amino)cyclopentyl)amino)-5-(trifluoromethyl)pyrimidin-4-yl)-7-(dimethylphosphoryl)-1H-indole-6-carboxylic acid as a yellow solid (244 mg, yield of 96%). The crude product was directly used in the next reaction step without purification. LCMS: MS m/z (ESI): [M+H]⁺ =569.2, t_{R} = 0.95 min.

Step 4: 2-(7-azabenzotriazole)-N,N,N',N'-tetramethyluronium hexafluorophosphate (149 mg, 0.39 mmol) and N,N-diisopropylethylamine (182 mg, 1.41 mmol) were sequentially added into a solution of 3-(2-(((1S,3S)-3-((2-(2-aminoethoxy)ethyl)amino)cyclopentyl)amino)-5-(trifluoromethyl)pyrimidin-4-yl)-7-(dimethylphosphoryl)-1H-indole-6-carboxylic acid (220 mg, 0.39 mmol) in N,N-dimethylformamide (8 mL), and the reaction solution was stirred at 60°C for 16 h. After monitoring complete reaction by LCMS, the reaction solution was concentrated, and reversed-phase preparation was carried out on the resulting crude product (chromatographic column: Ultimate XB-C18, 50× 250 mm, 10 um (PARP-02), chromatographic conditions: acetonitrile-water (0.1% trifluoroacetic acid), flow rate: 70 mL/min and column temperature: 25°C) to obtain (4¹S,4³S)-1⁷-(dimethylphosphoryl)-2⁵-(trifluoromethyl)-1¹H-8-oxa-3,5,11-triaza-1(3,6)-indola-2(4,2)-pyrimidina-4(1,3)-cyclopentanacyclododecaphan-12-one (Z7) trifluoroacetate (33.5 mg, yield of 16%). LCMS: MS m/z (ESI): [M+H]⁺ =551.2, t_{R} = 0.69 min. ¹H NMR (400 MHz, DMSO-*d₆*): δ 12.06 - 12.05 (m, 1H), 8.69 (d, J = 8.0 Hz, 2H), 8.64 - 8.48 (m, 2H), 8.13 (d, J = 4.4 Hz, 1H), 8.07 (d, J = 2.4 Hz, 1H), 7.86 - 7.75 (m, 1H), 7.42 (dd, J = 8.0, 3.2 Hz, 1H), 4.32 - 4.18 (m, 1H), 3.82 - 3.72 (m, 5H), 3.46 (t, J = 7.2 Hz, 1H), 3.31 - 3.17 (m, 2H), 3.16 - 3.08 (m, 1H), 2.72 (dd, J = 14.4, 7.2 Hz, 1H), 2.30 - 2.20 (m, 1H), 2.19 - 2.11 (m, 1H), 2.02 - 1.86 (m, 6H), 1.82 - 1.62 (m, 3H).

### Example 8 Synthesis of compound Z8

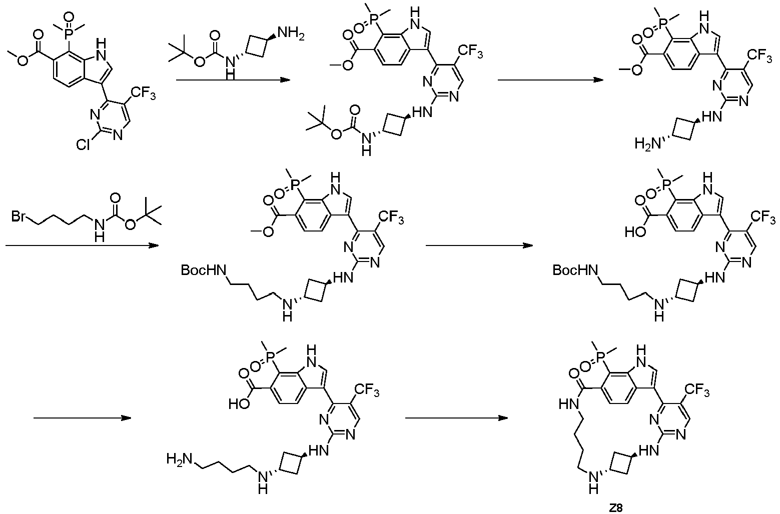

Step 1: Methyl 3-(2-chloro-5-(trifluoromethyl)pyrimidin-4-yl)-7-(dimethylphosphoryl)-1H-indole-6-carboxylate (810 mg, 1.88 mmol), N-methyl pyrrolidone (5 mL), N,N-diisopropylethylamine (0.93 mL, 5.63 mmol) and tert-butyl ((1r,3r)-3-aminocyclobutyl)carbamate (384 mg, 2.06 mmol) were sequentially added into a 10 mL microwave tube, the reaction solution was stirred at 80°C until the solution was dissolved, and microwave reaction was carried out at 130°C for 2 h. After monitoring complete reaction by LCMS, water (20 mL) was added into the reaction solution, extracting was carried out with ethyl acetate (20 mL × 3), the combined organic layer was washed with a saturated brine, dried over anhydrous sodium sulfate, filtered and concentrated, and the resulting crude product was purified by column chromatography (petroleum ether/ethyl acetate = 1/1) to obtain methyl 3-(2-(((1r,3r)-3-((t-butyloxycarbonyl)amino)cyclobutyl)amino)-5-(trifluoromethyl)pyrimidin-4-yl)-7-(dimethylphosphoryl)-1H-indole-6-carboxylate as an off-white solid (970 mg, yield of 89%). LCMS: MS m/z (ESI): [M+H]⁺ =582.2, t_{R} = 1.89 min.

Step 2: Methyl 3-(2-(((1r,3r)-3-((t-butyloxycarbonyl)amino)cyclobutyl)amino)-5-(trifluoromethyl) pyrimidin-4-yl)-7-(dimethylphosphoryl)-1H-indole-6-carboxylate (500 mg, 0.86 mmol) was dissolved in a mixed solution of hydrogen chloride/dioxane (5 mL, 4M) and methanol (2 mL) at room temperature, and reacting was carried out at 25°C for 16 h. After monitoring complete reaction by LCMS, a saturated sodium bicarbonate solution (40 mL) was added into the reaction solution, then extracting was carried out with ethyl acetate (30 mL × 10), the combined organic layer was washed with a saturated brine, dried over anhydrous sodium sulfate, filtered and concentrated, and the resulting crude product was purified by column chromatography on silica gel (dichloromethane/methanol = 10/1) to obtain methyl 3-(2-(((1r,3r)-3-aminocyclobutyl)amino)-5-(trifluoromethyl)pyrimidin-4-yl)-7-(dimethylphosphoryl)-1H-indole-6-carboxylate as a white solid (220 mg, yield of 53%). LCMS: MS m/z (ESI): [M+H]⁺ =482.2, t_{R} = 1.35 min.

Step 3: Tert-butyl (4-bromobutyl)carbamate (157 mg, 0.62 mmol) and N,N-diisopropylethylamine (322 mg, 2.49 mmol) were sequentially added into a solution (5 mL) of methyl 3-(2-(((1r,3r)-3-aminocyclobutyl)amino)-5-(trifluoromethyl)pyrimidin-4-yl)-7-(dimethylphosphoryl)-1H-indole-6-carboxylate (300 mg, 0.62 mmol) in N,N-dimethylformamide at room temperature, and the reaction solution was stirred to react for 16 h at 80°C under nitrogen protection. After monitoring complete reaction by LCMS, a saturated ammonium chloride solution (100 mL) was added into the reaction solution, extracting was carried out with ethyl acetate (100 mL of × 3), the combined organic layer was washed with a saturated brine, dried over anhydrous sodium sulfate, filtered and concentrated to dryness. The resulting crude product was purified by column chromatography on silica gel (dichloromethane/methanol = 50/1-10/1) to obtain methyl 3-(2-(((1r,3r)-3-((4-((t-butyloxycarbonyl)amino)butyl)amino)cyclobutyl)amino)-5-(trifluoromethyl)pyrimidin-4-yl)-7-(dimethylphosphoryl)-1H-indole-6-carboxylate as a yellow solid (225 mg, yield of 55%). LCMS: MS m/z (ESI): [M+H]⁺ =653.3, t_{R} = 1.19 min.

Step 4: A solution of lithium hydroxide monohydrate (72.0 mg, 1.72 mmol) in water (2 mL) was added into a mixed solution of 3-(2-(((1r,3r)-3-((4-((t-butyloxycarbonyl)amino)butyl)amino)cyclobutyl)amino)-5-(trifluoromethyl)pyrimidin-4-yl)-7-(dimethylphosphoryl)-1H-indole-6-carboxylate (225 mg, 0.35 mmol) in tetrahydrofuran (2 mL) and methanol (2 mL), and the reaction solution was stirred at 40°C to react for 16 h. After monitoring complete reaction by LCMS, the reaction solution was acidified with a 1.0 M hydrochloric acid aqueous solution until reaching pH of 3-4, extracting was carried out with dichloromethane/methanol (10:1, 60 mL × 6), the combined organic layer was washed with a saturated brine, dried over anhydrous sodium sulfate, filtered and concentrated to obtain 3-(2-(((1r,3r)-3-((4-((tert-butyloxycarbonyl)amino)butyl)amino)cyclobutyl)amino)-5-(trifluoromethyl)pyrimidin-4-yl)-7-(dimethylphosphoryl)-1H-indole-6-carboxylic acid as a yellow solid (300 mg, yield of 95%), and the crude product was directly used in the next reaction without purification. LCMS: MS m/z (ESI): [M+H]⁺ =639.2, t_{R} = 1.40 min.

Step 5: A hydrochloric acid/methanol (5 mL, 4 M) solution was added into a solution of 3-(2-(((1r,3r)-3-((4-((tert-butyloxycarbonyl)amino)butyl)amino)cyclobutyl)amino)-5-(trifluoromethyl)pyrimidin-4-yl)-7-(dimethylphosphoryl)-1H-indole-6-carboxylic acid (300 mg, 0.33 mmol) in methanol (5 mL), and the reaction solution was stirred at 40°C to react for 16 h. After monitoring complete reaction by LCMS, the reaction solution was concentrated to dryness to obtain 3-(2-(((1r,3r)-3-((4-aminobutyl)amino)cyclobutyl)amino)-5-(trifluoromethyl)pyrimidin-4-yl)-7-(dimethylphosphoryl)-1H-indole-6-carboxylic acid as a yellow solid (270 mg, yield of 94%). The crude product was directly used in the next reaction step without purification. LCMS: MS m/z (ESI): [M+H]⁺ =639.2, t_{R} = 1.31 min.

Step 6: 2-(7-azabenzotriazole)-N,N,N',N'-tetramethyluronium hexafluorophosphate (118 mg, 0.31 mmol) and N,N-diisopropylethylamine (200 mg, 1.55 mmol) were sequentially added into a solution of 3-(2-(((1r,3r)-3-((4-aminobutyl)amino)cyclobutyl)amino)-5-(trifluoromethyl)pyrimidin-4-yl)-7-(dimethylphosphoryl)-1H-indole-6-carboxylic acid (270 mg, 0.31 mmol) in N,N-dimethylformamide (5 mL), and the reaction solution was stirred at 70°C for 16 h. After monitoring complete reaction by LCMS, the reaction solution was concentrated until realizing dryness, and reversed-phase preparation was carried out on the resulting crude product (70 mL/min, 25°C, Ultimate XB-C18, 50× 250 mm, 10 um (PARP-02) and acetonitrile-water (0.1% trifluoroacetic acid)) to obtain (4¹r,4³r)-1⁷-(dimethylphosphoryl)-2⁵-(trifluoromethyl)-1¹H-3,5,10-triaza-1(3,6)-indola-2(4,2)-pyrimidina-4(1,3)-cyclobutanacycloundecaphan-11-one (Z8) trifluoroacetate (3.9 mg, yield of 2.4%). LCMS: MS m/z (ESI): [M+H]⁺ =551.3, t_{R} = 1.05 min. ¹H NMR (400 MHz, DMSO-*d₆*): δ 11.94 (s, 1H), 8.79 (d, *J=* 8.0 Hz, 1H), 8.56 (s, 1H), 8.29 (d, *J=* 4.4 Hz, 1H), 8.07 - 7.92 (m, 2H), 7.42 - 7.32 (m, 1H), 4.82 - 4.68 (m, 1H), 4.05 (s, 1H), 2.86 - 2.78 (m, 2H), 2.36 - 2.28 (m, 2H), 2.26 - 2.18 (m, 2H), 2.08 - 1.94 (m, 3H), 1.91 (s, 3H), 1.87 (s, 3H), 1.76 - 1.68 (m, 2H), 1.56 - 1.48 (m, 2H).

### Example 9 Synthesis of compound Z9

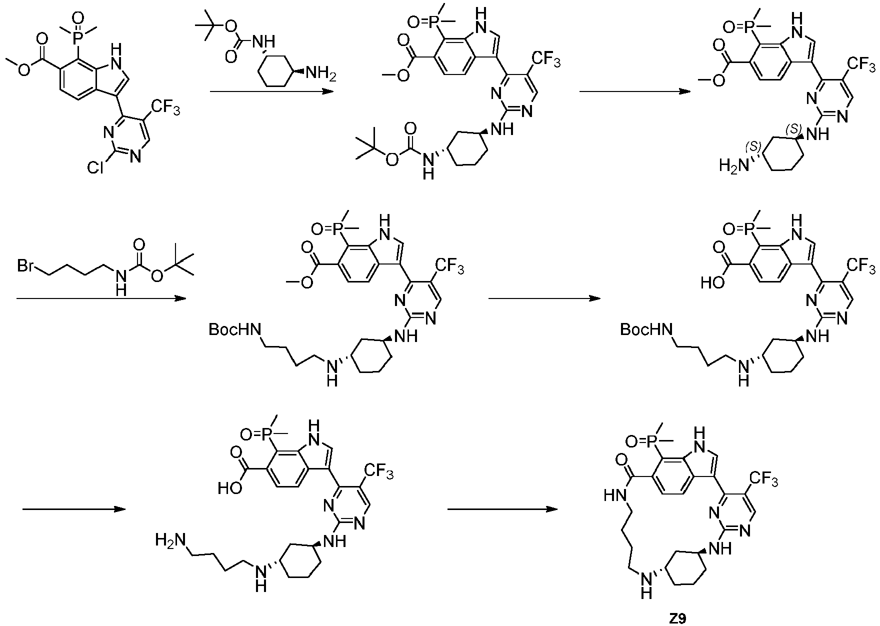

Step 1: Methyl 3-(2-chloro-5-(trifluoromethyl) pyrimidin-4-yl)-7-(dimethylphosphoryl)-1H-indole-6-carboxylate (450 mg, 1.04 mmol), N-methyl pyrrolidone (5 mL), N,N-diisopropylethylamine (0.52 mL, 3.13 mmol) and tert-butyl ((1S,3S)-3-aminocyclohexyl)carbamate (235 mg, 1.09 mmol) were sequentially added into a 10 mL microwave tube, the reaction solution was stirred at 80°C until the solution is dissolved, and microwave reaction was carried out at 130°C for 2 h. After monitoring complete reaction by LCMS, water (20 mL) was added into the reaction solution, then extracting was carried out with ethyl acetate (20 mL × 3), the combined organic layer was washed with a saturated brine, dried over anhydrous sodium sulfate, filtered and concentrated, and the resulting crude product was purified by column chromatography (petroleum ether/ethyl acetate = 1/1) to obtain methyl 3-(2-(((1S,3 S)-3-((t-butyloxycarbonyl)amino)cyclohexyl)amino)-5-(trifluoromethyl)pyrimidin-4-yl)-7-(dimethylphosphoryl)-1H-indole-6-carboxylate as an off-white solid (470 mg, yield of 74%). LCMS: m/z (ESI): [M+H]⁺ =610.2, t_{R} = 1.98 min.

Step 2: Methyl 3-(2-(((1S,3S)-3-((t-butyloxycarbonyl)amino)cyclohexyl)amino)-5-(trifluoromethyl)pyrimidin-4-yl)-7-(dimethylphosphoryl)-1H-indole-6-carboxylate (470 mg, 0.77 mmol) was dissolved in a mixed solution of hydrogen chloride/dioxane (5 mL, 4M) and methanol (2 mL) at room temperature, and reacting was carried out at 25°C for 16 h. After monitoring complete reaction by LCMS, a saturated sodium bicarbonate solution (40 mL) was into the reaction solution, then extracting was carried out with ethyl acetate (30 mL × 10), the combined organic layer was washed with a saturated brine, dried over anhydrous sodium sulfate, filtered and concentrated, and the resulting crude product was purified by column chromatograph on silica gel (dichloromethane/methanol = 10/1) to obtain methyl 3-(2-(((1S,3S)-3-aminocyclohexyl)amino)-5-(trifluoromethyl)pyrimidin-4-yl)-7-(dimethylphosphoryl)-1H-indole-6-carboxylate as a white solid (310 mg, yield of 79%). LCMS: m/z (ESI): [M+H]⁺ =510.5, t_{R} = 1.42 min.

Step 3: Methyl 3-(2-(((1S,3S)-3-aminocyclohexyl)amino)-5-(trifluoromethyl)pyrimidin-4-yl)-7-(dimethylphosphoryl)-1H-indole-6-carboxylate (310 mg, 0.61 mmol) was dissolved in N,N-dimethylformamide (3 mL), tert-butyl (4-bromobutyl)carbamate (153 mg, 0.61 mmol) and N,N-diisopropylethylamine (0.67 mL, 4.0 mmol) were sequentially added, reacting was carried out, and stirring was carried out at 80°C for 16 h. After monitoring complete reaction by LCMS, spin-drying was carried out, and purifying was carried out by column chromatography (dichloromethane/methanol = 100/7) to obtain methyl 3-(2-(((1S,3S)-3-((4-((t-butyloxycarbonyl)amino)butyl)amino)cyclohexyl)amino)-5-(trifluoromethyl)pyrimidin-4-yl)-7-(dimethylphosphoryl)-1H-indole-6-carboxylate as a yellow-like solid (200 mg, yield of 48%). LCMS: m/z (ESI): [M+H]⁺ =681.2, t_{R} = 1.60 min.

Step 4: Methyl 3-(2-(((1S,3S)-3-((4-((t-butyloxycarbonyl)amino)butyl)amino)cyclohexyl)amino)-5-(trifluoromethyl)pyrimidin-4-yl)-7-(dimethylphosphoryl)-1H-indole-6-carboxylate (200 mg, 0.29 mmol) was dissolved in a mixed system of water (1 mL), methanol (1 mL) and tetrahydrofuran (1 mL), then lithium hydroxide monohydrate (61.6 mg, 1.47 mmol) was added, and reacting was carried out while stirring at 40°C for 16 h. After monitoring complete reaction by LCMS, a solvent was spin-dried to obtain 3-(2-(((1S,3S)-3-((4-((t-butyloxycarbonyl)amino)butyl)amino)cyclohexyl)amino)-5-(trifluoromethyl)pyrimidin-4-yl)-7-(dimethylphosphoryl)-1H-indole-6-carboxylic acid as a yellow solid crude product (180 mg, yield of 92%), and the product was directly used in the next reaction without purification. LCMS: m/z (ESI): [M+H]⁺ =667.2, t_{R} = 1.43 min.

Step 5: 3-(2-(((1S,3 S)-3-((4-((t-butyloxycarbonyl)amino)butyl)amino)cyclohexyl)amino)-5-(trifluoromethyl)pyrimidin-4-yl)-7-(dimethylphosphoryl)-1H-indole-6-carboxylic acid (180 mg, 0.27 mmol) was dissolved in hydrogen chloride/methanol (5 mL, 4M), and the system was stirred at 15°C for 4 h. After monitoring complete reaction by LCMS, the solvent was directly spin-dried to obtain 3-(2-(((1S,3 S)-3-((4-aminobutyl)amino)cyclohexyl)amino)-5-(trifluoromethyl)pyrimidin-4-yl)-7-(dimethylphosphoryl)-1H-indole-6-carboxylic acid as a yellow solid (120 mg, yield of 78%), and the product was directly used in the next reaction without purification. LCMS: m/z (ESI): [M+H]⁺ =567.2, t_{R} = 1.43 min.

Step 6: 2-(7-azabenzotriazole)-N,N,N',N'-tetramethyluronium hexafluorophosphate (80.4 mg, 0.21 mmol) and N,N-diisopropylethylamine (0.14 mL, 0.84 mmol) were sequentially added into a solution of 3-(2-(((1S,3 S)-3-((4-aminobutyl)amino)cyclohexyl)amino)-5-(trifluoromethyl)pyrimidin-4-yl)-7-(dimethylphosphoryl)-1H-indole-6-carboxylic acid (120 mg, 0.21 mmol) in N,N-dimethylformamide (10 mL), and the reaction solution was stirred at 60°C for 16 h. After monitoring complete reaction by LCMS, the reaction solution was concentrated, and reversed-phase preparation was carried out on the resulting crude product (chromatographic column: Ultimate SFC18, 50× 250 mm, 10 um (PARP-02); mobile phase: acetonitrile-water (0.1% trifluoroacetic acid); flow rate: 70 mL/min; column temperature: 25°C) to obtain (4¹S,4³S)-1⁷-(dimethylphosphoryl)-2⁵-(trifluoromethyl)-1¹H-3,5,10-triaza-1(3,6)-indola-2(4,2)-pyrimidina-4(1,3)-cyclohexanacycloundecaphan-11-one (Z9) trifluoroacetate (8.2 mg, yield of 5.8%). LCMS: m/z (ESI): [M+H]⁺ =549.2, t_{R} = 0.72 min. ¹H NMR (400 MHz, DMSO-*d₆*): δ 11.87 (d, 1H), 8.62 (s, 1H), 8.27 - 8.09 (m, 2H), 7.97 (m, 2H), 7.88 - 7.76 (m, 2H), 7.29 (m, 1H), 4.05 (m, 1H), 2.83 (m, 3H), 2.71 - 2.58 (m, 2H), 2.36 (m, 1H), 1.88 (m, 8H), 1.58 (m, 9H).

### Example 10 Synthesis of compound Z10

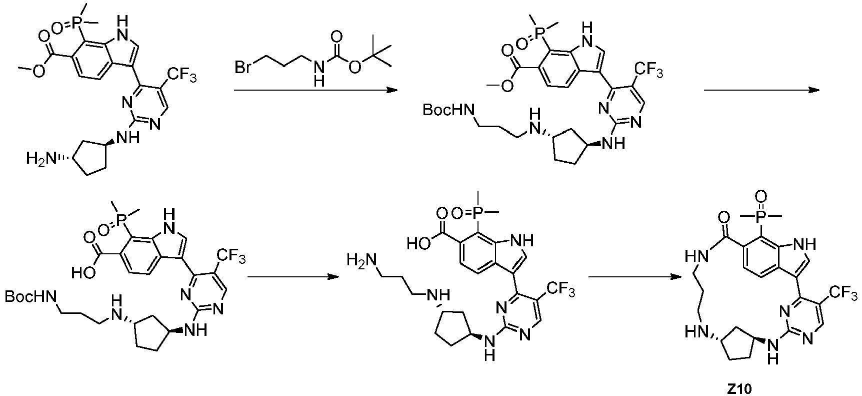

Step 1: 3-(tert-butyloxycarbonylamino)propyl bromide (144 mg, 0.61 mmol) and N,N-diisopropylethylamine (313 mg, 2.42 mmol) were sequentially added into a solution (3 mL) of methyl 3-(2-(((1S,3 S)-3-aminocyclopentyl)amino)-5-(trifluoromethyl)pyrimidin-4-yl)-7-(dimethylphosphoryl)-1H-indole-6-carboxylate (300 mg, 0.61 mmol) in N,N-dimethylformamide, and the reaction solution was reacted under nitrogen protection at 80°C for 16 h. After monitoring complete reaction by LCMS, a 10% ammonium chloride aqueous solution (50 mL) was added, extracting was carried out with ethyl acetate (50 mL × 3), the combined organic layer was washed with a saturated brine, dried over anhydrous sodium sulfate, filtered and concentrated. The resulting crude product was purified by column chromatography on silica gel (dichloromethane/methanol = 40/1-15/1) to obtain methyl 3-(2-(((1S,3 S)-3-(3-(tert-butyloxycarbonylaminopropyl)amino)cyclopentyl)amino)-5-(trifluoromethyl)pyrimidin-4-yl)-7-(dimethylphosphoryl)-1H-indole-6-carboxylate as a yellow solid (100 mg, yield of 25%). LCMS: MS m/z (ESI): [M+H]⁺ =653.6, t_{R} = 1.53 min.

Step 2: A solution of lithium hydroxide monohydrate (33 mg, 0.78 mmol) in water (1.5 mL) was added into a mixed solution of methyl 3-(2-(((1S,3 S)-3-(3-(tert-butyloxycarbonylaminopropyl)amino)cyclopentylamino)-5-(trifluoromethyl)pyrimidin-4-yl)-7-(dimethylphosphoryl)-1H-indole-6-carboxylate (100 mg, 0.16 mmol) in tetrahydrofuran (1.5 mL) and methanol (1.5 mL), and the reaction solution was stirred at 40°C to react for 16 h. After monitoring complete reaction by LCMS, the reaction solution was acidified with a 1.0 M hydrochloric acid aqueous solution until reaching pH of 3-4, extracting was carried out with dichloromethane (10 mL × 3), the combined organic layer was washed with a saturated brine, dried over anhydrous sodium sulfate, filtered and concentrated to obtain 3-(2-(((1S,3S)-3-((3-(tert-butyloxycarbonyl)amino)propyl)amino)cyclopentyl)amino)-5-(trifluoromethyl)pyrimidin-4-yl)-7-(dimethylphosphoryl)-1H-indole-6-carboxylic acid as a white solid (90 mg, yield of 92%), the product was directly used in the next reaction without purification. LCMS: MS m/z (ESI): [M+H]⁺ =639.2, t_{R} = 1.10 min.

Step 3: A hydrochloric acid/methanol (6 mL, 4 N) solution was added into a solution of 3-(2-(((1S,3S)-3-((3-(tert-butyloxycarbonyl)amino)propyl)amino)cyclopentyl)amino)-5-(trifluoromethyl)pyrimidin-4-yl)-7-(dimethylphosphoryl)-1H-indole-6-carboxylic acid (90 mg, 0.14 mmol) in methanol (3 mL) at 25°C, and the reaction solution was stirred at 40°C to react for 2 h. After monitoring complete reaction by LCMS, the reaction solution was concentrated to obtain 3-(2-(((1S,3S)-3-((3-aminopropyl)amino)cyclopentyl)amino)-5-(trifluoromethyl)pyrimidin-4-yl)-7-(dimethylphosphoryl)-1H-indole-6-carboxylic acid as a white solid (70 mg, yield of 93%). LCMS: MS m/z (ESI): [M+H]⁺ =539.6, t_{R} = 1.33 min.

Step 4: 2-(7-azabenzotriazole)-N,N,N',N'-tetramethyluronium hexafluorophosphate (80 mg, 0.21 mmol) and N,N-diisopropylethylamine (300 mg, 2.32 mmol) were sequentially added into a solution of 3-(2-(((1S,3S)-3-((3-aminopropyl)amino)cyclopentyl)amino)-5-(trifluoromethyl)pyrimidin-4-yl)-7-(dimethylphosphoryl)-1H-indole-6-carboxylic acid (65 mg, 0.12 mmol) in N,N-dimethylformamide (8 mL), and the reaction solution was stirred at 80°C for 16 h. After monitoring complete reaction by LCMS, the reaction solution was concentrated, and the resulting crude product was purified by reversed-phase preparation (chromatographic column: Ultimate XBC18, 50× 250 mm, 10 um (PARP-06); mobile phase: acetonitrile-water (0.1% trifluoroacetic acid); flow rate: 70 mL/min; room temperature: 25°C) to obtain (4¹S, 4³S)-1⁷-(dimethylphosphoryl)-2⁵-(trifluoromethyl)-1¹H-3,5,9-triaza-1(3,6)-indola-2(4,2)-pyrimidina-4(1,3)-cyclopentanacyclodecaphan-10-one (Z10) trifluoroacetate (1.6 mg, yield of 3%). LCMS: MS m/z (ESI): [M+H]⁺ =521.2, t_{R} = 1.26 min.

### Example 11 Synthesis of compound Z11

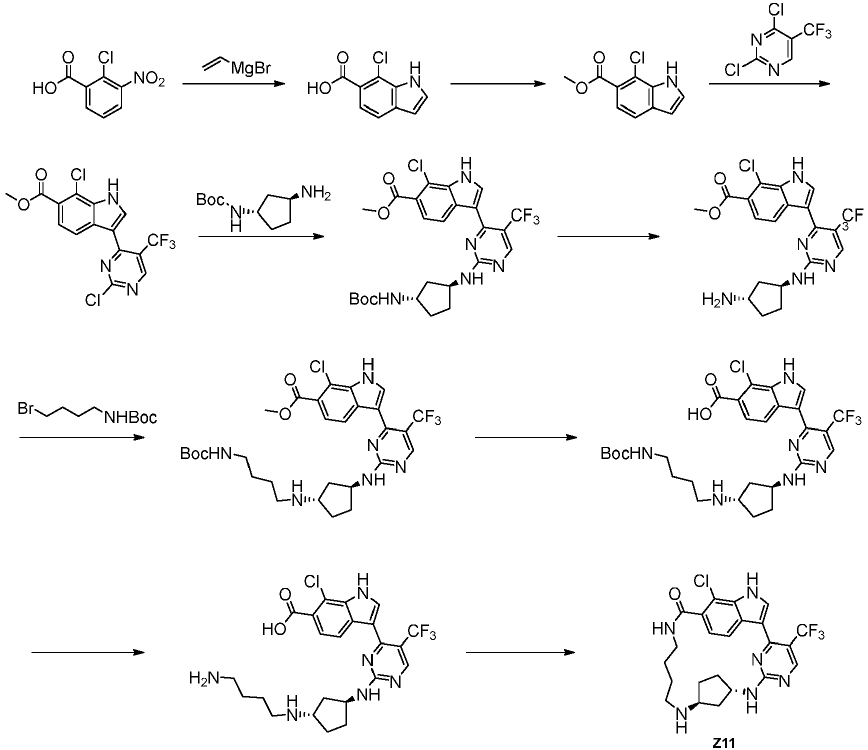

Step 1: A solution (198 mL, 198 mmol, 1 M) of vinyl magnesium bromide in tetrahydrofuran was slowly dropwise added into a solution of 2-chloro-3-nitrobenzoic acid (10.0 g, 49.6 mmol) in tetrahydrofuran (120 mL) at-78°C, and stirring was continued at -78 °C to react for 3 h under nitrogen protection after the dropwise adding. After realizing complete reaction by LCMS detection, a saturated ammonium chloride aqueous solution (300 mL) was slowly added into the reaction solution for quenching, the pH was adjusted to 4-5 with 1 M hydrochloric acid, and then extracting was carried out with dichloromethane (300 mL X3). Combined organic layers were washed with a saturated brine, dried over anhydrous sodium sulfate, filtered, and concentrated to obtain a brown yellow solid crude product 7-chloro-1H-indole-6-carboxylic acid (8.03 g, yield of 83%), and the product was directly used in the next reaction without purification. LCMS: m/z (ESI): [M+H]⁺ =196.0, t_{R} = 1.53 min.

Step 2: Iodomethane (6.97 g, 49.1 mmol) and potassium carbonate (11.3 g, 81.9 mmol) were added into a solution of 7-chloro-1H-indole-6-carboxylic acid (8.00 g, 40.9 mmol) in N,N-dimethylformamide (85 mL) at 25°C. The reaction solution was stirred at 25 °C for 16 h under nitrogen protection. After realizing complete reaction by TLC detection, water (200 mL) was added into the reaction solution, and then extracting was carried out with ethyl acetate (150 mL X3). Combined organic layers were washed with a saturated brine, dried over anhydrous sodium sulfate, and filtered and concentrated. The resulting crude product was purified with silica gel column chromatography (petroleum ether/ethyl acetate = 10/1) to obtain a yellow solid methyl 7-chloro-1H-indole-6-carboxylate (5.8 g, and yield of 67%). LCMS: m/z (ESI): [M+H]⁺ =210.1, t_{R} = 1.45 min. ¹H NMR (400 MHz, CDCl₃): δ 8.67 (s, 1H), 7.72 (d, *J=* 8.4 Hz, 1H), 7.55 (d, *J=* 8.4 Hz, 1H), 7.40 (m, 1H), 6.62 (dd, *J* = 3.2, 2.0 Hz, 1H), 3.95 (s, 3H).

Step 3: Aluminum trichloride (1.27 g, 9.54 mmol) was added into a 50 mL dried three-neck flask, a solution of methyl 7-chloro-1H-indole-6-carboxylate (1.00 g, 4.77 mmol) in 1.2-dichloroethane (10 mL) was added under nitrogen protection, the system was stirred at 80°C for 30 min, then a solution of 2,4-dichloro-5-(trifluoromethyl)pyrimidine (1.09 g, 5.01 mmol) in 1.2-dichloroethane (1 mL) was added, and then the reaction solution was reacted at 80°C for 16 h. After realizing complete reaction by TLC detection, water (30 mL) was added into the reaction solution, and then extracting was carried out with ethyl acetate (30 mL X3). Combined organic layers were washed with a saturated brine, dried over anhydrous sodium sulfate, filtered and concentrated. The resulting crude product was purified with column chromatography (petroleum ether/ethyl acetate = 3/5) to obtain a white solid methyl 7-chloro-3-(2-chloro-5-(trifluoromethyl)pyrimidin-4-yl)-1H-indole-6-carboxylate (0.38 g, yield of 20%). LCMS: m/z (ESI): [M+H]⁺ =392.0, t_{R} = 1.70 min. ¹H NMR (400 MHz, DMSO-*d₆*): δ 12.86 (s, 1H), 9.10 (s, 1H), 8.25 (d, *J* = 8.4 Hz, 1H), 8.08 (d, *J* = 2.4 Hz, 1H), 7.73 (d, *J* = 8.4 Hz, 1H), 3.91 (s, 3H).

Step 4: Methyl 7-chloro-3-(2-chloro-5-(trifluoromethyl)pyrimidin-4-yl)-1H-indole-6-carboxylate (420 mg, 1.08 mmol), N-methyl pyrrolidone (2 mL), N,N-diisopropylethylamine (0.53 mL, 3.23 mmol) and tert-butyl ((1S,3S)-3-aminocyclopentyl)carbamate (237 mg, 1.18 mmol) were sequentially added into a 10 mL microwave tube, the reaction solution was stirred at 80°C for dissolving, and then microwave reaction was carried out at 130°C for 2 h. After realizing complete reaction by LCMS detection, water (20 mL) was added into the reaction solution, and then extracting was carried out with ethyl acetate (20 mL X3). Combined organic layers were washed with a saturated brine, dried over anhydrous sodium sulfate, filtered and concentrated. The resulting crude product was purified with column chromatography (petroleum ether/ethyl acetate = 1/1) to obtain an off-white solid methyl 3-(2-(((1S,3S)-3-((t-butyloxycarbonyl)amino)cyclopentyl)amino)-5-(trifluoromethyl)pyrimidin-4-yl)-7-chloro-1H-indole-6-carboxylate(430 mg, yield of 72%). LCMS: m/z (ESI): [M+H]⁺ =554.0, t_{R} = 2.05 min.

Step 5: Methyl 3-(2-(((1S,3S)-3-((t-butyloxycarbonyl)amino)cyclopentyl)amino)-5-(trifluoromethyl)pyrimidin-4-yl)-7-chloro-1H-indole-6-carboxylate(430 mg, 0.72 mmol) was dissolved in a mixed solution of hydrogen chloride/dioxane (4 mL, 4 M) and methanol (2 mL) at room temperature, and reacting was carried out at 15°C for 16 h. After realizing complete reaction by LCMS detection, a saturated sodium bicarbonate solution (40 mL) was added into the reaction solution, and then extracting was carried out with dichloromethane (20 mL X3). Combined organic layers were washed with a saturated brine, dried over anhydrous sodium sulfate, filtered and concentrated. The resulting crude product was purified with column chromatography (dichloromethane/methanol = 30: 1) to obtain a white solid methyl 3-(2-(((1S,3S)-3-aminocyclopentyl)amino)-5-(trifluoromethyl)pyrimidin-4-yl)-7-chloro-1H-indole-6-carboxylate (320 mg, yield of 89%). LCMS: m/z (ESI): [M+H]⁺ =454.0, t_{R} = 1.38 min.

Step 6: Methyl 3-(2-(((1S,3S)-3-aminocyclopentyl)amino)-5-(trifluoromethyl)pyrimidin-4-yl)-7-chloro-1H-indole-6-carboxylate (320 mg, 0.71 mmol) was dissolved in N,N-dimethylformamide (2 mL), tert-butyl (4-bromobutyl)carbamate (178 mg, 0.71 mmol) and N,N-diisopropylethylamine (0.58 mL, 3.53 mmol) were sequentially added and reacted, and stirring was carried out at 80°C for 16 h. After realizing complete reaction by LCMS detection, spin-drying and purification with column chromatography (dichloromethane/methanol = 100/7) were carried out to obtain a yellow solid methyl 3-(2-(((1S,3S)-3-((4-((t-butyloxycarbonyl)amino)butyl)amino)cyclopentyl)amino)-5-(trifluoromethyl)pyrimidin-4-yl)-7-chloro-1H-indole-6-carboxylate (230 mg, yield of 52%). LCMS: m/z (ESI): [M+H]⁺ =625.4, t_{R} = 1.07 min.

Step 7: Methyl 3-(2-(((1S,3S)-3-((4-((t-butyloxycarbonyl)amino)butyl)amino)cyclopentyl)amino)-5-(trifluoromethyl)pyrimidin-4-yl)-7-chloro-1H-indole-6-carboxylate (230 mg, 0.37 mmol) was dissolved in a mixed system of water (1 mL), methanol (1 mL) and tetrahydrofuran (1 mL), then lithium hydroxide monohydrate (77.2 mg, 1.84 mmol) was added to react, and stirring was carried out at 40°C for 16 h. After realizing complete reaction by LCMS detection, a solvent was spin-dried to obtain a yellow solid crude product 3-(2-(((1S,3S)-3-((4-((t-butyloxycarbonyl)amino)butyl)amino)cyclopentyl)amino)-5-(trifluoromethyl)pyrimidin-4-yl)-7-chloro-1H-indole-6-carboxylic acid (lithium salt, 340 mg, yield of 91%), and the product was directly used in the next reaction without purification. LCMS: m/z (ESI): [M+H]⁺ =611.4, t_{R} = 0.98 min.

Step 8: 3-(2-(((1S,3 S)-3-((4-((t-butyloxycarbonylamino)pentylamino)cyclopentyl)amino)-5-(trifluoromethyl)pyrimidin-4-yl)-7-(dimethylphosphoryl)-1H-indole-6-carboxylic acid (lithium salt, 340 mg) was dissolved in hydrogen chloride/methanol (5 mL, 4M), and the system was stirred at 25°C for 4 h. After realizing complete reaction by LCMS detection, the solvent was spin-dried to obtain a brown solid 3-(2-(((1S,3S)-3-((4-aminobutyl)amino)cyclopentyl)amino)-5-(trifluoromethyl)pyrimidin-4-yl)-7-chloro-1H-indole-6-carboxylic acid (containing lithium chloride, 300 mg, yield of 88%), and the product was directly used in the next reaction without purification. LCMS: m/z (ESI): [M+H]⁺ =511.2, t_{R} = 0.61 min.

Step 9: 2-(7-azabenzotriazol)-N,N,N',N'-tetramethyluronium hexafluorophosphate (108 mg, 0.28 mmol) and N,N-diisopropylethylamine (0.24 mL, 1.42 mmol) were sequentially added into a solution of 3-(2-(((1S,3S)-3-((4-aminobutyl)amino)cyclopentyl)amino)-5-(trifluoromethyl)pyrimidin-4-yl)-7-chloro-1H-indole-6-carboxylic acid (containing lithium chloride, 290 mg, 0.28 mmol) in N,N-dimethylformamide (10 mL), and the reaction solution was stirred at 60°C for 16 h. After realizing complete reaction by LCMS detection, the reaction solution was concentrated, and the resulting crude product was purified with reversed-phase preparation (chromatographic column: Ultimate XB-C18, 50X250 mm, 10 um (PARP-02); mobile phase: acetonitrile-water (0.1% trifluoroacetic acid); flow rate: 70 mL/min; column temperature: 25°C) to obtain (4¹S,4³S)-1⁷-chloro-2⁵-(trifluoromethyl)-1¹H-3,5,10-triaza-1(3,6)-indola-2(4,2)-pyrimidina-4(1,3)-cyclopentanacycloundecaphan-11-one (Z11) trifluoroacetate (30.8 mg, yield of 22%). LCMS: m/z (ESI): [M+H]⁺ =493.2, t_{R} = 1.27 min. ¹H NMR (400 MHz, DMSO-*d₆*): δ 12.33 (s, 1H), 8.64 (s, 1H), 8.43 (s, 1H), 8.34 - 8.04 (m, 3H), 7.99 (s, 1H), 7.78 (s, 1H), 7.26 (d, *J=* 8.4 Hz, 1H), 3.60 (s, 2H), 2.88 (s, 1H), 2.55 (s, 1H), 2.47 - 2.38 (m, 1H), 2.30 - 1.95 (m, 3H), 1.83 - 1.41 (m, 6H), 1.30 - 1.25 (m, 2H).

### Example 12 Synthesis of compound Z12

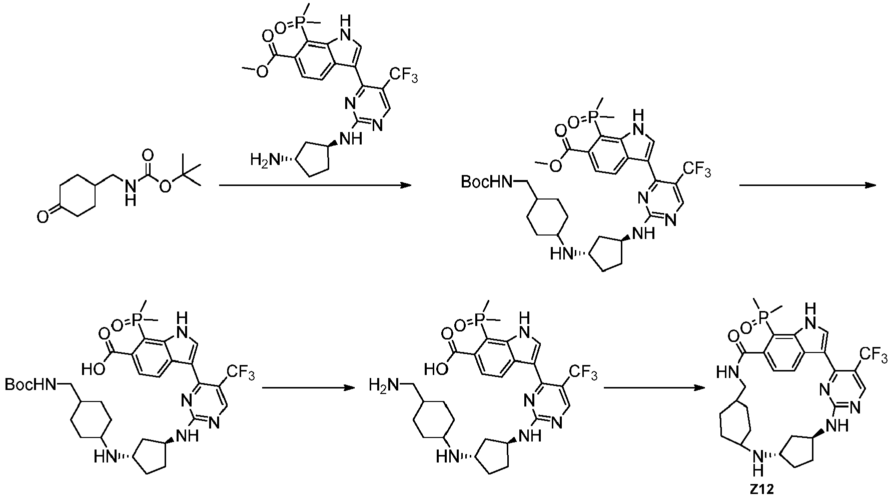

Step 1: Tert-butyl ((4-oxocyclohexyl)methyl)carbamate (414 mg, 1.82 mmol) and glacial acetic acid (0.1 mL) were sequentially added into a solution (10 mL) of methyl 3-(2-((1S,3S)-3-aminocyclopentyl)amino)-5-(trifluoromethyl)pyrimidin-4-yl)-7-(dimethylphosphoryl)-1H-indole-6-carboxylate (750 mg, 1.52 mmol) in methanol, and the reaction solution was stirred at 25°C to react under nitrogen protection for 16 h. Then sodium cyanoborohydride (287 mg, 4.56 mmol) was added, and the reaction solution was stirred to react at 25°C for 2 h. After realizing complete reaction by LCMS detection, a saturated ammonium chloride solution (20 mL) was added into the reaction solution, and extracting was carried out with ethyl acetate (30 mL X3). Combined organic layers were washed with a saturated brine, dried over anhydrous sodium sulfate, filtered and concentrated realize dryness. The resulting crude product was purified with reversed-phase preparation (chromatographic column: Ultimate XB-C18, 50X250 mm, 10 um (PARP-02); mobile phase: acetonitrile-water (0.1% trifluoroacetic acid), flow rate: 70 mL/min and column temperature: 25°C) to obtain a white solid methyl 3-(2-((1S,3S)-3-((t-butyloxycarbonyl)amino)methylcyclohexylamino)cyclopentylamino)-5-(trifluoromethyl)pyrimidin-4-yl)-7-(dimethylphosphoryl)-1H-indole-6-carboxylate (P1: 340 mg, yield of 32%). LCMS: MS m/z (ESI): 707.4 [M+H]⁺, t_{R} = 7.43 min. Methyl 3-(2-((1S,3S)-3-((t-butyloxycarbonyl)amino)methylcyclohexylamino)cyclopentylamino)-5-(trifluoromethyl)pyrimidin-4-yl)-7-(dimethylphosphoryl)-1H-indole-6-carboxylate (P2: 340 mg, yield of 32%). LCMS: MS m/z (ESI): 707.4 [M+H]⁺, t_{R} = 7.53 min.

Step 2: A solution of lithium hydroxide monohydrate (101 mg, 2.40 mmol) in water (3 mL) was added into a mixed solution of methyl 3-(2-((1S,3S)-3-((t-butyloxycarbonyl)amino)methyl)cyclohexyl)amino)cyclopentyl)amino)-5-(trifluoromethyl)pyrimidin-4-yl)-7-(dimethylphosphoryl)-1H-indole-6-carboxylate (P2: 340 mg, 0.48 mmol) in tetrahydrofuran (3 mL) and methanol (3 mL), and the reaction solution was stirred to react at 40°C overnight. After realizing complete reaction by LCMS detection, the reaction solution was acidified with 1.0 M hydrochloric acid aqueous solution until reaching pH of 3-4, and extracting was carried out with dichloromethane (10 mL X3). Combined organic layers were washed with a saturated brine, dried over anhydrous sodium sulfate, filtered and concentrated to obtain a white solid 3-(2-(((1S,3S)-3-((4-(((t-butyloxycarbonyl)amino)methyl)cyclohexyl)amino)cyclopentyl)amino)-5-(trifluoromethyl)pyrimidin-4-yl)-7-(dimethylphosphoryl)-1H-indole-6-carboxylic acid (300 mg, yield of 90%), and the crude product was directly used in the next reaction without purification. LCMS: MS m/z (ESI): [M+H]⁺ =692.6, t_{R} = 1.14 min.

Step 3: A hydrochloric acid/dioxane (3 mL, 4 N) solution was added into a solution of 3-(2-(((1S,3S)-3-((4-(((t-butyloxycarbonyl)amino)methyl)cyclohexyl)amino)cyclopentyl)amino)-5-(trifluoromethyl)pyrimidin-4-yl)-7-(dimethylphosphoryl)-1H-indole-6-carboxylic acid (300 mg, 0.43 mmol) in methanol (3 mL), and the reaction solution was stirred to react at 25°C for 16 h. After realizing complete reaction by LCMS detection, the reaction solution was concentrated, and reversed-phase preparation was carried out (chromatographic column: Ultimate XB-C18, 50X250 mm, 10 um (PARP-02); mobile phase: acetonitrile-water (0.1% trifluoroacetic acid), flow rate: 70 mL/min and column temperature: 25°C) to obtain a white solid 3-(2-(((1S,3S)-3-((4-(aminomethyl)cyclohexyl)amino)cyclopentyl)amino)-5-(trifluoromethyl)pyrimidin-4-yl)-7-(dimethylphosphoryl)-1H-indole-6-carboxylic acid (140 mg, yield of 55%). LCMS: MS m/z (ESI): [M+H]⁺ =592.8, t_{R} = 0.94 min.

Step 4: 2-(7-azabenzotriazol)-N,N,N',N'-tetramethyluronium hexafluorophosphate (19 mg, 0.05 mmol) and N,N-diisopropylethylamine (32 mg, 0.25 mmol) were sequentially added into a solution of 3-(2-(((1S,3S)-3-((4-(aminomethyl)cyclohexyl)amino)cyclopentyl)amino)-5-(trifluoromethyl)pyrimidin-4-yl)-7-(dimethylphosphoryl)-1H-indole-6-carboxylic acid (30 mg, 0.05 mmol) in N,N-dimethylformamide (5 mL), and the reaction solution was stirred at 70°C for 14 h. After realizing complete reaction by LCMS detection, the reaction solution was concentrated, and reversed-phase preparation was carried out on the resulting crude product (chromatographic column: Ultimate XBC18, 50X250 mm, 10 um (PARP-06); mobile phase: acetonitrile-water (0.1% trifluoroacetic acid), flow rate: 70 mL/min and column temperature: 25°C) to obtain (4¹S,4³S)-1⁷-(dimethylphosphoryl)-2⁵-(trifluoromethyl)-1¹H-3,5,8-triaza-1(3,6)-indola-2(4,2)-pyrimidina-6(1,4)-cyclohexana-4(1,3)-cyclopentanacyclononaphan-9-one (Z12) trifluoroacetate (2.0 mg, yield of 9%). LCMS: MS m/z (ESI): [M+H]⁺ =575.3, t_{R} = 1.55 min. ¹H NMR (400 MHz, CD₃OD): δ 11.44 (s, 1H), 8.63 (s, 1H), 7.94 (d, *J* = 6.8 Hz, 1H), 7.80 (s, 1H), 7.21 (dd, *J* = 8.0, 3.2 Hz, 1H), 3.94 - 3.68 (m, 2H), 3.17 - 2.94 (m, 3H), 2.68-2.63 (m, 1H), 2.48-2.41 (m, 1H), 2.28 (d, *J* = 12.8 Hz, 1H), 2.16-2.11 (m, 1H), 2.02-1.97 (m, 8H), 1.94-1.85 (m, 1H), 1.78 - 1.53 (m, 6H), 1.37-1.29 (m, 1H), 1.00-0.85 (m, 1H).

### Example 13 Synthesis of compound Z13

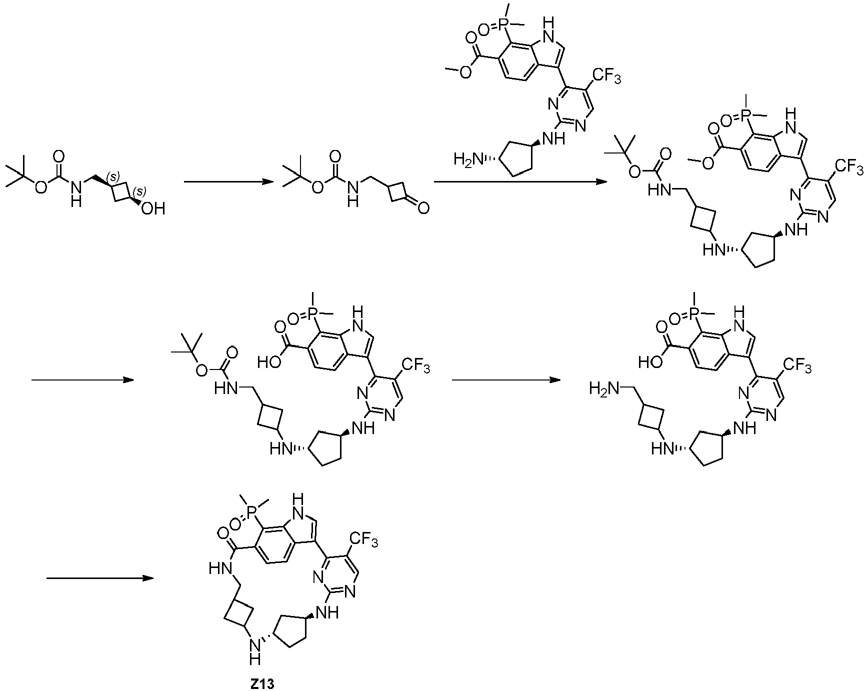

Step 1: A Dess-Martin reagent (843 mg, 2.0 mmol) was added into a solution of tert-butyl ((1S,3S)-3-hydroxycyclobutyl)(methyl)carbamate (200 mg, 1.0 mmol) in dichloromethane (4 mL) at 25°C under nitrogen protection, and the reaction solution was stirred to react at 25°C under nitrogen protection for 3 h. After realizing complete reaction by LCMS detection, the reaction solution was extracted with water (10 mL) and ethyl acetate (20 mL X3). Combined organic layers were washed with a saturated brine (10 mL X2), dried over anhydrous sodium sulfate, filtered and concentrated to dryness. The resulting crude product was purified with silica gel column chromatography (petroleum ether/ethyl acetate = 10/1-5/1) to obtain a white solid {[(3-oxocyclobutyl)methyl]amino}methanoic acid-2-methylpropyl-2-yl ester (170 mg, yield of 86%). LCMS: m/z (ESI): [M+H-56]⁺ =144.2, t_{R} = 1.58 min.

Step 2: Acetic acid (20 mg, 0.33 mmol) was added into a solution of methyl 7-[dimethyl(oxo)-λ⁵-phosphoranyl]-3-(2-{[(1S,3S)-3-aminocyclopentyl]amino}-5-(trifluoromethyl)pyrimidin-4-yl)-1H-indole-6-carboxylate (423 mg,0.85 mmol) and {[(3-oxocyclobutyl)methyl]amino}methanoic acid-2-methylpropyl-2-yl ester (170 mg, 0.85 mmol) in methanol (3 mL) at 25°C under nitrogen protection, and reacting was carried out while stirring at 25°C for 16 h under nitrogen protection. Sodium cyanoborohydride (268 mg, 4.26 mmol) was added into the reaction solution, and reacting continued at 25°C for 6 h under nitrogen protection. Complete reaction was realized by LCMS detection. The reaction solution was extracted with water (10 mL) and ethyl acetate (30 mL X3). Combined organic layers were washed with a saturated brine (10 mL X2), dried over anhydrous sodium sulfate, filtered and concentrated to dryness. The resulting crude product was purified with silica gel column chromatography (dichloromethane/methanol = 60/1-30/1) to obtain a white solid {[(3-{[(1S,3S)-3-[(4-{7-[dimethyl(oxo)-λ⁵-phosphoranyl]-6-(methoxycarbonyl)-1H-indole-3-yl}-5-(trifluoromethyl)pyrimidin-2-yl)amino]cyclopentyl]amino}cyclobutyl)methyl]amino}methanoic acid-2-methylpropyl-2-yl ester (380 mg, yield of 67%). LCMS: m/z (ESI):[M+H]⁺ =679.4, t_{R} = 1.19 min.

Step 3: Lithium hydroxide monohydrate (117 mg, 2.8 mmol) was added into a mixed solution of {[(3-{[(1S,3S)-3-[(4-{7-[dimethyl(oxo)-λ⁵-phosphoranyl]-6-(methoxycarbonyl)-1H-indole-3-yl}-5-(trifluoromethyl)pyrimidin-2-yl)amino]cyclopentyl]amino}cyclobutyl)methyl]amino}methanoic acid-2-methylpropyl-2-yl ester in methanol (4 mL), tetrahydrofuran (4 mL) and water (4 mL) at 25°C under nitrogen protection, the reaction solution was heated to 40°C under nitrogen protection, stirring was carried out to react for 16 h, and complete reaction was realized by LCMS detection. A 2 M hydrochloric acid solution was slowly added into the reaction solution to adjust the pH to 4-5, and extracting was carried out with ethyl acetate (50 mL X5). Combined organic layers were washed with a saturated brine (10 mL), dried over anhydrous sodium sulfate, filtered and concentrated to obtain a crude colorless oily substance 7-[dimethyl(oxo)-λ⁵-phosphoranyl]-3-(2-{[(1S,3 S)-3-{ [3-(2,2-dimethyl-4-oxo-5-aza-3-oxa-hex-6-yl)cyclobutyl]amino}cyclopentyl]amino}-5-(trifluoromethyl)pyrimidin-4-yl)-1H-indole-6-carboxylic acid (400 mg, purity of 70%, yield of 75%), and the product was directly used in the next reaction without purification. LCMS: m/z (ESI): [M+H]⁺ =664.6, t_{R} = 1.11 min. Step 4: A 4 M methanol hydrogen chloride solution (10 mL) was added into a solution of 7-[dimethyl(oxo)-λ⁵-phosphoranyl]-3-(2-{[(1S,3S)-3-{[3-(2,2-dimethyl-4-oxo-5-aza-3-oxa-hex-6-yl)cyclobutyl]amino}cyclopentyl]amino}-5-(trifluoromethyl)pyrimidin-4-yl)-1H-indole-6-carboxylic acid (300 mg, 0.45 mmol) in methanol (5 mL), the reaction solution was heated to 40°C under nitrogen protection, and reacting was while stirring for 2 h. After realizing complete reaction by LCMS detection, a saturated sodium bicarbonate solution (30 mL) was slowly added into the reaction solution, and extracting was carried out with ethyl acetate (50 mL X3). Combined organic layers were washed with a saturated brine, dried over anhydrous sodium sulfate, filtered and concentrated to dryness. The resulting crude product was purified with preparation (chromatographic column: Ultimate XB-C18, 50X250 mm, 10 um (PARP-02); mobile phase: acetonitrile-water (0.1% trifluoroacetic acid), flow rate: 70 mL/min and column temperature: 25°C) to obtain a white solid 7-[dimethyl(oxo)-λ⁵-phosphoranyl]-3-(2-{[(1*S*,3*S*)-3-{[3-(aminomethyl)cyclobutyl]amino}cyclopentyl]amino}-5-(trifluoromethyl)pyrimidin-4-yl)-1*H*-indole-6-carboxylic acid (185 mg, yield of 73%). LCMS: MS m/z (ESI): [M+H]⁺ =565.2, t_{R} = 1.25 min.

Step 5: 2-(7-azabenzotriazol)-N,N,N',N'-tetramethyluronium hexafluorophosphate (34 mg, 0.089 mmol) and N,N-diisopropylethylamine (0.059 mL, 0.35 mmol) were sequentially added into a solution of 7-[dimethyl(oxo)-λ⁵-phosphoranyl]-3-(2-{[(1S,3S)-3-{[3-(aminomethyl)cyclobutyl]amino}cyclopentyl]amino}-5-(trifluoromethyl)pyrimidin-4-yl)-1H-indole-6-carboxylic acid (50 mg, 0.089 mmol) in N,N-dimethylformamide (5 mL), and the reaction solution was stirred at 60°C for 2 h. After realizing complete reaction by LCMS detection, the reaction solution was concentrated, and the resulting crude product was purified with reversed-phase preparation (chromatographic column: Ultimate XB-C18, 50X250 mm, 10 um (PARP-02); mobile phase: acetonitrile-water (0.1% trifluoroacetic acid); flow rate: 70 mL/min; column temperature: 25°C) to obtain (4¹S,4³S)-1⁷-(dimethylphosphoryl)-2⁵-(trifluoromethyl)-1¹H-3,5,8-triaza-1(3,6)-indola-2(4,2)-pyrimidina-4(1,3)-cyclopentana-6(1,3)-cyclobutanacyclononaphan-9-one (Z13) trifluoroacetate (2.43 mg, yield of 5%). LCMS: MS m/z (ESI): [M+H]⁺ =547.3, t_{R} = 0.83 min. ¹H NMR (400 MHz, CD₃OD): δ 8.61 (s, 1H), 8.00 - 7.90 (m, 1H), 7.77 (s, 1H), 7.16 (dd, J = 8.0, 2.8 Hz, 1H), 4.00 - 3.90 (m, 1H), 3.70 - 3.65 (m, 1H), 3.61 - 3.52 (m, 1H), 3.25 - 3.15 (m, 1H), 2.90 - 2.80 (m, 1H), 2.65 - 2.56 (m, 2H), 2.40 - 2.30 (m, 3H), 2.15 - 2.05 (m, 1H), 1.99 (s, 3H), 1.96 (s, 3H), 1.84 - 1.75 (m, 1H), 1.73 - 1.61 (m, 2H), 1.56 - 1.40 (m, 2H).

### Example 14 Synthesis of compound Z14

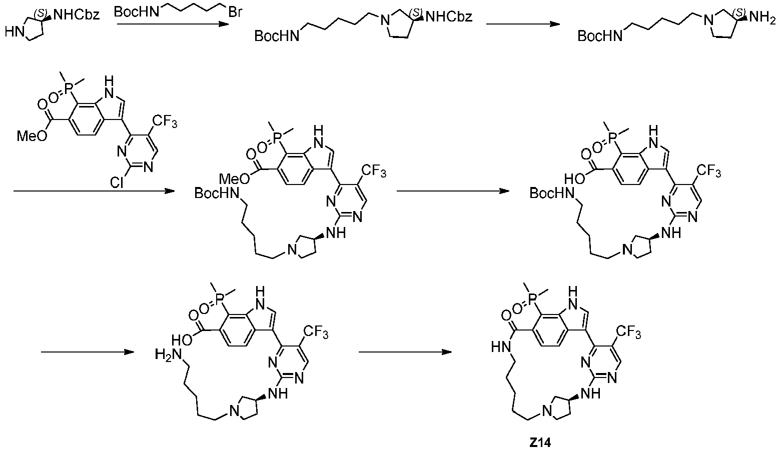

Step 1: [(5-bromopentyl)amino]methanoic acid-2-methylpropyl-2-yl ester (299 mg, 1.12 mmol) and N,N-diisopropylethylenediamine (528 mg, 4.08 mmol) were sequentially added into a solution of benzyl {[(3S)-tetrahydro-1H-pyrrol-3-yl]amino}carboxylate (225 mg, 1.02 mmol) in N,N-dimethylformamide (6 mL), and the reaction solution was stirred at 80°C in a nitrogen atmosphere for 16 h. After realizing complete reaction by LCMS detection, the reaction solution was extracted with water (15 mL) and ethyl acetate (25 mL X3). Combined organic layers were washed with a saturated brine (15 mL X2), dried over anhydrous sodium sulfate, filtered and concentrated to dryness. The resulting crude product was purified with silica gel column chromatography (dichloromethane/methanol = 30/1-10/1) to obtain a yellow solid benzyl {[(3S)-1-(2,2-dimethyl-4-oxo-5-aza-3-oxadeca-10-yl)tetrahydro-1H-pyrrol-3-yl]amino}carboxylate (373 mg, yield of 90%). LCMS: m/z (ESI): [M+H]⁺ =406.2, t_{R} = 0.80 min.

Step 2: Palladium on carbon (50 mg, 10% loading, water content of 60%) was added into a solution of benzyl {[(3S)-1-(2,2-dimethyl-4-oxo-5-aza-3-oxadeca-10-yl)tetrahydro-1H-pyrrol-3-yl]amino}carboxylate (500 mg, 1.23 mmol) in methanol (8 mL), and the reaction solution was stirred to react at 25°C in a hydrogen atmosphere for 2 h. Complete reaction was realized by LCMS detection. The reaction solution was filtered, and spin-drying was carried out to obtain a crude product ({5-[(3S)-3-aminotetrahydro-1H-pyrrol-1-yl]pentyl}amino)methanoic acid-2-methylpropyl-2-yl ester (370 mg, purity of about 85%, and yield of 94%), and the crude product was directly used in the next reaction without purification. LCMS: m/z (ESI): [M+H]⁺ =272.2, t_{R} = 0.46 min.

Step 3: Methyl 3-[2-chloro-5-(trifluoromethyl)pyrimidin-4-yl]-7-[dimethyl(oxo)-λ⁵-phosphoranyl]-1H-indole-6-carboxylate (507 mg, 1.23 mmol) and N,N-diisopropylethylenediamine (477 mg, 3.69 mmol) were sequentially added into ({5-[(3S)-3-aminotetrahydro-1H-pyrrol-1-yl]pentyl}amino)methanoic acid-2-methylpropyl-2-yl ester (334 mg, 1.23 mmol) in N,N- dimethylformamide (8 mL), the reaction solution was heated to 80°C under nitrogen protection and stirred to react for 16 h, and complete reaction was realized by LCMS detection. The reaction solution was extracted with water (20 mL) and dichloromethane (30 mL X5). Combined organic layers were washed with a saturated brine (20 mL X2), dried over anhydrous sodium sulfate, filtered and concentrated to dryness. The resulting crude product was purified with silica gel column chromatography (dichloromethane/methanol = 30/1-10/1) to obtain a yellow solid methyl 7-[dimethyl(oxo)-λ⁵-phosphoranyl]-3-(2-{[(3S)-1-(2,2-dimethyl-4-oxo-5-aza-3-oxadeca-10-yl)tetrahydro-1H-pyrrol-3-yl]amino}-5-(trifluoromethyl)pyrimidin-4-yl)-1H-indole-6-carboxylate (550 mg, and yield of 67%). LCMS: m/z (ESI): [M+H]⁺ =667.4, t_{R} = 0.92 min.

Step 4: Lithium hydroxide monohydrate (173 mg, 4.13 mmol) was added into a mixed solution of methyl 7-[dimethyl(oxo)-λ⁵-phosphoranyl]-3-(2-{[(3S)-1-(2,2-dimethyl-4-oxo-5-aza-3-oxadeca-10-yl)tetrahydro-1H-pyrrol-3-yl]amino}-5-(trifluoromethyl)pyrimidin-4-yl)-1H-indole-6-carboxylate (550 mg, 0.83 mmol) in methanol (3 mL), water (3 mL) and tetrahydrofuran (3 mL), the reaction solution was heated to 40°C under nitrogen protection and stirred to react for 2 h. After realizing complete reaction by LCMS detection, vacuum concentration was carried out to remove the solvent to obtain a crude product yellow solid 7-[dimethyl(oxo)-λ⁵-phosphoranyl]-3-(2-{[(3S)-1-(2,2-dimethyl-4-oxo-5-aza-3-oxadeca-10-yl)tetrahydro-1H-pyrrol-3-yl]amino}-5-(trifluoromethyl)pyrimidin-4-yl)-1H-indole-6-carboxylic acid (480 mg, purity of about 85% and yield of 95%). LCMS: MS m/z (ESI): [M+H]⁺ =653.2, t_{R} = 0.83 min.

Step 5: A 4 M 1,4-dioxane in hydrogen chloride solution (8 mL) was added into 7-[dimethyl(oxo)-λ⁵-phosphoranyl]-3-(2-{[(3S)-1-(2,2-dimethyl-4-oxo-5-aza-3-oxadeca-10-yl)tetrahydro-1H-pyrrol-3-yl]amino}-5-(trifluoromethyl)pyrimidin-4-yl)-1H-indole-6-carboxylic acid (480 mg, 0.70 mmol), the reaction solution was heated to 40°C under nitrogen protection and stirred to react for 2 h. After realizing complete reaction by LCMS detection, a saturated sodium bicarbonate solution (30 mL) was slowly added into the reaction solution, and extracting was carried out with ethyl acetate (40 mL X3). Combined organic layers were washed with a saturated brine, dried over anhydrous sodium sulfate, filtered and concentrated to dryness. The resulting crude product was purified with reversed-phase preparation (chromatographic column: Ultimate XB-C18, 50X250 mm, 10 um (PARP-02); mobile phase: acetonitrile-water (0.1% trifluoroacetic acid), flow rate: 70 mL/min and column temperature: 25°C) to obtain a yellow solid 7-[dimethyl(oxo)-λ⁵-phosphoranyl]-3-(2-{[(3S)-1-(5-aminopentyl)tetrahydro-1H-pyrrol-3-yl]amino}-5-(trifluoromethyl)pyrimidin-4-yl)-1H-indole-6-carboxylic acid (300 mg, yield of 77%). LCMS: MS m/z (ESI): 553.2 [M+H]⁺, t_{R} = 0.61 min.

Step 6: 2-(7-azabenzotriazol)-N,N,N',N'-tetramethyluronium hexafluorophosphate (69 mg, 0.18 mmol) and N,N-diisopropylethylamine (94 mg, 0.12 mmol) were sequentially added into a solution of 7-[dimethyl(oxo)-λ⁵-phosphoranyl]-3-(2-{[(3S)-1-(5-aminopentyl)tetrahydro-1H-pyrrol-3-yl]amino}-5-(trifluoromethyl)pyrimidin-4-yl)-1H-indole-6-carboxylic acid (100 mg, 0.18 mmol) in N,N-dimethylformamide (5 mL), and the reaction solution was stirred at 60°C for 16 h. After realizing complete reaction by LCMS detection, the reaction solution was concentrated, and the resulting crude product was purified with reversed-phase preparation (chromatographic column: Ultimate XB-C18, 50X250 mm, 10 um (PARP-02); mobile phase: acetonitrile-water (0.1% trifluoroacetic acid); flow rate: 70 mL/min; column temperature: 25°C) to obtain (4³S)-1⁷-(dimethylphosphoryl)-2⁵-(trifluoromethyl)-1¹H-3,10-diaza-1(3,6)-indola-2(4,2)-pyrimidina-4(3,1)-pyrrolidinacycloundecaphan-11-one (Z14) trifluoroacetate (13.8 mg, yield of 14%). LCMS: MS m/z (ESI): [M+H]⁺ =535.2, t_{R} = 0.63 min. ¹H NMR (400 MHz, CD₃OD): δ 8.63 (s, 1H), 8.43 (s, 1H), 7.97 (s, 1H), 7.32 (dd, J = 8.0, 3.6 Hz, 1H), 4.50 - 4.37 (m, 1H), 3.97 - 3.85 (m, 1H), 3.63 - 3.50 (m, 1H), 3.26 - 3.20 (m, 1H), 3.17 - 3.08 (m, 2H), 3.02 - 2.84 (m, 2H), 2.59 - 2.46 (m, 1H), 2.23 - 2.12 (m, 1H), 2.01 (d, J = 3.6 Hz, 3H), 1.98 (d, J = 3.6 Hz, 3H), 1.82 - 1.65 (m, 2H), 1.52 - 1.37 (m, 4H).

### Example 15 Synthesis of compound Z15

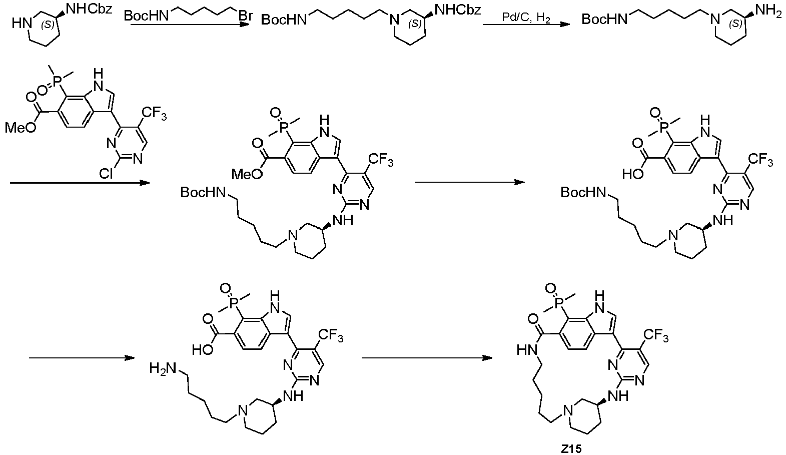

Step 1: [(5-bromopentyl)amino]methanoic acid-2-methylpropyl-2-yl ester (250 mg, 0.939 mmol) and N,N-diisopropylethylamine (363 mg, 2.82 mmol) were added into a solution of benzyl {[(3S)-hexahydropyridin-3-yl]amino}carboxylate (242 mg, 1.03 mmol) in N,N-dimethylformamide (8 mL) at 25°C, and the reaction solution was reacted at 80°C for 6 h under nitrogen protection while stirring. After realizing complete reaction by LCMS detection, water (50 mL) was added into the reaction solution, and extracting was carried out with ethyl acetate (50 mL X3). Combined organic layers were washed with a saturated brine (50 mL), dried over anhydrous sodium sulfate, filtered and concentrated. The resulting crude product was purified with column chromatography (petroleum ether/ethyl acetate = 10/1-3/1) to obtain a colorless oily substance ({5-[(3S)-3-{[(benzyloxy)carbonyl]amino}hexahydropyridin-1-yl]pentyl}amino)methanoic acid-2-methylpropyl-2-yl ester (280 mg, yield of 64%). LCMS: m/z (ESI): [M+H]⁺ =420.4, t_{R} = 0.80 min.

Step 2: ({5-[(3S)-3-{[(benzyloxy)carbonyl]amino}hexahydropyridin-1-yl]pentyl}amino)methanoic acid-2-methylpropyl-2-yl ester (250 mg, 0.596 mmol) was dissolved in a methanol (10 mL) solution, and palladium on carbon (100 mg, 10% loading, water content of 60%) was added. The reaction solution was stirred to react for 3 hours at 25 °C under hydrogen protection. After realizing complete reaction by LCMS detection, the reaction solution was filtered and concentrated to obtain a colorless oily substance ({ 5-[(3 S)-3-aminohexahydropyridin-1-yl]pentyl}amino)methanoic acid-2-methylpropyl-2-yl ester (180 mg, crude product). LCMS: m/z (ESI): [M+H]⁺ =286.0, t_{R} = 0.80 min.

Step 3: Methyl 3-[2-chloro-5-(trifluoromethyl)pyrimidin-4-yl]-7-[dimethyl(oxo)-λ⁵-phosphoranyl]-1*H*-indole-6-carboxylate (272 mg, 0.63 mmol) and N,N-diisopropylethylamine (244 mg, 1.89 mmol) were sequentially added into a solution of ({5-[(3*S*)-3-aminohexahydropyridin-1-yl]pentyl}amino)methanoic acid-2-methylpropyl-2-yl ester (180 mg, 0.63 mmol) in N,N-dimethylformamide (6 mL) at room temperature, and the reaction solution was stirred to react at 80°C under nitrogen protection for 16 h. After realizing complete reaction by LCMS detection, an aqueous solution (50 mL) was added into the reaction solution, and extracting was carried out with dichloromethane (50 mL X3). Combined organic layers were dried over anhydrous sodium sulfate, filtered and concentrated to dryness. The resulting crude product was purified with silica gel column chromatography (dichloromethane/methanol = 50/1-10/1) to obtain a colorless oily substance methyl 7-[dimethyl(oxo)-λ⁵-phosphoranyl]-3-(2-{[(3S)-1-(2,2-dimethyl-4-oxo-5-aza-3-oxadeca-10-yl)hexahydropyridin-3-yl]amino}-5-(trifluoromethyl)pyrimidin-4-yl)-1H-indole-6-carboxylate (300 mg, yield of 70%). LCMS: MS m/z (ESI): [M+H]⁺ =681.1, t_{R} = 1.11 min. Step 4: Lithium hydroxide hydrate (185 mg, 4.41 mmol) was added into methyl 7-[dimethyl(oxo)-λ⁵-phosphoranyl]-3-(2-{[(3*S*)-1-(2,2-dimethyl-4-oxo-5-aza-3-oxadeca-10-yl)hexahydropyridin-3-yl]amino}-5-(trifluoromethyl)pyrimidin-4-yl)-1*H*-indole-6-carboxylate (300 mg, 0.441 mmol) in methanol (4 mL), water (4 mL) and tetrahydrofuran (4 mL), and the reaction solution was stirred to react for 16 h at 40°C under nitrogen protection. After realizing complete reaction by LCMS detection, concentrating was carried out to dryness so as to obtain a white solid 7-[dimethyl(oxo)-λ⁵-phosphoranyl]-3-(2-{[(3S)-1-(2,2-dimethyl-4-oxo-5-aza-3-oxadeca-10-yl)hexahydropyridin-3-yl]amino}-5-(trifluoromethyl)pyrimidin-4-yl)-1H-indole-6-carboxylic acid (260 mg, crude product). LCMS: MS m/z (ESI): [M+H]⁺ =667.2, t_{R} = 0.79 min.

Step 5: Dioxane hydrochloride (1.95 mL, 4 mol/L) was added into 7-[dimethyl(oxo)-λ⁵-phosphoranyl]-3-(2-{[(3S)-1-(2,2-dimethyl-4-oxo-5-aza-3-oxadeca-10-yl)hexahydropyridin-3-yl]amino}-5-(trifluoromethyl)pyrimidin-4-yl)-1H-indole-6-carboxylic acid (260 mg, 0.390 mmol) in dioxane (4 mL) at room temperature. The reaction solution was stirred to react at 40°C under nitrogen protection for 3 h. After realizing complete reaction by LCMS detection, concentrating was carried out to remove hydrochloric acid, and the resulting crude product was purified with reversed-phase preparation (chromatographic column: Ultimate XB-C18, 50X250 mm, 10 um (PARP-02); mobile phase: acetonitrile-water (0.1% trifluoroacetic acid); flow rate: 70 mL/min; column temperature: 25°C) to obtain a white solid 7-[dimethyl(oxo)-λ⁵-phosphoranyl]-3-(2-{[(3S)-1-(5-aminopentyl)hexahydropyridin-3-yl]amino}-5-(trifluoromethyl)pyrimidin-4-yl)-1H-indole-6-carboxylic acid (200 mg, yield of 95%). LCMS: MS m/z (ESI): 567.2 [M+H]⁺, t_{R} = 0.82 min.

Step 6: 2-(7-azobenzotriazole)-N,N,N',N'-tetramethyluronium hexafluorophosphate (56.3 mg, 0.14 mmol) and N,N-diisopropylethylamine (77.4 mg, 0.6 mmol) were sequentially added into 7-[dimethyl(oxo)-λ⁵-phosphoranyl]-3-(2-{[(3S)-1-(5-aminopentyl)hexahydropyridin-3-yl]amino}-5-(trifluoromethyl)pyrimidin-4-yl)-1H-indole-6-carboxylic acid (70 mg, 0.12 mmol) in N,N-dimethylformamide (5 mL) at room temperature, and the reaction solution was reacted for 3 h at 65°C under liquid nitrogen protection. After realizing complete reaction by LCMS detection, the crude product resulted from concentration was purified with reversed phase (chromatographic column: Ultimate XB-C18, 50X250 mm, 10 um (PARP-02); mobile phase: acetonitrile-water (10 mmol NH₄HCO₃); flow rate: 70 mL/min; column temperature: 25°C) to obtain (4³S)-1⁷-(dimethylphosphoryl)-2⁵-(trifluoromethyl)-1¹H-3,10-diaza-1(3,6)-indola-2(4,2)-pyrimidina-4(3,1)-piperidinacycloundecaphan-11-one (Z15) (1.2 mg, yield of 1.9%). LCMS: MS m/z (ESI): 549.3 [M+H]⁺, t_{R} = 0.81 min. ¹H NMR (400 MHz, DMSO-*d₆*): δ 8.61 (s, 1H), 7.91 (d, J = 8.0 Hz, 1H), 7.79 (s, 1H), 7.22 - 7.19 (m, 1H), 2.96 - 2.78 (m, 4H), 2.46 - 2.40 (m, 2H), 2.08 - 2.03 (m, 1H), 1.96 (d, J = 14.4 Hz, 3H), 1.83 (d, J = 13.6 Hz, 3H), 1.71 (s, 1H), 1.73 - 1.56 (m, 5H), 1.38 - 1.14 (m, 6H).

### Example 16 Synthesis of compound Z16

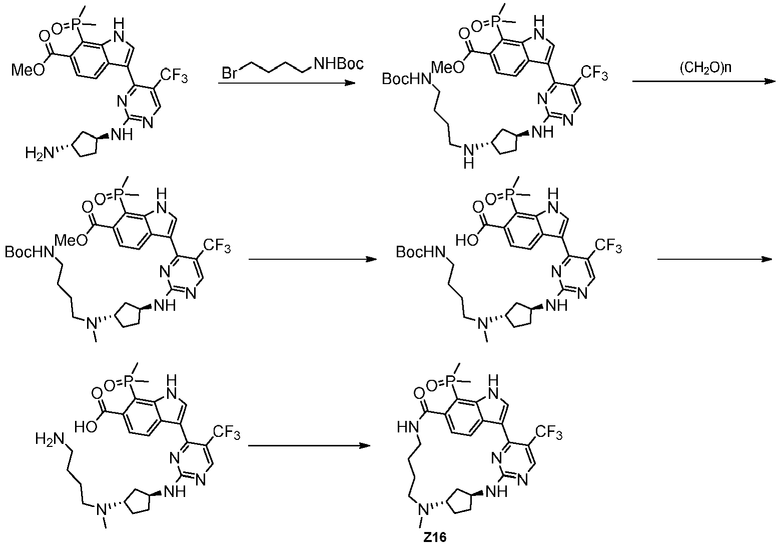

Step 1: Methyl 7-[dimethyl(oxo)-λ⁵-phosphoranyl]-3-(2-{[(1*S*,3*S*)-3-aminocyclopentyl]amino}-5-(trifluoromethyl)pyrimidin-4-yl)-1*H*-indole-6-carboxylate (300 mg, 0.61 mmol) was dissolved in N,N-dimethylformamide (3 mL), [(4-bromobutyl)amino]methanoic acid-2-methylpropyl-2-yl ester (153 mg, 0.61 mmol) and N,N-diisopropylethylamine (0.30 mL,1.83 mmol) were sequentially added, and stirring was carried out to react at 80°C for 16 h. After realizing complete reaction by LCMS detection, a right amount of silica gel was directly added, spin-drying and sample mixing were carried out, and purification was carried out by column chromatography (dichloromethane/methanol = 100/7) to obtain an off-white solid methyl 7-[dimethyl(oxo)-λ⁵-phosphoranyl]-3-(2-{[(1*S*,3*S*)-3-[(2,2-dimethyl-4-oxo-5-aza-3-oxanon-9-yl)amino]cyclopentyl]amino}-5-(trifluoromethyl)pyrimidin-4-yl)-1H-indole-6-carboxylate (200 mg, yield of 50%). LCMS: m/z (ESI): [M+H]⁺ =667.2, t_{R} = 0.91 min.

Step 2: Methyl 7-[dimethyl(oxo)-λ⁵-phosphoranyl]-3-(2-{[(1*S*,3*S*)-3-[(2,2-dimethyl-4-oxo-5-aza-3-oxanon-9-yl)amino]cyclopentyl]amino}-5-(trifluoromethyl)pyrimidin-4-yl)-1*H*-indole-6-carboxylate (150 mg, 0.225 mmol) was dissolved in methanol (3 mL), then paraformaldehyde (45.0 mg, 1.13 mmol), palladium on carbon (10 mg, 10% loading, 60% of water) and glacial acetic acid (0.1 mL) were added, and stirring was carried out to react at 70°C for 5 h. After realizing complete reaction by LCMS detection, the palladium on carbon was filtered out, silica gel was directly added into the filtrate for sample mixing, and purification was carried out by column chromatography (dichloromethane/methanol = 100/7) to obtain a white solid methyl 7-[dimethyl(oxo)-λ⁵-phosphoranyl]-3-(2-{[(1*S*,3*S*)-3-(2,2-dimethyl-4-oxo-5,10-diaza-3-oxaundecan-10-yl)cyclopentyl]amino}-5-(trifluoromethyl)pyrimidin-4-yl)-1H-indole-6-carboxylate (130 mg, yield of 85%). LCMS: m/z (ESI): [M+H]⁺ =681.2, t_{R} = 0.93 min.

Step 3: Methyl 7-[dimethyl(oxo)-λ⁵-phosphoranyl]-3-(2-{[(1*S*,3*S*)-3-(2,2-dimethyl-4-oxo-5,10-diaza-3-oxaundecan-10-yl)cyclopentyl]amino}-5-(trifluoromethyl)pyrimidin-4-yl)-1H-indole-6-carboxylate (130 mg, 0.191 mmol) was dissolved in a mixed system of water (1 mL), methanol (1 mL) and tetrahydrofuran (1 mL), then lithium hydroxide monohydrate (40.1 mg, 0.955 mmol) was added, and stirring was carried out to react at 40°C for 16 h. After realizing complete reaction by LCMS detection, the reaction solution was directly applied to the next reaction without any treatment. LCMS: m/z (ESI): 667.4 [M+H]⁺ =667.4, t_{R} = 0.76 min.

Step 4: A solution (5 mL, 4M) of 1,4-dioxane in hydrochloric acid was added into the reaction solution from the previous step, and the system was stirred at 10°C for 4 h. After realizing complete reaction by LCMS detection, a solvent was spin-dried, and reversed-phase preparation was carried out on the resulting crude product (chromatographic column: Ultimate XB-C18, 50X250 mm, 10 um (PARP-02); mobile phase: acetonitrile-water (0.1% ammonium bicarbonate), flow rate: 70 mL/min and column temperature: 25°C) to obtain a white solid 7-[dimethyl(oxo)-λ⁵-phosphoranyl]-3-(2-{[(1*S*,3*S*)-3-[(4-aminobutyl)(methyl)amino]cyclopentyl]amino}-5-(trifluoromethyl)pyrimidin-4-yl)-1H-indole-6-carboxylic acid (20 mg, yield of 13%). LCMS: m/z (ESI): [M+H]⁺ =567.2, t_{R} = 0.59 min.

Step 5: 2-(7-azabenzotriazol)-N,N,N',N'-tetramethyluronium hexafluorophosphate (14.8 mg, 0.04 mmol) and N,N-diisopropylethylamine (0.03 mL, 0.18 mmol) were sequentially added into a solution of 7-[dimethyl(oxo)-λ⁵-phosphoranyl]-3-(2-{[(1*S*,3*S*)-3-[(4-aminobutyl)(methyl)amino]cyclopentyl]amino}-5-(trifluoromethyl)pyrimidin-4-yl)-1H-indole-6-carboxylic acid (20 mg, 0.04 mmol) in N,N-dimethylformamide (3 mL), and the reaction solution was stirred at 60 °C for 16 h. After realizing complete reaction by LCMS detection, the reaction solution was concentrated, and reversed-phase preparation was carried out on the resulting crude product (chromatographic column: Ultimate XB-C18, 50X250 mm, 10 um (PARP-02); mobile phase: acetonitrile-water (0.1% trifluoroacetic acid), flow rate: 70 mL/min and column temperature: 25°C) to obtain (4¹S,4³S)-1⁷-(dimethylphosphoryl)-5-methyl-2⁵-(trifluoromethyl)-1¹H-3,5,10-triaza-1(3,6)-indola-2(4,2)-pyrimidina-4(1,3)-cyclopentanacycloundecaphan-11-one (Z16) trifluoroacetate (5.88 mg, yield of 30%). LCMS: m/z (ESI): [M+H]⁺ =549.2, t_{R} = 0.67 min. ¹H NMR (400 MHz, CD₃OD): δ 8.57 (s, 1H), 8.53 (d, J = 7.6 Hz, 1H), 7.99 (s, 1H), 7.31 (dd, *J* = 8.4, 3.2 Hz, 1H), 4.25-4.31 (m, 1H), 3.91-4.04 (m, 1H), 3.24 - 3.11 (m, 1H), 2.86-2.91 (m, 2H), 2.78 (s, 1H), 2.72 (s, 2H), 2.52-2.57 (m, 1H), 2.38 - 2.22 (m, 2H), 2.14 - 1.70 (m, 12H), 1.57-1.66 (m, 1H), 1.29-1.37 (m, 1H).

### Example 17 Synthesis of compound Z19

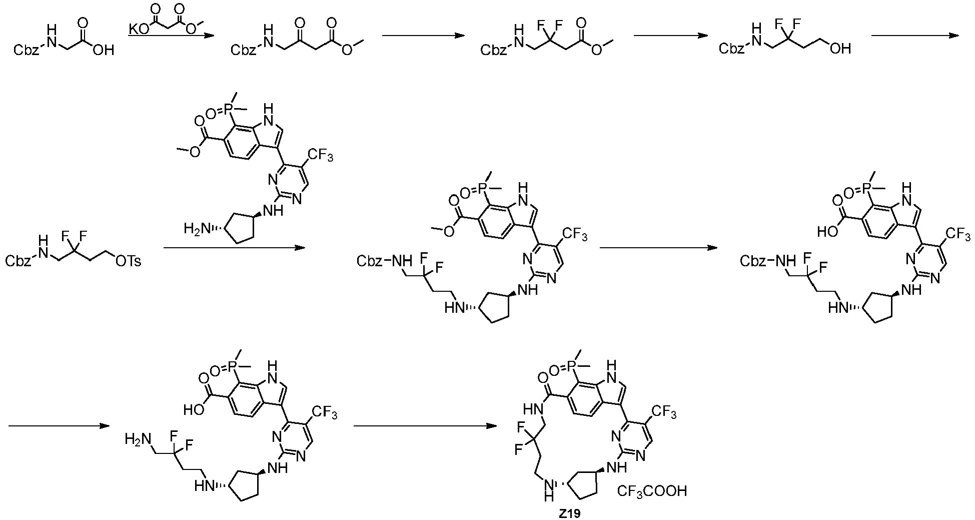

Step 1: *N*-[(benzyloxy)carbonyl]glycine (20 g, 95.6 mmol), tetrahydrofuran (130 mL), magnesium chloride (4.55 g, 47.8 mmol), carbonyldiimidazole (15.5 g, 95.6 mmol) and [(3-methoxy-1,3-dioxopropyl)oxy]potassium (12.2 g, 71.7 mmol) were sequentially added into a 250 mL three-neck flask, the reaction solution was replaced with nitrogen, refluxing was carried out at 70°C for 4 h, and complete reaction was realized by TLC detection. The reaction solution was concentrated, water (100 mL) was added for dilution, extracting was carried out with ethyl acetate (50 mL X3), anhydrous sodium sulfate was added for drying, concentrating was carried out to obtain a crude product, and the crude product was purified with silica gel column chromatography (dichloromethane/methanol = 10/3) to obtain an off-white solid benzyl [(1-methoxy-1,3-dioxobutyl-4-yl)amino]carboxylate (3.7 g, yield of 15%). ¹H NMR (400 MHz, CD₃OD): δ 7.42-7.26 (m, 5H), 5.12 (s, 2H), 4.21 (d, *J=* 5.2 Hz, 2H), 3.74 (s, 3H), 3.50 (s, 2H).

Step 2: Benzyl [(1-methoxy-1,3-dioxobutyl-4-yl)amino]carboxylate (1.50 g, 5.66 mmol) was dissolved in dichloromethane (30 mL), then diethylaminosulfur trifluoride (1.00 g, 6.22 mmol) was added dropwise at 0°C, stirring was carried out for 16 h at 15°C, then diethylaminosulfur trifluoride (1.00 g, 6.22 mmol) was supplemented at 15°C, and then stirring was carried out for 4 h at 15°C. After realizing complete reaction by LCMS detection, water (20 mL) was added for quenching the reaction, then extracting was carried out with ethyl acetate (20 mL X3), anhydrous sodium sulfate was added for drying, and reversed-phase preparation was carried out on a concentrated crude product (chromatographic column: Ultimate XB-C18, 50X250 mm, 10 um (PARP-02), mobile phase: acetonitrile-water (0.1% trifluoroacetic acid), flow rate: 70 mL/min and column temperature: 25°C) to obtain yellow oily benzyl (3,3-difluoro-1-methoxy-1-oxo-but-4-yl)amino]carboxylate (0.15 g, yield of 9%). LCMS: m/z (ESI): [M+H]⁺= 288.0, t_{R} = 0.97 min.

Step 3: Benzyl (3,3-difluoro-1-methoxy-1-oxo-but-4-yl)amino]carboxylate (130 mg, 0.45 mmol) was dissolved in tetrahydrofuran (3 mL), then a solution (1 mL, 1 M) of lithium aluminum hydride in tetrahydrofuran was added dropwise at 0°C, and the reaction was stirred for 2 h at 10°C. After realizing complete reaction by LCMS detection, water (20 mL) was added for quenching reaction, and extracting was carried out with ethyl acetate (20 mL X3). Anhydrous sodium sulfate was added for drying, and concentrating was carried out to obtain a crude yellow oily substance benzyl [(2,2-difluoro-4-hydroxybutyl)amino]carboxylate (100 mg, 85%), the substance was directly used in the next step without purification. LCMS: m/z (ESI): [M+H]⁺= 260.0, t_{R} = 0.88 min.

Step 4: Benzyl [(2,2-difluoro-4-hydroxybutyl)amino]carboxylate (120 mg, 0.46 mmol) was dissolved in dichloromethane (1 mL), then pyridine (1 mL) and p-methylsulfonyl chloride (265 mg, 1.39 mmol) were added, and the system was stirred at 10°C for 3 h. After realizing complete reaction by LCMS detection, silica gel was directly added, spin-drying and sample mixing were carried out by an oil pump, and purification was carried out with silica gel column chromatography (dichloromethane 100%) to obtain a yellow oily substance 4-toluenesulfonic acid-6,6-difluoro-3-oxo-1-phenyl-4-aza-2-oxaoct-8-yl ester (130 mg, yield of 68%). LCMS: m/z (ESI): [M+H]⁺ =414.0, t_{R} = 1.20 min.

Step 5: Methyl 7-[dimethyl(oxo)-λ⁵-phosphoranyl]-3-(2-{[(1*S*,3*S*)-3-aminocyclopentyl]amino}-5-(trifluoromethyl)pyrimidin-4-yl)-1*H*-indole-6-carboxylate (173 mg, 0.35 mmol) was dissolved in N,N-dimethylformamide (3 mL) in a 10 mL single-neck flask, then 4-toluenesulfonic acid-6,6-difluoro-3-oxo-1-phenyl-4-aza-2-oxaoct-8-yl ester (120 mg, 0.29 mmol) and N,N-diisopropylethylamine (0.24 mL, 1.45 mmol) were added. The system was stirred at 100°C for 20 h. After realizing complete reaction by LCMS detection, silica gel was added, an oil pump was used for spin-drying and sample mixing, and purification was carried out with silica gel column chromatography (dichloromethane: methanol = 10: 1) to obtain a yellow oily substance methyl 7-[dimethyl(oxo)-λ⁵-phosphoranyl]-3-(2-{[(1*S*,3*S*)-3-[(6,6-difluoro-3-oxo-1-phenyl-4-aza-2-oxaoct-8-yl)amino]cyclopentyl]amino}-5-(trifluoromethyl)pyrimidin-4-yl)-1*H*-indole-6-carboxylate (81 mg, yield of 38%). LCMS: m/z (ESI): [M+H]⁺ =737.2, t_{R} = 0.96 min.

Step 6: Methyl 7-[dimethyl(oxo)-λ⁵-phosphoranyl]-3-(2-{[(1*S*,3*S*)-3-[(6,6-difluoro-3-oxo-1-phenyl-4-aza-2-oxaoct-8-yl)amino]cyclopentyl]amino}-5-(trifluoromethyl)pyrimidin-4-yl)-1*H*-indole-6-carboxylate (90 mg, 0.12 mmol) was dissolved in a mixed system of water (1 mL), methanol (1 mL) and tetrahydrofuran (1 mL), then lithium hydroxide monohydrate (5.13 mg, 0.12 mmol) was added, and reacting was carried out while stirring at 40°C for 16 h. After realizing complete reaction by LCMS detection, filtering was carried out, and reversed-phase preparation was carried out on filtrate (chromatographic column: Ultimate XB-C18, 50X250 mm, 10 um (PARP-02), mobile phase: acetonitrile-water (0.1% ammonium bicarbonate), flow rate: 70 mL/min and column temperature: 25°C) to obtain a white solid 7-[dimethyl(oxo)-λ⁵-phosphoranyl]-3-(2-{[(1*S*,3*S*)-3-[(6,6-difluoro-3-oxo-1-phenyl-4-aza-2-oxaoct-8-yl)amino]cyclopentyl]amino}-5-(trifluoromethyl)pyrimidin-4-yl)-1*H*-indole-6-carboxylic acid (30 mg, yield of 34%). LCMS: m/z (ESI): [M+H]⁺=723.2, t_{R} = 0.87 min.

Step 7: 7-[dimethyl(oxo)-λ⁵-phosphoranyl]-3-(2-{[(1*S*,3*S*)-3-[(6,6-difluoro-3-oxo-1-phenyl-4-aza-2-oxaoct-8-yl)amino]cyclopentyl]amino}-5-(trifluoromethyl)pyrimidin-4-yl)-1H-indole-6-carboxylic acid (18 mg, 0.03 mmol) and trifluoroacetic acid (3 mL) were added into a 10 mL single-neck flask. Stirring was carried out at 60°C for 1.5 h. After realizing complete reaction by LCMS detection, the reaction solution was spin-dried to obtain a gray solid crude product 2,2-difluoro-4-{[(1*S*,3*S*)-3-[(4-{5-[(*Z*)-[dimethyl(oxo)-λ⁵-phosphoranyl]methylidene]-4-[(1*Z*)-ethylidene]-1*H*-pyrrol-3-yl}-5-(trifluoromethyl)pyrimidin-2-yl)amino]cyclopentyl]amino}butyl-1-amino carboxylic acid (14 mg, 96%), and the product is directly used in the next step without purification. LCMS: m/z (ESI): [M+H]⁺ =589.2, t_{R} = 0.62 min.

Step 8: 2-(7-azabenzotriazol)-N,N,N',N'-tetramethyluronium hexafluorophosphate (9.95 mg, 0.03 mmol) and N,N-diisopropylethylamine (0.02 mL, 0.12 mmol) were sequentially added into a solution of 2,2-difluoro-4-{ [(1*S*,3*S*)-3-[(4-{5-[(*Z*)-[dimethyl(oxo)-λ⁵-phosphoranyl]methylidene]-4-[(1*Z*)-ethylidene]-1*H*-pyrrol-3-yl}-5-(trifluoromethyl)pyrimidin-2-yl)amino]cyclopentyl]amino}butyl-1-amino carboxylic acid (14 mg, 0.02 mmol) in N,N-dimethylformamide (2 mL). Stirring was carried out at 60°C for 16 h. After realizing complete reaction by LCMS detection, the reaction solution was concentrated, and reversed-phase preparation was carried out on a crude product (chromatographic column: Ultimate XB-C18, 50X250 mm, 10 um (PARP-02), mobile phase: acetonitrile-water (0.1% trifluoroacetic acid), flow rate: 70 mL/min and column temperature: 25°C) to obtain (4¹S,4³S)-1⁷-(dimethylphosphoryl)-8,8-difluoro-2⁵-(trifluoromethyl)-1¹H-3,5,10-triaza-1(3,6)-indola-2(4,2)-pyrimidina-4(1,3)-cyclopentanacycloundecaphan-11-one (Z19) (1.22 mg, yield of 19%, as trifluoroacetate). LCMS: m/z (ESI): [M+H]⁺= 571.2, t_{R} = 0.67 min.¹H NMR (400 MHz, CD₃OD): δ 8.57-8.58 (m, 2H), 8.01 (s, 1H), 7.32 (d, *J* = 5.6 Hz, 1H), 4.16-4.18 (m, 1H), 3.86-3.87 (m, 1H), 3.47-3.49 (m, 1H), 2.89-2.93 (m, 2H), 2.70-2.73 (m, 1H), 2.48-2.27 (m, 4H), 1.98-2.02 (m, 6H), 1.81-1.86 (m, 2H), 1.28-1.29 (m, 2H).

### Example 18 Synthesis of compound Z27

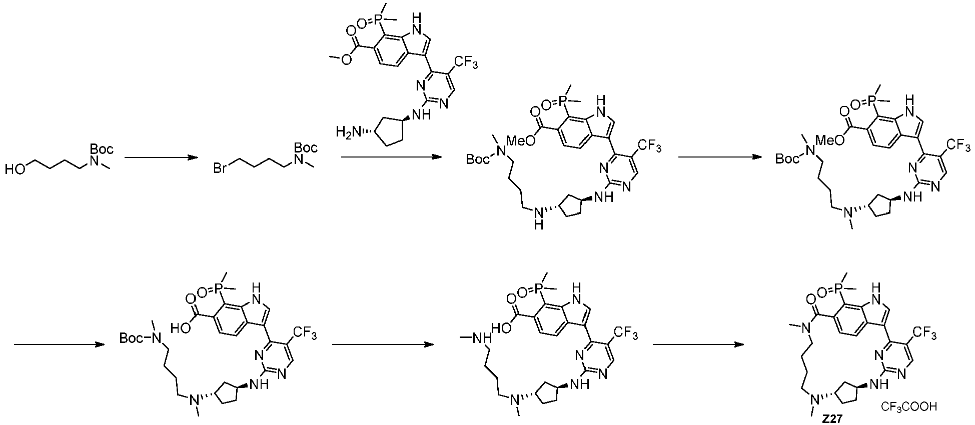

Step 1: Carbon tetrabromide (981 mg, 3.74 mmol) and triphenylphosphine (1.24 g, 3.74 mmol) were added into a solution of [(1-hydroxybutyl-4-yl)(methyl)amino]methanoic acid-2-methylpropyl-2-yl ester (400 mg, 1.97 mmol) in tetrahydrofuran (8 mL) at 25°C. The reaction solution was stirred to react at 25 °C under nitrogen protection for 16 h. After realizing complete reaction by LCMS detection, water (50 mL) was added into the reaction solution, and extracting was carried out with ethyl acetate (50 mL X3). Combined organic layers were washed with a saturated brine (50 mL), dried over anhydrous sodium sulfate, filtered and concentrated. The resulting crude product was purified with silica gel column chromatography (petroleum ether/ethyl acetate = 10/1-3/1) to obtain a colorless oily substance [(1-bromobutyl-4-yl)(methyl)amino]methanoic acid-2-methylpropyl-2-yl ester (400 mg, yield of 46%). LCMS: m/z (ESI): [M+H-56]⁺= 210.0, t_{R} = 2.03 min.

Step 2: [(1-bromobutyl-4-yl)(methyl)amino]methanoic acid-2-methylpropyl-2-yl ester (240 mg, 0.90 mmol) and methyl 3-(2-{[(1S,3S)-3-aminocyclopentyl]amino}-5-(trifluoromethyl)pyrimidin-4-yl)-7-[methyl(oxo)-λ⁵-phosphoranyl]-1H-indole-6-carboxylate (447 mg, 0.90 mmol) were dissolved in acetonitrile (10 mL), and N,N-diisopropylethylamine (349 mg, 2.71 mmol) was added. Reacting was carried out while stirring at 80°C under nitrogen protection for 16 h. After realizing complete reaction by LCMS detection, the reaction solution was concentrated to obtain a colorless oily substance methyl 7-[dimethyl(oxo)-λ⁵-phosphoranyl]-3-(2-{[(1S,3S)-3-[(2,2,5-trimethyl-4-oxo-5-aza-3-oxanon-9-yl)amino]cyclopentyl]amino}-5-(trifluoromethyl)pyrimidin-4-yl)-1H-indole-6-carboxylate (220 mg, yield of 36%). LCMS: m/z (ESI): [M+H]⁺=681.4, t_{R}= 0.96 min.

Step 3: Paraformaldehyde (145 mg, 1.62 mmol), acetic acid (244 mg, 1.89 mmol) and palladium on carbon (20 mg, 10% loading, and 60% of water) were sequentially added into a solution of methyl 7-[dimethyl(oxo)-λ⁵-phosphoranyl]-3-(2-{[(1S,3S)-3-[(2,2,5-trimethyl-4-oxo-5-aza-3-oxanon-9-yl)amino]cyclopentyl]amino}-5-(trifluoromethyl)pyrimidin-4-yl)-1H-indole-6-carboxylate (220 mg, 0.32 mmol) in methanol (8 mL) at room temperature. Reacting was carried out while stirring at 80°C under hydrogen for 16 h. After realizing complete reaction by LCMS detection, an aqueous solution (50 mL) was added into the reaction solution, and extracting was carried out with dichloromethane (50 mL X3). Combined organic layers were dried over anhydrous sodium sulfate, filtered and concentrated to dryness. The resulting crude product was purified with silica gel column chromatography (dichloromethane/methanol = 50/1 to 10/1) to obtain a colorless oily substance methyl 7-[dimethyl(oxo)-λ⁵-phosphoranyl]-3-(2-{[(1S,3S)-3-(2,2,5-trimethyl-4-oxo-5,10-diaza-3-undecan-10-yl)cyclopentyl]amino}-5-(trifluoromethyl)pyrimidin-4-yl)-1H-indole-6-carboxylate (200 mg, yield of 71%). LCMS: MS m/z (ESI): [M+H]⁺= 695.7, t_{R} = 1.19 min. Step 4: Lithium hydroxide hydrate (121 mg, 2.88 mmol) was added into methyl 7-[dimethyl(oxo)-λ⁵-phosphoranyl]-3-(2-{[(1S,3S)-3-(2,2,5-trimethyl-4-oxo-5,10-diaza-3-oxaundecan-10-yl)cyclopentyl]amino}-5-(trifluoromethyl)pyrimidin-4-yl)-1H-indole-6-carboxylate (200 mg, 0.29 mmol) in methanol (3 mL), water (3 mL) and tetrahydrofuran (3 mL). Stirring was carried out to react at 40°C under nitrogen protection for 16 h. After realizing complete reaction by LCMS detection, concentrating was carried out to dryness to obtain a crude white solid 7-[dimethyl(oxo)-λ⁵-phosphoranyl]-3-(2-{[(1S,3 S)-3-(2,2,5-trimethyl-4-oxo-5,10-diaza-3-oxaundecan-10-yl)cyclopentyl]amino}-5-(trifluoromethyl)pyrimidin-4-yl)-1H-indole-6-carboxylic acid (180 mg, yield of 83%). LCMS: MS m/z (ESI): [M+H]⁺= 681.4, t_{R} = 0.93 min.

Step 5: Dioxane in hydrochloric acid (1.32 mL, 5.29 mmol, 4 mol/L) was added into 7-[dimethyl(oxo)-λ⁵-phosphoranyl]-3-(2-{[(1S,3S)-3-(2,2,5-trimethyl-4-oxo-5,10-diaza-3-oxaundecan-10-yl)cyclopentyl]amino}-5-(trifluoromethyl)pyrimidin-4-yl)-1H-indole-6-carboxylic acid (180 mg, 0.26 mmol) in dioxane (4 mL) at room temperature. Reacting was carried out while stirring at 25°C under nitrogen protection for16 h. After realizing complete reaction by LCMS detection, concentrating was carried out, and the crude product was purified with reversed-phase preparation (chromatographic column: Ultimate XB-C18, 50X250 mm, 10 um (PARP-02), mobile phase: acetonitrile-water (0.1% trifluoroacetic acid), flow rate: 70 mL/min and column temperature: 25°C) to obtain a white solid 7-[dimethyl(oxo)-λ⁵-phosphoranyl]-3-(2-{[(1S,3S)-3-(2,7-diazaoct-2-yl)cyclopentyl]amino}-5-(trifluoromethyl)pyrimidin-4-yl)-1H-indole-6-carboxylic acid (80 mg, yield of 50%). LCMS: MS m/z (ESI): [M+H]⁺= 581.2, t_{R} = 1.00 min.

Step 6: 2-(7-azobenzotriazole)-N,N,N',N'- tetramethyluronium hexafluorophosphate (55 mg, 0.15 mmol) and N,N-diisopropylethylamine (78 mg, 0.61 mmol) were sequentially added into 7-[dimethyl(oxo)-λ⁵-phosphoranyl]-3-(2-{[(1S,3S)-3-(2,7-diazaoct-2-yl)cyclopentyl]amino}-5-(trifluoromethyl)pyrimidin-4-yl)-1H-indole-6-carboxylic acid (70 mg, 0.12 mmol) in N,N-dimethylformamide (5 mL) at room temperature. The reaction was carried out at 60°C under nitrogen protection for 18 h. After realizing complete reaction by LCMS detection, concentration was carried out, and the crude product was purified with reversed-phase preparation (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain (4¹S,4³S)-1⁷-(dimethylphosphoryl)-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z27) (26 mg, yield of 36%). LCMS: MS m/z (ESI): [M+H]⁺= 563.2, t_{R} = 1.30 min.¹H NMR (400 MHz, DMSO-d₆): δ 11.98 (s, 1H), 9.50 - 9.00 (m, 1H), 8.62 (s, 1H), 8.25 (d, J = 5.2 Hz, 1H), 7.97 (s, 1H), 7.23 (dd, J = 11.2, 1.2 Hz, 1H), 4.22 - 3.84 (m, 2H), 3.53 (s, 3H), 3.22 - 3.05 (m, 2H), 3.00 (s, 3H), 2.92 - 2.84 (m, 1H), 2.68 - 2.66 (m, 1H), 2.59 (d, J = 4.0 Hz, 2H), 2.45 - 2.37 (m, 1H), 2.17 - 2.08 (m, 2H), 1.91 - 1.49 (m, 11H).

### Example 19 Synthesis of compound Z58

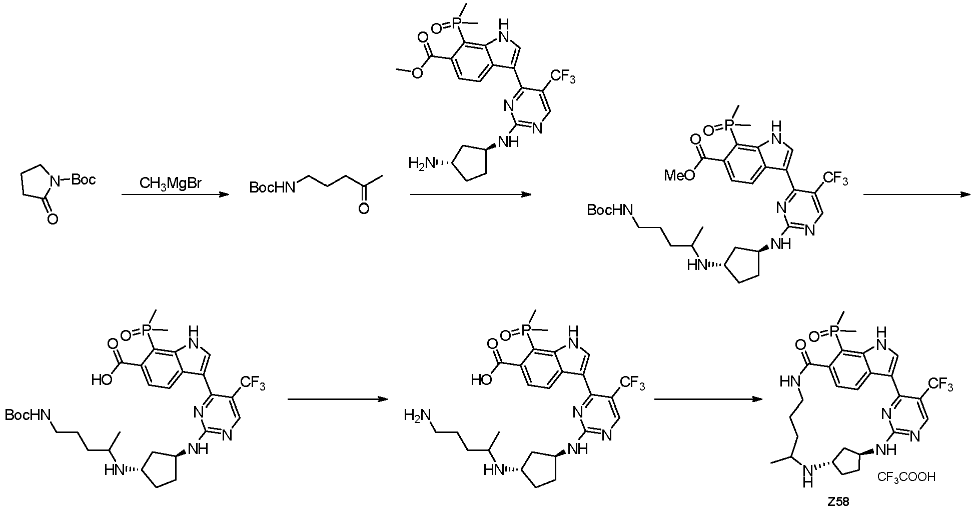

Step 1: Methyl magnesium bromide (15.1 mL, 15.1 mmol) was slowly dropwise added into a solution of 2-oxo-tetrahydropyrrole-1-carboxylic acid-2-methylpropan-2-yl ester (2.00 g, 10.8 mmol) in tetrahydrofuran (30 mL) at 0°C in a nitrogen atmosphere. The stirring was carried out to react at 0°C for 4 h. After realizing complete reaction by spot plate detection, water (30 mL) was added for quenching the reaction, and then extracting was carried out with ethyl acetate (100 mL of X3). Combined organic layers were washed with a saturated brine, dried over anhydrous sodium sulfate, filtered and concentrated to dryness. The resulting crude product was purified with silica gel column chromatography (petroleum ether/ethyl acetate = 10/1 to 3/1) to obtain a colorless oily substance [(4-oxo-pentyl)amino]methanoic acid-2-methylpropan-2-yl ester (1.20 g, yield of 56%). ¹H NMR (400 MHz, CD₃OD): δ 3.02 (t, J = 7.2 Hz, 2H), 2.50 (t, J = 7.2 Hz, 2H), 2.13 (s, 3H), 1.69 (p, J = 7.2 Hz, 1H), 1.43 (s, 9H).

Step 2: [(4-oxo-pentyl)amino]methanoic acid-2-methylpropan-2-yl ester (122 mg, 0.61 mmol) was dissolved in tetrahydrofuran (5 mL), then isopropyl titanate (574 mg, 2.0 mmol) and methyl 7-[dimethyl(oxo)-λ⁵-phosphoranyl]-3-(2-{[(1S,3S)-3-aminocyclopentyl]amino}-5-(trifluoromethyl)pyrimidin-4-yl)-1H-indole-6-carboxylate (250 mg, 0.51 mmol) were added, stirring was carried out to react at 70°C for 16 h, cooling was carried out to reach room temperature, sodium cyanoborohydride (159 mg, 2.53 mmol) was added, and stirring was carried out at 25°C for 6 h. After realizing complete reaction by LCMS detection, a reaction solution was filtered to remove solid impurities. Organic phases were concentrated to obtain a crude product. Then the crude product was purified with thin-layer chromatography (dichloromethane/methanol = 10/1) to obtain a yellow oily substance methyl 7-[dimethyl(oxo)-λ⁵-phosphoranyl]-3-(2-{[(1S,3S)-3-[(2,2-dimethyl-4-oxo-5-aza-3-oxadeca-9-yl)amino]cyclopentyl]amino}-5-(trifluoromethyl)pyrimidin-4-yl)-1H-indole-6-carboxylate (270 mg, yield of 79%). LCMS: m/z (ESI): [M+H]⁺= 681.4, t_{R} = 0.99 min.

Step 3: Methyl 7-[dimethyl(oxo)-λ⁵-phosphoranyl]-3-(2-{[(1S,3S)-3-[(2,2-dimethyl-4-oxo-5-aza-3-oxadeca-9-yl)amino]cyclopentyl]amino}-5-(trifluoromethyl)pyrimidin-4-yl)-1H-indole-6-carboxylate (220 mg, 0.32 mmol) was dissolved in a mixed system of water (2 mL), methanol (2 mL) and tetrahydrofuran (2 mL), lithium hydroxide (1 crystal water, 68 mg, 1.62 mmol) was added, and stirring was carried out to react at 40°C for 14 h. After realizing complete reaction by LCMS detection, the reaction solution was directly applied to the next reaction without any treatment. LCMS: m/z (ESI): [M+H]⁺= 667.4, t_{R} = 0.91 min.

Step 4: A hydrogen chloride/1,4-dioxane solution (5 mL, 4 M) was added into the reaction solution resulted from step 3, and the system was stirred at 40°C for 4 h. After realizing complete reaction by LCMS detection, a solvent was directly spin-dried to obtain a crude product, and reversed-phase preparation was carried out on the crude product (chromatographic column: Ultimate XB-C18, 50X250 mm, 10 um (PARP-02), mobile phase: acetonitrile-water (0.1% trifluoroacetic acid), flow rate: 70 mL/min and column temperature: 25°C) to obtain a white solid 7-[dimethyl(oxo)-λ⁵-phosphoranyl]-3-(2-{[(1S,3S)-3-[(5-aminopent-2-yl)amino]cyclopentyl]amino}-5-(trifluoromethyl)pyrimidin-4-yl)-1H-indole-6-carboxylic acid (150 mg, yield of 94%). LCMS: m/z (ESI): [M+H]⁺= 567.2, t_{R} = 0.87 min.

Step 5: 2-(7-azabenzotriazol)-N,N,N',N'-tetramethyluronium hexafluorophosphate (47 mg, 0.12 mmol) and N,N-diisopropylethylamine (64 mg, 0.50 mmol) were sequentially added into a solution of 7-[dimethyl(oxo)-λ⁵-phosphoranyl]-3-(2-{[(1S,3S)-3-[(5-aminopent-2-yl)amino]cyclopentyl]amino}-5-(trifluoromethyl)pyrimidin-4-yl)-1H-indole-6-carboxylic acid (70 mg, 0.12 mmol) in N,N-dimethylformamide (3 mL), and the reaction solution was stirred at 80°C for 18 h. After realizing complete reaction by LCMS detection, the reaction solution was concentrated, and reversed-phase preparation was carried out on the resulting crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain (4¹S,4³S)-1⁷-(dimethylphosphoryl)-6-methyl-2⁵-(trifluoromethyl)-1¹H-3,5,10-triaza-1(3,6)-indola-2(4,2)-pyrimidina-4(1,3)-cyclopentanacycloundecaphan-11-one (Z58) (10.50 mg, yield of 15%, as trifluoroacetate). LCMS: m/z (ESI): [M+H]⁺= 549.2, t_{R} = 0.69 min.¹H NMR (400 MHz, CD₃OD): δ 8.58 (s, 1H), 8.51 - 8.36 (m, 1H), 7.97 (s, 1H), 7.45 - 7.29 (m, 1H), 4.42 - 4.11 (m, 1H), 3.95 - 3.74 (m, 1H), 3.30 - 3.17 (m, 1H), 3.13 - 2.99 (m, 1H), 2.98 - 2.74 (m, 2H), 2.66 - 2.50 (m, 1H), 2.36 - 2.27 (m, 1H), 2.27 - 2.19 (m, 1H), 2.15 - 2.05 (m, 1H), 2.03 - 1.96 (m, 6H), 1.95 - 1.85 (m, 1H), 1.80 - 1.66 (m, 2H), 1.56 - 1.50 (m, 1H), 1.47 - 1.34 (m, 1H), 1.26 (t, J = 6.0 Hz, 3H).

### Example 20 Synthesis of compound Z59

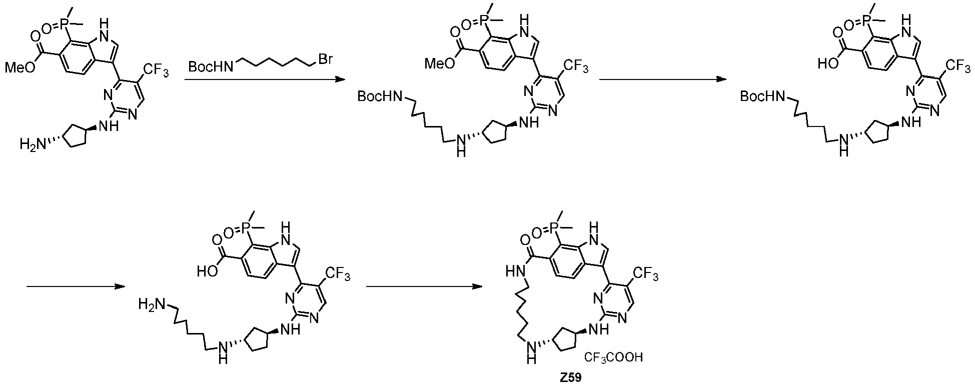

Step 1: [(6-bromohexyl)amino]methanoic acid-2-methylpropyl-2-yl ester (353 mg, 0.71 mmol) and N,N-diisopropylethylamine (460 mg, 3.57 mmol) were added into a solution of methyl 7-[dimethyl(oxo)-λ⁵-phosphoranyl]-3-(2-{[(1S,3S)-3-aminocyclopentyl]amino}-5-(trifluoromethyl)pyrimidin-4-yl)-1H-indole-6-carboxylate (200 mg, 0.71 mmol) in N,N-dimethylformamide (6 mL) at 25°C. Stirring was carried out to react at 80°C under nitrogen protection for 16 h. After realizing complete reaction by LCMS detection, water (50 mL) was added into the reaction solution, and extracting was carried out with ethyl acetate (50 mL X3). Combined organic layers were washed with a saturated brine (50 mL), dried over anhydrous sodium sulfate, filtered and concentrated. The resulting crude product was purified with silica gel column chromatography (petroleum ether/ethyl acetate = 10/1 to 3/1) to obtain a colorless oily substance methyl 7-[dimethyl(oxo)-λ⁵-phosphoranyl]-3-(2-{[(1S,3S)-3-[(2,2-dimethyl-4-oxo-5-aza-3-oxaundecan-11-yl)amino]cyclopentyl]amino}-5-(trifluoromethyl)pyrimidin-4-yl)-1H-indole-6-carboxylate (180 mg, yield of 33%). LCMS: m/z (ESI): [M+H]⁺= 694.7, t_{R} = 1.33 min.

Step 2: Lithium hydroxide hydrate (109 mg, 2.59 mmol) was added into methyl 7-[dimethyl(oxo)-λ⁵-phosphoranyl]-3-(2-{[(1S,3S)-3-[(2,2-dimethyl-4-oxo-5-aza-3-oxaundecan-11-yl)amino]cyclopentyl]amino}-5-(trifluoromethyl)pyrimidin-4-yl)-1H-indole-6-carboxylate (180 mg, 0.26 mmol) in methanol (3 mL), water (3 mL) and tetrahydrofuran (3 mL) at room temperature. Stirring was carried out to react at 40°C under nitrogen protection for 16 h. After realizing complete reaction by LCMS detection, concentrating was carried out to dryness to obtain a crude white solid 7-[dimethyl(oxo)-λ⁵-phosphoranyl]-3-(2-{[(1*S*,3*S*)-3-[(2,2-dimethyl-4-oxo-5-aza-3-oxaundecan-11-yl)amino]cyclopentyl]amino}-5-(trifluoromethyl)pyrimidin-4-yl)-1*H*-indole-6-carboxylic acid (180 mg, crude product). LCMS: MS m/z (ESI): [M+H]⁺= 681.3, t_{R} = 0.87 min.

Step 3: Methanol in hydrochloric acid (1.12 mL, 4 mol/L) was added into 7-[dimethyl(oxo)-λ⁵-phosphoranyl]-3-(2-{[(1*S*,3*S*)-3-[(2,2-dimethyl-4-oxo-5-aza-3-oxaundecan-11-yl)amino]cyclopentyl]amino}-5-(trifluoromethyl)pyrimidin-4-yl)-1*H*-indole-6-carboxylic acid (160 mg, 0.24 mmol) in methanol (4 mL) at room temperature. The reaction solution was stirred to react at 40°C under nitrogen protection for 16 h. After realizing complete reaction by LCMS detection, concentrating was carried out to remove hydrochloric acid, the resulting crude product was purified with reversed-phase preparation (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid 7-[dimethyl(oxo)-λ⁵-phosphoranyl]-3-(2-{[(1S,3S)-3-[(6-aminohexyl)amino]cyclopentyl]amino}-5-(trifluoromethyl)pyrimidin-4-yl)-1H-indole-6-carboxylic acid (80 mg, yield of 56%). LCMS: MS m/z (ESI): [M+H]⁺ =581.4, t_{R} = 0.68 min.

Step 4: 2-(7-azobenzoftriazole)-N,N,N',N'-tetramethyluronium hexafluorophosphate (55.2 mg, 0.15 mmol) and N,N-diisopropylethylamine (78 mg, 0.61 mmol) were sequentially added into 7-[dimethyl(oxo)-λ⁵-phosphoranyl]-3-(2-{[(1S,3S)-3-[(6-aminohexyl)amino]cyclopentyl]amino}-5-(trifluoromethyl)pyrimidin-4-yl)-1H-indole-6-carboxylic acid (70 mg, 0.12 mmol) in N,N-dimethylformamide (5 mL) at room temperature. The reaction was carried out at 60°C under nitrogen protection for 16 h. After realizing complete reaction by LCMS detection, the crude product obtained by concentrated was purified with reversed phase (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain (4¹S,4³S)-1⁷-(dimethylphosphoryl)-2⁵-(trifluoromethyl)-1¹H-3,5,12-triaza-1(3,6)-indola-2(4,2)-pyrimidina-4(1,3)-cyclopentanacyclotridecaphan-13-one (Z59) 2,2,2-trifluoroacetic acid (14 mg, yield of 18%). LCMS: MS m/z (ESI): [M+H]⁺= 563.0, t_{R} = 0.68 min.¹H NMR (400 MHz, DMSO-*d₆*): δ 11.99 (s, 1H), 8.62 (s, 1H), 8.60 - 8.57 (m, 1H), 8.51 (d, J = 8.4 Hz, 1H), 8.04 (s, 1H), 7.46 (dd, J = 8.4, 3.2 Hz, 1H), 4.27 - 4.20 (m, 1H), 3.73 - 3.71 (m, 1H), 3.63 - 3.55 (m, 2H), 3.19 - 3.14 (m, 1H), 2.95 - 2.92 (m, 2H), 2.18 - 2.05 (m, 2H), 2.01- 1.89 (m, 7H), 1.82- 1.74 (m, 1H), 1.69 - 1.48 (m, 9H).

### Example 21 Synthesis of compound Z60

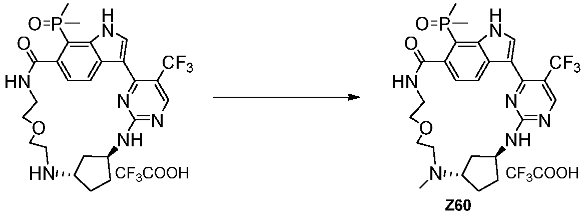

Step 1: Paraformaldehyde (20 mg, 0.22 mmol) and isopropyl titanate (10.3 mg, 0.036 mmol) were sequentially added into a solution (5 mL) of (4¹S,4³S)-1⁷-(dimethylphosphoryl)-2⁵-(trifluoromethyl)-1¹H-8-oxa-3,5,11-triaza-1(3,6)-indole-2(4,2)-pyrimidin-4(1,3)-cyclopentanecyclododecan-12-one-2,2,2-trifluoroacetate (20 mg, 0.030 mmol) in tetrahydrofuran at room temperature. Stirring was carried out to react at 60°C under nitrogen protection for 14 h. The reaction solution was cooled to 30°C, sodium cyanoborohydride (20 mg, 0.318 mmol) was added, and reacting was carried out for 4 h at a constant temperature. After realizing complete reaction by LCMS detection, the reaction solution was concentrated, reversed-phase preparation was carried out on the resulting crude product (chromatographic column: Ultimate XB-C18, 50X250 mm, 10 um (PARP-02), mobile phase: acetonitrile-water (0.1% trifluoroacetic acid), flow rate: 70 mL/min and column temperature: 25°C) to obtain (4¹S,4³S)-1⁷-(dimethylphosphoryl)-5-methyl-2⁵-(trifluoromethyl)-1¹H-8-oxa-3,5,11-triaza-1(3,6)-indola-2(4,2)-pyrimidina-4(1,3)-cyclopentanacyclododecaphan-12-one (Z60) (7.8 mg, yield of 38%). LCMS: MS m/z (ESI): [M+H]⁺= 565.2, t_{R} = 0.73 min.¹H NMR (400 MHz, CD₃OD): δ 8.58 (s, 1H), 8.50 - 8.28 (m, 1H), 7.94 - 7.89 (m, 1H), 7.69 - 7.50 (m, 1H), 4.28 - 4.15 (m, 1H), 3.99 - 3.68 (m, 6H), 3.52 - 3.42 (m, 1H), 3.24 - 3.14 (m, 1H), 2.97 - 2.85 (m, 3H), 2.78 - 2.59 (m, 1H), 2.37 - 2.15 (m, 3H), 2.14 - 1.92 (m, 7H), 1.87 - 1.70 (m, 2H).

### Example 22 Synthesis of compound Z61

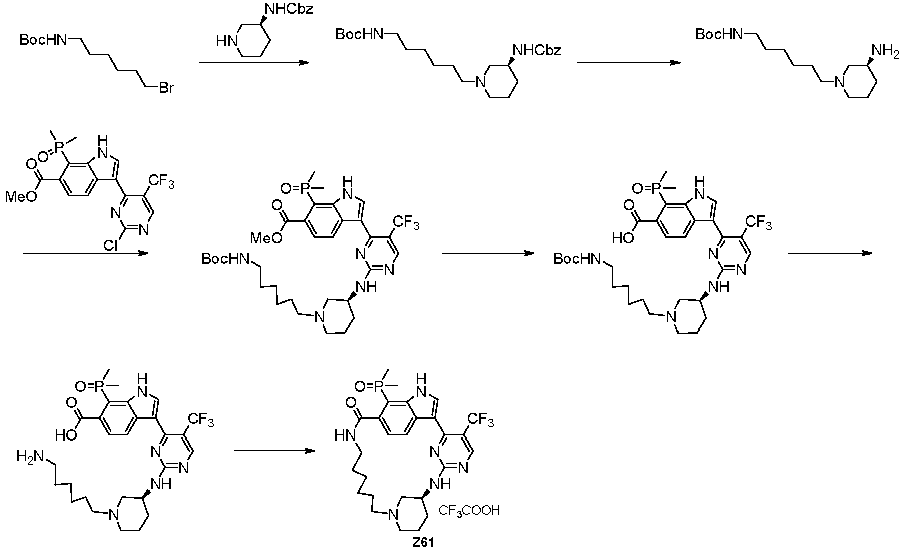

Step 1: Benzyl {[(3S)-hexahydropyridin-3-yl]amino}carboxylate (395 mg, 1.41 mmol) and N,N-diisopropylethylamine (662 mg, 5.12 mmol) were added into a solution of [(6-bromohexyl)amino]methanoic acid-2-methylpropyl-2-yl ester (300 mg, 1.28 mmol) in N,N-dimethylformamide (8 mL) at 25°C. The reaction solution was stirred to react at 80°C under nitrogen protection for 6 h. After realizing complete reaction by LCMS detection, water (30 mL) was added into the reaction solution, and extracting was carried out with ethyl acetate (50 mL X3). Combined organic layers were washed with a saturated brine (50 mL), dried over anhydrous sodium sulfate, filtered and concentrated. The resulting crude product was purified with silica gel column chromatography (dichloromethane/methanol = 30/1 to 10/1) to obtain a colorless oily substance benzyl {[(3S)-1-(2,2-dimethyl-4-oxo-5-aza-3-oxa-undecan-11-yl)hexahydropyridin-3-yl]amino}carboxylate (555 mg, yield of 92%). LCMS: m/z (ESI): [M+H]⁺= 434.2, t_{R} = 1.57 min.

Step 2: Benzyl {[(3S)-1-(2,2-dimethyl-4-oxo-5-aza-3-oxa-undecan-11-yl)hexahydropyridin-3-yl]amino}carboxylate (555 mg, 1.17 mmol) was dissolved in a methanol (10 mL) solution, and palladium on carbon (60 mg, 10% loading, 60% of water content) was added. The reaction solution was stirred to react for 3 hours at 25 °C under hydrogen protection. After realizing complete reaction by LCMS detection, the reaction solution was filtered and concentrated to obtain a colorless oily substance ({6-[(3S)-3-aminohexahydropyridin-1-yl]hexyl}amino)methanoic acid-2-methylpropyl-2-yl ester (350 mg, crude product). LCMS: m/z (ESI): [M+H]⁺= 300.0, t_{R} = 0.98 min.

Step 3: Methyl 3-[2-chloro-5-(trifluoromethyl)pyrimidin-4-yl]-7-[dimethyl(oxo)-λ⁵-phosphoranyl]-1H-indole-6-carboxylate (404 mg, 0.94 mmol) and N,N-diisopropylethylamine (483 mg, 3.74 mmol) were sequentially added into a solution of {6-[(3S)-3-aminohexahydropyridin-1-yl]hexyl}amino (280 mg, 0.94 mmol) in N,N-dimethylformamide (8 mL) at room temperature, and the reaction solution was stirred to react at 80°C under nitrogen protection for 16 h. After realizing complete reaction by LCMS detection, an aqueous solution (40 mL) was added into the reaction solution, and extracting was carried out with dichloromethane (50 mL X3). Combined organic layers were dried over anhydrous sodium sulfate, filtered and concentrated to dryness. The resulting crude product was purified with silica gel column chromatography (dichloromethane/methanol = 30/1 to 10/1) to obtain a yellow oily substance methyl 7-[dimethyl(oxo)-λ⁵-phosphoranyl]-3-(2-{[(3S)-1-(2,2-dimethyl-4-oxo-5-aza-3-oxaundecan-11-yl)hexahydropyridin-3-yl]amino}-5-(trifluoromethyl)pyrimidin-4-yl)-1H-indole-6-carboxylate (473 mg, yield of 72%). LCMS: MS m/z (ESI): [M+H]⁺ =695.4, t_{R} = 1.15 min.

Step 4: Lithium hydroxide hydrate (74 mg, 1.75 mmol) was added into methyl 7-[dimethyl(oxo)-λ⁵-phosphoranyl]-3-(2-{[(3S)-1-(2,2-dimethyl-4-oxo-5-aza-3-oxaundecan-11-yl)hexahydropyridin-3-yl]amino}-5-(trifluoromethyl)pyrimidin-4-yl)-1H-indole-6-carboxylate (243 mg, 0.35 mmol) in methanol (3 mL), water (3 mL) and tetrahydrofuran (3 mL), and the reaction solution was stirred to react at 40°C under nitrogen protection for 14 h. After realizing complete reaction by LCMS detection, concentrating was carried out to dryness so as to obtain a crude yellow solid 7-[dimethyl(oxo)-λ⁵-phosphoranyl]-3-(2-{[(3S)-1-(2,2-dimethyl-4-oxo-5-aza-3-oxaundecan-11-yl)hexahydropyridin-3-yl]amino}-5-(trifluoromethyl)pyrimidin-4-yl)-1H-indole-6-carboxylic acid (320 mg, crude product, purity of 90%). LCMS: MS m/z (ESI): [M+H]⁺ =680.8, t_{R} = 1.21 min.

Step 5: A hydrogen chloride/methanol solution (6 mL, 4 mol/L) was added into a solution of 7-[dimethyl(oxo)-λ⁵-phosphoranyl]-3-(2-{[(3S)-1-(2,2-dimethyl-4-oxo-5-aza-3-oxaundecan-11-yl)hexahydropyridin-3-yl]amino}-5-(trifluoromethyl)pyrimidin-4-yl)-1H-indole-6-carboxylic acid (420 mg, crude product, purity of 90%) in methanol (4 mL) at room temperature. The reaction solution was stirred to react at 40°C under nitrogen protection for 4 h. After realizing complete reaction by LCMS detection, concentrating was carried out to remove hydrochloric acid, and the resulting crude product was purified with reversed-phase preparation (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid substance 7-[dimethyl(oxo)-λ⁵-phosphoranyl]-3-(2-λ[(3S)-1-(6-amino hexyl)hexahydropyridin-3-yl]amino}-5-(trifluoromethyl)pyrimidin-4-yl)-1H-indole-6-carboxylic acid (275 mg, yield of 85%). LCMS: MS m/z (ESI): [M+H]⁺= 581.4, t_{R} = 0.64 min.

Step 6: 2-(7-azobenzotriazole)-N,N,N',N'-tetramethyluronium hexafluorophosphate (65.4 mg, 0.17 mmol) and N,N-diisopropylethylamine (88.9 mg, 0.69 mmol) were sequentially added into 7-[dimethyl(oxo)-λ⁵-phosphoranyl]-3-(2-{[(3S)-1-(6-aminohexyl)hexahydropyridin-3-yl]amino}-5-(trifluoromethyl)pyrimidin-4-yl)-1H-indole-6-carboxylic acid (100 mg, 0.17 mmol) in N,N-dimethylformamide (3 mL) at room temperature, and the reaction solution was reacted at 80°C for 16 h under liquid nitrogen protection. After realizing complete reaction by LCMS detection, the crude product obtained by concentration was purified with reversed-phase preparation (chromatographic column: Ultimate XB-C18, 50X250 mm, 10 um (PARP-02), mobile phase (acetonitrile-water (0.1% trifluoroacetic acid), flow rate of 70 mL/min and column temperature of 25°C) to obtain (4³S)-1⁷-(dimethylphosphoryl)-2⁵-(trifluoromethyl)-1¹H-3,11-diaza-1(3,6)-indola-2(4,2)-pyrimidina-4(3,1)-piperidinacyclododecaphan-12-one (Z61) (18.90 mg, yield of 19%, as trifluoroacetate). LCMS: MS m/z (ESI): [M+H]⁺= 563.2, t_{R} = 0.67 min.¹H NMR (400 MHz, CD₃OD): δ 8.64 (s, 1H), 8.22 (dd, J = 8.4, 1.2 Hz, 1H), 7.91 (s, 1H), 7.65 (dd, J = 8.4, 3.6 Hz, 1H), 4.22 - 4.02 (m, 1H), 4.01 - 3.49 (m, 3H), 3.29 - 3.21 (m, 1H), 3.19 - 3.09 (m, 2H), 2.95 - 2.87 (m, 1H), 2.87 - 2.69 (m, 1H), 2.16 - 1.89 (m, 9H), 1.80 - 1.27 (m, 9H).

### Example 23 Synthesis of compound Z56

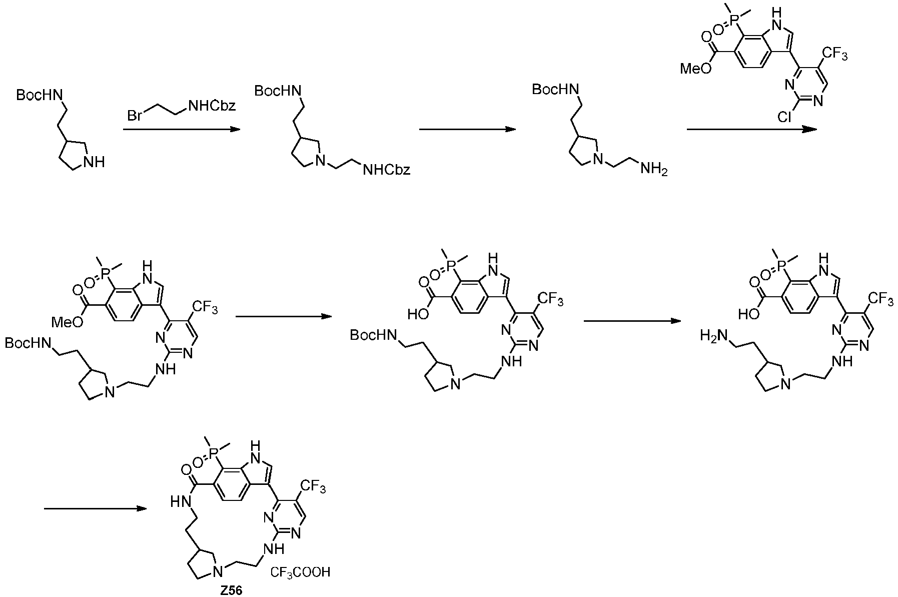

Step 1: Benzyl [(2-bromoethyl)amino]carboxylate (241 mg, 0.93 mmol) and N,N-diisopropylethylamine (602 mg, 4.67 mmol) were added into a solution of { [2-(tetrahydro-1H-pyrrol-3-yl)ethyl]amino}methanoic acid-2-methylpropyl-2-yl ester (200 mg, 0.93 mmol) in N,N-dimethylformamide (6 mL) at 25°C. The reaction solution was stirred to react at 80°C under nitrogen protection for 5 h. After realizing complete reaction by LCMS detection, water (20 mL) was added into the reaction solution, and extracting was carried out with ethyl acetate (50 mL X 3). Combined organic layer was washed with saturated brine (20 mL), dried over anhydrous sodium sulfate, and filtered and concentrated. The resulting crude product was purified with silica gel column chromatography (petroleum ether/ethyl acetate = 10/1 to 3/1) to obtain a colorless oily substance ({2-[1-(3-oxo-1-phenyl-4-aza-2-oxa-hexyl-6-yl)tetrahydro-1H-pyrrol-3-yl]ethyl}amino)methanoic acid-2-methylpropyl-2-yl ester (218 mg, yield of 54%). LCMS: m/z (ESI): [M+H]⁺= 392.4, t_{R} = 1.40 min.

Step 2: Pd/C (20 mg, 10% loading, 60% of water content) was added into {2-[1-(3-oxo-1-phenyl-4-aza-2-oxa-hexyl-6-yl)tetrahydro-1H-pyrrol-3-yl]ethyl}amino)methanoic acid-2-methylpropyl-2-yl ester (200 mg, 0.51 mmol) in methanol at room temperature. The reaction solution was stirred to react at 25°C under hydrogen protection for 5 h. After realizing complete reaction by LCMS detection, concentrating and drying were carried out to obtain a crude white solid {2-[1-(2-aminoethyl)tetrahydro-1H-pyrrol-3-yl]ethyl}amino)methanoic acid-2-methylpropyl-2-yl ester (100 mg, yield of 53%). LCMS: MS m/z (ESI): [M+H]⁺= 258.2, t_{R} = 0.47 min.

Step 3: Methyl 3-[2-chloro-5-(trifluoromethyl)pyrimidin-4-yl]-7-[dimethyl(oxo)-λ⁵-phosphoranyl]-1H-indole-6-carboxylate (168 mg, 0.39 mmol) and N,N-diisopropylethylamine (150 mg, 1.17 mmol) were added into ({2-[1-(2-aminoethyl)tetrahydro-1H-pyrrol-3-yl]ethyl}amino)methanoic acid-2-methylpropyl-2-yl ester (100 mg, 0.39 mmol) in N,N-dimethylformamide (6 mL) at room temperature. The reaction solution was stirred to react at 80°C under nitrogen protection for 16 h. After realizing complete reaction by LCMS detection, water (20 mL) was added into the reaction solution, and then extracting was carried out with ethyl acetate (30 mL X 3). Combined organic layers were washed with saturated brine (30 mL), dried over anhydrous sodium sulfate, and filtered and concentrated. The resulting crude product was purified with silica gel column chromatography (petroleum ether/ethyl acetate = 10/1 to 3/1) to obtain a colorless oily substance methyl 7-[dimethyl(oxo)-λ⁵-phosphoranyl]-3-[2-({2-[3-(2,2-dimethyl-4-oxo-5-aza-3-oxahept-7-yl)tetrahydro-1H-pyrrol-1-yl]ethyl}amino)-5-(trifluoromethyl)pyrimidin-4-yl]-1H-indole-6-carboxylate (220 mg, yield of 78%). LCMS: MS m/z (ESI): [M+H]⁺ = 653.2, t_{R} = 1.41 min.

Step 4: Lithium hydroxide hydrate (141 mg, 3.37 mmol) was added into methyl 7-[dimethyl(oxo)-λ⁵-phosphoranyl]-3-[2-({2-[3-(2,2-dimethyl-4-oxo-5-aza-3-oxahept-7-yl)tetrahydro-1H-pyrrol-1-yl]ethyl}amino)-5-(trifluoromethyl)pyrimidin-4-yl]-1H-indole-6-carboxylate (220 mg, 0.34 mmol) in methanol (3 mL), water (3 mL) and tetrahydrofuran (3 mL) at room temperature. Stirring was carried out to react at 40°C under nitrogen protection for 16 h. After realizing complete reaction by LCMS detection, concentrating was carried out to dryness so as to obtain a crude white solid 7-[dimethyl(oxo)-λ⁵-phosphoranyl]-3-[2-({2-[3-(2,2-dimethyl-4-oxo-5-aza-3-oxahept-7-yl)tetrahydro-1H-pyrrol-1-yl]ethyl}amino)-5-(trifluoromethyl)pyrimidin-4-yl]-1H-indole-6-carboxylic acid (200 mg). LCMS: MS m/z (ESI): [M+H]⁺ = 638.7, t_{R} = 1.11 min.

Step 5: 1,4-dioxane in hydrochloric acid (0.78 mL, 3.13 mmol, 4 mol/L) was added into 7-[dimethyl(oxo)-λ⁵-phosphoranyl]-3-[2-({2-[3-(2,2-dimethyl-4-oxo-5-aza-3-oxahept-7-yl)tetrahydro-1H-pyrrol-1-yl]ethyl}amino)-5-(trifluoromethyl)pyrimidin-4-yl]-1H-indole-6-carboxylic acid (200 mg, 0.31 mmol) in 1,4-dioxane (4 mL) at room temperature. Stirring was carried out at 40°C under nitrogen protection for 3 h. After realizing complete reaction by LCMS detection, concentrating was carried out, the crude product was purified with reversed-phase preparation (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid 3-[2-({2-[3-(2-aminoethyl)tetrahydro-1H-pyrrol-1-yl]ethyl}amino)-5-(trifluoromethyl)pyrimidin-4-yl]-7-[dimethyl(oxo)-λ5-phosphoranyl]-1H-indole-6-carboxylic acid (70 mg, yield of 37%). LCMS: MS m/z (ESI): [M+H]⁺ =538.8, t_{R} = 0.98 min.

Step 6: 2-(7-azobenzotriazole)-N,N,N',N'-tetramethyluronium hexafluorophosphate (51 mg, 0.13 mmol) and N,N-diisopropylethylamine (43 mg, 0.33 mmol) were sequentially added into 3-[2-({2-[3-(2-aminoethyl)tetrahydro-1H-pyrrol-1-yl]ethyl}amino)-5-(trifluoromethyl)pyrimidin-4-yl]-7-[dimethyl(oxo)-λ⁵-phosphoranyl]-1H-indole-6-carboxylic acid (70 mg, 0.11 mmol) in N,N-dimethylformamide (5 mL) at room temperature. Reacting was carried out at 65°C for 3 h in a nitrogen atmosphere. After realizing complete reaction by LCMS detection, concentrating was carried out. The crude product was purified with reversed-phase preparation (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain 1⁷-(dimethylphosphoryl)-2⁵-(trifluoromethyl)-1¹H-3,9-diaza-1(3,6)-indola-2(4,2)-pyrimidina-6(1,3)-pyrrolidinacyclodecaphan-10-one (Z56) (6.0 mg, yield of 10%, as trifluoroacetate). LCMS: MS m/z (ESI): [M+H]⁺= 521.3, t_{R} = 0.71 min.¹H NMR (400 MHz, CD₃OD): δ 8.65 (s, 1H), 8.58 - 8.43 (m, 1H), 8.03 (s, 1H), 7.57 (s, 1H), 4.09 - 3.82 (m, 3H), 3.63 - 3.36 (m, 4H), 3.18 - 2.98 (m, 2H), 2.78 - 2.57 (m, 1H), 2.49 - 2.35 (m, 1H), 2.23 - 1.99 (m, 7H), 1.70 - 1.14 (m, 3H).

### Example 24 Synthesis of compound Z64

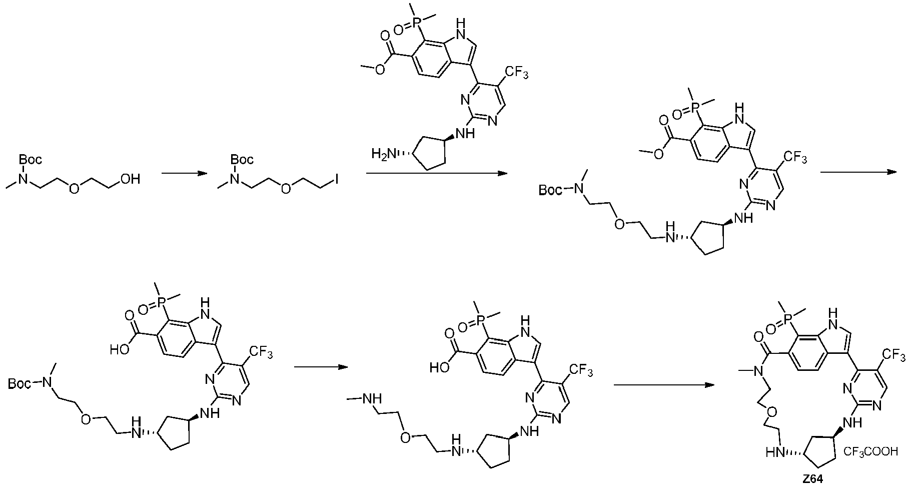

Step 1: Imidazole (140 mg, 2.05 mmol), triphenylphosphine (431 mg, 1.64 mmol) and iodine (521 mg, 2.05 mmol) were sequentially added into a solution of (1-hydroxy-6-aza-3-oxahept-6-yl)methanoic acid-2-methylpropyl-2-yl ester (300 mg, 1.37 mmol) in dichloromethane (10 mL) in a 50 mL single-neck flask. Stirring was carried out at 5°C under nitrogen protection for 2 h. After realizing complete reaction by LCMS detection, water (20 mL) was added into the reaction solution for dilution. Then the reaction solution was extracted with dichloromethane (10 mL X 3), dried over anhydrous sodium sulfate, and concentrated to obtain a crude product. The crude product was purified with silica gel column chromatography (petroleum ether: ethyl acetate = 5:1) to obtain a light yellow oily substance (1-iodo-6-aza-3-oxahept-6-yl)methanoic acid-2-methylpropyl-2-yl ester (314 mg, yield of 70%). LCMS: m/z (ESI): [M-100+H]⁺= 230.0, t_{R} = 1.20 min.

Step 2: Methyl 7-[dimethyl(oxo)-λ⁵-phosphoranyl]-3-(2-{[(1*S*,3*S*)-3-aminocyclopentyl]amino}-5-(trifluoromethyl)pyrimidin-4-yl)-1*H*-indole-6-carboxylate (553 mg, 1.12 mmol) and N,N-diisopropylethylamine (0.88 mL, 5.32 mmol) were added into a solution of (1-iodo-6-aza-3-oxahept-6-yl)methanoic acid-2-methylpropyl-2-yl ester (350 mg, 1.06 mmol) in N,N-dimethylformamide (5 mL) in a 50 mL single-neck flask. Stirring was carried out at 100°C under nitrogen protection for 16 h. After realizing complete reaction by LCMS detection, water (30 mL) was added into the reaction solution for dilution. The reaction solution was extracted with ethyl acetate (15 mL X 3), dried over anhydrous sodium sulfate, and concentrated to obtain a crude product. The crude product was purified with silica gel column chromatography (dichloromethane: methanol = 100:6) to obtain a light yellow solid methyl 7-[dimethyl(oxo)-λ⁵-phosphoranyl]-3-(2-{[(1S,3*S*)-3-[(2,2,5-trimethyl-4-oxo-5-aza-3,8-dioxadeca-10-yl)amino]cyclopentyl]amino}-5-(trifluoromethyl)pyrimidin-4-yl)-1*H*-indole-6-carboxylate (610 mg, yield of 82%). LCMS: m/z (ESI): [M+H]⁺= 697.4, t_{R} = 0.97 min.

Step 3: Methyl 7-[dimethyl(oxo)-λ⁵-phosphoranyl]-3-(2-{[(1*S*,3*S*)-3-[(2,2,5-trimethyl-4-oxo-5-aza-3,8-dioxadeca-10-yl)amino]cyclopentyl]amino}-5-(trifluoromethyl)pyrimidin-4-yl)-1*H*-indole-6-carboxylate (610 mg, 0.88 mmol) was dissolved in a mixed system of water (3 mL), methanol (3 mL) and tetrahydrofuran (3 mL). Lithium hydroxide monohydrate (184 mg, 4.38 mmol) was added, and stirring was carried out at 40°C for 16 h. After realizing complete reaction by LCMS detection, a reaction solution containing 7-[dimethyl(oxo)-λ⁵-phosphoranyl]-3-(2-{[(1*S*,3*S*)-3-[(2,2,5-trimethyl-4-oxo-5-aza-3,8-dioxadeca-10-yl)amino]cyclopentyl]amino}-5-(trifluoromethyl)pyrimidin-4-yl)-1*H*-indole-6-carboxylic acid was obtained, the reaction solution was directly used in the next reaction without treatment. LCMS: m/z (ESI): [M+H]⁺= 683.3, t_{R} = 0.86 min.

Step 4: A solution (5 mL, 4 M) of hydrochloric acid in methanol was added into the reaction solution from the previous step, and stirring was carried out at 5°C for 5 h. After realizing complete reaction by LCMS detection, the reaction solution was concentrated. Reversed-phase preparation was carried out on a crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid 7-[dimethyl(oxo)-λ⁵-phosphoranyl]-3-(2-{[(1*S*,3*S*)-3-(6-aza-3-oxahept-1-ylamino)cyclopentyl]amino}-5-(trifluoromethyl)pyrimidin-4-yl)-1*H*-indole-6-carboxylic acid (260 mg, 2-step yield of 51%). LCMS: m/z (ESI): [M+H]⁺= 582.6, t_{R} = 0.92 min.

Step 5: 2-(7-azabenzotriazole)-N,N,N',N'-tetramethyluronium hexafluorophosphate (187 mg, 0.49 mmol) and N,N-diisopropylethylamine (0.37 mL,2.23 mmol) were sequentially added into a solution of 7-[dimethyl(oxo)-λ⁵-phosphoranyl]-3-(2-{[(1*S*,3*S*)-3-(6-aza-3-oxahept-1-ylamino)cyclopentyl]amino}-5-(trifluoromethyl)pyrimidin-4-yl)-1*H*-indole-6-carboxylic acid (260 mg, 0.45 mmol) in N,N-dimethylformamide (10 mL). Stirring was carried out to react at 60°C for 16 h. After realizing complete reaction by LCMS detection, the reaction solution was concentrated. Reversed-phase preparation was carried out on the crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a light yellow solid (4¹S,4³S)-1⁷-(dimethylphosphoryl)-11-methyl-2⁵-(trifluoromethyl)-1¹H-8-oxa-3,5,11-triaza-1(3,6)-indola-2(4,2)-pyrimidina-4(1,3)-cyclopentanacyclododecaphan-12-one (Z64) (120 mg, yield of 48%, as trifluoroacetate). LCMS: m/z (ESI): [M+H]⁺= 564.8, t_{R} = 0.97 min.¹H NMR (400 MHz, CD₃OD): δ 8.57 (s, 1H), 8.43 (br, 1H), 7.96 (s, 1H), 7.44 (dd, *J* = 8.4, 3.2 Hz, 1H), 4.36-4.39 (m, 1H), 3.88-3.91 (m, 1H), 3.78-3.83 (m, 2H), 3.53-3.72 (m, 3H), 3.67-3.42 (m, 1H), 3.23-3.61 (m, 1H), 3.13 (s, 3H), 2.65-2.71 (m, 1H), 2.32-2.39 (m, 1H), 2.21-2.23 (m, 1H), 2.14 - 1.62 (m, 10H).

### Example 25 Synthesis of compound Z63

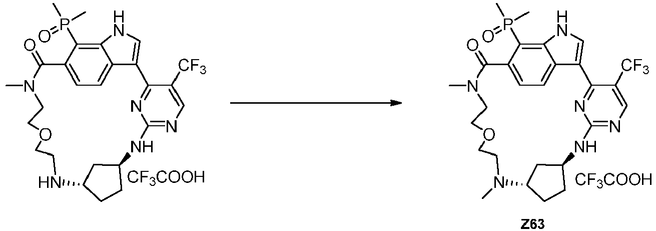

Step 1: Paraformaldehyde (9.30 mg, 0.31 mmol) and tetraisopropyl titanate (35.2 mg, 0.12 mmol) were sequentially added into a solution of (4¹S,4³S)-1⁷-(dimethylphosphoryl)-11-methyl-2⁵-(trifluoromethyl)-1¹H-8-oxa-3,5,11-triaza-1(3,6)-indola-2(4,2)-pyrimidina-4(1,3)-cyclopentanacyclododecaphan-12-one trifluoroacetate (35 mg, 0.06 mmol) in tetrahydrofuran (3 mL) in a 10 mL single-neck flask. The reaction solution was stirred at 70°C for 16 h. The reaction solution was cooled to 35°C, sodium cyanoborohydride (7.79 mg, 0.12 mmol) was added, and stirring continued at 35°C for 3 h. After realizing complete reaction by LCMS detection, the reaction solution was concentrated. Reversed-phase preparation was carried out on the resulting crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain (4¹S,4³S)-1⁷-(dimethylphosphoryl)-5,11-dimethyl-2⁵-(trifluoromethyl)-1¹H-8-oxa-3,5,11-triaza-1(3,6)-indola-2(4,2)-pyrimidina-4(1,3)-cyclopentanacyclododecaphan-12-one (2 mg, yield of 6%, as trifluoroacetate). LCMS: m/z (ESI): [M+H]⁺= 579.2, t_{R} = 0.77 min. ¹H NMR (400 MHz, CD₃OD): δ 8.24-8.58 (m, 2H), 7.88-8.01 (m, 1H), 7.41-7.43 (m, 1H), 4.11-4.18 (m, 1H), 3.94-3.97 (m, 1H), 3.79-3.85 (m, 3H), 3.66-3.70 (m, 2H), 3.36-3.41 (m, 1H), 3.13-3.18 (m, 4H), 2.86 (s, 2H), 2.55-2.73 (m, 2H), 2.25-2.32 (m, 3H), 1.82-1.97 (m, 9H).

### Example 26 Synthesis of compound Z23

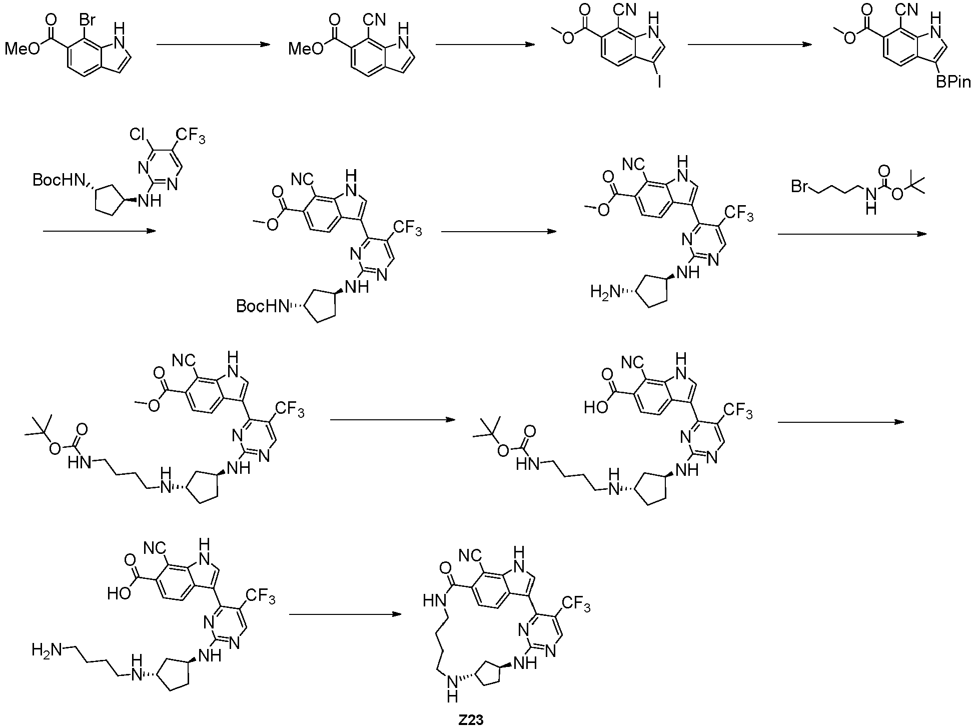

Step 1: Tetra(triphenylphosphino)palladium (0.45 g, 0.39 mmol) and zinc cyanide (0.46 g, 3.9 mmol) were added into a solution of methyl 7-bromo-1H-indole-6-carboxylate (1.0 g, 3.9 mmol) in N,N-dimethylformamide (10 mL) at 25°C. Nitrogen was used for blowing for 1 min, and reacting was carried out at 150°C in microwaves for 1 h. After realizing complete reaction by LCMS detection, water (20 mL) was slowly added into the reaction solution, and extracting was carried out with ethyl acetate (30 mL X 3). Combined organic layers were washed with saturated brine, dried over anhydrous sodium sulfate, and filtered and concentrated. The crude product was purified with column chromatography (petroleum ether/ethyl acetate = 5/1) to obtain methyl 7-cyano-1H-indole-6-carboxylate (800 mg, yellow solid, yield of 81%). LCMS: m/z (ESI): 201.2 [M+H]⁺, t_{R} = 1.22 min.

Step 2: N-iodosuccinimide (1.35 g, 6.0 mmol) was added into a solution of methyl 7-cyano-1H-indole-6-carboxylate (800 mg, 4.0 mmol) in N,N-dimethylformamide (10 mL) at 25°C. Nitrogen was replaced, stirring was carried out at 40°C for 3 h, complete reaction was realized by LCMS detection. Water (30 mL) was added into the reaction solution, and extracting was carried out with ethyl acetate (40 mL X 2). Combined organic layers were washed with saturated brine (40 mL X 4), dried over anhydrous sodium sulfate, filtered and concentrated. The crude product was purified with silica gel column chromatography (petroleum ether/ethyl acetate = 1/1) to obtain methyl 7-cyano-3-iodo-1H-indole-6-carboxylate (off-white solid, 740 mg, yield of 51%). LCMS: m/z (ESI): 327.0 [M+H]⁺, t_{R} = 1.95 min.

Step 3: Tris(dibenzylideneacetone)dipalladium (312 mg, 0.34 mmol), 2-dicyclohexylphosphine-2',4',6'-triisopropylbiphenyl (108 mg, 0.227 mmol) and triethylamine (689 mg, 6.81 mmol) were sequentially added into a solution of methyl 7-cyano-3-iodo-1H-indole-6-carboxylate (740 mg, 2.27 mmol) and 4,4,5,5-tetramethyl-1,3,2-dioxaborolane (871 mg, 6.81 mmol) in tetrahydrofuran (15 mL) at 25°C. Nitrogen was replaced, reacting was carried out at 90°C for 14 h, and complete reaction was realized by LCMS detection. Water (20 mL) was added into the reaction solution, and extracting was carried out with ethyl acetate (30 mL X 3). Combined organic layers were washed with saturated brine, dried over anhydrous sodium sulfate, filtered and concentrated. The crude product was purified with silica gel column chromatography (petroleum ether/ethyl acetate = 5/1 to 1/1) to obtain 6-formyl-3-(4,4,5,5-tetramethyl-1,3,2-dioxaborolan-2-yl)-1H-indole-7-formonitrile methane (yellow solid, 600 mg, yield of 81%). LCMS: m/z (ESI): 327.2 [M+H]⁺, t_{R} = 1.26 min.

Step 4: Chlorine[(n-butyldi(1-adamantyl)phosphino)-2-(2-aminobiphenyl)]palladium (II) (102 mg, 0.153 mmol) and a solution of potassium phosphate (976 mg, 4.6 mmol) in water (2 mL) were sequentially added into a solution of 6-formyl-3-(4,4,5,5-tetramethyl-1,3,2-dioxaborolan-2-yl)-1H-indole-7-formonitrile methane (600 mg, 1.84 mmol) and {[(1S,3S)-3-{[4-chloro-5-(trifluoromethyl)pyrimidin-2-yl]amino}cyclopentyl]amino}methanoic acid-2-methylpropyl-2-yl ester (583 mg, 1.53 mmol) in tetrahydrofuran (10 mL) at 25°C. Nitrogen was replaced, reacting was carried out at 60°C for 14 h, and complete reaction was realized by LCMS detection. Water (20 mL) was added into the reaction solution, and extracting was carried out with ethyl acetate (20 mL X 3). Combined organic layers were washed with saturated brine, dried over anhydrous sodium sulfate, filtered and concentrated. The crude product was purified with column chromatography (petroleum ether/ethyl acetate = 5/1) to obtain methyl 7-cyano-3-(2-{[(1S,3S)-3-({[(2-methylpropyl-2-yl)oxy]carbonyl}amino)cyclopentyl]amino}-5-(trifluoromethyl)pyrimidin-4-yl)-1H-indole-6-carboxylate (yellow oily, 580 mg, yield of 69%). LCMS: m/z (ESI): 544.8 [M+H]⁺, t_{R} = 1.66 min.

Step 5: Trifluoroacetic acid (2.5 mL) was added into a solution of methyl 7-cyano-3-(2-{[(1S,3S)-3-({[(2-methylpropyl-2-yl)oxy]carbonyl amino}cyclopentyl]amino}-5-(trifluoromethyl)pyrimidin-4-yl)-1H-indole-6-carboxylate (500 mg, 0.92 mmol) in dichloromethane (5 mL) at 25°C. Reacting was carried out at 20°C for 3 h, and complete reaction was realized by LCMS detection. A saturated sodium bicarbonate solution (30 mL) was added into the reaction solution, and extracting was carried out with a mixed solution of dichloromethane and methanol (5:1) (50 mL X 6). Combined organic layers were washed with saturated brine, dried over anhydrous sodium sulfate, and filtered and concentrated to obtain a crude product methyl 7-cyano-3-(2-{[(1S,3S)-3-aminocyclopentyl]amino}-5-(trifluoromethyl)pyrimidin-4-yl)-1H-indole-6-carboxylate (500 mg, purity of 75% and yield of 92%). LCMS: MS m/z (ESI): 445.2 [M+H]⁺, t_{R} = 0.79 min.

Step 6: [(4-bromobutyl)amino]methanoic acid-2-methylpropyl-2-yl ester (56 mg, 0.225 mmol) and N,N-diisopropylethylamine (116 mg, 0.9 mmol) were sequentially added into a solution of methyl 7-cyano-3-(2-{[(1S,3 S)-3-aminocyclopentyl]amino}-5-(trifluoromethyl)pyrimidin-4-yl)-1H-indole-6-carboxylate (100 mg, 0.225 mmol) in N,N-dimethylformamide (2 mL) at room temperature. Nitrogen was replaced, stirring was carried out at 80°C to react for 16 h, and complete reaction was realized by LCMS detection. A saturated ammonium chloride solution (10 mL) was added into the reaction solution, and extracting was carried out with ethyl acetate (20 mL X 3). Combined organic layers were washed with saturated brine, dried over anhydrous sodium sulfate, filtered and concentrated. The resulting crude product was purified with silica gel column chromatography (dichloromethane/methanol = 20/1 to 10/1) to obtain methyl 7-cyano-3-(2-{[(1S,3S)-3-[(2,2-dimethyl-4-oxo-5-aza-3-oxa-non-9-yl)amino]cyclopentyl]amino}-5-(trifluoromethyl)pyrimidin-4-yl)-1H-indole-6-carboxylate (yellow oily, 70 mg, yield of 40%). LCMS: MS m/z (ESI): 615.8 [M+H]⁺, t_{R} = 1.28 min.

Step 7: A solution of lithium hydroxide monohydrate (19.0 mg, 0.45 mmol) in water (3 mL) was added into a mixed solution of methyl 7-cyano-3-(2-{[(1S,3S)-3-[(2,2-dimethyl-4-oxo-5-aza-3-oxa-non-9-yl)amino]cyclopentyl]amino}-5-(trifluoromethyl)pyrimidin-4-yl)-1H-indole-6-carboxylate (70 mg, 0.09 mmol) in tetrahydrofuran (3 mL) and methanol (3 mL). Stirring was carried out to react at 40°C for 12 h, and complete reaction was realized by LCMS detection. The reaction solution was directly concentrated. Reversed-phase preparation was carried out on a crude product (chromatographic column: Ultimate XB-C18-08, 50X250 mm, 10 um (PARP-02), mobile phase: acetonitrile-water (0.1% ammonium bicarbonate), flow rate: 70 mL/min, and column temperature: 25°C) to obtain 7-cyano-3-(2-{[(1S,3S)-3-[(2,2-dimethyl-4-oxo-5-aza-3-oxanon-9-yl)amino]cyclopentyl]amino}-5-(trifluoromethyl)pyrimidin-4-yl)-1H-indole-6-carboxylic acid (white solid, 30 mg, yield of 55%). LCMS: MS m/z (ESI): 602.2 [M+H]⁺, t_{R} = 0.86 min.

Step 8: Trifluoroacetic acid (0.1 mL) was added into a solution of 7-cyano-3-(2-{[(1S,3S)-3-[(2,2-dimethyl-4-oxo-5-aza-3-oxanon-9-yl)amino]cyclopentyl]amino}-5-(trifluoromethyl)pyrimidin-4-yl)-1H-indole-6-carboxylic acid (20 mg, 0.033 mmol) in dichloromethane (0.9 mL). Stirring was carried out to react at 15°C for 45 min. After realizing complete reaction by LCMS detection, N,N-diisopropylethylamine was added into the reaction solution to adjust the pH to be 8. Concentrating was carried out to obtain 7-cyano-3-(2-{[(1S,3S)-3-[(4-aminobutyl)amino]cyclopentyl]amino}-5-(trifluoromethyl)pyrimidin-4-yl)-1H-indole-6-carboxylic acid (yellow oily substance, 12 mg, yield of 72%). The crude product was directly used in the next reaction. LCMS: MS m/z (ESI): 502.0 [M+H]⁺, t_{R} = 1.20 min.

Step 9: 2-(7-azabenzotriazole)-N,N,N',N'-tetramethyluronium hexafluorophosphate (15 mg, 0.04 mmol) and N,N-diisopropylethylamine (21 mg, 0.16 mmol) were sequentially added into a solution of 7-cyano-3-(2-{ [(1S,3S)-3-[(4-aminobutyl)amino]cyclopentyl]amino}-5-(trifluoromethyl)pyrimidin-4-yl)-1H-indole-6-carboxylic acid (12 mg, 0.02 mmol) in N,N-dimethylformamide (2 mL). Stirring was carried out at 80°C for 14 h, and complete reaction was realized by LCMS detection. Concentrating was carried out, and reversed-phase preparation was carried out on a crude product (chromatographic column: Ultimate XB-C18-08, 50X250 mm, 10 um (PARP-02), mobile phase: acetonitrile-water (0.1% ammonium bicarbonate), flow rate: 70 mL/min, and column temperature: 25°C) to obtain (4¹S,4³S)-11-oxo-2⁵-(trifluoromethyl)-1¹H-3,5,10-triaza-1(3,6)-indola-2(4,2)-pyrimidina-4(1,3)-cyclopentanacycloundecaphane-17-carbonitrile (Z23, 0.8 mg, yield of 7%). LCMS: MS m/z (ESI): 484.2 [M+H]⁺, t_{R} = 0.84 min.¹H NMR (400 MHz, CD₃OD): δ 8.73 (d, J = 8.4 Hz, 1H), 8.53 (s, 1H), 7.96 (s, 1H), 7.46 (d, J = 8.4 Hz, 1H), 4.18-4.13 (m, 1H), 3.28-3.23 (m, 1H), 3.11-3.00 (m, 1H), 2.91-2.80 (m, 1H), 2.52-2.48 (m, 1H), 2.34-2.08 (m, 4H), 1.84-1.73 (m, 1H), 1.72-1.61 (m, 1H), 1.56-1.39 (m, 5H).

### Example 27 Synthesis of compound Z62

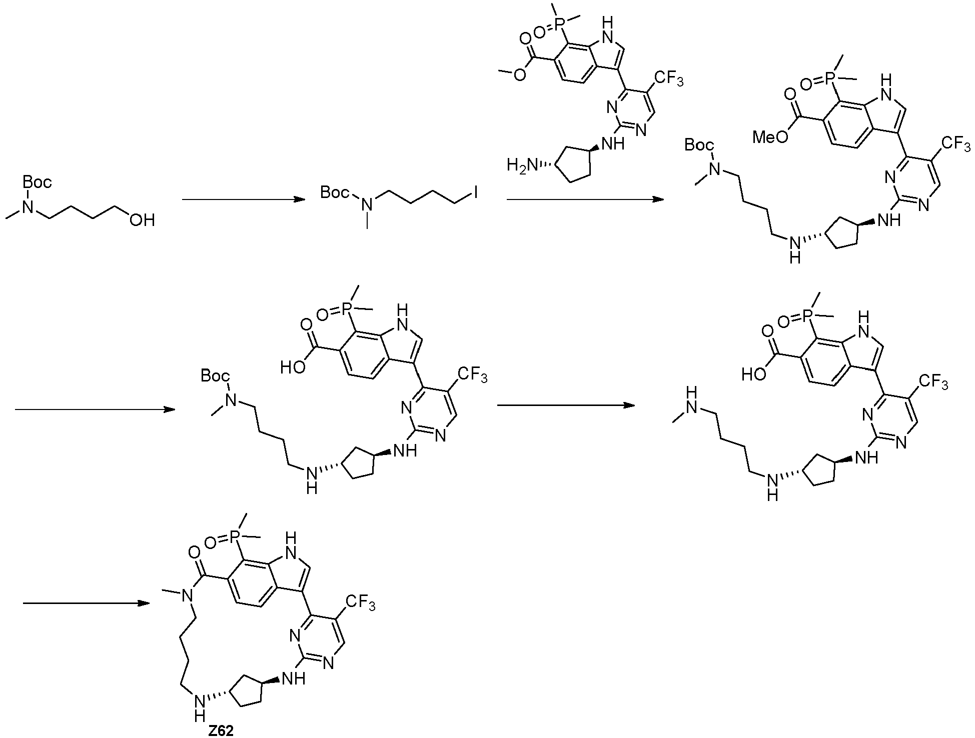

Step 1: [(4-hydroxybutyl) (methyl)amino]methanoic acid-2-methylpropyl-2-yl ester (100 mg, 0.49mmol), iodine (125 mg, 0.49mmol) and imidazole (50 mg, 0.74 mmol) were sequentially added into a solution of triphenylphosphine (129 mg, 0.49 mmol) in dichloromethane (3 mL) at 0°C in a nitrogen atmosphere. Stirring was carried out at 0°C for 5 min, then heating was carried out to reach room temperature, and stirring was carried out at room temperature for 4 h. After realizing complete reaction by LCMS detection, water (10 mL) was added for dilution, extracting was carried out with dichloromethane (20 mL X 3), organic phases were combined, washed with saturated brine, dried over anhydrous sodium sulfate, filtered and concentrated. The crude product was purified with silica gel column chromatography (petroleum ether/ethyl acetate = 10/1-3/1) to obtain a colorless oily substance [(4-iodobutyl) (methyl)amino]methanoic acid-2-methylpropyl-2-yl ester (120 mg, yield of 78%). LCMS: m/z (ESI): 258.0 [M+H-56]⁺, t_{R} = 1.63 min.

Step 2: Methyl 7-[dimethyl(oxo)-λ⁵-phosphoranyl]-3-(2-{[(1S,3S)-3-aminocyclopentyl]amino}-5-(trifluoromethyl)pyrimidin-4-yl)-1H-indole-6-carboxylate (27 mg, 0.054 mmol) was dissolved in N,N-dimethylformamide (2 mL). [(4-iodobutyl) (methyl)amino]methanoic acid-2-methylpropyl-2-yl ester (20 mg, 0.065 mmol) and N,N-diisopropylethylamine (28 mg, 0.218 mmol) were sequentially added. Stirring was carried out at 80°C for 16 h, and complete reaction was realized by LCMS detection. The reaction solution was filtered to remove solid impurities, the organic phase was concentrated to obtain a crude product, and preparation was carried out by TLC (dichloromethane/methanol = 10/1), and purification was carried out to obtain methyl 7-[dimethyl(oxo)-λ⁵-phosphoranyl]-3-(2-{[(1S,3S)-3-[(2,2,5-trimethyl-4-oxo-5-aza-3-oxanon-9-yl)amino]cyclopentyl]amino}-5-(trifluoromethyl)pyrimidin-4-yl)-1H-indole-6-carboxylate (yellow solid,28 mg, yield of 75%). LCMS: m/z (ESI): 681.3 [M+H]⁺, t_{R} = 1.17 min.

Step 3: Methyl 7-[dimethyl(oxo)-λ⁵-phosphoranyl]-3-(2-{[(1S,3S)-3-[(2,2,5-trimethyl-4-oxo-5-aza-3-oxanon-9-yl)amino]cyclopentyl]amino}-5-(trifluoromethyl)pyrimidin-4-yl)-1H-indole-6-carboxylate (110 mg, 0.162 mmol) was dissolved in a mixed system of water (2 mL), methanol (2 mL) and tetrahydrofuran (2 mL). Lithium hydroxide (as monohydrate, 34 mg, 0.808 mmol) was added. Stirring was carried out at 40°C for 6 h. After realizing complete reaction by LCMS detection, spin-drying was carried out to remove the solvent to obtain a crude product 7-[dimethyl(oxo)-λ⁵-phosphoranyl]-3-(2-{[(1S,3S)-3-[(2,2,5-trimethyl-4-oxo-5-aza-3-oxanon-9-yl)amino]cyclopentyl]amino}-5-(trifluoromethyl)pyrimidin-4-yl)-1H-indole-6-carboxylic acid (110 mg, 94%), and the product was directly used in the next reaction. LCMS: m/z (ESI): 667.4 [M+H]⁺, t_{R} = 1.04 min.

Step 4: A hydrogen chloride/methanol solution (5 mL, 4 M) was added into 7-[dimethyl(oxo)-λ⁵-phosphoranyl]-3-(2-{[(1S,3S)-3-[(2,2,5-trimethyl-4-oxo-5-aza-3-oxanon-9-yl)amino]cyclopentyl]amino}-5-(trifluoromethyl)pyrimidin-4-yl)-1H-indole-6-carboxylic acid (110 mg, 0.164 mmol), and the system was stirred at 40°C for 4 h. After realizing complete reaction by LCMS detection, spin-drying was carried out, and reversed-phase preparation was carried out on a crude product (chromatographic column: Ultimate XB-C18, 50X250 mm, 10 um (PARP-02), mobile phase: acetonitrile-water (0.1% trifluoroacetic acid), flow rate: 70 mL/min, column temperature: 25°C) to obtain 7-[dimethyl(oxo)-λ⁵-phosphoranyl]-3-(2-{[(1S,3S)-3-{[4-(methylamino)butyl]amino}cyclopentyl]amino}-5-(trifluoromethyl)pyrimidin-4-yl)-1H-indole-6-carboxylic acid (white solid, 80 mg, yield of 94%). LCMS: m/z (ESI): 567.3 [M+H]⁺, t_{R} = 0.83 min.

Step 5: 2-(7-azabenzotriazole)-N,N,N',N'-tetramethyluronium hexafluorophosphate (40 mg, 0.106 mmol) and N,N-diisopropylethylamine (68 mg, 0.530 mmol) were sequentially added into a solution of 7-[dimethyl(oxo)-λ⁵-phosphoranyl]-3-(2-{[(1S,3S)-3-{[4-(methylamino)butyl]amino}cyclopentyl]amino}-5-(trifluoromethyl)pyrimidin-4-yl)-1H-indole-6-carboxylic acid (60 mg, 0.106 mmol) in N,N-dimethylformamide (5 mL). Stirring was carried out at 80°C for 4 h, and complete reaction was realized by LCMS detection. Concentrating was carried out, and reversed-phase preparation was carried out on a crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain (4¹S,4³S)-1⁷-(dimethylphosphoryl)-10-methyl-2⁵-(trifluoromethyl)-1¹H-3,5,10-triaza-1(3,6)-indola-2(4,2)-pyrimidina-4(1,3)-cyclopentanacycloundecaphan-11-one (Z62, 16 mg, yield of 28%, as trifluoroacetate). LCMS: m/z (ESI): 549.2 [M+H]⁺, t_{R} = 0.84 min.¹H NMR (400 MHz, CD₃OD): δ 8.72-8.36 (m, 2H), 8.05-7.89 (m, 1H), 7.36-7.19 (m, 1H), 3.86-3.73 (m, 1H), 3.23-3.13 (m, 1H), 3.11 (s, 3H), 3.08-2.98 (m, 1H), 2.83-2.63 (m, 3H), 2.41-2.06 (m, 4H), 2.00-1.55 (m, 12H).

### Example 28 Synthesis of compounds Z68-1 and Z68-2

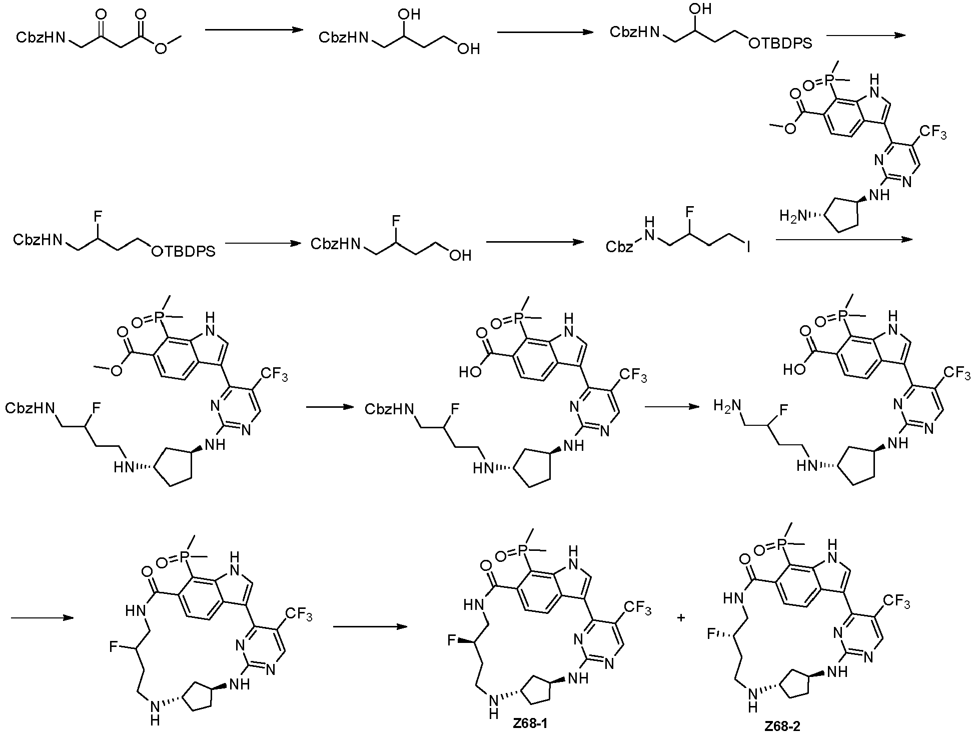

Step 1: A solution of lithium aluminum hydride in tetrahydrofuran (28.3 mL, 1 M,28.3 mmol) was added into a solution of methyl 4-((benzyloxy)carbonyl)amino)-3-oxobutyrate (5.0 g, 18.9 mmol) in tetrahydrofuran (100 mL) at 5°C. The reaction solution was stirred to react at 5°C under nitrogen protection for 2 h. After realizing complete reaction by LCMS detection, a saturated ammonium chloride aqueous solution (100 mL) was added into the reaction solution, extracting was carried out with ethyl acetate (100 mL X 3), the combined organic layer was washed with saturated brine (100 mL), dried over anhydrous sodium sulfate, filtered and concentrated. The crude product was purified with column chromatography (petroleum ether/ethyl acetate = 10/1 to 0/1) to obtain a colorless oily substance benzyl (2,4-dihydroxybutyl)carbamate (1.2 g, yield of 27%). LCMS: m/z (ESI): 239.8 [M+H]⁺, t_{R} = 0.99 min.

Step 2: Imidazole (0.68 g, 10.04 mmol) and tert-butyl diphenylchlorosilane (1.38 g, 5.02 mmol) were added into a solution of benzyl (2,4-dihydroxybutyl)carbamate (1.2 g, 5.02 mmol) in dichloromethane (50 mL) at 5°C. Stirring was carried out to react at 5°C under nitrogen protection for 3 h. After realizing complete reaction by LCMS detection, water (50 mL) was added into the reaction solution, extracting was carried out with ethyl acetate (50 mL X 3), the combined organic layer was washed with saturated brine (50 mL), dried over anhydrous sodium sulfate, filtered and concentrated. The crude product was purified with column chromatography (petroleum ether/ethyl acetate = 10/1 to 1/1) to obtain a colorless oily substance benzyl (4-((tert-butyl diphenylsilyl)oxy)-2-hydroxybutyl)carbamate (2.2 g, yield of 92%). LCMS: m/z (ESI): 400.2 [M+H]⁺, t_{R} = 1.69 min.

Step 3: Benzyl (4-((tert-butyl diphenylsilyl)oxy)-2-hydroxybutyl)carbamate (2.2 g, 4.61 mmol) was dissolved into dichloromethane (30 mL), then diethylamino sulfur trifluoride (1.1 g, 6.92 mmol) was slowly added at 0°C, and stirring was carried out at 5°C for 3 h. After realizing complete reaction by TLC detection, water (20 mL) was added for quenching, extracting was carried out with ethyl acetate (30 mL X 3), the combined organic layer was washed with saturated brine (5 mL), dried over anhydrous sodium sulfate, filtered and concentrated. The crude product was purified with column chromatography (petroleum ether/ethyl acetate = 15/1 to 1/1) to obtain a colorless oily substance benzyl (4-((tert-butyldiphenylsilyl)oxy)-2-hydroxybutyl)carbamate (0.7 g, yield of 29%). ¹H NMR (400 MHz, DMSO-*d₆*): δ 7.80 - 7.18 (m, 16H), 5.04 (s, 2H), 4.77 (d, *J* = 50.4 Hz, 1H), 3.74 (d, *J* = 5.6 H, 2H), 3.32 - 3.22 (m, 2H), 1.85 - 1.70 (m, 2H), 0.99 (s, 9H).

Step 4: Benzyl (4-((tert-butyldiphenylsilyl)oxy)-2-hydroxybutyl)carbamate (700 mg, 1.46 mmol) was dissolved in N,N-dimethylformamide(5 mL), and then cesium fluoride added(888 mg, 5.84 mmol) was added. Stirring was carried out for 18 h at 5°C in the reaction. After realizing complete reaction by LCMS detection, water (20 mL) was added, extracting was carried out with ethyl acetate (30 mL X 3), the combined organic layer was washed with saturated brine (50 mL), dried over anhydrous sodium sulfate, filtered and concentrated. The crude product was purified with column chromatography (petroleum ether/ethyl acetate = 15/1 to 0/1) to obtain a colorless oily substance benzyl (2-fluoro-4-hydroxybutyl)carbamate (300 mg, yield of 85%). LCMS: m/z (ESI): 242.0 [M+H]⁺, t_{R} = 1.13 min.

Step 5: Benzyl (2-fluoro-4-hydroxybutyl)carbamate (300 mg, 1.24 mmol) was dissolved in dichloromethane (10 mL) in a 50 mL single-neck flask, and imidazole (126 mg, 1.86 mmol), triphenylphosphine (390 mg, 1.50 mmol) and iodine (381 mg, 1.50 mmol) were added. Stirring was carried out for 1 h at 10°C in the reaction. After realizing complete reaction by TLC spot plate detection, silica gel was directly added, an oil pump was used for performing spin-drying and sample mixing, and column chromatography purification was carried out (petroleum ether/ethyl acetate = 20/1 to 3/1) to obtain a colorless oily substance benzyl (2-fluoro-4-iodobutyl)carbamate (330 mg, yield of 76%).

Step 6: Methyl 3-(2-((1S,3S)-3-aminocyclopentyl)amino)-5-(trifluoromethyl)pyrimidin-4-yl)-7-(dimethylphosphoryl)-1H-indole-6-carboxylate (220 mg, 0.44 mmol) was dissolved in N,N-dimethylformamide (5 mL), benzyl (2-fluoro-4-iodobutyl)carbamate (156 mg, 0.44 mmol) and N,N-diisopropylethylamine (286 mg, 2.20 mmol) were added. Stirring was carried out at 100°C for 18 h. After realizing complete reaction by LCMS detection, silica gel was directly added, an oil pump is used for performing spin-drying and sample mixing, and column chromatography purification was carried out (dichloromethane/methanol = 40/1 to 5/1) to obtain white solid methyl 3-(2-((1S,3S)-3-((benzyloxy)carbonyl)amino)-3-fluorobutylamino)cyclopentylamino)-5-(trifluoromethyl)pyrimidin-4-yl)-7-(dimethylphosphoryl)-1H-indole-6-carboxylate (290 mg, yield of 92%). LCMS: m/z (ESI): 719.2 [M+H]⁺, t_{R} = 1.12 min.

Step 7: Methyl 3-(2-((1S,3S)-3-((benzyloxy)carbonyl)amino)-3-fluorobutylamino)cyclopentylamino)-5-(trifluoromethyl)pyrimidin-4-yl)-7-(dimethylphosphoryl)-1H-indole-6-carboxylate (290 mg, 0.40 mmol) was dissolved in a mixed system of methanol (5 mL), tetrahydrofuran (5 mL) and water (5 mL), and lithium hydroxide monohydrate (84 mg, 2.00 mmol) was added. The reaction solution was stirred at 40°C for 18 h, complete reaction was realized by LCMS detection, the reaction solution was concentrated to obtain a crude white solid 3-(2-((1S,3S)-3-((benzyloxy)carbonyl)amino)-3-fluorobutylamino)cyclopentyl amino)-5-(trifluoromethyl)pyrimidin-4-yl)-7-(dimethylphosphoryl)-1H-indole-6-carboxylic acid (300 mg). LCMS: m/z (ESI): 705.2 [M+H]⁺, t_{R} = 0.94 min.

Step 8: Trifluoroacetic acid (5 mL) was added into a reaction flask of 3-(2-((1S,3S)-3-((benzyloxy)carbonyl)amino)-3-fluorobutylamino)cyclopentyl amino)-5-(trifluoromethyl)pyrimidin-4-yl)-7-(dimethylphosphoryl)-1H-indole-6-carboxylic acid (300 mg, 0.43 mmol), and stirring was carried out at 60°C for 3 h. After realizing complete reaction by LCMS detection, the reaction solution was concentrated, and reversed-phase preparation was carried out on a crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid 3-(2-((1S,3S)-3-((4-amino-3-fluorobutyl)amino)cyclopentyl)amino)-5-(trifluoromethyl)pyrimidin-4-yl)-7-(dimethylphosphoryl)-1H-indole-6-carboxylic acid (140 mg, yield of 57%, as trifluoroacetate). LCMS: m/z (ESI): 571.2 [M+H]⁺, t_{R} = 0.97 min.

Step 9: 2-(7-azobenzotriazole)-N,N,N',N'-tetramethyluronium hexafluorophosphate (55 mg, 0.15 mmol) and N,N-diisopropylethylamine (78 mg, 0.61 mmol) were sequentially added into 3-(2-((1S,3S)-3-((4-amino-3-fluorobutyl)amino)cyclopentyl)amino)-5-(trifluoromethyl)pyrimidin-4-yl)-7-(dimethylphosphoryl)-1H-indole-6-carboxylic acid (70 mg, 0.12 mmol) in N,N-dimethylformamide (5 mL) at room temperature. Reacting was carried out for 18 h at 60°C under nitrogen protection. After realizing complete reaction by LCMS detection, concentrating was carried out to obtain a crude product (4¹S,4³S)-1⁷-(dimethylphosphoryl)-8-fluoro-2⁵-(trifluoromethyl)-1¹H-3,5,10-triaza-1(3,6)-indole-2(4,2)-pyrimidin-4(1,3)-cyclopentanundecan-11-one.

Step 10: A crude product obtained by the reaction from the previous step was subjected to reversed-phase preparation and purification (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain two isomer compounds. One isomer compound is arbitrarily designated as a a white solid (4¹S,4³S,8R)-1⁷-(dimethylphosphoryl)-8-fluoro-2⁵-(trifluoromethyl)-1¹H-3,5,10-triaza-1(3,6)-indola-2(4,2)-pyrimidina-4(1,3)-cyclopentanacycloundecaphan-11-one (Z68-1, 4 mg, yield of 6%, as trifluoroacetate). LCMS: MS m/z (ESI): 553.2 [M+H]⁺, t_{R} = 1.07 min.¹H NMR (400 MHz, CD₃OD): δ 8.57 (s, 1H), 8.45 - 8.42 (m, 1H), 7.96 (s, 1H), 7.32 (dd, *J* = 8.0, 3.2 Hz, 1H), 4.80-4.61 (m, 1H), 4.22-4.08 (m, 1H), 3.88-3.78 (m, 1H), 3.28-3.17 (m, 2H), 2.96-2.86 (m, 1H), 2.85 - 2.64 (m, 2H), 2.40-2.26 (m, 2H), 2.11 - 1.94 (m, 8H), 1.92 - 1.69 (m, 3H). The other isomer compound is arbitrarily designated as (4¹S,4³S,8S)-1⁷-(dimethylphosphoryl)-8-fluoro-2⁵-(trifluoromethyl)-1¹H-3,5,10-triaza-1(3,6)-indola-2(4,2)-pyrimidina-4(1,3)-cyclopentanacycloundecaphan-11-one (Z68-2,3 mg, yield of 5%, as trifluoroacetate). LCMS: MS m/z (ESI): 553.3 [M+H]⁺, t_{R} = 1.08 min.¹H NMR (400 MHz, CD₃OD): δ 8.57 (s, 1H), 8.50 (d, *J=* 8.0 Hz, 1H), 7.99 (s, 1H), 7.28 (dd, *J* = 8.0, 3.2 Hz, 1H), 4.83-4.73 (m, 1H), 4.27 - 4.09 (m, 1H), 3.88-3.78 (m, 1H), 3.27 - 3.08 (m, 2H), 2.92-2.72 (m, 2H), 2.67 - 2.51 (m, 1H), 2.40 - 2.17 (m, 2H), 2.08 - 1.71 (m, 11H).

### Example 29 Synthesis of compound Z66

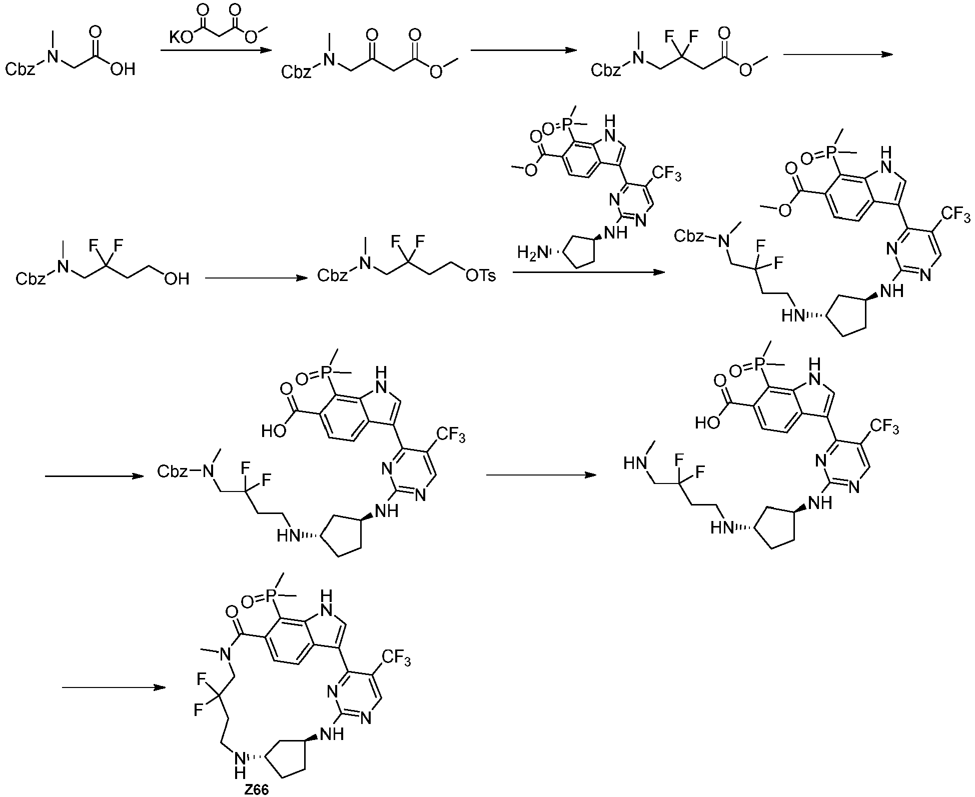

Step 1: N-[(benzyloxy)carbonyl]-N-methylglycine(10.8 g, 48.4 mmol), tetrahydrofuran (100 mL) and carbonyldiimidazole (7.8 g, 48.4 mmol) were sequentially added into a 250 mL three-neck flask. The reaction solution was replaced with nitrogen, stirring was carried out at 10°C for 12 h, magnesium chloride (2.3 g, 24.2 mmol) and monomethyl malonate potassium salt (7.6 g, 48.4 mmol) were added, heated to reach 60°C, and stirred for 6 h, and complete reaction was realized by TLC detection. The reaction solution was concentrated, water (100 mL) was added for dilution, extracting was carried out with ethyl acetate (50 mL X 3), dried was carried out with anhydrous sodium sulfate, concentrating was carried out to obtain a crude product, and column chromatography purification was carried out (ethyl acetate/petroleum ether = 50/50) to obtain light yellow oily liquid benzyl (3,5-dioxo-7-aza-2-oxa-octyl-7-yl)carboxylate (5.8 g, yield of 43%). ¹H NMR (400 MHz, CD₃OD): δ 7.47 - 7.21 (m, 5H), 5.13 - 5.06 (m, 2H), 4.29 - 4.25 (m, 2H), 3.70 - 3.64 (m, 3H), 2.94 - 2.91 (m, 3H).

Step 2: Benzyl (3,5-dioxo-7-aza-2-oxa-octyl-7-yl)carboxylate (5.8 g, 20.8 mmol) was dissolved in dichloromethane (50 mL), and diethylaminosulfur trifluoride (5.0 g, 31.2 mmol) was added dropwise at 0°C. Stirring was carried out at 25°C for 16 h. After realizing complete reaction by LCMS detection, water (40 mL) was added for quenching, extracting was carried out with dichloromethane (50 mL X 3), drying was carried out with anhydrous sodium sulfate, a concentrated crude product was subjected to reversed-phase preparation (chromatographic column: Ultimate XB-C18, 50X250 mm, 10 um (PARP-02), flow rate: acetonitrile-water (0.1% trifluoroacetic acid), flow rate: 70 mL/min, and column temperature: 25°C) to obtain an off-white solid benzyl (5,5-difluoro-3-dioxo-7-aza-2-oxaoct-7-yl)carboxylate (1.4 g, yield of 22%). LCMS: m/z (ESI): 301.8 [M+H]⁺, t_{R} = 1.48 min.

Step 3: Benzyl (5,5-difluoro-3-dioxo-7-aza-2-oxaoct-7-yl)carboxylate (1.4 g, 4.7 mmol) was dissolved in tetrahydrofuran (20 mL), a tetrahydrofuran solution (4.7 mL, 1M) of lithium aluminum hydride was added dropwise at 0°C, and stirring was carried out for 2 h at 10°C. After realizing complete reaction by LCMS detection, water (20 mL) was added for quenching, extracting was carried out with ethyl acetate (20 mL X 3), drying was carried out with anhydrous sodium sulfate, concentrating was carried out to obtain a crude product, and column chromatography purification was carried out (ethyl acetate/petroleum ether = 80/20) to obtain colorless oily liquid benzyl [(2,2-difluoro-4-hydroxybutyl)(methyl)amino]carboxylate (1.0 g, yield of 80%). LCMS: m/z (ESI): 274.2 [M+H]⁺, t_{R} = 1.14 min.

Step 4: P-methylsulfonyl chloride (558 mg, 2.93 mmol) was added into a solution of benzyl [(2,2-difluoro-4-hydroxybutyl) (methyl)amino]carboxylate (400 mg, 1.46 mmol) in pyridine (5 mL), and stirring was carried out at 10°C for 3 h. After realizing complete reaction by LCMS detection, silica gel was directly added, an oil pump was used for performing spin-drying and sample mixing, and column chromatography purification was carried out (dichloromethane 100%) to obtain a yellow oily substance 4-methylbenzenesulfonic acid-6,6-difluoro-4-methyl-3-oxo-1-phenyl-4-aza-2-oxaoct-8-yl ester (370 mg, yield of 59%). LCMS: m/z (ESI): 428.2 [M+H]⁺, t_{R} = 1.44 min.

Step 5: Methyl 7-[dimethyl(oxo)-λ⁵-phosphoranyl]-3-(2-{[(1S,3S)-3-aminocyclopentyl]amino}-5-(trifluoromethyl)pyrimidin-4-yl)-1H-indole-6-carboxylate (426 mg, 0.86 mmol) was dissolved in N,N-dimethylformamide (7 mL), and 4-methylbenzenesulfonic acid-6,6-difluoro-4-methyl-3-oxo-1-phenyl-4-aza-2-oxaoct-8-yl ester (350 mg, 0.82 mmol) and N,N-diisopropylethylamine (0.68 mL, 4.09 mmol) were added. Stirring was carried out at 100°C for 16 h. After realizing complete reaction by LCMS detection, silica gel was added, an oil pump was used for performing spin-drying and sample mixing, and column chromatography purification was carried out (dichloromethane: methanol = 100:6) to obtain a yellow solid methyl 7-[dimethyl(oxo)-λ⁵-phosphoranyl]-3-(2-{[(1S,3S)-3-[(6,6-difluoro-4-methyl-3-oxo-1-phenyl-4-aza-2-oxaoct-8-yl)amino]cyclopentyl]amino}-5-(trifluoromethyl)pyrimidin-4-yl)-1H-indole-6-carboxylate (350 mg, yield of 57%). LCMS: m/z (ESI): 751.3 [M+H]⁺, t_{R} = 1.15 min.

Sep 6: Methyl 7-[dimethyl(oxo)-λ⁵-phosphoranyl]-3-(2-{[(1S,3S)-3-[(6,6-difluoro-4-methyl-3-oxo-1-phenyl-4-aza-2-oxaoct-8-yl)amino]cyclopentyl]amino}-5-(trifluoromethyl)pyrimidin-4-yl)-1H-indole-6-carboxylate (330 mg, 0.46 mmol) was dissolved in a mixed system of water (3 mL), methanol (3 mL) and tetrahydrofuran (3 mL), lithium hydroxide monohydrate (96.6 mg, 2.30 mmol) was added, and stirring was carried out at 40°C for 16 h. After realizing complete reaction by LCMS detection, concentrating was directly carried out to obtain 7-[dimethyl(oxo)-λ⁵-phosphoranyl]-3-(2-{[(1S,3S)-3-[(6,6-difluoro-4-methyl-3-oxo-1-phenyl-4-aza-2-oxaoct-8-yl)amino]cyclopentyl]amino}-5-(trifluoromethyl)pyrimidin-4-yl)-1H-indole-6-carboxylic acid, and the product was directly used in the next reaction without any treatment. LCMS: m/z (ESI): 737.4 [M+H]⁺, t_{R} = 1.43 min.

Step 7: Trifluoroacetic acid (5 mL) was added into 7-[dimethyl(oxo)-λ⁵-phosphoranyl]-3-(2-{[(1S,3S)-3-[(6,6-difluoro-4-methyl-3-oxo-1-phenyl-4-aza-2-oxaoct-8-yl)amino]cyclopentyl]amino}-5-(trifluoromethyl)pyrimidin-4-yl)-1H-indole-6-carboxylic acid (350 mg, 0.48 mmol). Stirring was carried out at 60°C for 1.5 h, complete reaction was realized by LCMS detection, then concentrating was carried out, and reversed-phase preparation was carried out on a crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid 7-[dimethyl(oxo)-λ⁵-phosphoranyl]-3-(2-{[(1S,3S)-3-{[3,3-difluoro-4-(methylamino)butyl]amino}cyclopentyl]amino}-5-(trifluoromethyl)pyrimidin-4-yl)-1H-indole-6-carboxylic acid (215 mg, two steps of yield of 81%). LCMS: m/z (ESI): 603.2 [M+H]⁺, t_{R} = 0.78 min.

Step 8: 2-(7-azabenzotriazole)-N,N,N',N'-tetramethyluronium hexafluorophosphate (149 mg, 0.39 mmol) and N,N-diisopropylethylamine (0.30 mL, 1.78 mmol) were sequentially added into a solution of 7-[dimethyl(oxo)-λ⁵-phosphoranyl]-3-(2-{[(1S,3S)-3-{[3,3-difluoro-4-(methylamino)butyl]amino}cyclopentyl]amino}-5-(trifluoromethyl)pyrimidin-4-yl)-1H-indole-6-carboxylic acid (215 mg, 0.36 mmol) in N,N-dimethylformamide (10 mL). Stirring was carried out at 60°C for 16 h, complete reaction was realized by LCMS detection, concentrating was carried out, and reversed-phase preparation was carried out on a crude product (chromatographic column: Ultimate XB-C18, 50X250 mm, 10 um (PARP-02), mobile phase: acetonitrile-water (0.1 percent trifluoroacetic acid), flow rate: 70 mL/min, and column temperature: 25°C) to obtain (4¹S,4³S)-1⁷-(dimethylphosphoryl)-8,8-difluoro-10-methyl-2⁵-(trifluoromethyl)-1¹H-3,5,10-triaza-1(3,6)-indola-2(4,2)-pyrimidina-4(1,3)-cyclopentanacycloundecaphan-11-one (Z66, 22 mg, yield of 11%, as trifluoroacetate). LCMS: m/z (ESI): 585.2 [M+H]⁺, t_{R} = 0.87 min.¹H NMR (400 MHz, CD₃OD): δ 8.45-8.65 (m, 2H), 7.93 (s, 1H), 7.26 (s, 1H), 4.00-4.38 (m, 1H), 3.96-3.47 (m, 3H), 3.25 (s, 3H), 3.01-2.52 (m, 3H), 2.52-2.09 (m, 4H), 2.08-1.51 (m, 9H).

### Example 30 Synthesis of compound Z65

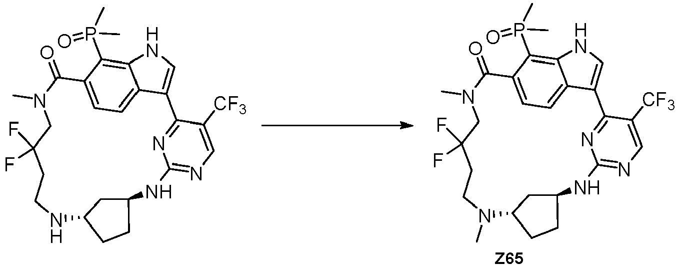

Step 1: Paraformaldehyde (9 mg, 0.10 mmol) and tetraisopropyl titanate (28 mg, 0.10 mmol) were sequentially added into a solution of (4¹S,4³S)-1⁷-(dimethylphosphoryl)-8,8-difluoro-10-methyl-2⁵-(trifluoromethyl)-1¹H-3,5,10-triaza-1(3,6)-indola-2(4,2)-pyrimidina-4(1,3)-cyclopentanacycloundecaphan-11-one (14 mg, 0.02 mmol, as trifluoroacetate) in tetrahydrofuran (3 mL) in a 10 mL single-neck flask. Stirring was carried out at 70°C for 14 h, the reaction solution was cooled to reach 25°C, sodium cyanoborohydride (4 mg, 0.06 mmol) was added, and stirring was carried out at 25°C for 2 h. After realizing complete reaction by LCMS detection, the reaction solution was concentrated, and reversed-phase preparation was carried out on a crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain (4¹S,4³S)-1⁷-(dimethylphosphoryl)-8,8-difluoro-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z65,2 mg, yield of 14%, as trifluoroacetate). LCMS: m/z (ESI): 599.2 [M+H]⁺, t_{R} = 1.31 min.¹H NMR (400 MHz, CD₃OD): δ 8.86 - 8.41 (m, 2H), 8.00 (s, 1H), 7.23 (dd, *J* = 8.0, 3.2 Hz, 1H), 4.28 - 4.06 (m, 1H), 3.92-3.76 (m, 1H), 3.68-3.47 (m, 2H), 3.25 (s, 3H), 2.90-2.77 (m, 1H), 2.46-2.38 (m, 1H), 2.33-2.16 (m, 5H), 2.13 - 1.94 (m, 6H), 1.90 - 1.86 (m, 3H), 1.78 - 1.55 (m, 3H).

### Example 31 Synthesis of compounds Z72 and Z75

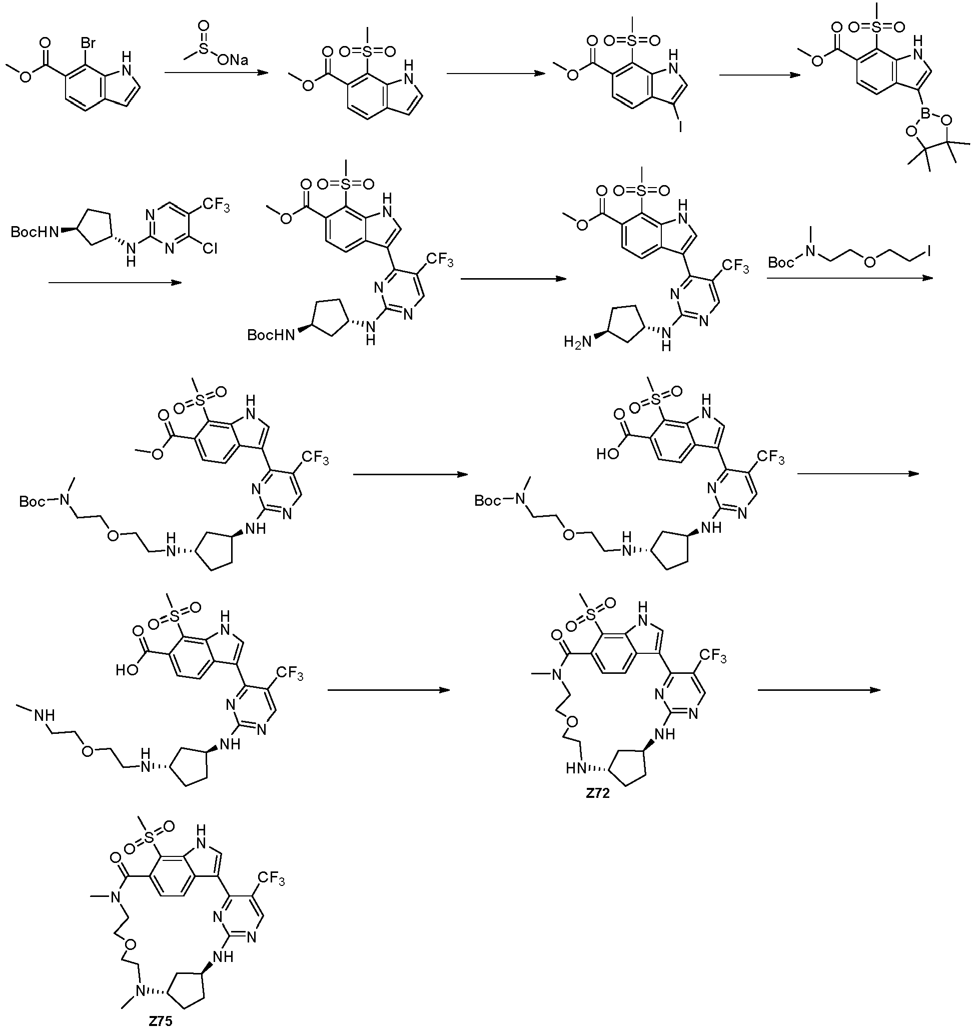

Step 1: L-proline (0.18 g, 1.6 mmol), cuprous iodide (0.95 g, 0.8 mmol) and sodium methyl sulfite (1.1 g, 9.5 mmol) were added into a solution of methyl 7-bromo-1H-indole-6-carboxylate (2.0 g, 7.9 mmol) in dimethyl sulfoxide (15 mL) at room temperature. Stirring was carried out to react at 90°C under nitrogen protection for 16 h. After realizing complete reaction by LCMS detection, a saturated ammonium chloride aqueous solution (30 mL) was added into a reaction solution for quenching, extracting was carried out with ethyl acetate (30 mL X 3), a combined organic layer was washed for three times by saturated brine, dried over anhydrous sodium sulfate, filtered and concentrated. The crude product was purified with silica gel column chromatography (petroleum ether/ethyl acetate = 30/1-2/1) to obtain a white solid methyl 7-(methylsulfonyl)-1H-indole-6-carboxylate (1.4 g, yield of 70%). LCMS: m/z (ESI): 254.0 [M+H]⁺, t_{R} = 1.10 min.

Step 2: N-iodosuccinimide (1.5 g, 6.6 mmol) was added into a solution of methyl 7-(methylsulfonyl)-1H-indole-6-carboxylate (1.4 g, 5.5 mmol) in N,N dimethylformamide (15 mL) at room temperature. The reaction solution was stirred for 16 h at room temperature under nitrogen protection. After realizing complete reaction by LCMS detection, water (30 mL) was added into the reaction solution, then extracting was carried out with ethyl acetate (30 mL X 3), a combined organic layer was washed with saturated brine, dried over anhydrous sodium sulfate, filtered and concentrated. The crude product was purified with silica gel column chromatography (petroleum ether/ethyl acetate = 20/1-1: 1) to obtain methyl 3-iodine-7-(methylsulfonyl)-1H-indole-6-carboxylate (1.8 g, yield of 86%). LCMS: m/z (ESI): 380.0 [M+H]⁺, t_{R} = 1.25 min.

Step 3: Methyl 3-iodine-7-(methylsulfonyl)-1H-indole-6-carboxylate (1.8 g, 4.7 mmol), Tris(dibenzylideneacetone)dipalladium (0.44 g, 0.47 mmol), 2-dicyclohexylphosphine-2,4,6-triisopropylbiphenyl (0.45 g, 0.94 mmol), pinacolborane (0.91 g, 7.1 mmol), triethylamine (1.4 g, 14.1 mmol) and tetrahydrofuran (15 mL) solution were sequentially added into a sealed tube at room temperature. Stirring was carried out for 16 h at 90°C under nitrogen protection. After realizing complete reaction by LCMS detection, spin-evaporation and concentration were carried out on reaction solution to obtain a crude product. The crude product was purified with silica gel column chromatography purification (petroleum ether/ethyl acetate = 10/1-2/1) to obtain white solid methyl 7-(methylsulfonyl)-3-(4,4,5,5-tetramethyl-1,3,2-dioxaboropentan-2-yl)-1H-indole-6-carboxylate (1.1 g, yield of 61%). LCMS: m/z (ESI): 380.2 [M+H]⁺, t_{R} = 1.35 min.

Step 4: A mixed solution of methyl 7-(methylsulfonyl)-3-(4,4,5,5-tetramethyl-1,3,2-dioxaboropentan-2-yl)-1H-indole-6-carboxylate (1.1 g, 2.9 mmol), chlorine [(n-butyl di(1-adamantyl)phosphino)-2-(2-aminobiphenyl)]palladium (II) (0.19 g, 0.29 mmol), potassium phosphate (1.8 g, 8.7 mmol), tert-butyl ((1S,3S)-3-((4-chlorine-5-(trifluoromethyl)pyrimidin-2-yl)amino)cyclopentyl)carbamate (1.1 g, 2.9 mmol), tetrahydrofuran (25 mL) and water (5 mL) into a dry 50 mL single-neck flask at room temperature. Stirring was carried out for 16 h at 60°C under nitrogen protection. After realizing complete reaction by LCMS detection, spin-evaporation and concentration were carried out on a reaction solution to obtain a crude product. The crude product was purified with silica gel column chromatography purification (petroleum ether/ethyl acetate = 10/1-0/1) to obtain a white solid methyl 3-(2-((1S,3S)-3-((t-butyloxycarbonyl)amino)cyclopentyl)amino)-5-(trifluoromethyl)pyrimidin-4-yl)-7-(methylsulfonyl)-1H-indole-6-carboxylate (0.57 g, yield of 33%). LCMS: m/z (ESI): 598.2 [M+H]⁺, t_{R} = 1.38 min.

Step 5: Methyl 3-(2-((1S,3S)-3-((t-butyloxycarbonyl)amino)cyclopentyl)amino)-5-(trifluoromethyl)pyrimidin-4-yl)-7-(methylsulfonyl)-1H-indole-6-carboxylate (570 mg, 0.95 mmol) was dissolved in a mixed solution of hydrogen chloride/ethyl acetate (5 mL, 4 M) and ethyl acetate (3 mL) at room temperature, and reacting was carried out for 16 h at 30°C. After realizing complete reaction by LCMS detection, a saturated sodium bicarbonate solution (30 mL) was added into the reaction solution, then extracting was carried out with dichloromethane (20 mL X 3), the combined organic layer was washed with saturated brine, dried over anhydrous sodium sulfate, filtered and concentrated. The resulting crude product was purified with column chromatography (dichloromethane/methanol = 20: 1) to obtain a white solid methyl 3-(2-((1S,3S)-3-aminocyclopentyl)amino)-5-(trifluoromethyl)pyrimidin-4-yl)-7-(methylsulfonyl)-1H-indole-6- carboxylate (300 mg, yield of 64%). LCMS: m/z (ESI): 498.3 [M+H]⁺, t_{R} = 1.12 min.

Step 6: Tert-butyl (2-(2-iodoethoxy)ethyl) (methyl)carbamate (197 mg, 0.60 mmol) and N,N-diisopropylethylamine (310 mg, 2.40 mmol) were sequentially added into a solution of methyl 3-(2-((1S,3S)-3-aminocyclopentyl)amino)-5-(trifluoromethyl)pyrimidin-4-yl)-7-(methylsulfonyl)-1H-indole-6-carboxylate (300 mg, 0.60 mmol) in N,N-dimethylformamide (10 mL) at room temperature, and stirring was carried out at 80°C for 16 h. After realizing complete reaction by LCMS detection, a saturated sodium bicarbonate solution (30 mL) was added into the reaction solution, extracting was carried out with ethyl acetate (20 mL X 3), the combined organic layer was washed with saturated brine, dried over anhydrous sodium sulfate, filtered and concentrated. The resulting crude product was purified with column chromatography (dichloromethane/methanol = 30:1) to obtain a white solid methyl 3-(2-((1S,3S)-3-((2-(2-((t-butyloxycarbonyl) (methyl)amino)ethoxy)ethyl)amino)cyclopentyl)amino)-5-(trifluoromethyl)pyrimidin-4-yl)-7-(methylsulfonyl)-1H-indole-6-carboxylate (180 mg, yield of 43%). LCMS: m/z (ESI): 699.4 [M+H]⁺, t_{R} = 1.29 min.

Step 7: Methyl 3-(2-((1S,3S)-3-((2-(2-((t-butyloxycarbonyl) (methyl)amino)ethoxy)ethyl)amino)cyclopentyl)amino)-5-(trifluoromethyl)pyrimidin-4-yl)-7-(methylsulfonyl)-1H-indole-6-carboxylate (180 mg, 0.26 mmol) was dissolved in a mixed system of water (3 mL), methanol (3 mL) and tetrahydrofuran (3 mL), then lithium hydroxide monohydrate (55 mg, 1.3 mmol) was added, and stirring was carried out at 40°C for 16 h. After realizing complete reaction by LCMS detection, spin-drying was directly carried out on the reaction solution by rotary evaporation to obtain a crude product 3-(2-((1S,3S)-3-((2-(2-((t-butyloxycarbonyl) (methyl)amino)ethoxy)ethyl)amino)cyclopentyl)amino)-5-(trifluoromethyl)pyrimidin-4-yl)-7-(methylsulfonyl)-1H-indole-6-carboxylic acid (176 mg, yield of 100%), the product was directly used in next step without any purification. LCMS: m/z (ESI): 685.3 [M+H]⁺, t_{R} = 1.21 min.

Step 8: 3-(2-((1S,3S)-3-((2-(2-((t-butyloxycarbonyl) (methyl)amino)ethoxy)ethyl)amino)cyclopentyl)amino)-5-(trifluoromethyl)pyrimidin-4-yl)-7-(methylsulfonyl)-1H-indole-6-carboxylic acid (176 mg, 0.26 mmol) was dissolved in methanol (5 mL), then hydrochloric acid/methanol (5 mL, 4 M) was added, and stirring was carried out at 40°C for 16 h. After realizing complete reaction by LCMS detection, the reaction solution was concentrated, and reversed-phase preparation was carried out (chromatographic column: Ultimate XB-C18, 50X250 mm, 10 um (PARP-02), mobile phase: acetonitrile-water (0.1% trifluoroacetic acid), flow rate: 70 mL/min, column temperature: 25°C) on the resulting crude product to obtain a white solid 3-(2-((1S,3S)-3-((2-(methylamino)ethoxy)ethyl)amino)cyclopentyl amino)-5-(trifluoromethyl)pyrimidin-4-yl)-7-(methylsulfonyl)-1H-indole-6-carboxylic acid (110 mg, yield of 72%, as trifluoroacetate). LCMS: m/z (ESI): 585.2 [M+H]⁺, t_{R} = 0.78 min.

Step 9: 2-(7-azabenzotriazole)-N,N,N',N'-tetramethyluronium hexafluorophosphate (61 mg, 0.16 mmol) and N,N-diisopropylethylamine (83 mg, 0.64 mmol) were sequentially added into a solution of 3-(2-((1S,3S)-3-((2-(methylamino)ethoxy)ethyl)amino)cyclopentyl amino)-5-(trifluoromethyl)pyrimidin-4-yl)-7-(methylsulfonyl)-1H-indole-6-carboxylic acid (110 mg, 0.16 mmol, as trifluoroacetate) in N,N-dimethylformamide (10 mL), and stirring was carried out at 60°C for 16 h. After realizing complete reaction by LCMS detection, the reaction solution was concentrated, and reversed-phase preparation was carried out (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid (4¹S,4³S)-11-methyl-1⁷-(methylsulfonyl)-2⁵-(trifluoromethyl)-1¹H-8-oxa-3,5,11-triaza-1(3,6)-indola-2(4,2)-pyrimidina-4(11,3)-cyclopentanacyclododecaphan-12-one (Z72 mg, 55 mg, yield of 51%, as trifluoroacetate). LCMS: m/z (ESI): 567.2 [M+H]⁺, t_{R} = 1.12 min.¹H NMR (400 MHz, CD₃OD): δ 8.59 (s, 1H), 8.34 (d, *J* = 8.0 Hz, 1H), 7.86 (s, 1H), 7.39 (d, *J* = 8.0, 1H), 4.48 - 4.10 (m, 1H), 4.05 - 3.89 (m, 1H), 3.86-3.70 (m, 2H), 3.68-3.60 (m, 1H), 3.57 - 3.39 (m, 3H), 3.33 (s, 3H), 3.25-3.16 (m, 2H), 3.16 - 3.09 (m, 3H), 2.80 - 2.52 (m, 1H), 2.44 - 2.25 (m, 1H), 2.24 - 1.90 (m, 2H), 1.86 - 1.53 (m, 2H).

Step 10: Paraformaldehyde (20 mg, 0.221 mmol) and tetraisopropyl titanate (63 mg, 0.221 mmol) were sequentially added into a solution of (4¹S,4³S)-11-methyl-1⁷-(methylsulfonyl)-2⁵-(trifluoromethyl)-1¹H-8-oxa-3,5,11-triaza-1(3,6)-indola-2(4,2)-pyrimidina-4(1,3)-cyclopentanacyclododecaphan-12-one (30 mg, 0.044 mmol, as trifluoroacetate) in tetrahydrofuran (10 mL) in a 10 mL single-neck flask, the reaction solution was stirred at 70°C for 14 h and then cooled to 30°C, sodium cyanoborohydride (41 mg, 0.660 mmol) was added, and stirring continued at 30°C for 2 h. After realizing complete reaction by LCMS detection, the reaction solution was concentrated, and reversed-phase preparation was carried out on the resulting crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain (4¹S,4³S)-5,11-dimethyl-1⁷-(methylsulfonyl)-2⁵-(trifluoromethyl)-1¹H-8-oxa-3,5,11-triaza-1(3,6)-indola-2(4,2)-pyrimidina-4(1,3)-cyclopentanacyclododecaphan-12-one (Z75, 8 mg, yield of 26%. trifluoroacetate). LCMS: m/z (ESI): 581.2 [M+H]⁺, t_{R} = 1.12 min.¹H NMR (400 MHz, CD₃OD): δ 8.60 (s, 1H), 8.25 (d, *J* = 8.0 Hz, 1H), 7.82 (s, 1H), 7.36 (d, *J* = 8.0 Hz, 1H), 4.19 - 4.03 (m, 1H), 3.98-3.89 (m, 1H), 3.88 - 3.72 (m, 3H), 3.70-3.52 (m, 2H), 3.49-3.37 (m, 1H), 3.32 (s, 3H), 3.19-3.11 (m, 5H), 2.87 - 2.82 (m, 3H), 2.61 - 2.43 (m, 1H), 2.31 - 2.21 (m, 3H), 1.86-1.73 (m, 2H).

### Example 32 Synthesis of compounds Z76, Z77, Z78 and Z79

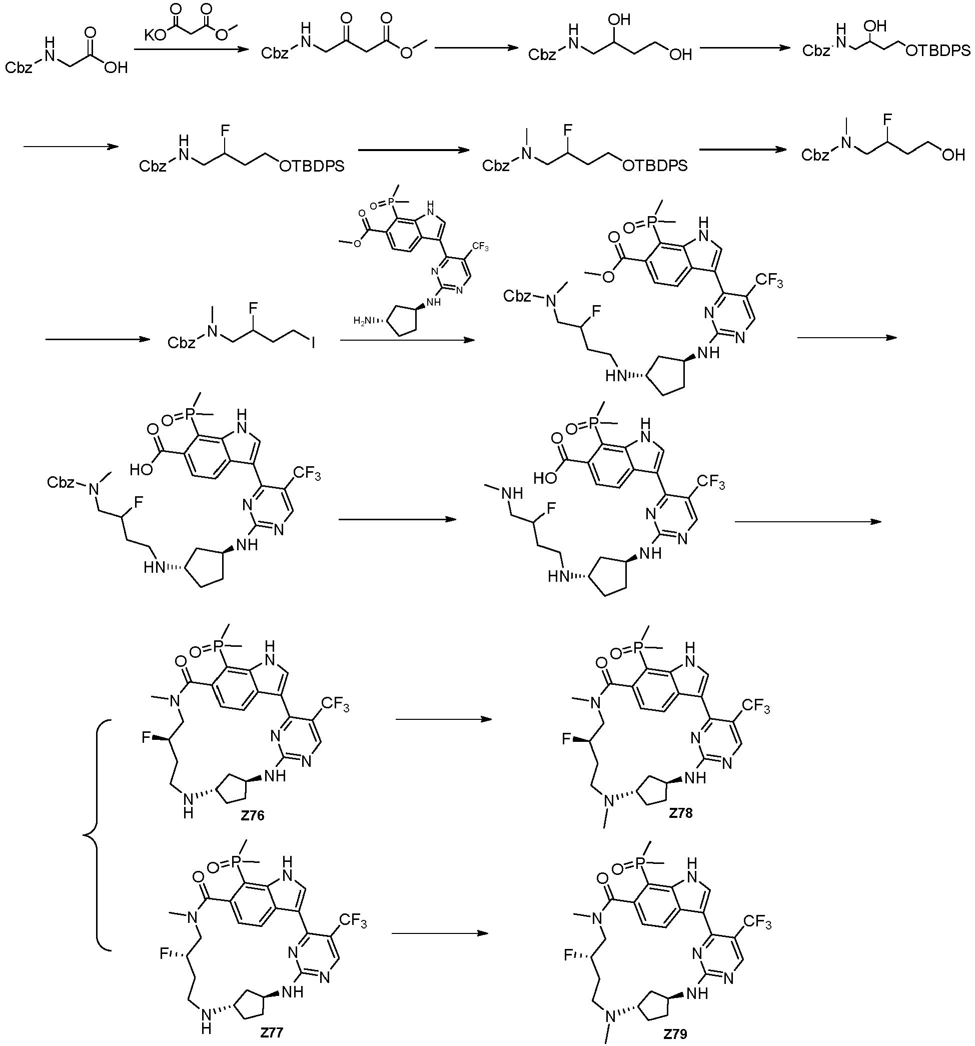

Step 1: Carbonyl diimidazole (85.3 g, 526.34 mmol) are added into a solution of N-[(benzyloxy)carbonyl]glycine (100.0 g, 478.49 mmol) in tetrahydrofuran (1000 mL) at 15°C. The reaction solution was stirred to react at 15°C under nitrogen protection for 18 h. Then magnesium chloride (22.8 g, 239.25 mmol) and potassium monomethyl malonate (82.2 g, 526.34 mmol) were added into the reaction solution. The reaction solution was continuously stirred to react for 6 h at 45°C under nitrogen protection. After realizing complete reaction by LCMS detection, water (600 mL) was added into the reaction solution, then extracting was carried out with ethyl acetate (500 mL X 3), a combined organic layer was washed with saturated brine (500 mL), dried over anhydrous sodium sulfate, and filtered and concentrated to obtain a crude product. The crude product was purified with column chromatography (petroleum ether/ethyl acetate = 15/1 to 3/1) to obtain a colorless oily substance benzyl [(1-methoxy-1,3-dioxo-but-4-yl)amino]carboxylate (85.3 g, yield of 67%). LCMS: m/z (ESI): 220.0 [M-44+H]⁺, t_{R} = 1.58 min.

Step 2: Lithium borohydride (14.0 g, 643.78 mmol) was added into a solution of benzyl [(1-methoxy-1,3-dioxo-but-4-yl)amino]carboxylate (85.3 g, 321.89 mmol) in tetrahydrofuran (900 mL) in batches at 15°C. The reaction solution was stirred to react at 15°C under nitrogen protection for 18 h. After realizing complete reaction by TLC detection, a saturated ammonium chloride solution (400 mL) was added into the reaction solution, then extracting was carried out with ethyl acetate (600 mL X 3), the combined organic layer was washed with saturated brine (600 mL), dried over anhydrous sodium sulfate, and filtered and concentrated to obtain a crude product. The crude product was purified with column chromatography (petroleum ether/ethyl acetate = 5/1 to 1/2) to obtain a colorless oily substance benzyl [(2,4-dihydroxy butyl)amino]carboxylate (60.0 g, yield of 78%).

Step 3: Imidazole (22.2 g, 326.37 mmol) was added into a solution of benzyl [(2,4-dihydroxy butyl)amino]carboxylate (60.0 g, 251.05 mmol) in dichloromethane (600 mL) at 15°C, and then tert-butyl diphenylchlorosilane (69.0 g, 251.05 mmol) was slowly added dropwise. The reaction solution was stirred to react at 15°C under nitrogen protection for 2 h. After realizing complete reaction by LCMS detection, a saturated ammonium chloride solution (400 mL) was added into the reaction solution, then extracting was carried out with ethyl acetate (600 mL X 3), the combined organic layer was washed with saturated brine (600 mL), dried over anhydrous sodium sulfate, filtered and concentrated. The resulting crude product was purified with column chromatography (petroleum ether/ethyl acetate = 10/1 to 2/1) to obtain a colorless oily substance benzyl [(7-hydroxy-2,2-dimethyl-3,3-diphenyl-4-oxa-3-silazaoct-8-yl)amino]carboxylate (88.0 g, yield of 74%). LCMS: m/z (ESI): 402.2 [M-77+H]⁺, t_{R} = 1.74 min.

Step 4: Benzyl [(7-hydroxy-2,2-dimethyl-3,3-diphenyl-4-oxa-3-silazaoct-8-yl)amino]carboxylate (88.0 g, 184.49 mmol) was dissolved in dichloromethane (900 mL), and then diethylaminosulfur trifluoride (38.7 g, 239.83 mmol) was slowly added dropwise at room temperature. Stirring was carried out for 2 h at 15°C in the reaction. After realizing complete reaction by TLC detection, a saturated ammonium chloride solution (400 mL) was added into the reaction solution, then extracting was carried out with ethyl acetate (600 mL X 3), the combined organic layer was washed with saturated brine (600 mL), dried over anhydrous sodium sulfate, filtered and concentrated. The resulting crude product was purified with column chromatography (petroleum ether/ethyl acetate = 30/1 to 5/1) to obtain a colorless oily substance benzyl [(7-fluoro-2,2-dimethyl-3,3-diphenyl-4-oxa-3-silazaoct-8-yl)amino]carboxylate (20.5 g, yield of 23%). LCMS: m/z (ESI): 502.2 [M+Na]⁺, t_{R} = 1.74 min.

Step 5: Benzyl [(7-fluoro-2,2-dimethyl-3,3-diphenyl-4-oxa-3-silazaoct-8-yl)amino]carboxylate (20.5 g, 42.80 mmol) was dissolved in tetrahydrofuran (150 mL), sodium hydrogen (5.1 g, 60% dispersed in mineral oil, 128.40 mmol) was added in batches at room temperature, and methyl iodide (12.2 g, 85.60 mmol) was slowly added dropwise. Stirring was carried out for 12 h at 15°C in the reaction. After realizing complete reaction by LCMS detection, a saturated ammonium chloride solution (200 mL) was added into the reaction solution, then extracting was carried out with ethyl acetate (200 mL X 3), a combined organic layer was washed with saturated brine (200 mL), dried over anhydrous sodium sulfate, filtered and concentrated. The resulting crude product was purified with column chromatography (petroleum ether/ethyl acetate = 30/1 to 5/1) to obtain a colorless oily substance benzyl (7-fluoro-2,2-dimethyl-3,3-diphenyl-9-aza-4-oxa-3-silazaoct-9-yl)carboxylate (16.5 g, yield of 78%). LCMS: m/z (ESI): 516.2 [M+Na]⁺, t_{R} = 1.85 min.

Step 6: Benzyl (7-fluoro-2,2-dimethyl-3,3-diphenyl-9-aza-4-oxa-3-silazaoct-9-yl)carboxylate (10.0 g, 20.24 mmol) was dissolved in a solution of N,N-dimethylformamide (60 mL), then cesium fluoride (15.4 g, 101.20 mmol) was added, and stirring was carried out for 8 h at 50°C. After realizing complete reaction by LCMS detection, a saturated ammonium chloride solution (100 mL) was added into the reaction solution, then extracting was carried out with ethyl acetate (100 mL X 3), the combined organic layer was washed with saturated brine (100 mL), dried over anhydrous sodium sulfate, filtered and concentrated. The resulting crude product was purified with column chromatography (petroleum ether/ethyl acetate = 10/1 to 2/1) to obtain a colorless oily substance benzyl [(2-fluoro-4-hydroxybutyl) (methyl)amino]carboxylate (4.4 g, yield of 85%). LCMS: m/z (ESI): 256.2 [M+H]⁺, t_{R} = 1.08 min.

Step 7: Benzyl [(2-fluoro-4-hydroxybutyl) (methyl)amino]carboxylate (4.4 g, 17.25 mmol) was dissolved in a dichloromethane (40 mL) solution at room temperature, and then imidazole (1.4 g, 20.70 mmol), triphenylphosphine (5.4 g, 20.70 mmol) and iodine (5.3 g, 20.70 mmol) are sequentially added. The reaction continued for 2 h at room temperature. After realizing complete reaction by LCMS detection, a saturated ammonium chloride solution (50 mL) was added into the reaction solution, then extracting was carried out with dichloromethane (50 mL X 3), the combined organic layer was washed with saturated brine (50 mL), dried over anhydrous sodium sulfate, filtered and concentrated. The resulting crude product was purified with column chromatography (petroleum ether/ethyl acetate = 30/1 to 10/1) to obtain a colorless oily substance benzyl [(2-fluoro-4-iodobutyl) (methyl)amino]carboxylate (5.0 g, yield of 79%). LCMS: m/z (ESI): 366.0 [M+H]⁺, t_{R} = 1.44 min.

Step 8: Methyl 7-[dimethyl(oxo)-λ⁵-phosphoranyl]-3-(2-{[(1S,3S)-3-aminocyclopentyl]amino}-5-(trifluoromethyl)pyrimidin-4-yl)-1H-indole-6-carboxylate (5.0 g, 10.10 mmol) was dissolved in N,N-dimethylformamide (70 mL) in a 250 mL single-neck flask, then benzyl [(2-fluoro-4-iodobutyl) (methyl)amino]carboxylate (3.7 g, 10.10 mmol) and N,N-diisopropylethylamine (5.2 g, 40.40 mmol) were added, and stirring was carried out for 16 h at 100°C. After realizing complete reaction by LCMS detection, silica gel was directly added, an oil pump was used for performing spin-drying and sample mixing, and column chromatography purification was carried out (dichloromethane: methanol = 100:8) to obtain a yellow solid methyl 7-[dimethyl(oxo)-λ⁵-phosphoranyl]-3-(2-{[(1S,3S)-3-[(6-fluoro-4-methyl-3-oxo-1-phenyl-4-aza-2-oxaoct-8-yl)amino]cyclopentyl]amino}-5-(trifluoromethyl)pyrimidin-4-yl)-1H-indole-6-carboxylate (7.0 g, yield of 95%). LCMS: m/z (ESI): 733.2 [M+H]⁺, t_{R} = 1.14 min.

Step 9: Methyl 7-[dimethyl(oxo)-λ⁵-phosphoranyl]-3-(2-{[(1S,3S)-3-[(6-fluoro-4-methyl-3-oxo-1-phenyl-4-aza-2-oxaoct-8-yl)amino]cyclopentyl]amino}-5-(trifluoromethyl)pyrimidin-4-yl)-1H-indole-6-carboxylate (7.0 g, 9.56 mmol) was dissolved in a mixed system of water (20 mL), methanol (20 mL) and tetrahydrofuran (20 mL), then lithium hydroxide monohydrate (1.6 g, 38.24 mmol) was added, reacting and stirring were carried out for 16 h at 40°C. After realizing complete reaction by LCMS detection, the solvent in the reaction was removed by an oil pump in a rotating manner to obtain 7-[dimethyl(oxo)-λ⁵-phosphoranyl]-3-(2-{[(1S,3S)-3-[(6-fluoro-4-methyl-3-oxo-1-phenyl-4-aza-2-oxaoct-8-yl)amino]cyclopentyl]amino}-5-(trifluoromethyl)pyrimidin-4-yl)-1H-indole-6-carboxylic acid (crude product containing salt), and the product was directly used in the next reaction without purification. LCMS: m/z (ESI): 719.2 [M+H]⁺, t_{R} = 1.09 min.

Step 10: Trifluoroacetic acid (30 mL) was added into the crude product obtained from the previous step, stirring was carried out for 3 h at 60°C, after complete reaction was realized by LCMS detection, a reaction solution was concentrated, and reversed-phase preparation was carried out on a resulting crude product (70 mL/min, 25°C, Ultimate XB-C18, 50X250 mm, 10 um (PARP-02), and acetonitrile-water (0.1% trifluoroacetic acid)) to obtain 7-[dimethyl(oxo)-λ⁵-phosphoranyl]-3-(2-{[(1S,3S)-3-{[3-fluoro-4-(methylamino)butyl]amino}cyclopentyl]amino}-5-(trifluoromethyl)pyrimidin-4-yl)-1H-indole-6-carboxylic acid (4.0 g, two-step yield of 72%, white solid). LCMS: m/z (ESI): 585.2 [M+H]⁺, t_{R} = 1.13 min.

Step 11: 2-(7-azabenzotriazole)-N,N,N',N'-tetramethyluronium hexafluorophosphate (2.6 g, 6.85 mmol) and N,N-diisopropylethylamine (3.6 g, 27.4 mmol) were sequentially added into a solution of 7-[dimethyl(oxo)-λ⁵-phosphoranyl]-3-(2-{[(1S,3S)-3-{[3-fluoro-4-(methylamino)butyl]amino}cyclopentyl]amino}-5-(trifluoromethyl)pyrimidin-4-yl)-1H-indole-6-carboxylic acid (4.0 g, 6.85 mmol) in N,N-dimethylformamide (60 mL), and the reaction solution was stirred for 16 h at 60°C. After realizing complete reaction by LCMS detection, the reaction solution was concentrated, and two isomer compounds were obtained from a resulting crude product through reversed-phase preparation (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C). One isomer compound was arbitrarily designated as a off-white solid (4¹S,4³S,8R)-1⁷-(dimethylphosphoryl)-8-fluoro-10-methyl-2⁵-(trifluoromethyl)-1¹H-3,5,10-triaza-1(3,6)-indola-2(4,2)-pyrimidina-4(1,3)-cyclopentanacycloundecaphan-11-one (Z76, 0.7 g, yield of 18%, as trifluoroacetate). LCMS: m/z (ESI): 567.2 [M+H]⁺, t_{R} = 0.86 min.¹H NMR (400 MHz, CD₃OD): δ 8.55 (s, 1H), 8.30-7.70 (m, 2H), 7.30 (m, 1H), 5.69-4.98 (m, 1H), 4.28-3.95 (m, 1H), 3.87-3.76 (m, 1H), 3.69-3.37 (m, 2H), 3.21 (s, 3H), 2.89-2.78 (m, 1H), 2.74-2.64 (m, 1H), 2.48-2.14 (m, 3H), 2.12-1.54 (m, 11H). And the other isomer compound was arbitrarily designated as a off-white solid (4¹S,4³S,8S)-1⁷-(dimethylphosphoryl)-8-fluoro-10-methyl-2⁵-(trifluoromethyl)-1¹H-3,5,10-triaza-1(3,6)-indola-2(4,2)-pyrimidina-4(1,3)-cyclopentanacycloundecaphan-11-one (Z77, 0.8 g, yield of 21%, as trifluoroacetate). LCMS: m/z (ESI): 567.2 [M+H]⁺, t_{R} = 0.87 min.¹H NMR (400 MHz, CD₃OD): δ 8.58 (s, 1H), 8.40 (s, 1H), 7.93 (s, 1H), 7.21 (dd, *J* = 8.0, 3.2 Hz, 1H), 5.26-4.96 (m, 1H), 4.32-3.97 (m, 1H), 3.86-3.72 (m, 1H), 3.54-3.31 (m, 2H), 3.22 (s, 3H), 2.91-2.79 (m, 1H), 2.73-2.37 (m, 2H), 2.36-2.16 (m, 2H), 2.12-1.65 (m, 11H).

Step 12: Paraformaldehyde (176 mg, 1.95 mmol) and tetraisopropyl titanate (554 mg, 1.95 mmol) were sequentially added into a solution of (4¹S,4³S,8S)-1⁷-(dimethylphosphoryl)-8-fluoro-10-methyl-2⁵-(trifluoromethyl)-1¹H-3,5,10-triaza-1(3,6)-indola-2(4,2)-pyrimidina-4(1,3)-cyclopentanacycloundecaphan-11-one (Z76,220 mg, 0.39 mmol) in tetrahydrofuran (20 mL), and the reaction solution was stirred at 70°C for 16 h. The reaction was cooled to 10°C, sodium cyanoborohydride (74 mg, 1.17 mmol) was added into the reaction solution, and stirring continued for 2 h. After realizing complete reaction by LCMS detection, the reaction solution was concentrated, and reversed-phase preparation was carried out on the resulting crude product (chromatographic column: Ultimate XB-C18, 50X250 mm, 10 um (PARP-02), mobile phase: acetonitrile-water (0.1% trifluoroacetic acid), flow rate: 70 mL/min and column temperature: 25°C) to obtain a off-white solid (4¹S,4³S,8R)-1⁷-(dimethylphosphoryl)-8-fluoro-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z78, 45 mg, yield of 20%, as trifluoroacetate). LCMS: m/z (ESI): 581.2 [M+H]⁺, t_{R} = 0.92 min.¹H NMR (400 MHz, CD₃OD): δ 8.57 (s, 1H), 8.24-7.73 (m, 2H), 7.27 (s, 1H), 5.46-4.85 (m, 1H), 4.49-3.80 (m, 2H), 3.80-3.46 (m, 1H), 3.27-3.09 (m, 4H), 2.83-2.66 (m, 4H), 2.58-2.47 (m, 1H), 2.45-2.21 (m, 3H), 2.13-1.65 (m, 11H).

Step 13: Paraformaldehyde (158 mg, 1.75 mmol) and tetraisopropyl titanate (497 mg, 1.75 mmol) were sequentially added into a solution of (41S,43S,8S)-17-(dimethylphosphoryl)-8-fluoro-10-methyl-25-(trifluoromethyl)-11H-3,5,10-triaza-1(3,6)-indola-2(4,2)-pyrimidina-4(1,3)-cyclopentanacycloundecaphan-11-one (Z77,200 mg, 0.35 mmol) in tetrahydrofuran (20 mL), and the reaction solution was stirred at 70°C for 16 h. The reaction was cooled to 10°C, sodium cyanoborohydride (66 mg, 1.05 mmol) was added into the reaction solution, and stirring continued for 2 h. After realizing complete reaction by LCMS detection, the reaction solution was concentrated, and reversed-phase preparation was carried out on a resulting crude product (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain (4¹S,4³S,8S)-1⁷-(dimethylphosphoryl)-8-fluoro-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z79, 30 mg, yield of 15, as trifluoroacetate). LCMS: m/z (ESI): 581.2 [M+H]⁺, t_{R} = 0.92 min.¹H NMR (400 MHz, CD₃OD): δ 8.57 (s, 2H), 8.22-7.91 (m, 1H), 7.56-7.05 (m, 1H), 5.27-4.99 (m, 1H), 4.37-4.19 (m, 1H), 4.07-3.82 (m, 1H), 3.74-3.44 (m, 1H), 3.41-3.35 (m, 1H), 3.24 (s, 3H), 2.88-2.64 (m, 3H), 2.57-2.44 (m, 1H), 2.40-2.23 (m, 3H), 2.16-1.64 (m, 12H).

### Example 33 Synthesis of compound Z84

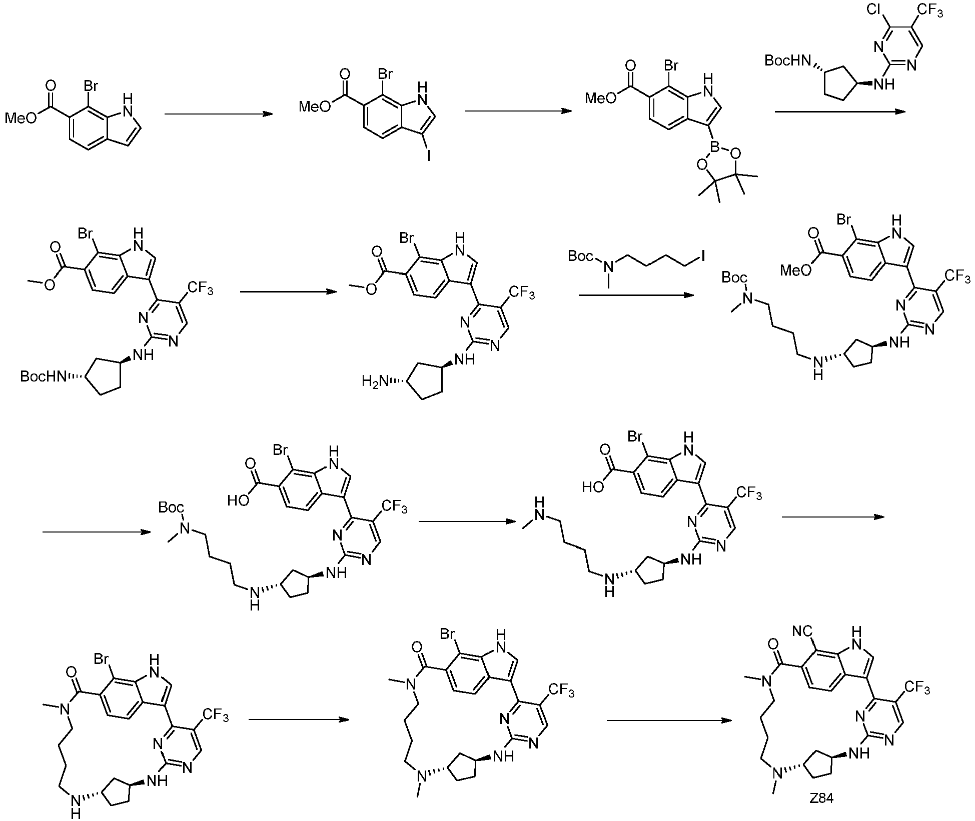

Step 1: N-iodosuccinimide (6.7 g, 29.7 mmol) was added into a solution of methyl 7-bromo-1H-indole-6-carboxylate (5.0 g, 19.8 mmol) in N,N-dimethylformamide (30 mL) at room temperature. Stirring was carried out for 2 h at 40°C under nitrogen protection. After realizing complete reaction by LCMS detection, a saturated ammonium chloride solution (40 mL) was added into the reaction solution, then extracting was carried out with 40 mL of ethyl acetate (40 mL X 3), the combined organic layer was washed with saturated brine, dried over anhydrous sodium sulfate, filtered and concentrated. The crude product was purified with silica gel column chromatography (petroleum ether/ethyl acetate = 20/1-1: 1) to obtain an off-white solid methyl 7-bromo-3-iodo-1H-indole-6-carboxylate (6.5 g, yield of 87%). LCMS: m/z (ESI): 379.8 [M+H]⁺, t_{R} = 1.32 min.

Step 2: Methyl 7-bromo-3-iodo-1H-indole-6-carboxylate (6.5 g, 17.2 mmol), tris(dibenzylideneacetone)dipalladium (1.6 g, 1.7 mmol), 2-dicyclohexylphosphine-2,4,6-triisopropylbiphenyl (0.8 g, 1.7 mmol), pinacolborane (6.5 g, 51.6 mmol), triethylamine (5.2 g, 51.6 mmol) and tetrahydrofuran (60 mL) were sequentially added into a sealed tube at room temperature. Stirring was carried out for 15 h at 90°C under nitrogen protection. After realizing complete reaction by LCMS detection, rotary evaporation and concentration were directly carried out on reaction solution to obtain a crude product. The crude product was purified with silica gel column chromatography (petroleum ether/ethyl acetate = 10/1-2/1) to obtain an off-white solid methyl 7-bromo-3-(4,4,5,5-tetramethyl-1,3,2-dioxaborolan-2-yl)-1H-indole-6-carboxylate (2.4 g, yield of 37%). LCMS: m/z (ESI): 380.0 [M+H]⁺, t_{R} = 1.48 min.

Step 3: A mixed solution of methyl 7-bromo-3-(4,4,5,5-tetramethyl-1,3,2-dioxaborolan-2-yl)-1H-indole-6-carboxylate (2.4 g, 6.3 mmol), bis(triphenylphosphino)palladium dichloride (0.44 g, 0.63 mmol), potassium carbonate (1.7 g, 12.6 mmol), tert-butyl ((1S,3S)-3-((4-chloro-5-(trifluoromethyl)pyrimidin-2-yl)amino)cyclopentyl)carbamate (2.4 g, 6.3 mmol), dioxane (30 mL) and water (6 mL) were sequentially added into a dry 100 mL single-neck flask at room temperature. Stirring was carried out for 16 h at 90°C under nitrogen protection. After realizing complete reaction by LCMS detection, rotary evaporation and concentration were directly carried out on a reaction solution to obtain a crude product. The crude product was purified with silica gel column chromatography (petroleum ether/ethyl acetate = 10/1-0/1) to obtain a white solid methyl 7-bromo-3-(2-(((1S,3S)-3-((t-butyloxycarbonyl)amino)cyclopentyl)amino)-5-(trifluoromethyl)pyrimidin-4-yl)-1H-indole-6-carboxylate (1.4 g, yield of 37%). LCMS: m/z (ESI): 598.2 [M+H]⁺, t_{R} = 1.46 min.

Step 4: Methyl 7-bromo-3-(2-(((1S,3S)-3-((t-butyloxycarbonyl)amino)cyclopentyl)amino)-5-(trifluoromethyl)pyrimidin-4-yl)-1H-indole-6-carboxylate (1.4 g, 2.3 mmol) was dissolved in a mixed solution of hydrogen chloride/ethyl acetate (15 mL, 4 M) and ethyl acetate (10 mL) at room temperature, and reacting was carried out at 40°C for 16 h. After realizing complete reaction by LCMS detection, a saturated sodium bicarbonate solution (50 mL) was added into the reaction solution, then extracting was carried out with dichloromethane (40 mL X 3), the combined organic layer was washed with saturated brine, dried over anhydrous sodium sulfate, filtered and concentrated. The resulting crude product was purified with column chromatography (dichloromethane/methanol = 20: 1) to obtain a white solid methyl 3-(2-(((1S,3S)-3-aminocyclopentyl)amino)-5-(trifluoromethyl)pyrimidin-4-yl)-7-bromo-1H-indole-6-carboxylate (0.8 g, yield of 69%). LCMS: m/z (ESI): 498.0 [M+H]⁺, t_{R} = 1.08 min. Step 5: Tert-butyl(4-iodobutyl)(methyl)carbamate (344 mg, 1.1 mmol) and N,N-diisopropylethylamine (645 mg, 5.0 mmol) were sequentially added into a solution of methyl 3-(2-(((1S,3S)-3-aminocyclopentyl)amino)-5-(trifluoromethyl)pyrimidin-4-yl)-7-bromo-1H-indole-6-carboxylate (500 mg, 1.0 mmol) in N,N-dimethylformamide (10 mL), and stirring was carried out at 70°C for 16 h. After realizing complete reaction by LCMS detection, a saturated sodium bicarbonate solution (30 mL) was added into the reaction solution, extracting was carried out with ethyl acetate (20 mL X 3), the combined organic layer was washed with saturated brine, dried over anhydrous sodium sulfate, filtered and concentrated. The resulting crude product was purified with column chromatography (dichloromethane/methanol = 30: 1) to obtain the white solid methyl 7-bromo-3-(2-(((1S,3S)-3-((4-((t-butyloxycarbonyl) (methyl)amino)butyl)amino)cyclopentyl)amino)-5-(trifluoromethyl)pyrimidin-4-yl)-1H-indole-6-carboxylate (60 mg, yield of 9%). LCMS: m/z (ESI): 683.2 [M+H]⁺, t_{R} = 1.32 min.

Step 6: Methyl 7-bromo-3-(2-(((1S,3S)-3-((4-((t-butyloxycarbonyl) (methyl)amino)butyl)amino)cyclopentyl)amino)-5-(trifluoromethyl)pyrimidin-4-yl)-1H-indole-6-carboxylate (60 mg, 0.09 mmol) was dissolved in a mixed system of water (3 mL), methanol (3 mL) and tetrahydrofuran (3 mL), then lithium hydroxide monohydrate (19 mg, 0.45 mmol) was added, and stirring was carried out at 40°C for 6 h. After realizing complete reaction by LCMS detection, spin-drying was directly carried out on the reaction solution by rotary evaporation to obtain a crude product 7-bromo-3-(2-(((1S,3S)-3-((4-((t-butyloxycarbonyl) (methyl)amino)butyl)amino)cyclopentyl)amino)-5-(trifluoromethyl)pyrimidin-4-yl)-1H-indole-6-carboxylic acid (60 mg, yield of greater than 100%), and the crude product was directly used in the next reaction without any purification. LCMS: m/z (ESI): 669.2 [M+H]⁺, t_{R} = 1.34 min.

Step 7: 7-bromo-3-(2-(((1S,3S)-3-((4-((t-butyloxycarbonyl) (methyl)amino)butyl)amino)cyclopentyl)amino)-5-(trifluoromethyl)pyrimidin-4-yl)-1H-indole-6-carboxylic acid (60 mg, 0.09 mmol) was dissolved in methanol (5 mL), then hydrochloric acid/methanol (5 mL, 4 M) was added, and stirring was carried out at 20°C for 16 h. After realizing complete reaction by LCMS detection, the reaction solution was concentrated, and the resulting crude product was purified by reversed-phase prep-HPLC (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid 7-bromo-3-(2-(((1S,3S)-3-((4-(methylamino)butyl)amino)cyclopentyl)amino)-5-(trifluoromethyl)pyrimidin-4-yl)-1H-indole-6-carboxylic acid (30 mg, yield of 59%, as trifluoroacetate). LCMS: m/z (ESI): 569.3 [M+H]⁺, t_{R} = 8.51 min.

Step 8: 2-(7-azabenzotriazole)-N,N,N',N'-tetramethyluronium hexafluorophosphate (19 mg, 0.05 mmol) and N,N-diisopropylethylamine (32 mg, 0.25 mmol) were sequentially added into a solution of 7-bromo-3-(2-(((1S,3S)-3-((4-(methylamino)butyl)amino)cyclopentyl)amino)-5-(trifluoromethyl)pyrimidin-4-yl)-1H-indole-6-carboxylic acid (30 mg, 0.053 mmol) in N,N-dimethylformamide (5 mL). The reaction was stirred for 18 h at 60°C. After realizing complete reaction by LCMS detection, a reaction solution was concentrated, and the resulting crude product was purified by reversed-phase prep-HPLC (chromatographic column: Ultimate XB-C18, 50X250 mm, 10 um (PARP-02); mobile phase: acetonitrile-water (0.1% trifluoroacetic acid), flow rate: 70 mL/min, and column temperature: 25°C) to obtain a white solid (8S,11S)-20-bromo-17-methyl-3-(trifluoromethyl)-5,7,12,17,22,28-hexaazapentacyclo[17.4.3.1^{2,6}.1^{8,11}.0^{21,24}]octacosyl-1(23),2(3),4,6(28),19(20),21(24),25-heptaene-18-one (12 mg, yield of 41%, as trifluoroacetate). LCMS: m/z (ESI): 551.2 [M+H]⁺, t_{R} = 0.87 min.

Step 9: Paraformaldehyde (10 mg, 0.11 mmol) and tetraisopropyl titanate (31 mg, 0.11 mmol) were sequentially added into a solution of (8S,11S)-20-bromo-17-methyl-3-(trifluoromethyl)-5,7,12,17,22,28-hexaazapentacyclo[17.4.3.1^{2,6}.1^{8,11}.0^{21,24}]octacosyl-1(23),2(3),4,6(28),19(20),21(24),25-heptaene-18-one (12 mg, 0.022 mmol, as trifluoroacetate) tetrahydrofuran (5 mL) in a 50 mL single-neck flask, the reaction solution was stirred at 60°C for 14 h, then the reaction solution was cooled to 30°C, sodium cyanoborohydride (7 mg, 0.11 mmol) was added, and stirring continued at 30°C for 4 h. After realizing complete reaction by LCMS detection, the reaction solution was concentrated, and the resulting crude product was purified by reversed-phase prep-HPLC (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid (8S,11S)-20-bromo-12, 17-dimethyl-3-(trifluoromethyl)-5,7,12,17,22,28-hexaazapentacyclo[17.4.3.1^{2,6}.1⁸,¹¹.0^{21,24}]octacosyl-1(23),2(3),4,6(28),19(20),21(24),25-heptaene-18-one (5 mg, yield of 40%, as trifluoroacetate). LCMS: m/z (ESI): 567.2 [M+H]⁺, t_{R} = 0.86 min.

Step 10: (8S, 11S)-20-bromo-12,17-dimethyl-3-(trifluoromethyl)-5,7,12,17,22,28-hexaazapentacyclo[17.4.3.1^{2,6}.1^{8,11}.0^{21,24}]octacosyl-1(23),2(3),4,6(28),19(20),21(24),25-heptaene-18-one (5 mg, 0.009 mmol), tetrakis (triphenylphosphino)palladium (2 mg, 0.002 mmol), zinc cyanide (3 mg, 0.027 mmol) and N,N-dimethylformamide (1 mL) solution were sequentially added into a microwave tube at room temperature. Microwave reaction was carried out for 1 h at 150°C under nitrogen protection. After realizing complete reaction by LCMS detection, the reaction solution was concentrated, and the resulting crude product was purified by reversed-phase prep-HPLC (chromatographic column: Ultimate XB-C18, 50X250 mm, 10 um (PARP-02), mobile phase: acetonitrile-water, flow rate: 70 mL/min, and column temperature: 25°C) to obtain (4¹S,4³S)-5,10-dimethyl-11-oxo-2⁵-(trifluoromethyl)-1¹H-3,5,10-triaza-1(3,6)-indola-2(4,2)-pyrimidina-4(1,3)-cyclopentanacycloundecaphane-1⁷-carbonitrile (Z84, 1.6 mg, yield of 35%). LCMS: m/z (ESI): 512.2 [M+H]⁺, t_{R} = 0.85 min.¹H NMR (400 MHz, CD₃OD): δ 8.59 (d, *J* = 8.4 Hz, 1H), 8.54 (s, 1H), 7.94 (s, 1H), 7.37 (d, *J* = 8.4 Hz, 1H), 4.15-4.06 (m, 1H), 3.19-3.04 (m, 5H), 2.57-2.45 (m, 1H), 2.41-2.32 (m, 1H), 2.23-2.11 (m, 4H), 2.05-1.90 (m, 3H), 1.76-1.61 (m, 3H), 1.47-1.36 (m, 3H), 1.35-1.29 (m, 1H).

### Example 34 Synthesis of compounds Z85 and Z86

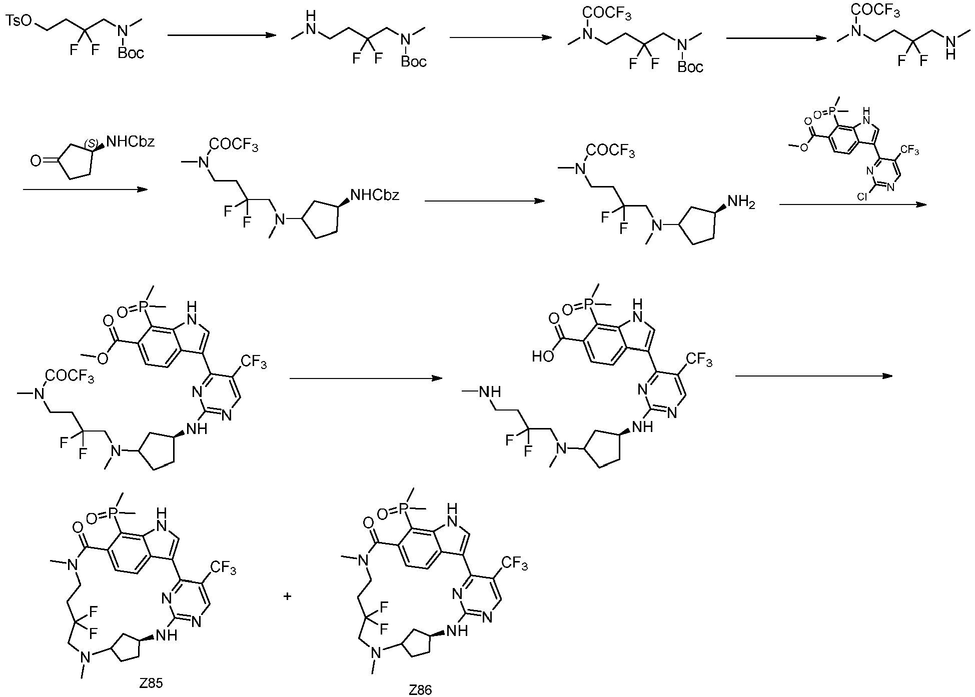

Step 1: A solution of methylamine in methanol (30% content, 20 mL) and sodium iodide (60 mg, 0.38 mmol) were added into a solution of 4-toluenesulfonic acid-7,7-difluoro-2,2,5-trimethyl-4-oxo-5-aza-3-oxanon-9-yl ester (1.50 g, 3.81 mmol) in methanol (10 mL), and stirring was carried out at 50°C for 14 h. After realizing complete reaction by LCMS detection, the reaction solution was concentrated to remove a solvent. The crude product was purified with silica gel column chromatography (dichloromethane/methanol = 100/1 to 10/1) to obtain a colorless oily substance (4,4-difluoro-2,7-diazaoct-2-yl)methanoic acid-2-methylpropyl-2-yl ester (1.0 g, yield of 83%). LCMS: m/z (ESI): 253.2 [M+H]⁺, t_{R} = 1.09 min.

Step 2: (4,4-difluoro-2,7-diazaoct-2-yl)methanoic acid-2-methylpropyl-2-yl ester (1.10 g, 3.49 mmol) was dissolved in tetrahydrofuran (10 mL), then ethyl trifluoroacetate (2.48 g, 17.4 mmol) and triethylamine (1.5 mL, 10.5 mmol) were sequentially added, stirring was carried out for 14 h at 50°C, after realizing complete reaction by LCMS detection, ethyl acetate (30 mL) and water (20 mL) were added into the reaction solution, an organic phase was separated, and concentrated. The crude product was purified with silica gel column chromatography (petroleum ether/ethyl acetate = 100/1 to 5/1) to obtain a colorless oily substance (4,4,9,9,9-pentafluoro-7-methyl-8-oxo-2,7-diazanon-2-yl)methanoic acid-2-methylpropyl-2-yl ester (660 mg, yield of 54%). LCMS: m/z (ESI): 249.1 [M+H-99]⁺, t_{R} = 1.35 min.

Step 3: (4,4,9,9,9-pentafluoro-7-methyl-8-oxo-2,7-diazanon-2-yl) methanoic acid-2-methylpropyl-2-yl ester (566 mg, 1.63 mmol) was dissolved in dichloromethane (5 mL), then trifluoroacetic acid (1.0 mL) was added into the reaction solution, stirring was carried out for 3 h at 25°C, after realizing complete reaction by LCMS detection, ethyl acetate (10 mL) and a saturated sodium bicarbonate solution (5 mL) were added into the reaction solution, and an organic phase was concentrated to obtain a crude product which is a colorless oily substance N-[3,3-difluoro-4-(methylamino)butyl]-2,2,2-trifluoro-N-methylacetamide (400 mg, yield of 99%), the product was directly used in next reaction. LCMS: m/z (ESI): 249.2 [M+H]⁺, t_{R} = 0.89 min.

Step 4: N-[3,3-difluoro-4-(methylamino)butyl]-2,2,2-trifluoro-N-methylacetamide (399 mg, 1.61 mmol) was dissolved in methanol (5 mL), then benzyl {[(3S)-1-oxocyclopenta-3-yl]amino}carboxylate (562 mg, 2.41 mmol), a 4A molecular sieve (100 mg) and glacial acetic acid (2 drops) were sequentially added, stirring was carried out for 2 h at 25°C, then sodium cyanoborohydride (303 mg, 4.82 mmol) was added into the reaction solution, and stirring continued for 14 h at 25°C. After realizing complete reaction by LCMS detection, filtering was carried out to remove solid impurities, and the resulting crude product was purified by reversed-phase prep-HPLC (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a colorless oily substance benzyl {[(3S)-1-(4,4,9,9, 9-pentafluoro-7-methyl-8-oxo-2,7-diazanon-2-yl)cyclopenta-3-yl]amino}carboxylate (380 mg, yield of 50%). LCMS: m/z (ESI): 466.4 [M+H]⁺, t_{R} = 1.33 min.

Step 5: f [(3S)-1-(4,4,9,9,9-pentafluoro-7-methyl-8-oxo-2,7-diazanon-2-yl)cyclopenta-3-yl]amino}methanoic acid benzyl ester (330 mg, 2.21 mmol) was dissolved in methanol (10 mL), palladium on carbon (30 mg, 10% loading, 55% of water content) and glacial acetic acid (30 mg, 0.50 mmol) were added into the reaction solution, hydrogen was replaced, stirring was carried out for 2 h at 25°C, and after realizing complete reaction by TLC detection, filtering was carried out, and the organic phase was concentrated to obtain a crude product which is a colorless oily substance N-(3,3-difluoro-4-{[(3S)-3-aminocyclopentyl](methyl)amino}butyl)-2,2,2-trifluoro-N-methylacetamide (310 mg, yield of 96%), the product was directly used in the next reaction. LCMS: m/z (ESI): 332.2 [M+H]⁺, t_{R} = 0.99 min.

Step 6: N-(3,3-difluoro-4-{[(3S)-3-aminocyclopentyl](methyl)amino}butyl)-2,2,2-trifluoro-N-methylacetamide (310 mg, 0.94 mmol) was dissolved in N,N-dimethylformamide (4 mL), methyl 3-[2-chloro-5-(trifluoromethyl)pyrimidin-4-yl]-7-[dimethyl(oxo)-λ⁵-phosphoranyl]-1H-indole-6-carboxylate (424 mg, 0.98 mmol) and N,N-diisopropylethylamine (0.77 mL, 4.68 mmol) were sequentially added into the reaction solution, reacting was carried out in a nitrogen atmosphere, stirring was carried out at 130°C for 5 h, and after realizing complete reaction by LCMS detection, water (10 mL) was added into the reaction solution, then extracting was carried out with ethyl acetate (10 mL X2), the organic layers were combined, dried over anhydrous sodium sulfate, filtered and concentrated. The resulting crude product was purified by reversed-phase prep-HPLC (chromatographic column: Ultimate XB-C18, 50X250 mm, 10 um (PARP-02), mobile phase: acetonitrile-water (0.1% trifluoroacetic acid), flow rate: 70 mL/min, and column temperature: 25°C) to obtain a white solid methyl 7-[dimethyl(oxo)-λ⁵-phosphoranyl]-3-(2-{[(1S,3S)-3-(4,4,9,9-pentafluoro-7-methyl-8-oxo-2,7-diaza-2-yl)cyclopentyl]-5-(trifluoromethyl)pyrimidin-4-yl)-1H-indole-6-carboxylate (125 mg, yield of 18%). LCMS: m/z (ESI): 727.4 [M+H]⁺, t_{R} = 1.38 min.

Step 7: Methyl 7-[dimethyl(oxo)-λ⁵-phosphoranyl]-3-(2-{[(1S,3S)-3-(4,4,9,9-pentafluoro-7-methyl-8-oxo-2,7-diaza-2-yl)cyclopentyl]-5-(trifluoromethyl)pyrimidin-4-yl)-1H-indole-6-carboxylate (125 mg, 0.17 mmol)was dissolved in a mixed solvent of water (1 mL), methanol (3 mL) and tetrahydrofuran (3 mL), lithium hydroxide (as monohydrate, 300 mg, 7.09 mmol) was added, and stirring was carried out at 40°C for 14 h. After realizing complete reaction by LCMS detection, the reaction solution was concentrated to obtain a crude product, and the resulting crude product was purified by reversed-phase prep-HPLC (chromatographic column: Ultimate XB-C18, 50X250 mm, 10 um (PARP-02), mobile phase: acetonitrile-water (0.1% ammonium bicarbonate), flow rate: 70 mL/min, and column temperature: 25°C) to obtain a white solid 7-[dimethyl(oxo)-λ⁵-phosphoranyl]-3-(2-{[(1S,3S)-3-(4,4-difluoro-2,7-diazaoct-2-yl)cyclopentyl]amino}-5-(trifluoromethyl)pyrimidin-4-yl)-1H-indole-6-carboxylic acid (60 mg, yield of 57%). LCMS: m/z (ESI): 617.4 [M+H]⁺, t_{R} = 1.13 min.

Step 8: N,N-diisopropylethylamine (37.7 mg, 0.29 mmol) and 2-(7-azabenzotriazole)-N,N,N',N'-tetramethyluronium hexafluorophosphate (74 mg, 0.20 mmol) were sequentially added into a solution of 7-[dimethyl(oxo)-λ⁵-phosphoranyl]-3-(2-{[(1S,3S)-3-(4,4-difluoro-2,7-diazaoct-2-yl)cyclopentyl]amino}-5-(trifluoromethyl)pyrimidin-4-yl)-1H-indole-6-carboxylic acid (30 mg, 0.05 mmol) in N,N-dimethylformamide (4 mL), and stirring was carried out at 60°C for 14 h. After realizing complete reaction by LCMS detection, the reaction solution was concentrated, and the resulting crude product was purified by reversed-phase prep-HPLC (chromatographic column: Ultimate XB-C18, 50X250 mm, 10 um (PARP-02), mobile phase: acetonitrile-water (0.1% ammonium bicarbonate), flow rate: 70 mL/min, and column temperature: 25°C) to obtain a white solid (4¹S,4³S)-1⁷-(dimethylphosphoryl)-7,7-difluoro-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z85, 1.51 mg, yield of 5.2%). LCMS: m/z (ESI): 599.2 [M+H]⁺, t_{R} = 0.91 min.¹H NMR (400 MHz, CD₃OD): δ 8.65 (d, J = 7.6 Hz, 1H), 8.51 (s, 1H), 8.02 (d, J = 5.6 Hz, 1H), 7.32 (dd, J = 8.0, 3.6 Hz, 1H), 4.31 - 4.16 (m, 1H), 3.29 - 3.18 (m, 3H), 3.13 (s, 3H), 2.88 - 2.77 (m, 1H), 2.70 - 2.53 (m, 1H), 2.47 - 2.08 (m, 8H), 2.05 - 1.93 (m, 4H), 1.91 - 1.81 (m, 3H), 1.65 - 1.52 (m, 2H). (4¹S,4³S)-1⁷-(dimethylphosphoryl)-7,7-difluoro-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z86, 1.27 mg, yield of 4.4%). LCMS: m/z (ESI): 599.3 [M+H]⁺, t_{R} = 0.93 min. ¹H NMR (400 MHz, CD₃OD): δ 8.53 (s, 1H), 8.16 - 8.12 (m, 1H), 7.83 (s, 1H), 7.24 (dd, J = 8.0, 3.2 Hz, 1H), 3.74 - 3.68 (m, 1H), 3.12 (s, 3H), 2.96 - 2.77 (m, 2H), 2.68 - 2.41 (m, 4H), 2.32 (s, 3H), 2.06 - 1.84 (m, 9H), 1.76 - 1.58 (m, 4H).

### Example 35 Synthesis of compounds Z87 and Z88

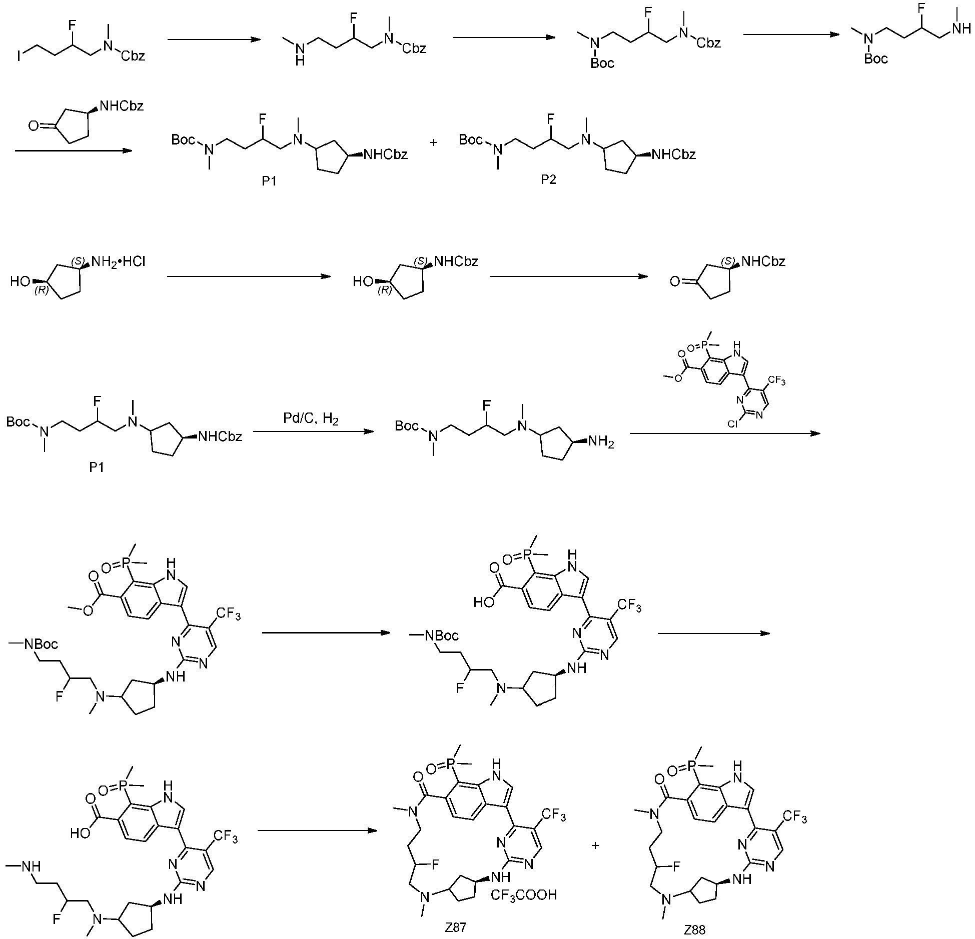

Step 1: A solution of methylamine in methanol (30% content, 5.87 g, 56.7 mmol) was added into a solution of (2-fluoro-4-iodobutyl)(methyl)benzyl carbamate (2.07 g, 5.67 mmol) in methanol (20 mL) at room temperature, and stirring was carried out at 50°C for 16 h. After realizing complete reaction by LCMS detection, the reaction solution was concentrated to remove the solvent. The crude product was purified with silica gel column chromatography (dichloromethane/methanol = 100/1 to 20/1) to obtain a colorless oily substance benzyl (4-fluoro-2,7-diazaoct-2-yl)carboxylate (1.50 g, yield of 98%). LCMS: m/z (ESI): 269.2 [M+H]⁺, t_{R} = 1.17 min.

Step 2: Benzyl (4-fluoro-2,7-diazaoct-2-yl)carboxylate (1.50 g, 5.59 mmol) was dissolved in dichloromethane (20 mL), then 4-dimethylaminopyridine (0.07 g, 0.56 mmol) and di-tert-butyl dicarbonate (1.46 g, 6.71 mmol) were sequentially added, stirring was carried out for 14 h at 25°C, after realizing complete reaction by LCMS detection, the reaction solution was concentrated to remove a solvent. The crude product was purified with silica gel column chromatography (dichloromethane/methanol = 100/1 to 20/1) to obtain a colorless oily substance benzyl (8-fluoro-2,2,5-trimethyl-4-oxo-5,10-diaza-3-oxaundecan-10-yl)carboxylate (1.20 g, yield of 58%). LCMS: m/z (ESI): 269.2 [M+H-99]⁺, t_{R} = 1.71 min.

Sstep 3: Benzyl (8-fluoro-2,2,5-trimethyl-4-oxo-5,10-diaza-3-oxaundecan-10-yl)carboxylate (1.20 g, 3.26 mmol) was dissolved in methanol (20 mL), and palladium on carbon (120 mg, 10% loading, 55% of water content) was added into the reaction solution. Hydrogen was replaced, stirring was carried out for 14 h at 25°C, after realizing complete reaction by LCMS detection, filtering was carried out, and an organic phase was concentrated to obtain a crude product which was a colorless oily substance (5-fluoro-2,7-diazaoct-2-yl)benzyl methanoic acid-2-methylpropyl-2-yl ester (0.70 g, yield of 92%), and the product was directly used in the next reaction. LCMS: m/z (ESI): 235.2 [M+H]⁺, t_{R} = 1.08 min.

Step 4: (5-fluoro-2,7-diazaoct-2-yl)benzyl methanoic acid-2-methylpropyl-2-yl ester (600 mg, 2.56 mmol) was dissolved in methanol (15 mL), {[(3S)-1-oxocyclopenta-3-yl]amino}methanoic acid benzyl ester (597 mg, 2.56 mmol) and glacial acetic acid (30 mg, 0.50 mmol) were sequentially added, stirring was carried out for 2 h at 25°C, sodium cyanoborohydride (483 mg, 7.68 mmol) was added into the reaction solution, and stirring continued for 16 h at 25°C. After realizing complete reaction by LCMS detection, filtering and spin-drying were carried out, and the resulting crude product was purified by reversed-phase prep-HPLC (chromatographic column: Ultimate XB-C18, 50X250 mm, 10 um (PARP-02); mobile phase: acetonitrile-water (0.1% trifluoroacetic acid); flow rate: 70 mL/min; and column temperature: 25°C) to obtain a white solid {7-[(3S)-3-{ [(benzyloxy)carbonyl]amino}cyclopentyl]-5-fluoro-2,7-diazaoct-2-yl}methanoic acid-2-methylpropyl-2-yl ester (P1, 240 mg, yield of 20.7%). LCMS: m/z (ESI): 452.3 [M+H]⁺, t_{R} = 3.21 min. {7-[(3S)-3-{[(benzyloxy)carbonyl]amino}cyclopentyl]-5-fluoro-2,7-diazaoct-2-yl}methanoic acid-2-methylpropyl-2-yl ester (P2, 360 mg, yield of 31.1%). LCMS: m/z (ESI): 452.3 [M+H]⁺, t_{R} = 3.22 min.

Step 5: 3-aminocyclopentan-1-ol hydrochloride (10.0 g, 72.7 mmol) was dissolved in dichloromethane (100 mL), and benzoyl carbonyl succinimide (21.7 g, 87.2 mmol) and triethylamine (14.7 g, 145 mmol) were added into the reaction solution. Stirring was carried out for 14 h at 25°C in a nitrogen atmosphere, after realizing complete reaction by LCMS detection, water (100 mL) was added into the reaction solution, extracting was carried out with dichloromethane (200 mL X 3), the combined organic layer was washed by saturated brine, dried over anhydrous sodium sulfate, and filtered and concentrated. The crude product was purified with silica gel column chromatography (dichloromethane/methanol = 100/1 to 20/1), an organic phase was concentrated to obtain a colorless oily substance benzyl [(3-hydroxycyclopentyl)amino]carboxylate (9.0 g, yield of 53%). LCMS: m/z (ESI): 236.2 [M+H]⁺, t_{R} = 1.26 min.

Step 6: Dimethyl sulfoxide (1.69 g, 21.7 mmol) and oxalyl chloride (1.38 g, 10.8 mmol) were sequentially added into dichloromethane (20 mL) at -78°C, and the reaction solution was stirred at -78°C for 1 h. Benzyl [(3-hydroxycyclopentyl)amino]carboxylate (1.7 g, 7.23 mmol) was added into the reaction solution, the reaction solution was continuously reacted for 3 h, and triethylamine (3.66 g, 36.1 mmol) was added into the reaction solution. After realizing complete reaction by LCMS detection, water (30 mL) was added into the reaction solution, extracting was carried out with dichloromethane (50 mL X 3), the combined organic layer was washed by saturated brine, dried over anhydrous sodium sulfate, filtered and concentrated. The crude product was purified with silica gel column chromatography (dichloromethane/methanol = 100/1 to 30/1), an organic phase was concentrated to obtain a colorless oily substance benzyl [(3-hydroxycyclopentyl)amino]carboxylate (1.0 g, yield of 59%). LCMS: m/z (ESI): 256.1 [M+Na]⁺, t_{R} = 1.00 min.

Step 7: {7-[(3S)-3-{[(benzyloxy)carbonyl]amino}cyclopentyl]-5-fluoro-2,7-diazaoct-2-yl}methanoic acid-2-methylpropyl-2-yl ester (P1, 1.00 g, 2.21 mmol) was dissolved in methanol (20 mL), palladium on carbon (100 mg, 10% loading, 55% of water content) and glacial acetic acid (30.0 mg, 0.50 mmol) were added into the reaction solution, reacting was carried out in a hydrogen atmosphere, and stirring was carried out at 25°C for 14 h. After realizing complete reaction by LCMS detection, filtering was carried out to remove solid impurities, and the organic phase was concentrated to obtain a crude product which was a colorless oily substance {7-[(3S)-3-aminocyclopentyl]-5-fluoro-2,7-diazaoct-2-yl}methanoic acid-2-methylpropyl-2-yl ester (0.75 g, purity of 90%, yield of 96%), and the product was directly used in the next reaction. LCMS: m/z (ESI): 318.4 [M+H]⁺, t_{R} = 0.99 min.

Step 8: {7-[(3S)-3-aminocyclopentyl]-5-fluoro-2,7-diazaoct-2-yl}methanoic acid-2-methylpropyl-2-yl ester (0.75 g, 90% purity,2.13 mmol) was dissolved in N,N-dimethylformamide (10 mL), and N,N-diisopropylethylamine (1.37 g, 10.63 mmol) and methyl 3-[2-chloro-5-(trifluoromethyl)pyrimidin-4-yl]-7-[dimethyl(oxo)-λ⁵-phosphoranyl]-1H-indole-6-carboxylate (1.01 g, 2.34 mmol) were added into the reaction solution. Stirring was carried out for 5 h at 130°C in a nitrogen atmosphere, after realizing complete reaction by TLC detection, water (20 mL) was added into the reaction solution, extracting was carried out with ethyl acetate (30 mL X 3), the combined organic layer was washed with saturated brine, dried over anhydrous sodium sulfate, and filtered and concentrated. The resulting crude product was purified with silica gel column chromatography (dichloromethane/methanol = 100/1 to 20/1) and concentrated to obtain white solid methyl 7-[dimethyl(oxo)-λ⁵-phosphoranyl]-3-(2-{[(1S)-3-(8-fluoro-2,2,5-trimethyl-4-oxo-5,10-diaza-3-undecan-10-yl)cyclopentyl]amino}-5-(trifluoromethyl)pyrimidin-4-yl)-1H-indole-6-carboxylate (1.01 g, yield of 67%).

Step 9: Methyl 7-[dimethyl(oxo)-λ⁵-phosphoranyl]-3-(2-{[(1S)-3-(8-fluoro-2,2,5-trimethyl-4-oxo-5,10-diaza-3-undecan-10-yl)cyclopentyl]amino}-5-(trifluoromethyl)pyrimidin-4-yl)-1H-indole-6-carboxylate (1.01 g, 1.42 mmol) was dissolved in a mixed system of water (5 mL), methanol (5 mL) and tetrahydrofuran (5 mL), then lithium hydroxide (as monohydrate, 0.30 g, 7.09 mmol) was added, and then stirring was carried out at 40°C for 5 h. After realizing complete reaction by LCMS detection, the reaction solution was directly applied to the next reaction without any treatment. LCMS: m/z (ESI): 699.4 [M+H]⁺, t_{R} = 0.93 min.

Step 10: A hydrogen chloride/methanol solution (10 mL, 4 M) was added into the reaction solution in the step 7, and the system was stirred at 40°C for 5 h. After realizing complete reaction by LCMS detection, spin-drying was directly carried out to remove the solvent, and the resulting crude product was purified by reversed-phase prep-HPLC (chromatographic column: Ultimate XB-C18, 50X250 mm, 10 um (PARP-02), mobile phase: acetonitrile-water (0.1% ammonium bicarbonate), mobile phase: 70 mL/min, and column temperature: 25°C) to obtain a white solid 7-[dimethyl(oxo)-λ⁵-phosphoranyl]-3-(2-{[(1S)-3-(4-fluoro-2,7-diazaoct-2-yl)cyclopentyl]amino}-5-(trifluoromethyl)pyrimidin-4-yl)-1H-indole-6-carboxylic acid (530 mg, yield of 62%). LCMS: m/z (ESI): 599.2 [M+H]⁺, t_{R} = 0.73 min.

Step 11: N,N-diisopropylethylamine (108 mg, 0.84 mmol) and 2-(7-azabenzotriazole)-N,N,N',N'-tetramethyluronium hexafluorophosphate (254 mg, 0.67 mmol) were sequentially added into a solution of 7-[dimethyl(oxo)-λ⁵-phosphoranyl]-3-(2-{ [(1S)-3-(4-fluoro-2,7-diazaoct-2-yl)cyclopentyl]amino}-5-(trifluoromethyl)pyrimidin-4-yl)-1H-indole-6-carboxylic acid (100 mg, 0.17 mmol) in N,N-dimethylformamide (8 mL), and the reaction solution was stirred at 60°C for 14 h. After realizing complete reaction by LCMS detection, the reaction solution was concentrated, and reversed-phase preparation was carried out on the resulting crude product (chromatographic column: Ultimate XB-C18, 50X250 mm, 10 um (PARP-02); mobile phase: acetonitrile-water (0.1% trifluoroacetic acid); mobile phase: 70 mL/min; column temperature: 25°C) to obtain (4¹S)-1⁷-(dimethylphosphoryl)-7-fluoro-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z87, 12.22 mg, yield of 12.4 mg, as trifluoroacetate). LCMS: m/z (ESI): 581.3 [M+H]⁺, t_{R} = 0.85 min.¹H NMR (400 MHz, CD₃OD): δ 8.70 - 8.16 (m, 2H), 7.88 (s, 1H), 7.18 (dd, J = 7.6, 2.4 Hz, 1H), 4.34 - 4.08 (m, 1H), 3.96 - 3.80 (m, 1H), 3.35 - 3.25 (m, 1H), 3.14 - 3.05 (m, 2H), 3.04 (s, 3H), 2.96 - 2.81 (m, 1H), 2.71 (s, 3H), 2.48 - 2.36 (m, 1H), 2.30 - 2.12 (m, 3H), 2.06 - 1.46 (m, 11H). (4¹S)-1⁷-(dimethylphosphoryl)-7-fluoro-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z88, 8.24 mg, yield of 8.34 mg, as trifluoroacetate). LCMS: m/z (ESI): 581.2 [M+H]⁺, t_{R} = 0.88 min. ¹H NMR (400 MHz, CD₃OD): δ 8.63 - 8.39 (m, 2H), 7.92 (s, 1H), 7.21 (dd, J = 8.0, 3.2 Hz, 1H), 4.36 - 4.20 (m, 1H), 3.98 - 3.86 (m, 1H), 3.55 - 3.43 (m, 1H), 3.13 - 3.00 (m, 5H), 2.72 - 2.55 (m, 4H), 2.49 - 2.40 (m, 1H), 2.28 - 1.69 (m, 13H), 1.63 - 1.44 (m, 1H).

### Example 36 Synthesis of compounds Z89 and Z90

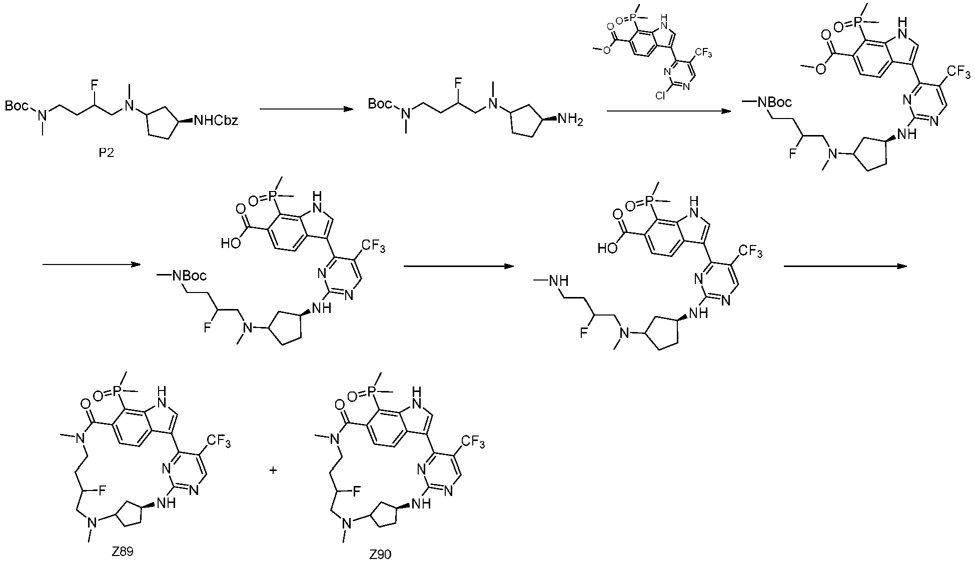

Step 1: {7-[(3S)-3-{[(benzyloxy)carbonyl]amino}cyclopentyl]-5-fluoro-2,7-diazaoct-2-yl}methanoic acid-2-methylpropyl-2-yl ester (P2, 530 mg, 1.17 mmol) was dissolved in methanol (10 mL), and then palladium on carbon (53 mg, 10% loading, 55% of water content) and glacial acetic acid (30 mg, 0.50 mmol) were added into the reaction solution. Stirring was carried out for 14 h at 25°C in a hydrogen atmosphere, after realizing complete reaction by LCMS detection, filtering was carried out to remove solid impurities, and an organic phase was concentrated to obtain a crude product which is a colorless oily substance {7-[(3S)-3-aminocyclopentyl]-5-fluoro-2,7-diazaoct-2-yl}methanoic acid-2-methylpropyl-2-yl ester (380 mg, purity of 90%, yield of 92%), the product was directly used in the next reaction. LCMS: m/z (ESI): 318.3 [M+H]⁺, t_{R} = 1.01 min.

Step 2: {7-[(3S)-3-aminocyclopentyl]-5-fluoro-2,7-diazaoct-2-y}methanoic acid-2-methylpropyl-2-yl ester (380 mg, purity of 90%, 1.08 mmol) was dissolved in N,N-dimethylformamide (8 mL), and N,N-diisopropylethylamine (696 mg, 5.39 mmol) and methyl 3-[2-chloro-5-(trifluoromethyl)pyrimidin-4-yl]-7-[dimethyl(oxo)-λ⁵-phosphoranyl]-1H-indole-6-carboxylate (465 mg, 1.08 mmol) were added into the reaction solution. Stirring was carried out for 5 h at 130°C in a nitrogen atmosphere, after realizing complete reaction by LCMS detection, water (20 mL) was added into the reaction solution, extracting was carried out with ethyl acetate (30 mL X 3), the combined organic layer was washed with saturated brine, dried over anhydrous sodium sulfate, and filtered and concentrated. The crude product was purified with silica gel column chromatography (dichloromethane/methanol = 100/1 to 20/1) to obtain a white solid methyl 7-[dimethyl(oxo)-λ⁵-phosphoranyl]-3-(2-{[(1S)-3-(8-fluoro-2,2,5-trimethyl-4-oxo-5,10-diaza-3-oxaundecan-10-yl)cyclopentyl]amino}-5-(trifluoromethyl)pyrimidin-4-yl)-1H-indole-6-carboxylate (440 mg, yield of 57%). LCMS: m/z (ESI): 713.3 [M+H]⁺, t_{R} = 1.34 min.

Step 3: Methyl 7-[dimethyl(oxo)-λ⁵-phosphoranyl]-3-(2-{[(1S)-3-(8-fluoro-2,2,5-trimethyl-4-oxo-5,10-diaza-3-oxaundecan-10-yl)cyclopentyl]amino}-5-(trifluoromethyl)pyrimidin-4-yl)-1H-indole-6-carboxylate (330 mg, 0.46 mmol) was dissolved in a mixed system of water (3 mL), methanol (3 mL) and tetrahydrofuran (3 mL), lithium hydroxide (as monohydrate, 97 mg, 2.32 mmol) was added, reacting and stirring were carried out for 5 h at 40°C. After realizing complete reaction by LCMS detection, the reaction solution was directly applied to the next reaction without any treatment. LCMS: m/z (ESI): 699.3 [M+H]⁺, t_{R} = 1.22 min. Step 4: A hydrogen chloride/methanol solution (8 mL, 4 M) was added into the reaction solution in the step 3, and the system was stirred at 40°C for 5 h. After realizing complete reaction by LCMS detection, spin-drying was directly carried out to remove the solvent, and the resulting crude product was purified by reversed-phase prep-HPLC (chromatographic column: Ultimate XB-C18, 50X250 mm, 10 um (PARP-02); mobile phase: acetonitrile-water (0.1% ammonium bicarbonate); flow rate: 70 mL/min; column temperature: 25°C) to obtain a white solid 7-[dimethyl(oxo)-λ⁵-phosphoranyl]-3-(2-{[(1S)-3-(4-fluoro-2,7-diazaoct-2-yl)cyclopentyl]amino}-5-(trifluoromethyl)pyrimidin-4-yl)-1H-indole-6-carboxylic acid (190 mg, yield of 74%). LCMS: m/z (ESI): 599.4 [M+H]⁺, t_{R} = 0.72 min.

Step 5: N,N-diisopropylethylamine (172 mg, 1.33 mmol) and 2-(7-azabenzotriazole)-N,N,N',N'-tetramethyluronium hexafluorophosphate (254 mg, 0.67 mmol) were sequentially added into a solution of 7-[dimethyl(oxo)-λ⁵-phosphoranyl]-3-(2-{[(1S)-3-(4-fluoro-2, 7-diazaoct-2-yl)cyclopentyl]amino}-5-(trifluoromethyl)pyrimidin-4-yl)-1H-indole-6-carboxylic acid (100 mg, 0.17 mmol) in N,N-dimethylformamide (8 mL), and the reaction solution was stirred for 14 h at 60°C. After realizing complete reaction by LCMS detection, the reaction solution was concentrated. The resulting crude product was purified by reversed-phase prep-HPLC (chromatographic column: Ultimate XB-C18, 50X250 mm, 10 um (PARP-02); mobile phase: acetonitrile-water (0.1% trifluoroacetic acid); flow rate: 70 mL/min; and column temperature: 25°C) to obtain products (P1 and P2, as trifluoroacetate), the obtained products were respectively dissolved with ethyl acetate, and were sequentially washed with a saturated sodium bicarbonate solution and saturated brine, an organic phase was concentrated and freeze-dried to obtain (4¹S)-1⁷-(dimethylphosphoryl)-7-fluoro-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z89, 17.0 mg, yield of 17.21%). LCMS: m/z (ESI): 581.1 [M+H]⁺, t_{R} = 0.73 min. ¹H NMR (400 MHz, CD₃OD): δ 8.55 (s, 1H), 8.10 - 7.90 (m, 1H), 7.80 (s, 1H), 7.31 - 7.20 (m, 1H), 4.73 - 4.47 (m, 1H), 3.92 - 3.59 (m, 1H), 3.29 - 2.98 (m, 5H), 2.84 (m, 2H), 2.58 - 2.46 (m, 1H), 2.38 - 2.17 (m, 4H), 2.16 - 1.95 (m, 5H), 1.92 - 1.72 (m, 6H), 1.67 - 1.54 (m, 2H). (4¹S)-1⁷-(dimethylphosphoryl)-7-fluoro-5,10-dimethyl-2⁵-(trifluoromethyl)-1¹H-3,5,10-triaza-1(3,6)-indola-2(4,2)-pyrimidina-4(1,3)-cyclopentanacycloundecaphan-11-one (Z90, 15.0 mg, yield of 15.18%). LCMS: m/z (ESI): 581.3 [M+H]⁺, t_{R} = 0.76 min. ¹H NMR (400 MHz, CDCl₃): δ 11.76 (s, 1H), 8.60 - 8.45 (m, 2H), 8.00 - 7.90 (m, 1H), 7.13 (s, 1H), 6.00 - 5.50 (br, 1H), 4.55 - 4.45 (s, 1H), 3.15 - 3.10 (m, 2H), 2.99 - 2.90 (m, 3H), 2.60 - 2.20 (m, 6H), 2.13 - 1.82 (m, 15H).

### Example 37 Synthesis of compound Z91

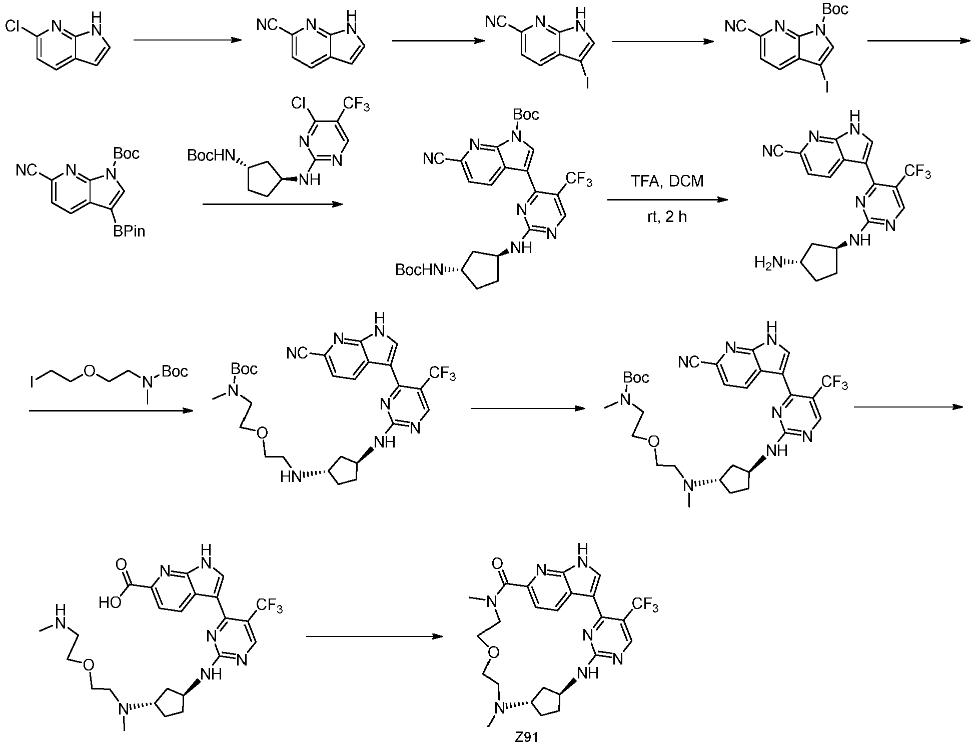

Step 1: 6-chloro-1H-pyrrolo[2,3-b]pyridine (1.0 g, 6.55 mmol), N-methyl pyrrolidone (15 mL), zinc cyanide (0.77 g, 6.55 mmol), tetra(triphenylphosphino)palladium (0.76 g, 0.66 mmol) and zinc powder (0.21 g, 3.28 mmol) were sequentially added into a 25 mL microwave tube, and reacting was carried out for 6 h at 150°C by microwaves. After realizing complete reaction by LCMS detection, water (20 mL) was added into a reaction solution, extracting was carried out with ethyl acetate (30 mL X 3), a combined organic layer was washed with saturated brine, dried over anhydrous sodium sulfate, and filtered and concentrated to obtain a crude product. The crude product was purified with silica gel column chromatography (petroleum ether/ethyl acetate = 1.5/1) to obtain an off-white solid 1H-pyrrolo[2,3-b]pyridine-6-formonitrile (250 mg, yield of 26%). LCMS: m/z (ESI): 144.2 [M+H]⁺, t_{R} = 1.20 min.

Step 2: N-iodosuccinimide (2.26 g, 10.1 mmol) was added into a solution of 1H-pyrrolo[2,3-b]pyridine-6-formonitrile (1.20 g, 8.38 mmol) in N,N-dimethylformamide (25 mL). Stirring was carried out for 2 h at 45°C under nitrogen protection. After realizing complete reaction by LCMS detection, water (50 mL) was added into a reaction solution, extracting was carried out with ethyl acetate (50 mL X 3), a combined organic layer was washed with saturated brine, dried over anhydrous sodium sulfate, filtered and concentrated. The crude product was purified with silica gel column chromatography (petroleum ether/ethyl acetate = 4/1) to obtain a brown solid 3-iodine-1H-pyrrolo[2,3-b]pyridine-6-formonitrile (1.87 g, yield of 83%). LCMS: m/z (ESI): 270.0 [M+H]⁺, t_{R} = 1.49 min.

Step 3: Triethylamine (1.76 g, 17.4 mmol), 4-dimethylaminopyridine (0.17 g, 1.39 mmol) and di-tert-butyl dicarbonate (2.28 g, 10.4 mmol) were added into a solution of 3-iodine-1H-pyrrolo[2,3-b ]pyridine-6-formonitrile (1.87 g, 6.95 mmol) in dichloromethane (25 mL). Stirring was carried out at room temperature for 14 h. After realizing complete reaction by LCMS detection, water (50 mL) was added into the reaction solution, then extracting was carried out with ethyl acetate (50 mL X 3), a combined organic layer was washed with saturated brine, dried over anhydrous sodium sulfate, filtered and concentrated. The crude product was purified with silica gel column chromatography (petroleum ether/ethyl acetate = 10/1) to obtain a yellow solid 6-cyano-3-iodopyrrolo[2,3-b ]pyridine-1-carboxylic acid-2-methylpropyl-2-yl ester (1.90 g, yield of 74%). LCMS: m/z (ESI): 314.0 [M+H-56]⁺, t_{R} = 1.77 min.

Step 4: 6-cyano-3-iodopyrrolo[2,3-b ]pyridine-1-carboxylic acid-2-methylpropyl-2-yl ester (1.90 g, 5.15 mmol), tris(dibenzylideneacetone)dipalladium (470 mg, 0.515 mmol), tetrahydrofuran (20 mL), 2-dicyclohexylphosphorus-2',4',6' -triisopropylbiphenyl (242 mg, 0.515 mmol), triethylamine (1.56 g, 15.4 mmol) and 4,4,5,5-tetramethyl-1,3,2-dioxaborolane (1.98 g, 15.4 mmol) were sequentially added into a sealed tube at room temperature. Stirring was carried out at 90°C under nitrogen protection for 14 h. After realizing complete reaction by LCMS detection, water (20 mL) was added into reaction solution, extracting was carried out with ethyl acetate (20 mL X 3), a combined organic layer was washed with saturated brine, dried over anhydrous sodium sulfate, filtered and concentrated. The crude product was purified with silica gel column chromatography (petroleum ether/ethyl acetate = 5/1) to obtain a white solid 6-cyano-3-(4,4,5,5-tetramethyl-1,3,2-dioxaborolan-2-yl)pyrrolo[2,3-b]pyridine-1-carboxylic acid-2-methylpropan-2-yl ester (1.30 g, yield of 68%). LCMS: m/z (ESI): 314.4 [M+H-56]⁺, t_{R} = 1.61 min.

Step 5: An aqueous solution (3 mL) of {[(1S,3S)-3-{[4-chloro-5-(trifluoromethyl)pyrimidin-2-yl]amino}cyclopentyl]amino}methanoic acid-2-methylpropyl-2-yl ester (1.34 g, 3.52 mmol), chloro[(n-butyldi(1-adamantyl)phosphino)-2-(2-aminobiphenyl)]palladium (II) (0.24 g, 0.352 mmol) and potassium phosphate (2.24 g, 10.6 mmol) were added into a solution of 6-cyano-3 -(4,4, 5, 5-tetramethyl-1,3,2-dioxaborolan-2-yl)pyrrolo [2,3 -b]pyridine-1-carboxylic acid-2-methylpropyl-2-yl ester (1.30 g, 3.52 mmol) in tetrahydrofuran (13 mL)at room temperature. The reaction solution was stirred for 16 h at 60°C under nitrogen protection. After realizing complete reaction by LCMS detection, water (20 mL) was added into the reaction solution, then extracting was carried out with ethyl acetate (20 mL X 3), the combined organic layer was washed with saturated brine, dried over anhydrous sodium sulfate, and filtered and concentrated to obtain a crude product. The crude product was purified with silica gel column chromatography (petroleum ether/ethyl acetate = 2: 1) to obtain a off-white solid 6-cyano-3-(2-{[(1S,3S)-3-({[(2-methylpropyl-2-yl)oxy]carbonyl}amino)cyclopentyl]amino}-5-(trifluoromethyl)pyrimidin-4-yl)pyrrolo[2,3-b]pyridine-1-carboxylic acid-2-methylpropyl-2-yl ester (1.20 g, yield of 58%). LCMS: m/z (ESI): 588.2 [M+H]⁺, t_{R} = 1.88 min.

Step 6: Trifluoroacetic acid (2 mL) was added into a solution of 6-cyano-3-(2-{[(1S,3S)-3-({[(2-methylpropyl-2-yl) oxy ]carbonyl}amino)cyclopentyl]amino}-5-(trifluoromethyl)pyrimidin-4-yl)pyrrolo[2,3-b]pyridine-1-carboxylic acid-2-methylpropyl-2-yl ester (700 mg, 1.20 mmol) in dichloromethane (10 mL), and the reaction solution was reacted at 25°C for 2 h. After realizing complete reaction by LCMS detection, the pH was adjusted to about 8 with a saturated sodium bicarbonate solution (10 mL), extracting was carried out with ethyl acetate (50 mL X 3), the combined organic layer was washed with saturated brine, dried over anhydrous sodium sulfate, and filtered and concentrated to obtain a crude product. The crude product was purified with silica gel column chromatography (water/methanol = 5:1) to obtain a yellow solid 6-cyano-3-(2-{[(1S,3S)-3-aminocyclopentyl]amino}-5-(trifluoromethyl)pyrimidin-4-yl)pyrrolo[2,3-b]pyridine-1-carboxylic acid-2-methylpropan-2-yl ester (450 mg, yield of 98%). LCMS: m/z (ESI): 388.2 [M+H]⁺, t_{R} = 1.18 min.

Step 7: (1-iodine-6-aza-3-oxahept-6-yl) methanoic acid-2-methylpropyl-2-yl ester (255 mg, 0.77 mmol) and N,N-diisopropylethylamine (0.384 mL,2.32 mmol) were sequentially added into 6-cyano-3-(2-{[(1S,3S)-3-aminocyclopentyl]amino}-5-(trifluoromethyl)pyrimidin-4-yl)pyrrolo[2,3-b]pyridine-1-carboxylic acid-2-methylpropan-2-yl ester (300 mg, 0.774 mmol) in N-methylpyrrolidone (5 mL) at room temperature, reacting and stirring were carried out at 80°C for 16 h. After realizing complete reaction by LCMS detection, water (30 mL) was added into the reaction solution, extracting was carried out with ethyl acetate (50 mL X 3), the combined organic layer was washed with saturated brine, dried over anhydrous sodium sulfate, filtered and concentrated. The crude product was purified with silica gel column chromatography (dichloromethane/methanol = 10:1) to obtain a yellow oily substance (1-{[(1S,3S)-3-{[4-(6-cyano-1H-pyrrolo[2,3-b]pyridin-3-yl)-5-(trifluoromethyl)pyrimidin-2-yl]amino}cyclopentyl]amino}-6-aza-3-oxahept-6-yl) methanoic acid-2-methylpropyl-2-yl ester (75 mg, yield of 17%). LCMS: m/z (ESI): 589.4 [M+H]⁺, t_{R} = 1.45 min.

Step 8: Paraformaldehyde (100 mg, 1.11 mmol), acetic acid (0.1 mL) and a 4A molecular sieve (130 mg) were added into a solution of (1-{[(1S,3S)-3-{[4-(6-cyano-1H-pyrrolo[2,3-b]pyridin-3-yl)-5-(trifluoromethyl)pyrimidin-2-yl]amino}cyclopentyl]amino}-6-aza-3-oxahept-6-yl) methanoic acid-2-methylpropyl-2-yl ester (130 mg, 0.221 mmol) methanol (5 mL) at room temperature, and stirring was carried out for 2 h at 25°C under nitrogen protection. Sodium cyanoborohydride (41.7 mg, 0.663 mmol) was added into the reaction solution, and the reaction continued for 14 h. After realizing complete reaction by LCMS detection, filtering and concentrating were directly carried out. The crude product was purified with silica gel column chromatography (dichloromethane/methanol = 10: 1) to obtain a yellow oily substance {8-[(1S,3S)-3-{[4-(6-cyano-1H-pyrrolo[2,3-b]pyridin-3-yl)-5-(trifluoromethyl)pyrimidin-2-yl]amino}cyclopentyl]-2,8-diaza-5-oxanon-2-yl}methanoic acid-2-methylpropyl-2-yl ester (100 mg, yield of 75%). LCMS: m/z (ESI): 603.4 [M+H]⁺, t_{R} = 1.10 min.

Step 9: Concentrated hydrochloric acid (2 mL) was added into a solution of {8-[(1S,3S)-3-{[4-(6-cyano-1H-pyrrolo[2,3-b]pyridin-3-yl)-5-(trifluoromethyl)pyrimidin-2-yl]amino}cyclopentyl]-2,8-diaza-5-oxanon-2-yl}methanoic acid-2-methylpropyl-2-yl ester (100 mg, 0.167 mmol) in acetic acid (4 mL) at room temperature, and the reaction solution was stirred at 120°C for 16 h. After realizing complete reaction by LCMS detection, spin-drying was directly carried out to remove the solvent to obtain a crude product, and the resulting crude product was purified by reversed-phase prep-HPLC (chromatographic column: Ultimate XB-C18-08,25X150 mm, 10 um (PARP-02); mobile phase: acetonitrile-water (0.1% NH₄HCO₃); flow rate: 25 mL/min; column temperature: 25°C) to obtain 3-(2-{[(1S,3S)-3-(2,8-diaza-5-oxanon-2-yl)cyclopentyl]amino}-5-(trifluoromethyl)pyrimidin-4-yl)-1H-pyrrolo[2,3-b]pyridine-6-carboxylic acid (30 mg, yield of 35%). LCMS: m/z (ESI): 522.2 [M+H]+, tR = 1.06 min. LCMS: m/z (ESI): 522.2 [M+H]⁺, t_{R} = 1.06 min.

Step 10: N,N-diisopropylethylamine (0.048 mL, 0.29 mmol) and 2-(7-azabenzotriazole)-N,N,N',N'-tetramethyluronium hexafluorophosphate (54.7 mg, 0.144 mmol) were sequentially added into a solution of 3-(2-{[(1S,3S)-3-(2,8-diaza-5-oxanon-2-yl)cyclopentyl]amino}-5-(trifluoromethyl)pyrimidin-4-yl)-1H-pyrrolo[2,3-b]pyridine-6-carboxylic acid (15 mg, 0.029 mmol) in N,N-dimethylformamide (3 mL), and the reaction solution was stirred at 60°C for 4 h. After realizing complete reaction by LCMS detection, the reaction solution was concentrated, and the resulting crude product was purified by reversed-phase prep-HPLC (chromatographic column: Ultimate XB-C18-08, 25X150 mm, 10 um (PARP-02); mobile phase: acetonitrile-water (0.1% NH₄HCO₃); flow rate: 25 mL/min; column temperature: 25°C) to obtain (4¹S,4³S)-5,11-dimethyl-2⁵-(trifluoromethyl)-1¹H-8-oxa-3,5,11-triaza-1(3,6)-pyrrolo[2,3-b]pyridina-2(4,2)-pyrimidina-4(1,3)-cyclopentanacyclododecaphan-12-one (Z91, 2.5 mg, yield of 17%). LCMS: m/z (ESI): 504.2 [M+H]⁺, t_{R} = 0.77 min. ¹H NMR (400 MHz, CD₃OD): δ 8.66 (d, J = 8.0 Hz, 1H), 8.50 (s, 1H), 7.92 (s, 1H), 7.41 (d, J = 8.0 Hz, 1H), 4.31 - 4.13 (m, 1H), 3.71 - 3.36 (m, 5H), 3.22 (m, 2H), 3.15 (s, 3H), 2.72 - 2.56 (m, 1H), 2.46 - 2.26 (m, 2H), 2.16 (s, 3H), 2.14 - 2.08 (m, 1H), 2.00 (m, 1H), 1.70 - 1.53 (m, 3H).

### Example 38 Synthesis of compound Z92

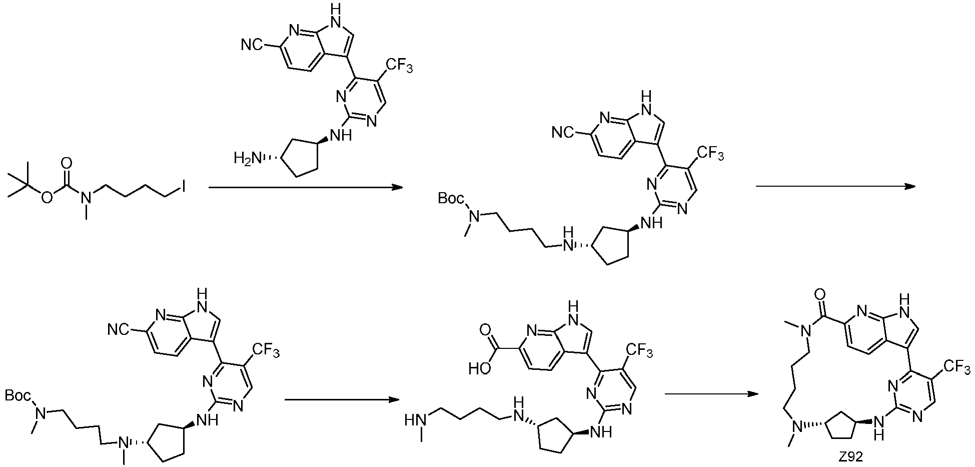

Step 1: [(4-iodobutyl)(methyl)amino]methanoic acid-2-methylpropyl-2-yl ester (178 mg, 0.57 mmol) and N,N-diisopropylethylamine (147 mg, 1.14 mmol) were sequentially added into 3-(2-{[(1S,3S)-3-aminocyclopentyl]amino}-5-(trifluoromethyl)pyrimidin-4-yl)-1H-pyrrolo[2,3-b]pyridine-6-carbonitrile (220 mg, 0.57 mmol) in N-methylpyrrolidone (5 mL). Stirring was carried out at 130°C for 16 h, after realizing complete reaction by LCMS detection, water (30 mL) was added into the reaction solution, extracting was carried out with ethyl acetate (50 mL X 3), the combined organic layer was washed with saturated brine, dried over anhydrous sodium sulfate, and filtered and concentrated to obtain a crude product. The crude product was purified with silica gel column chromatography (dichloromethane/methanol = 10:1) to obtain a yellow oily substance [(4-{[(1S,3S)-3-{[4-(6-cyano-1H-pyrrolo[2,3-b]pyridin-3-yl)-5-(trifluoromethyl)pyrimidin-2-yl]amino}cyclopentyl]amino}butyl)(methyl)amino]methanoic acid-2-methylpropyl-2-yl ester (70.0 mg, yield of 22%). LCMS: m/z (ESI): 573.4 [M+H]⁺, t_{R} = 1.45 min.

Step 2: Paraformaldehyde (94.0 mg, 1.05 mmol), acetic acid (30.0 mg) and a 4A molecular sieve (120 mg) were added into a solution of [(4-{[(1S,3S)-3-{[4-(6-cyano-1H-pyrrolo[2,3-b]pyridin-3-yl)-5-(trifluoromethyl)pyrimidin-2-yl]amino}cyclopentyl]amino}butyl)(methyl)amino]methanoic acid-2-methylpropyl-2-yl ester (120 mg, 0.21 mmol) in methanol (6 mL) at room temperature, reacting and stirring were carried out under nitrogen protection for 2.0 h at 25°C. Sodium cyanoborohydride (40.0 mg, 0.63 mmol) was added into the reaction solution, and the reaction continued for 14 h. After realizing complete reaction by LCMS detection, filtering and concentrating were directly carried out. The crude product was purified with silica gel column chromatography (dichloromethane/methanol = 10:1) to obtain a yellow oily substance {7-[(1S,3S)-3-{[4-(6-cyano-1H-pyrrolo[2,3-b]pyridin-3-yl)-5-(trifluoromethyl)pyrimidin-2-yl]amino}cyclopentyl]-2,7-diazaoct-2-yl}methanoic acid-2-methylpropyl-2-yl ester (120 mg, yield of 98%). LCMS: m/z (ESI): 587.0 [M+H]⁺, t_{R} = 1.13 min.

Step 3: Concentrated hydrochloric acid (2 mL) was added into a solution of {7-[(1S,3S)-3-{[4-(6-cyano-1H-pyrrolo[2,3-b]pyridin-3-yl)-5-(trifluoromethyl)pyrimidin-2-yl]amino}cyclopentyl]-2,7-diazaoct-2-yl}methanoic acid-2-methylpropyl-2-yl ester (110 mg, 0.19 mmol) in acetic acid (4 mL), and stirring was carried out at 120°C for 14 h. After realizing complete reaction by LCMS detection, spin-drying was directly carried out to remove the solvent to obtain a crude product, and the resulting crude product was purified by reversed-phase prep-HPLC (chromatographic column: Ultimate XB-C18-08, 25X150 mm, 10 um (PARP-02), mobile phase: acetonitrile-water (0.1% NH₄HCO₃), flow rate: 25 mL/min, and column temperature: 25°C) to obtain a white solid 3-(2-{[(1S,3S)-3-{[4-(methylamino)butyl]amino}cyclopentyl]amino}-5-(trifluoromethyl)pyrimidin-4-yl)-1H-pyrrolo[2,3-b]pyridine-6-carboxylic acid (54 mg, yield of 57%). LCMS: m/z (ESI): 506.2 [M+H]⁺, t_{R} = 0.96 min.

Step 4: N,N-diisopropylethylamine (51.0 mg, 0.40 mmol) and 2-(7-azabenzotriazole)-N,N,N',N'-tetramethyluronium hexafluorophosphate (36.0 mg, 0.10 mmol) were sequentially added into a solution of 3-(2-{[(1S,3S)-3-{[4-(methylamino)butyl]amino}cyclopentyl]amino}-5-(trifluoromethyl)pyrimidin-4-yl)-1H-pyrrolo[2,3-b]pyridine-6-carboxylic acid (40.0 mg, 0.08 mmol) in N,N-dimethylformamide (5 mL), and the reaction solution was stirred at 60°C for 16 h. After realizing complete reaction by LCMS detection, the reaction solution was concentrated, and the resulting crude product was purified by reversed-phase prep-HPLC (chromatographic column: Ultimate XB-C18-08, 25X150 mm, 10 um (PARP-02), mobile phase: acetonitrile-water (0.1% NH₄HCO₃), flow rate: 25 mL/min, and column temperature: 25°C) to obtain (4¹S,4³S)-5,10-dimethyl-2⁵-(trifluoromethyl)-1¹H-3,5,10-triaza-1(3,6)-pyrrolo[2,3-b]pyridina-2(4,2)-pyrimidina-4(1,3)-cyclopentanacycloundecaphan-11-one (Z92, 4.70 mg, yield of 12%). LCMS: m/z (ESI): 488.2 [M+H]⁺, t_{R} = 0.77 min. ¹H NMR (400 MHz, CD₃OD): δ 8.77 (d, J = 8.0 Hz, 1H), 8.56 (s, 1H), 8.01 (s, 1H), 7.44 (d, J = 8.0 Hz, 1H), 4.26 - 4.07 (m, 1H), 3.33 - 3.27 (m, 1H), 3.17 (s, 3H), 3.14 - 2.98 (m, 2H), 2.55 - 2.27 (m, 2H), 2.25 - 2.10 (m, 4H), 2.09 - 1.84 (m, 3H), 1.80 - 1.63 (m, 3H), 1.54 - 1.43 (m, 1H), 1.42 - 1.27 (m, 2H).

### Example 39 Synthesis of compound Z93

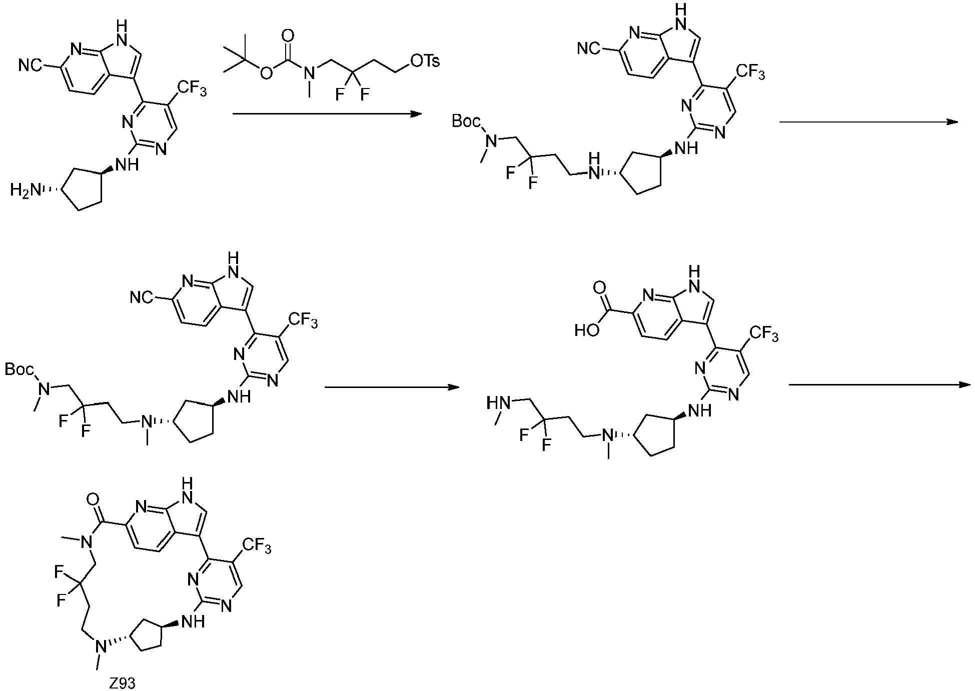

Step 1: 4-methylbenzenesulfonic acid-7,7-difluoro-2,2,5-trimethyl-4-oxo-5-aza-3-oxanon-9-yl ester (2.44 g, 6.20 mmol), sodium iodide (0.15 g, 1.03 mmol) and N,N-diisopropylethylamine (2.56 mL, 15.49 mmol) were sequentially added into a solution of 6-cyano-3-(2-{[(1S,3S)-3-aminocyclopentyl]amino}-5-(trifluoromethyl)pyrimidin-4-yl)pyrrolo[2,3-b]pyridine-1-carboxylic acid-2-methylpropan-2-yl ester (2.0 g, 5.16 mmol) in N-methylpyrrolidone (20 mL) at room temperature, and the reaction was carried out at 80°C for 16 h while stirring. After realizing complete reaction by LCMS detection, water (50 mL) was added into the reaction solution, extracting was carried out with ethyl acetate (100 mL X 3), the combined organic layer was washed with saturated brine, dried over anhydrous sodium sulfate, filtered and concentrated, and the resulting crude product was purified with column chromatography (dichloromethane/methanol = 10: 1) to obtain a yellow oily substance [(2,2-difluoro-4-{[(1S,3S)-3-{[4-(6-cyano-1H-pyrrolo[2,3-b]pyridin-3-yl)-5-(trifluoromethyl)pyrimidin-2-yl]aminocyclopentyl]amino}butyl)(methyl)amino]methyl-2-methylpropan-2-yl ester (480 mg, yield of 15%). LCMS: m/z (ESI): 609.5 [M+H]⁺, t_{R} = 1.33 min.

Step 2: Paraformaldehyde (118 mg, 3.95 mmol), acetic acid (0.1 mL) and a 4A molecular sieve (480 mg) were added into a solution of [(2,2-difluoro-4-{[(1S,3S)-3-{[4-(6-cyano-1H-pyrrolo[2,3-b]pyridin-3-yl)-5-(trifluoromethyl)pyrimidin-2-yl]aminocyclopentyl]amino}butyl)(methyl)amino]methyl-2-methylpropan-2-yl ester (480 mg, 0.789 mmol) in methanol (5 mL) at room temperature, reacting and stirring were carried out under nitrogen protection for 2.0 h at 25°C. Sodium cyanoborohydride (149 mg, 2.37 mmol) was added into the reaction solution, and the reaction continued for 14 h. After realizing complete reaction by LCMS detection, filtering and concentrating were directly carried out. The crude product was purified with silica gel column chromatography (dichloromethane/methanol = 10:1) to obtain a yellow oily substance {7-[(1S,3S)-3-{[4-(6-cyano-1H-pyrrolo[2,3-b]pyridin-3-yl)-5-(trifluoromethyl)pyrimidin-2-yl]amino}cyclopentyl]-4, 4-difluoro-2, 7-diazaoct-2-yl}methanoic acid-2-methylpropyl-2-yl ester (330 mg, yield of 67%). LCMS: m/z (ESI): 623.3 [M+H]⁺, t_{R} = 1.12 min.

Step 3: Concentrated hydrochloric acid (1 mL) was added into a solution of {7-[(1S,3S)-3-{[4-(6-cyano-1H-pyrrolo[2,3-b]pyridin-3-yl)-5-(trifluoromethyl)pyrimidin-2-yl]amino}cyclopentyl]-4, 4-difluoro-2,7-diazaoct-2-yl}methanoic acid-2-methylpropyl-2-yl ester (330 mg, 0.53 mmol) in acetic acid (2 mL), and the reaction solution was stirred at 120°C for 5 h. After realizing complete reaction by LCMS detection, spin-drying was directly carried out to remove the solvent to obtain a crude product, and the resulting crude product was purified by reversed-phase prep-HPLC (chromatographic column: Ultimate XB-C18-08,25X150 mm, 10 um (PARP-02), mobile phase: acetonitrile-water (0.1% NH₄HCO₃), flow rate: 25 mL/min, and column temperature: 25°C) to obtain a white solid 3-(2-{[(1S,3S)-3-(5,5-difluoro-2,7-diaza-2-yl)cyclopentyl]amino}-5-(trifluoromethyl)pyrimidin-4-yl)-1H-pyrrolo[2,3-b]pyridine-6-carboxylic acid (220 mg, yield of 76%). LCMS: m/z (ESI): 542.2 [M+H]⁺, t_{R} = 1.05 min.

Step 4: N,N-diisopropylethylamine (0.15 mL, 0.92 mmol) and 2-(7-azabenzotriazole)-N,N,N',N'-tetramethyluronium hexafluorophosphate (280 mg, 0.74 mmol) were sequentially added into a solution of 3-(2-{ [(1S,3S)-3-(5,5-difluoro-2,7-diazaoct-2-yl)cyclopentyl]amino}-5-(trifluoromethyl)pyrimidin-4-yl)-1H-pyrrolo[2,3-b]pyridine-6-carboxylic acid (100 mg, 0.19 mmol) in N,N-dimethylformamide (2 mL), and the reaction solution was stirred at 60°C for 16 h. The reaction solution was concentrated, and the resulting crude product was purified by reversed-phase prep-HPLC (chromatographic column: Ultimate XB-C18-08,25X150 mm, 10 um (PARP-02), mobile phase: acetonitrile-water (0.1% NH₄HCO₃), flow rate: 25 mL/min, and column temperature: 25°C) to obtain (4¹S,4³S)-8,8-difluoro-5,10-dimethyl-2⁵-(trifluoromethyl)-1¹H-3,5,10-triaza-1(3,6)-pyrrolo[2,3-b]pyridine-2(4,2)-pyrimidina-4(1,3)-cyclopentanacycloundecaphan-11-one (Z93, 2.50 mg, yield of 2.6%). LCMS: m/z (ESI): 524.4 [M+H]⁺, t_{R} = 1.10 min. ¹H NMR (400 MHz, CD₃OD): δ 9.06 - 8.61 (m, 1H), 8.56 - 8.23 (m, 1H), 8.20 - 7.91 (m, 1H), 7.71 - 7.32 (m, 1H), 4.77 - 4.18 (m, 2H), 4.13 - 3.38 (m, 2H), 3.29 - 3.17 (m, 3H), 3.15 - 2.38 (m, 3H), 2.34 - 2.15 (m, 3H), 2.13 - 1.82 (m, 5H), 1.79 - 1.48 (m, 2H).

The compounds shown in the following table can be prepared according to the synthesis method in the above examples or according to a method in the existing literature.

**Table A**

| No. | Structure | MS [M+H]⁺ | No. | Structure | MS [M+H]⁺ |
|---|---|---|---|---|---|
| Z17 | 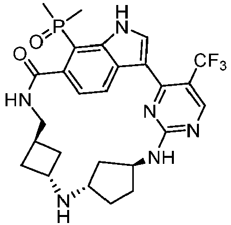 | 547.2 | Z18 | 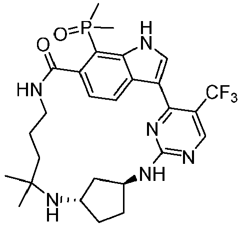 | 563.2 |
| Z20 | 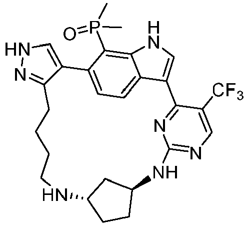 | 558.2 | Z21 | 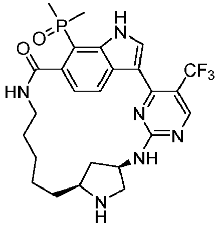 | 535.2 |
| Z22 | 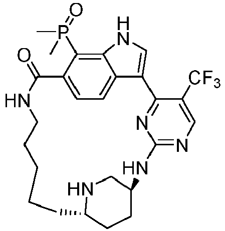 | 549.2 | Z24 | 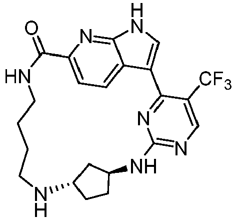 | 460.2 |
| Z25 | 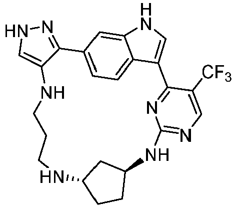 | 483.2 | Z26 | 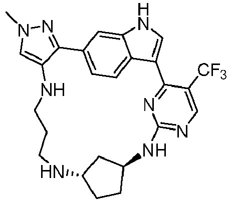 | 497.2 |
| Z28 | 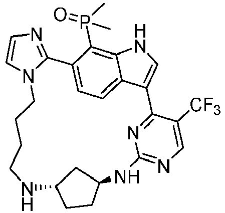 | 558.2 | Z29 | 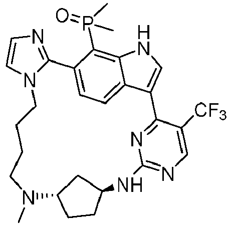 | 572.2 |
| Z30 | 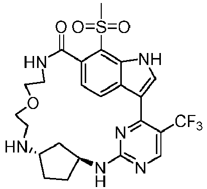 | 553.2 | Z31 | 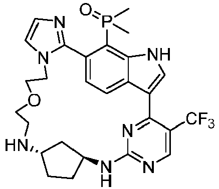 | 574.2 |
| Z32 | 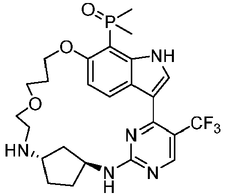 | 538.2 | Z33 | 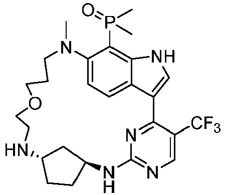 | 551.2 |
| Z34 | 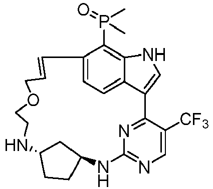 | 520.2 | Z35 | 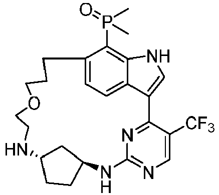 | 522.2 |
| Z36 | 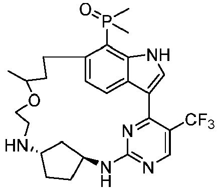 | 536.2 | Z37 | 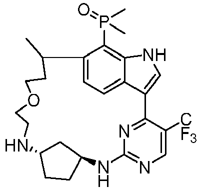 | 536.2 |
| Z38 | 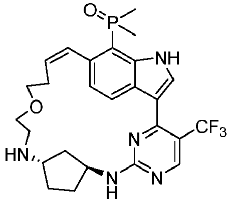 | 534.2 | Z39 | 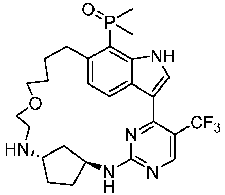 | 536.2 |
| Z40 | 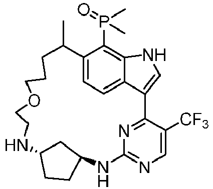 | 550.3 | Z41 | 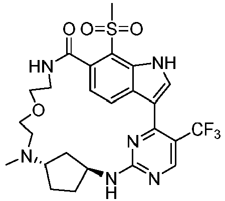 | 567.2 |
| Z42 | 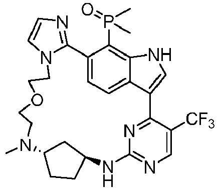 | 588.2 | Z43 | 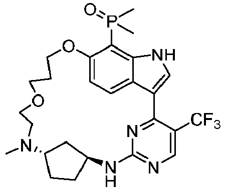 | 552.2 |
| Z44 | 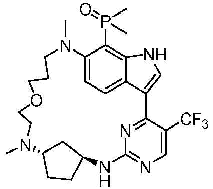 | 565.3 | Z45 | 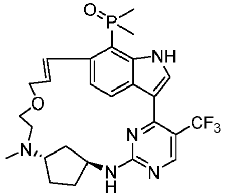 | 534.2 |
| Z46 | 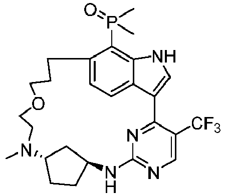 | 536.2 | Z47 | 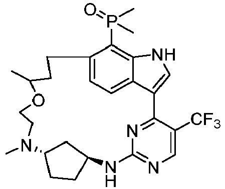 | 550.3 |
| Z48 | 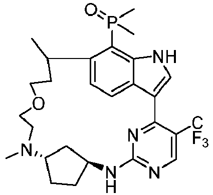 | 550.3 | Z49 | 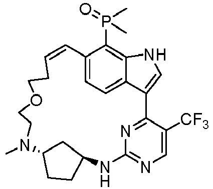 | 548.2 |
| Z50 | 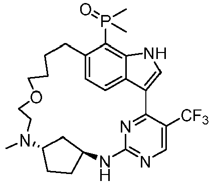 | 550.3 | Z51 | 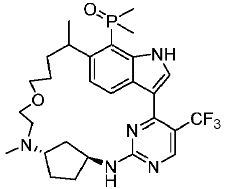 | 564.3 |
| Z52 | 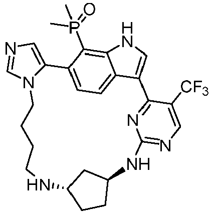 | 558.2 | Z53 | 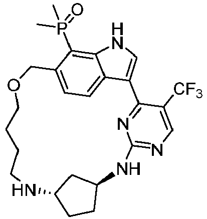 | 522.2 |
| Z54 | 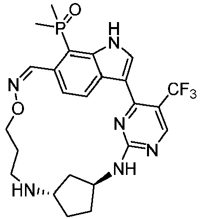 | 521.2 | Z55 | 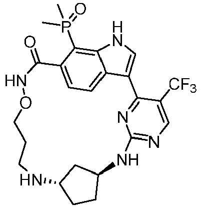 | 537.2 |
| Z57 | 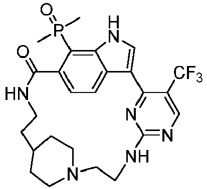 | 535.2 | Z67 | 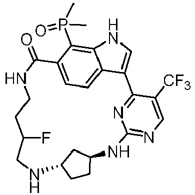 | 553.2 |
| Z69 | 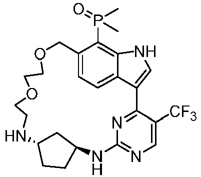 | 538.2 | Z70 | 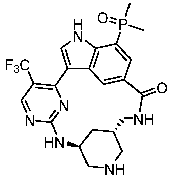 | 493.2 |
| Z71 | 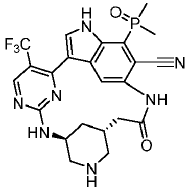 | 518.2 | Z73 | 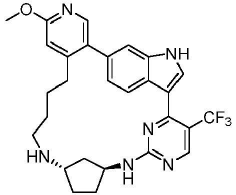 | 523.2 |
| Z74 | 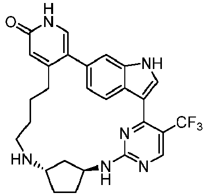 | 509.2 | Z80 | 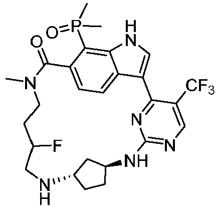 | 567.2 |
| Z81 | 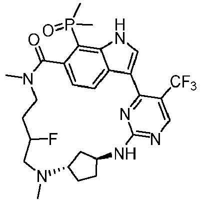 | 581.2 | Z82 | 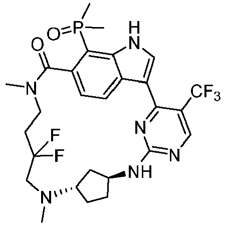 | 599.2 |
| Z83 | 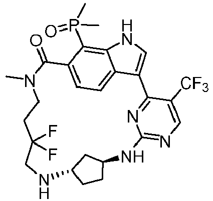 | 585.2 | | | |

### Test example 1: In-vitro inhibition of CDK kinase activity

CDK2/Cyclin E1 was purchased from Biortus; batch No.: 20150228-BP469/477/691; CDK7/Cyclin H/MAT1 was purchased from Biortus; Batch No.: 20190326-BP487/492/479; CDK9/Cyclin T1 was purchased from Biortus; Batch No.: 20200727-BP488/792/691; CDK12/Cyclin K was purchased from Biortus; Batch No.: 20200526-BP1642/1648/691; MES was purchased from BiRoYee (Baoruyi); Batch No.: 67GR9637; BSA was purchased from Aladdin; Batch No.: H1601024; ATP was purchased from VWR; Batch No.: 97061-226; EDTA was purchased from Sinopharm Group; Batch No.: 20200521; and staurosporine was purchased from Selleckchem; Batch No.: S1421.

This experiment was used for determining the inhibition of the activities of CDK2, CDK7, CDK9 and CDK12 kinases by the compound. The kinase reaction carried out by the present invention was measured in a 384-well plate, the final measured volume was 16µl, and the reaction temperature was 27°C. The concentrations of the kinases were determined by optimization experiment. The specific experimental process was as follows:
1) preparation of kinase solutions:
   kinase solution (CDK2/Cyclin E1): the kinase was diluted in a assay buffer solution ((20 mM MES pH 6.75, 0.01% Tween 20, 0.05 mg/mL BSA, 2 mM MgCl₂) to obtain an enzyme solution with the corresponding 2.4 X concentration;
   kinase solution (CDK7/Cyclin H/MAT1): the kinase was diluted in the assay buffer solution (20 mM MES pH 6.75, 0.01% Tween 20, 0.05 mg/mL BSA, 6 mM MgCl₂) to obtain an enzyme solution with the corresponding 2.4 X concentration;
   kinase solution (CDK9/Cyclin T1): the kinase was diluted in the assay buffer solution (20 mM MES pH 6.75, 0.01% Tween 20, 0.05 mg/mL BSA, 10 mM MgCl₂) to obtain an enzyme solution with the corresponding 2.4 X concentration; and
   kinase solution (CDK12/Cyclin K): the kinase was diluted in a assay buffer solution (80 mM MES pH 6.5, 0.01% Tween 20, 0.05 mg/mL BSA, 10 mM MgCl₂) to obtain an enzyme solution with the corresponding 2.4 X concentration.
2) Preparation of compound solutions: the compound was dissolved in dimethyl sulfoxide (DMSO) at a concentration of 10 mM, the compound was diluted to 10 concentration gradients of 25 nM to 500 µM with DMSO during use, diluted in ultrapure water by 8.3 times respectively to obtain compound solutions with 6 X concentration.
3) Preparation of a polypeptide substrate and ATP solution: the polypeptide substrate and ATP were diluted in the assay buffer solution to obtain a polypeptide substrate and ATP mixed solution with 2.4 X concentration.
4) Kinase reaction process:
   2 ul of the test compound solution, 5 ul of the polypeptide substrate and ATP mixed solution and 5 ul of an enzyme solution were mixed and incubated at 27°C (CDK2 was incubated for 60 min, CDK7 was incubated for 70 min, CDK9 was incubated for 70 min, and CDK12 was incubated for 280 min), and then 4 ul of EDTA with a concentration of 150 mM was added into each sample to terminate the reaction. The assay buffer solution containing 20 µM of staurosporine was used for replacing the compound solution as 100% inhibition, and DMSO was used for replacing the compound solution as 0% inhibition. Each test had at least two parallel controls.

Final concentration of a reagent in CDK2 assay: ATP was 100µM, the polypeptide substrate (5-FAM-YSPTSPSYSPTSPSYSPT SPSKKKK) was 2µM, and CDK2/Cyclin E1 was 0.5 nM;
final concentration of a reagent in CDK7 assay: ATP was 50 µM, the polypeptide substrate (5-FAM-YSPTSPSYSPTSPSYSPT SPSKKKK) was 2 µM, and CDK7/Cyclin H/MAT1 was 3 nM;
final concentration of a reagent in CDK9 assay: ATP was 50 µM, the polypeptide substrate (FITC-Ahx-GSRTPMY-NH2) was 2 µM, and CDK9/Cyclin T1 was 8 nM; and final concentration of a reagent in CDK12 assay: ATP was 30 µM, the polypeptide substrate (FITC-Ahx-GSRTPMY-NH2) was 2 µM, and CDK12/Cyclin K was 50 nM. 5) Data calculation and analysis: electrophoretic separation was carried out on a fluorescent substrate and a phosphorylated product on a Caliper EZ Reader II to analyze a reaction mixture. The data was calculated with GraphPad Prism version 9.0, and an IC₅₀ value was obtained by adjusting a nonlinear regression model using a dose reaction curve. The calculation formula was Y=Bottom + (Top-Bottom)/(1+10^((LogIC50-X)*HillSlope)). X represents a log value (log of dose or concentration); Y represents an inhibition rate (%inhibition, increasing as x increases) which was increased along with the increase of X; Top represents maximum response; Bottom represents baseline response; and HillSlope represents a curve slope. Due to the limitation of the detection lower limit of a CDK7/Cyclin H/MAT1 kinase activity experiment, the inhibition of the compound in the present invention on the CDK7 kinase activity reached the experiment detection lower limit, IC₅₀ could not accurately reflect the kinase activity inhibition capability of the compound, and the binding affinity K_{D} of the compound was detected by adopting a surface plasmon resonance (SPR) method. The compound had an inhibition effect on the activity of CDK2, CDK9 and CDK12 kinases, and IC₅₀ obtained by detection fitting under a Km ATP condition was used for calculating Ki (Cheng and Prusoff, Biochem. Pharmacol.,22(23)3099-3108, 1973), and the conversion formula was as follows: IC₅₀ = Kᵢ (1+ [substrate/Kₘ]) + [enzyme/2]. The results of the compounds are shown in Table 1.

**Table 1**

| No. | CDK7/CyclinH/MAT1 IC50 assay (nM) | CDK2 Ki | CDK9 Ki | CDK12 Ki |
|---|---|---|---|---|
| Z1 | 11.0 | 153.7 | 52.25 | 62.65 |
| Z2 | 4.58 | 9.8 | 10.6 | 22.74 |
| Z5 | - | 25.65 | 95.55 | 26.06 |
| Z7 | - | 33.4 | 216.8 | 61.35 |
| Z65 | - | 30.33 | 257.7 | 61.6 |
| Z79 | - | 23.29 | 134.25 | 92.8 |

### Test example 2: CDK7/Cyclin H surface plasmon resonance (SPR) assay method

CDK7/Cyclin H was purchased from Biortus; Batch No.: 20200309-BP487/492; MES buffer solution was purchased from BioRoYee; Batch No.: 67GR9637; CM5 sensor chip was purchased from Cytiva; Batch No.: 10305527; and HEPES buffer solution was purchased from Cytiva; Batch No.: 32349.

The experiment adopted a Biacare S200 surface plasmon resonance device (GE Healthcare) to test the dynamics and affinity parameters of a CDK7/Cyclin H dimer and the compounds. Under a condition of 10 mM MES buffer solution with pH of 6.5, CDK7/Cyclin H dimer with concentration of 50 µg/mL was coupled to a CM5 sensing chip by amino group at flow rate of 5 µL/min. Target protein was fixed on a chip channel within 600 seconds, and 7,000-10,000 response value was achieved in general. The compound underwent 2 times of gradient dilution for 5 steps in 10 mM HEPES buffer solution with pH of 7.4 and comprising 150 mM NaCl, 0.05% of surfactant P20 and 2% of DMSO to reach a concentration range of 0.6-10 nM. Each concentration cycle of the compound was operated at 100µL/min, 180 seconds of contact time and 1800 seconds of dissociation time. For each compound, the combination of a 0 nM compound control and a reference channel was deducted to remove background signals and normalization data. Biaccore S200 evaluation software and a kinetic model were used for carrying out titration overall fitting on the compound. Fitting data was optimized, the combination rate and the dissociation rate parameter of CDK7/Cyclin H were determined, and the affinity parameter K_{D} of the compound was calculated by the following equation. K_{D} (M) = k_{off} (s⁻¹) / Kₒₙ (M⁻¹s⁻¹), wherein Kₒₙ (ka) represents the combination rate, K_{off} (kd) represents the dissociation rate, and s⁻¹ (per second) and M⁻¹s⁻¹ (per mole per second) were the units of k_{off} and kₒₙ. Based on the ratio of the Kᵢ value of off-target CDK to the direct compound combination K_{D} of CDK7 (measured by SPR), the selectivity of CDK7 to the compound of CDK2, CDK9 or CDK12 was determined according to the following equation. Selectivity = K_{i, off-target}/K_{D, CDK7}. The result showed that the compound had excellent selectivity to CDK7 relative to CDK2/CDK9/CDK12, and the specificity of the compound to CDK7 was at least 100 times or 300 times and even more than 500 times to other CDK7. The results of a part of the compounds are shown in Table 2.

**Table 2**

| No. | Selectivity of CDK7 to CDK2, CDK9 and CDK12 | |
|---|---|---|
| Z2 | CDK7/Cyclin H SPR K_{D} assay=0.14 | |
| | CDK2 Kᵢ: 9.8 | (CDK2 Kᵢ/CDK7 K_{D}): 70 |
| | CDK9 Kᵢ: 10.6 | (CDK9 Kᵢ/CDK7 K_{D}): 76 |
| | CDK12 Kᵢ: 22.74 | (CDK12 Kᵢ/CDK7 K_{D}): 162 |
| Z5 | CDK7/Cyclin H SPR KD assay=0.16 | |
| | CDK2 Kᵢ: 25.65 | (CDK2 Kᵢ/CDK7 K_{D}): 160 |
| | CDK9 Kᵢ: 95.55 | (CDK9 Kᵢ/CDK7 K_{D}): 597 |
| | CDK12 Kᵢ: 26.06 | (CDK12 Kᵢ/CDK7 K_{D}): 163 |
| Z7 | CDK7/Cyclin H SPR KD assay=0.059 | |
| | CDK2 Kᵢ: 33.4 | (CDK2 Kᵢ/CDK7 K_{D}): 566 |
| | CDK9 Kᵢ: 216.8 | (CDK9 Kᵢ/CDK7 K_{D}): 3675 |
| | CDK12 Kᵢ: 61.35 | (CDK12 Kᵢ/CDK7 K_{D}): 61.35 |
| Z65 | CDK7/Cyclin H SPR KD assay=0.18 | |
| | CDK2 Kᵢ: 30.33 | (CDK2 Kᵢ/CDK7 K_{D}): 165 |
| | CDK9 Kᵢ: 257.7 | (CDK9 Kᵢ/CDK7 K_{D}): 1400 |
| | CDK12 Kᵢ: 61.6 | (CDK12 Kᵢ/CDK7 K_{D}): 335 |
| Z79 | CDK7/Cyclin H SPR KD assay=0.12 | |
| | CDK2 Kᵢ: 23.29 | (CDK2 Kᵢ/CDK7 K_{D}): 192 |
| | CDK9 Kᵢ: 134.25 | (CDK9 Kᵢ/CDK7 K_{D}): 1103 |
| | CDK12 Kᵢ: 92.8 | (CDK12 Kᵢ/CDK7 K_{D}): 763 |

### Test example 3: HCC70 tumor cell proliferation inhibition activity experiment

HCC70 was purchased from ATCC; article No.: CRL-2315; RPMI1640 was purchased from Gibco; article No.: 11875-093; Pancreatin (including EDTA) was purchased from Gibco; article No.: 25200-072; FBS was purchased from Gibco; article No.: 10099-141C; CellTiter-Glo was purchased from Promega; article No.: G7573; DMSO was purchased from VWR AMRESCO; article No.: 0231-500ML; Staurosporine was purchased from Selleck; article No.: S1421; a cell counter was purchased from CHEMOMETEC; model: NC-200; and a microplate reader was purchased from PerkinElmer; Model: Envison.

HCC70 was human breast ductal cancer cells cultured in an RPMI-1640 culture medium containing 10% FBS. Cells in logarithmic phase were taken, and the cells were subjected to digesting, collecting and counting with pancreatin-EDTA, 2000 HCC70 cells/well were inoculated in a 384-well cell plate, and were cultured in 5% CO₂ overnight. A 1000X compound stock solution with 3.16 times of gradient concentration was prepared with DMSO, and was diluted 100 times to 10X compound stock solution with a culture medium, and 10X compound stock solution was added into each cell culture well on the next day after cell inoculation, wherein the final concentration was 1X, and the DMSO content was 0.1%. The cell group was treated with DMSO as an experimental control (control), and the cell group was treated with 5 µM of Staurosporine as a blank control (blank). After the compound was added and the cells were continuously cultured for 3 d, 25 µl of CellTiter-Glo working solution was added into each well and uniformly mixed and then incubated at room temperature for 5 min, then the luminescence chemiluminescence value was read, the cell proliferation inhibition ratio was calculated as IR (%) = (RLU _{control}-RLU _{compound})/(RLU _{control}-RLU _{blank}) X 100%; and the compound gradient dilution concentration and the corresponding cell proliferation inhibition ratio were fit by an XLFit four-parameter method, and the IC₅₀ value was calculated. The result indicated that the compound of the present invention had strong inhibitory action on HCC70 human breast ductal cancer cell proliferation. The IC₅₀ of part of compounds was lower than 1,000 nM or 500 nM, and even lower than 100 nM or 50 nM. The results of a part of exemplary compounds are shown in the following Table 3.

**Table 3**

| No. | HCC70 IC₅₀ (nM) | No. | HCC70 IC₅₀ (nM) |
|---|---|---|---|
| Z2 | 207.0 | Z16 | 23.0 |
| Z5 | 42.7 | Z19 | 15.4 |
| Z6 | 222.4 | Z27 | 9.7 |
| Z7 | 41.1 | Z59 | 422 |
| Z9 | 99.77 | Z60 | 89 |
| Z10 | 161.53 | Z58 | 233 |
| Z11 | 186 | Z61 | 389.7 |
| Z23 | 66.60 | Z63 | 134.23 |
| Z64 | 42.78 | Z62 | 24.64 |
| Z68-1 | 10.35 | Z68-2 | 3.18 |
| Z66 | 10.51 | Z65 | 46.93 |
| Z72 | 869.32 | Z73 | 233.42 |
| Z74 | 1250 | Z78 | 43.97 |
| Z79 | 66.5 | Z76 | 21.91 |
| Z77 | 9.87 | Z84 | 263.1 |
| Z85 | 517.14 | Z86 | >10000 |
| Z89 | >10000 | Z90 | >10000 |
| Z87 | 242.9 | Z88 | 241.1 |
| Z91 | 438.5 | Z92 | 106.76 |
| Z93 | 5211.0 | | |

### Test example 4: OVCAR-3 tumor cell proliferation inhibition activity experiment

OVCAR-3 was purchased from ATCC; article No.: HTB-161; RPMI1640 was purchased from Gibco; article No.: 11875-093; Pancreatin (including EDTA) was purchased from Gibco; article No.: 25200-072; FBS was purchased from Gibco; Article No.: 10091-148; CellTiter-Glo was purchased from Promega; article No.: G7573; Bovine insulin was purchased from absin; article No.: abs9169; DMSO was purchased from VWR AMRESCO; article No.: 0231-500ML; Staurosporine was purchased from Selleck; article No.: S1421; a cell counter was purchased from CHEMOMETEC; model: NC-200; and a microplate reader was purchased from PerkinElmer; Model: Envison.

OVCAR-3 was human ovarian adenocarcinoma cells cultured in an RPMI-1640 culture medium containing 20% FBS and 0.01 mg/mL of Bovine insulin. Cells in logarithmic phase were taken, and the cells were subjected to digesting, collecting and counting with pancreatin-EDTA, 2000 OVCAR-3 cells/well were inoculated in a 384-well cell plate, and were cultured in 5% CO₂ overnight. A 1000X compound stock solution with 3.16 times of gradient concentration was prepared with DMSO, and was diluted 100 times to 10X compound stock solution with a culture medium, and 10X compound stock solution was added into each cell culture well on the next day after cell inoculation, wherein the final concentration was 1X, and the DMSO content was 0.1%. The cell group was treated with DMSO as an experimental control (control), and the cell group was treated with 5 µM of Staurosporine as a blank control (blank). After the compound was added and the cells were continuously cultured for 3 d, 25 µl of CellTiter-Glo working solution was added into each well and uniformly mixed and then incubated at room temperature for 5 min, then the luminescence chemiluminescence value was read, the cell proliferation inhibition ratio was calculated as IR (%) = (RLU _{control}-RLU _{compound})/(RLU _{control}-RLU _{blank}) X 100%; and the compound gradient dilution concentration and the corresponding cell proliferation inhibition ratio were fit by an XLFit four-parameter method, and the IC₅₀ value was calculated. The result showed that the compound had a strong inhibition effect on OVCAR-3 for human ovarian adenocarcinoma cell proliferation. The IC₅₀ of part of compounds was lower than 1,000 nM or 500 nM, and even lower than 100 nM or 50 nM. The results of a part of exemplary compounds are shown in the following Table 4.

**Table 4**

| No. | OVCAR-3 IC₅₀ (nM) | No. | OVCAR-3 IC₅₀ (nM) |
|---|---|---|---|
| Z23 | 134.85 | Z63 | 195.06 |
| Z64 | 98.6 | Z62 | 15.91 |
| Z68-1 | 35.8 | Z68-2 | 8.84 |
| Z66 | 21.24 | Z65 | 253.96 |
| Z73 | 1054 | Z74 | 3457 |
| Z78 | 62.6 | Z79 | 84.05 |
| Z76 | 31.5 | Z77 | 11.8 |
| Z84 | 198.2 | Z85 | 1802.3 |
| Z86 | >10000 | Z89 | >10000 |
| Z90 | >10000 | Z91 | 810.54 |
| Z92 | 342.9 | Z93 | >10000 |

### Test example 5: In-vivo pharmacokinetic experiment of compound of the present invention

Experimental method and conditions: male beagles with weight of 8-11 kg were purchased from Jiangsu Marshall Biotechnology Co., Ltd. The to-be-tested compound was administered to 3 dogs at 0.2 mg/kg or 1.0 mg/kg (solvent: 2% DMSO + 10% Kolliphor HS 15 + 88% (6.818% of hydroxypropyl-beta-cyclodextrin aqueous solution), 0.2 mg/mL or 1.0 mg/mL) by intravenous injection, and administered to 3 dogs at 2 mg/kg (solvent: 5% DMSO + 95% (10% hydroxypropyl-beta-cyclodextrin salt aqueous solution, 0.4 mg/mL) by oral intragastric administration. Head vein blood sampling was carried out 5 min, 15 min, 30 min, 1 h, 2 h, 4 h, 8 h and 24 h after the administration, and 500 µL of each sample was collected. After collection, the samples were placed on ice, and plasma was centrifugally separated within 15 min for testing. The concentration of the medicine in the plasma was detected by liquid chromatography-tandem mass spectrometry (LC/MS/MS), and pharmacokinetic parameters were calculated with Phoenix WinNonlin software when the concentration was measured. The experimental results are shown in Table 5 and Table 6.

**Table 5 In-vivo pharmacokinetics of the compound of the present invention by oral administration (2 mg/kg).**

| No. | T_{1/2} (h) | Cₘₐₓ (ng/mL) | AUC_{0∼inf} (ng*h/mL) | F(%) |
|---|---|---|---|---|
| Z65 | 4.44 | 6623 | 45620 | 147 |
| Compound 101 | 6.61 | 123 | 1591 | 65.8 |

**Table 6 In-vivo pharmacokinetics of the compound of the present invention by intravenous injection**

| No. | Dose (mg/kg) | T_{1/2} (h) | AUC_{0∼inf} (ng*h/mL) | Vₛₛ (L/kg) | CL (mL/min/kg) |
|---|---|---|---|---|---|
| Z65 | 0.2 | 3.42 | 3097 | 0.24 | 1.23 |
| Compound 101 | 1.0 | 6.88 | 1228 | 7.57 | 14.5 |

Therefore, compared with the prior art (Compound 101, WO2020093006, 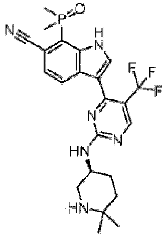 ), the compound provided by the present invention has significantly lower metabolic clearance (CL) and higher plasma exposure (AUC_{0-inf} ) in pharmacokinetics.

Although the specific embodiments of the present invention have been described in detail, according to all the teachings already disclosed, those skilled in the art can make various modifications and replacements to the details of the technical solution of the present invention, and these changes are within the protection scope of the present invention. The full scope of the present invention is given by the appended claims and any equivalents thereof.

## Claims

1. A compound shown in Formula (A) or a pharmaceutically acceptable salt thereof, wherein 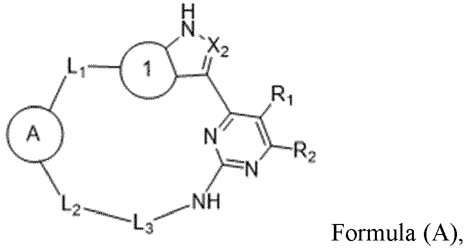 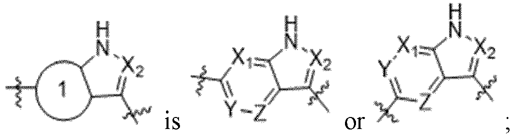
ring A is none; or the ring A is selected from the group consisting of 3-14 membered carbocycle, 3-14 membered heterocycle, 5-12 membered heteroaryl and C₅₋₁₂ aryl; the ring A is unsubstituted or substituted by 1, 2, 3, 4 or 5 groups selected from a group S1; the 3-14 membered heterocycle has 1, 2, 3 or 4 heteroatoms independently selected from nitrogen, oxygen and sulfur; the 5-12 membered heteroaryl has 1, 2, 3 or 4 heteroatoms independently selected from nitrogen, oxygen and sulfur;
L₃ is none, C₁₋₆ alkylene, C₂₋₆ alkenylene or ring B, wherein 1, 2, 3, 4 or 5 hydrogen atoms on the C₁₋₆ alkylene may be each independently and optionally substituted by R¹³; 1, 2, 3, 4 or 5 hydrogen atoms on the C₂₋₆ alkenylene may be each independently and optionally substituted by R¹³;
the ring B is selected from the group consisting of 3-14 membered carbocycle, 3-14 membered heterocycle, 5-12 membered heteroaryl and C₅₋₁₂ aryl; the ring B is unsubstituted or substituted by 1, 2, 3, 4 or 5 groups selected from the group S1; the 3-14 membered heterocycle has 1, 2, 3 or 4 heteroatoms independently selected from nitrogen, oxygen and sulfur; the 5-12 membered heteroaryl has 1, 2, 3 or 4 heteroatoms independently selected from nitrogen, oxygen and sulfur;
L₁ is none, C₁₋₆ alkylene, C₂₋₆ alkenylene, -O-, -NR¹⁰-, -C=N-, C₅₋₁₂ aryl, 5-12 membered heteroaryl, -C(O)-, -C(O)-NR¹⁰- or -NR¹⁰-C(O)-, wherein 1, 2, 3, 4 or 5 hydrogen atoms on the C₁₋₆ alkylene may be each independently and optionally substituted by R¹³; 1, 2, 3, 4 or 5 hydrogen atoms on the C₂₋₆ alkenylene may be each independently and optionally substituted by R¹³; the 5-12 membered heteroaryl has 1, 2, 3 or 4 heteroatoms independently selected from nitrogen, oxygen and sulfur; the C₅₋₁₂ aryl and the 5-12 membered heteroaryl are each independently unsubstituted or substituted by 1, 2, 3, 4 or 5 groups selected from the group S1;
L₂ is selected from the group consisting of:
- (O)ₘ₂-(CR¹³R¹⁴)ₘ₁-(NR¹²)ₘ₃-,
- (NR¹¹)ₘ₂-(CR¹³R¹⁴)ₘ₁-(NR¹²)ₘ₃-,
- (O)ₘ₂-(CR¹³R¹⁴)ₘ₅-(3-7 membered carbocyclyl)ₘ₄-(CR¹³R¹⁴)ₘ₆-(NR¹²)ₘ₃-,
- (NR¹¹)ₘ₂-(CR¹³R¹⁴)ₘ₅-(3-7 membered carbocyclyl)ₘ₄-(CR¹³R¹⁴)ₘ₆-(NR¹²)ₘ₃-,
- (O)ₘ₂-(CR¹³R¹⁴)ₘ₅-(3-7 membered heterocyclyl)ₘ₄-(CR¹³R¹⁴)ₘ₆-(NR¹²)ₘ₃-,
- (NR¹¹)ₘ₂-(CR¹³R¹⁴)ₘ₅-(3-7 membered heterocyclyl)ₘ₄-(CR¹³R¹⁴)ₘ₆-(NR¹²)ₘ₃-,
- (O)ₘ₂-(CR¹³R¹⁴)ₘ₅-NR¹⁰-(CR¹³R¹⁴)ₘ₆-(NR¹²)ₘ₃-,
- (NR¹¹)ₘ₂-(CR¹³R¹⁴)ₘ₅-NR¹⁰-(CR¹³R¹⁴)ₘ₆-(NR¹²)ₘ₃-,
- (O)ₘ₂-(CR¹³R¹⁴)ₘ₅-NR¹⁰C(O)-(CR¹³R¹⁴)ₘ₆-(NR¹²)ₘ₃-,
- (NR¹¹)ₘ₂-(CR¹³R¹⁴)ₘ₅-NR¹⁰C(O)-(CR¹³R¹⁴)ₘ₆-(NR¹²)ₘ₃-,
- (O)ₘ₂-(CR¹³R¹⁴)ₘ₅-C(O)NR¹⁰-(CR¹³R¹⁴)ₘ₆-(NR¹²)ₘ₃-,
- (NR¹¹)ₘ₂-(CR¹³R¹⁴)ₘ₅-C(O)NR¹⁰-(CR¹³R¹⁴)ₘ₆-(NR¹²)ₘ₃-,
- (O)ₘ₂-(CR¹³R¹⁴)ₘ₅-O-(CR¹³R¹⁴)ₘ₆-(NR¹²)ₘ₃-,
- (NR¹¹)ₘ₂-(CR¹³R¹⁴)ₘ₅-O-(CR¹³R¹⁴)ₘ₆-(NR¹²)ₘ₃-,
- (O)ₘ₂-(CR¹³R¹⁴)ₘ₅-OC(O)-(CR¹³R¹⁴)ₘ₆-(NR¹²)ₘ₃-,
- (NR¹¹)ₘ₂-(CR¹³R¹⁴)ₘ₅-OC(O)-(CR¹³R¹⁴)ₘ₆-(NR¹²)ₘ₃-,
- (O)ₘ₂-(CR¹³R¹⁴)ₘ₅-C(O)O-(CR¹³R¹⁴)ₘ₆-(NR¹²)ₘ₃-,
- (NR¹¹)ₘ₂-(CR¹³R¹⁴)ₘ₅-C(O)O-(CR¹³R¹⁴)ₘ₆-(NR¹²)ₘ₃-,
- (O)ₘ₂-(CR¹³R¹⁴)ₘ₅-(CH=CH)ₘ₇-(CR¹³R¹⁴)ₘ₆-(NR¹²)ₘ₃-, and
- (NR¹¹)ₘ₂-(CR¹³R¹⁴)ₘ₅-(CH=CH)ₘ₇-(CR¹³R¹⁴)ₘ₆-(NR¹²)ₘ₃-;
each R¹⁰ is independently hydrogen, C₁₋₆ alkyl, deuterated C₁₋₆ alkyl, -3-7 membered carbocyclyl, -C(O)-C₁₋₆ alkyl, -C(O)-C₁₋₄ alkyl-C₁₋₆ alkoxy, -C(O)-3-7 membered carbocyclyl, -C₁₋₄ alkyl-hydroxyl, -C₁₋₄ alkyl-cyano, -C₁₋₄ alkyl-C₁₋₆ alkoxy, -C₁₋₄ alkyl-NHC(O)-C₁₋₆ alkyl, -C₁₋₄ alkyl-NHC(O)-C₁₋₄ alkyl-C₁₋₆ alkoxy, -C₁₋₄ alkyl-NHC(O)-3-7 membered carbocyclyl, or -C₁₋₄ alkyl-NRR';
each 3-7 membered heterocyclyl independently has 1, 2 or 3 heteroatoms independently selected from nitrogen, oxygen and sulfur; the 3-7 membered carbocycle is each independently unsubstituted or substituted by 1, 2, 3, 4 or 5 groups selected from the group S1;
each 3-7 membered carbocyclyl is independently unsubstituted or substituted by 1, 2, 3, 4 or 5 groups selected from the group S1;
each R¹¹ is independently hydrogen, C₁₋₆ alkyl or deuterated C₁₋₆ alkyl;
each R¹² is independently hydrogen, C₁₋₆ alkyl or deuterated C₁₋₆ alkyl;
each R¹³ is independently hydrogen, cyano, hydroxyl, halogen, C₁₋₆ alkyl, C₁₋₆ alkoxy, halogenated C₁₋₆ alkyl, halogenated C₁₋₆ alkoxy, -3-7 membered carbocyclyl, -C₀₋₆ alkylene-NRR', -C₁₋₆ alkylene-hydroxyl or -C₀₋₆ alkylene-cyano;
each R¹⁴ is independently hydrogen, cyano, hydroxyl, halogen, C₁₋₆ alkyl, C₁₋₆ alkoxy, halogenated C₁₋₆ alkyl, halogenated C₁₋₆ alkoxy, -3-7 membered carbocyclyl, -C₀₋₆ alkylene-NRR', -C₁₋₆ alkylene-hydroxyl or -C₀₋₆ alkylene-cyano;
each m1 is independently 1, 2, 3, 4, 5 or 6;
each m2 is independently 0 or 1;
each m3 is independently 0 or 1;
each m4 is independently 1 or 2;
each m5 is independently 0, 1, 2, 3, 4, 5 or 6;
each m6 is independently 0, 1, 2, 3, 4, 5 or 6;
each m7 is independently 1 or 2;
R₁ is hydrogen, halogen, cyano, C₁₋₆ alkyl or 3-14 membered carbocyclyl; the C₁₋₆ alkyl and the 3-14 membered carbocyclyl are independently unsubstituted or substituted by 1, 2, 3, 4 or 5 groups selected from the group consisting of oxo, hydroxyl, halogen, C₁₋₆ alkyl and halogenated C₁₋₆ alkyl;
R₂ is hydrogen, halogen, cyano, C₁₋₆ alkyl or 3-14 membered carbocyclyl; the C₁₋₆ alkyl and the 3-14 membered carbocyclyl are independently unsubstituted or substituted by 1, 2, 3, 4 or 5 groups selected from the group consisting of oxo, hydroxyl, halogen, C₁₋₆ alkyl and halogenated C₁₋₆ alkyl;
X₂ is N or C(R₃);
X₁ is N or C(R₄);
Y is N or C(R₄);
Z is N or C(R₃);
each R₃ is independently hydrogen, halogen, cyano, C₁₋₆ alkyl or halogenated C₁₋₆ alkyl;
each R₄ is independently hydrogen, halogen, hydroxyl, cyano, -C₂₋₄ alkenylene-phenyl, -C₂₋₄ alkynylene-phenyl, -S(O)-OH, -S(O)₂-OH, -S-(C₁₋₆ alkyl), C₁₋₆ alkyl, -O-C₁₋₆ alkyl, -C₁₋₆ alkylene-O-C₁₋₆ alkyl, -O-C₁₋₆ alkylene-O-C₁₋₆ alkyl, -C₀₋₆ alkylene-NRR', -C₀₋₆ alkylene-C(O)OH, -C₀₋₆ alkylene-C(O)-C₁₋₆ alkyl, -C₀₋₆ alkylene-C(O)-NRR', -C₀₋₆ alkylene-NR-C(O)-C₁₋₆ alkyl, -C₀₋₆ alkylene-S(O)₂-C₁₋₆ alkyl, -C₀₋₆ alkylene-S(O)₂-NRR', -C₀₋₆ alkylene-NR-S(O)₂-C₁₋₆ alkyl, -C₀₋₆ alkylene-NR-S(O)₂-NRR', -C₀₋₆ alkylene-P(O)O-(C₁₋₆ alkyl)₂, -C₀₋₆ alkylene-P(O)-(C₁₋₆ alkyl)(O-C₁₋₆ alkyl), -C₀₋₆ alkylene-P(O)-(C₁₋₆ alkyl)₂, -C₀₋₆ alkylene-3-14 membered carbocyclyl, -C₀₋₆ alkylene-3-14 membered heterocyclyl, -C₀₋₆ alkylene-5-12 membered heteroaryl, -C₀₋₆ alkylene-C₅₋₁₂ aryl, -C₀₋₆ alkylene-C(O)-3-14 membered heterocyclyl, -C₀₋₆ alkylene-C(O)-5-12 membered heteroaryl, -O-C₀₋₆ alkylene-O-C₁₋₆ alkyl, -O-C₀₋₆ alkylene-3-14 membered carbocyclyl, -O-C₀₋₆ alkylene-3-14 membered heterocyclyl, -O-C₀₋₆ alkylene-5-12 membered heteroaryl, -O-C₀₋₆ alkylene-C₅₋₁₂ aryl, and - S(O)-C₁₋₆ alkyl, wherein the C₁₋₆ alkyl, the C₀₋₆ alkylene, the C₂₋₄ alkenylene, the 3-14 membered carbocyclyl, the 3-14 membered heterocyclyl, the 5-12 membered heteroaryl and the C₅₋₁₂ aryl are independently unsubstituted or substituted by 1, 2, 3, 4 or 5 groups selected from the group S1; the 3-14 membered heterocycle has 1, 2, 3 or 4 heteroatoms independently selected from nitrogen, oxygen and sulfur; the 5-12 membered heteroaryl has 1, 2, 3 or 4 heteroatoms independently selected from nitrogen, oxygen and sulfur;
the groups in the group S1 comprise: oxo (=O), halogen, hydroxyl, cyano, C₁₋₆ alkyl, - O-C₁₋₆ alkyl, -C₁₋₆ alkylene-O-C₁₋₆ alkyl, -O-C₁₋₆ alkylene-O-C₁₋₆ alkyl, -C₀₋₆ alkylene-NRR', -C₀₋₆ alkylene-C(O)OH, -C₀₋₆ alkylene-C(O)-C₁₋₆ alkyl, -C₀₋₆ alkylene-C(O)-NRR', -C₀₋₆ alkylene-NR-C(O)-C₁₋₆ alkyl, -C₀₋₆ alkylene-S(O)₂-C₁₋₆ alkyl, -C₀₋₆ alkylene-S(O)₂-NRR', - C₀₋₆ alkylene-NR-S(O)₂-C₁₋₆ alkyl, -C₀₋₆ alkylene-NR-S(O)₂-NRR', -C₀₋₆ alkylene-P(O)O-(C₁₋₆ alkyl)₂, -C₀₋₆ alkylene-P(O)-(C₁₋₆ alkyl)(O-C₁₋₆ alkyl), -C₀₋₆ alkylene-P(O)-(C₁₋₆ alkyl)₂, -C₀₋₆ alkylene-3-14 membered carbocyclyl, -C₀₋₆ alkylene-3-14 membered heterocyclyl, -C₀₋₆ alkylene-5-12 membered heteroaryl, -C₀₋₆ alkylene-C₅₋₁₂ aryl, -C₀₋₆ alkylene-C(O)-3-14 membered heterocyclyl, -C₀₋₆ alkylene-C(O)-5-12 membered heteroaryl, -O-C₀₋₆ alkylene-O-C₁₋₆ alkyl, -O-C₀₋₆ alkylene-3-14 membered carbocyclyl, -O-C₀₋₆ alkylene-3-14 membered heterocyclyl, -O-C₀₋₆ alkylene-5-12 membered heteroaryl, -O-C₀₋₆ alkylene-C₅₋₁₂ aryl, and - S(O)-C₁₋₆ alkyl;
R and R' are each independently hydrogen, C₁₋₆ alkyl or deuterated C₁₋₆ alkyl, or R and R' optionally form the 3-14 membered heterocyclyl or 5-12 membered heteroaryl together with nitrogen atoms connected to R and R', wherein the heterocyclyl and the heteroaryl each independently contain 0, 1 or 2 heteroatoms selected from N, O and S in addition to the existing nitrogen atoms;
in the above groups, 2 hydrogen atoms on any one carbon atom on the C₀₋₆ alkylene may further be optionally substituted by 3-7 membered carbocycle or 3-7 membered heterocyclic spirocycle at the same time; and
in the above groups, 2 hydrogen atoms on any one carbon atom on the C₁₋₆ alkylene may further be optionally substituted by 3-7 membered carbocycle or 3-7 membered heterocyclic spirocycle at the same time.

2. The compound or the pharmaceutically acceptable salt thereof according to claim 1, wherein
the compound shown in Formula (A) is a compound shown in Formula (I-A): 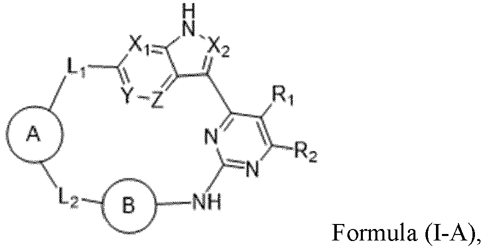
wherein X₁, Y, Z, X₂, R₁, R₂, L₁, L₂, ring A and ring B are each defined as in claim 1; or
the compound shown in Formula (A) is a compound shown in Formula (I-B): 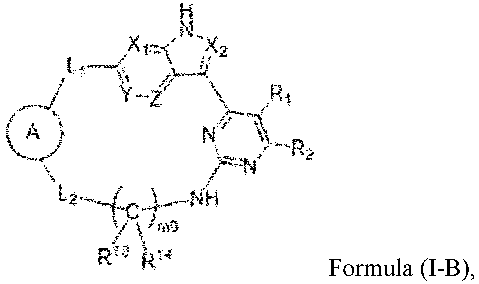
wherein X₁, Y, Z, X₂, R₁, R₂, L₁, L₂, ring A, R¹³ and R¹⁴ are each defined as in claim 1, and m0 is 1, 2, 3, 4, 5 or 6;
or
the compound shown in Formula (A) is a compound shown in Formula (A1): 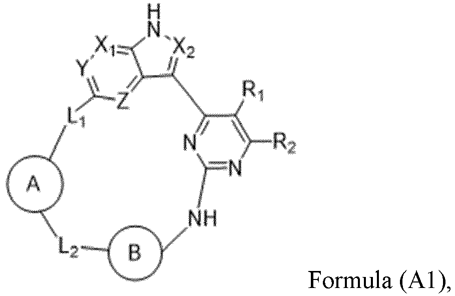
wherein X₁, Y, Z, X₂, R₁, R₂, L₁, L₂, ring A and ring B are each defined as in claim 1; or
the compound shown in Formula (A) is a compound shown in Formula (A2): 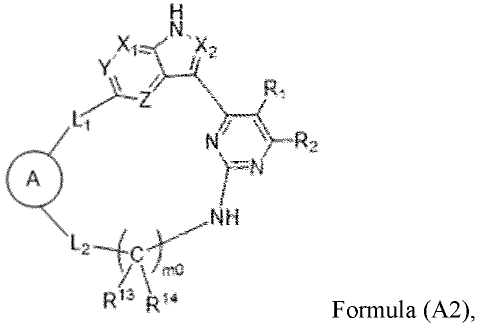
wherein X₁, Y, Z, X₂, R₁, R₂, L₁, L₂, ring A, R¹³ and R¹⁴ are each defined as in claim 1, and m0 is 1, 2, 3, 4, 5 or 6.

3. The compound or the pharmaceutically acceptable salt thereof according to claim 1, wherein
the compound shown in Formula (A) is a compound shown in Formula (II-A): 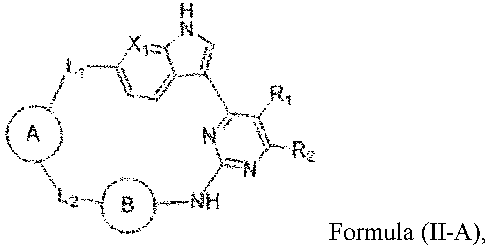
wherein R₁, R₂, L₁, L₂, ring A, ring B and X₁ are each defined as in claim 1; or
the compound shown in Formula (A) is a compound shown in Formula (II-B): 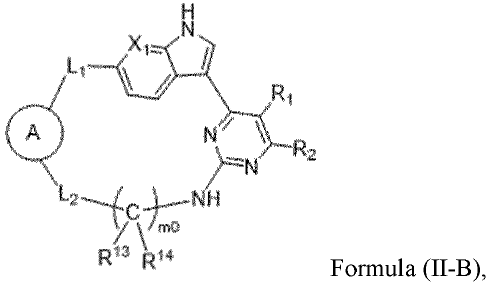
wherein X₁, R₁, R₂, L₁, L₂, ring A, R¹³ and R¹⁴ are each defined as in claim 1, and m0 is 1, 2, 3, 4, 5 or 6;
or
the compound shown in Formula (A) is a compound shown in Formula (A3): 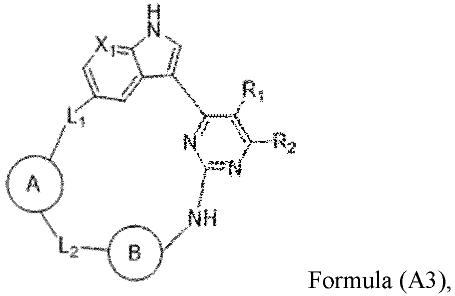
wherein X₁, R₁, R₂, L₁, L₂, ring A and ring B are each defined as in claim 1; or
the compound shown in Formula (A) is a compound shown in Formula (A4): 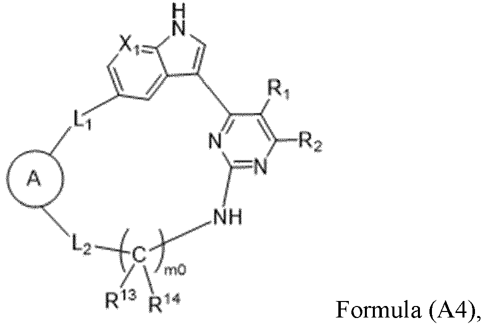
wherein X₁, R₁, R₂, L₁, L₂, ring A, R¹³ and R¹⁴ are each defined as in claim 1, and m0 is 1, 2, 3, 4, 5 or 6.

4. The compound or the pharmaceutically acceptable salt thereof according to claim 1, wherein
the compound shown in Formula (A) is a compound shown in Formula (III-A): 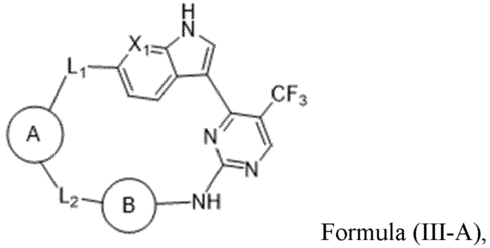
wherein L₁, L₂, ring A, ring B and X₁ are each defined as in claim 1;
or
the compound shown in Formula (A) is a compound shown in Formula (III-B): 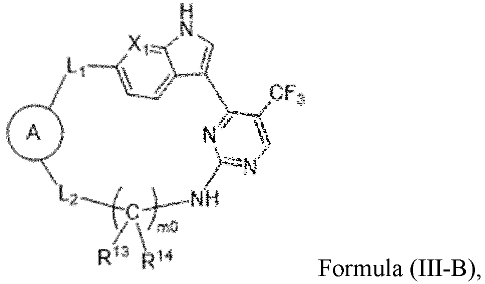
wherein X₁, L₁, L₂, ring A, R¹³ and R¹⁴ are each defined as in claim 1, and m0 is 1, 2, 3, 4, 5 or 6;
or
the compound shown in Formula (A) is a compound shown in Formula (A5): 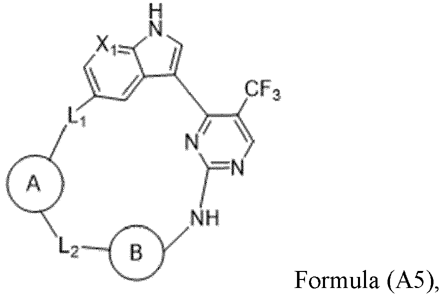
wherein X₁, L₁, L₂, ring A and ring B are each defined as in claim 1;
or
the compound shown in Formula (A) is a compound shown in Formula (A6): 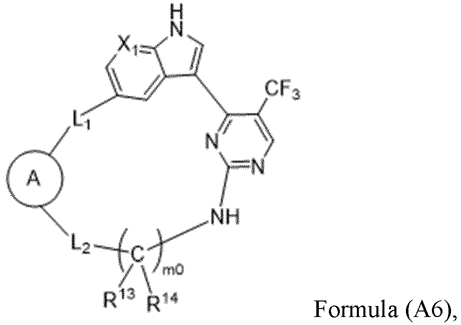
wherein X₁, L₁, L₂, ring A, R¹³ and R¹⁴ are each defined as in claim 1, and m0 is 1, 2, 3, 4, 5 or 6;
or, the compound shown in Formula (A) is a compound shown in Formula (IV-a): 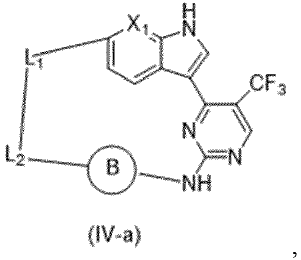
wherein X₁, L₁, L₂ and ring B are each defined as in claim 1;
or, the compound shown in Formula (A) is a compound shown in Formula (IV-b): 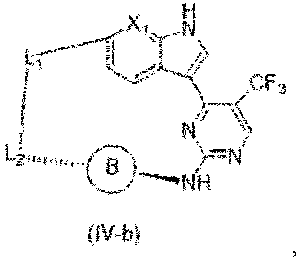
wherein X₁, L₁, L₂ and ring B are each defined as in claim 1; 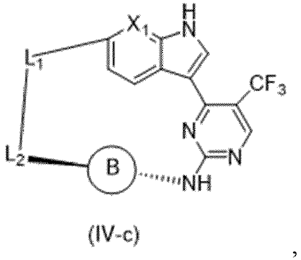
wherein X₁, L₁, L₂ and ring B are each defined as in claim 1.

5. The compound or the pharmaceutically acceptable salt thereof according to claims 1 to 4, wherein the ring A is none; or the ring A is selected from the group consisting of a cyclobutyl ring, a cyclopentyl ring, a cyclohexyl ring, a piperidinyl ring, a piperazinyl ring, a tetrahydropyrrolidinyl ring, a pyrazolyl ring, an imidazolyl ring and a pyridinyl ring; the ring A is unsubstituted or substituted by 1, 2, 3, 4 or 5 groups selected from the group S1.

6. The compound or the pharmaceutically acceptable salt thereof according to claims 1 to 4, wherein the ring B is selected from the group consisting of a cyclobutyl ring, a cyclopentyl ring, a cyclohexyl ring, a piperidinyl ring, a piperazinyl ring, a tetrahydropyrrolidinyl ring, a pyrazolyl ring and an imidazolyl ring; the ring B is unsubstituted or substituted by 1, 2, 3, 4 or 5 groups selected from the group S1.

7. The compound or the pharmaceutically acceptable salt thereof according to claims 1 to 4, wherein X₁ is N or C(R₄), and R₄ is hydrogen, halogen, cyano, -S(O)₂CH₃ or - P(O)(CH₃)₂.

8. The compound or the pharmaceutically acceptable salt thereof according to claims 1 to 4, wherein the compound is selected from Table (I).

9. A pharmaceutical composition, comprising the compound or the pharmaceutically acceptable salt thereof according to any one of claims 1 to 8 and a pharmaceutically acceptable carrier.

10. Use of the compound or the pharmaceutically acceptable salt thereof according to any one of claims 1 to 8 or the pharmaceutical composition according to claim 9 in preparation of drugs for preventing and/or treating CDK7-related diseases.

11. Use of the compound or the pharmaceutically acceptable salt thereof according to any one of claims 1 to 8 or the pharmaceutical composition according to claim 9 in preparation of CDK7 inhibitors.

12. The use according to claim 11, wherein the CDK7-related diseases are proliferative diseases (such as tumors or cancers), infectious diseases, immune diseases, autoimmune diseases and inflammatory diseases.
